(19) Europäisches Patentamt
European Patent Office
Office européen des brevets

(11) EP 2 228 455 B1

(12) **EUROPEAN PATENT SPECIFICATION**

(45) Date of publication and mention of the grant of the patent:
**04.07.2012 Bulletin 2012/27**

(51) Int Cl.:
***C12Q 1/68*** *(2006.01)*

(21) Application number: **10155167.9**

(22) Date of filing: **02.03.2010**

# (54) Diagnosis of HER-2 positive breast cancer

Diagnose von HER2-positivem Brustkrebs

Diagnostic du cancer du sein HER-2 positif

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **04.03.2009 KR 20090018575**
**27.11.2009 KR 20090116139**

(43) Date of publication of application:
**15.09.2010 Bulletin 2010/37**

(73) Proprietor: **Macrogen Inc.**
**Geumcheon-gu**
**Seoul 153-801 (KR)**

(72) Inventors:
- **Lee, Jong-Ho**
  **153-801 Seoul (KR)**
- **Kim, Jong-Yun**
  **153-801 Seoul (KR)**
- **Kang, Hyun-Woong**
  **153-801 Seoul (KR)**
- **Lee, Jung-Wan**
  **153-801 Seoul (KR)**
- **Lee, Seung-Heui**
  **153-801 Seoul (KR)**
- **Seo, Jeong-Sun**
  **153-801 Seoul (KR)**

(74) Representative: **Nemec, Harald**
**Schwarz & Partner**
**Patentanwälte**
**Wipplingerstrasse 30**
**1010 Wien (AT)**

(56) References cited:
**WO-A1-2008/030065** **WO-A1-2008/089577**
**WO-A2-2006/133460** **WO-A2-2007/081613**

- **MARCHIO C ET AL: "The genomic profile of HER2-amplified breast cancers: The influence of ER status" JOURNAL OF PATHOLOGY 200812 GB LNKD- DOI:10.1002/PATH.2423, vol. 216, no. 4, December 2008 (2008-12), pages 399-407, XP7912728**
- **ALAN MACKAY ET AL: "A high-resolution integrated analysis of genetic and expression profiles of breast cancer cell lines" BREAST CANCER RESEARCH AND TREATMENT, KLUWER ACADEMIC PUBLISHERS, BO LNKD- DOI:10.1007/S10549-008-0296-7, vol. 118, no. 3, 24 January 2009 (2009-01-24), pages 481-498, XP019746598 ISSN: 1573-7217**
- **NATRAJAN R ET AL: "Tiling path genomic profiling of grade 3 invasive ductal breast cancers" CLINICAL CANCER RESEARCH 20090415 AMERICAN ASSOCIATION FOR CANCER RESEARCH INC. USA LNKD- DOI: 10.1158/1078-0432.CCR-08-1878, vol. 15, no. 8, 15 April 2009 (2009-04-15) , pages 2711-2722, XP7912729 ISSN: 1078-0432**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 228 455 B1

<Sequence Listing>

<110> MACROGEN INC.

<120> METHOD OF DIAGNOSING HER-2 GENE SPECIFIC BREAST CANCER

<130> M 13872

<150> KR 10-2009-0018575
<151> 2009-03-04

<160> 4

<170> KopatentIn 1.71

<210> 1
<211> 94220
<212> DNA
<213> Artificial Sequence

<220>
<223> nucleotide sequence of human genome 17q12 region based on NCBI Build 36.1 (March, 2006)

<400> 1

tctctgttcc ctctgtctgg aatgcaaagt ggtataatag taaagaggac aagcttgtgg 60
tcaaacaggc cagaactgga aactccactt tactacatac tggctgtgtg accttgggca 120
gggcatttaa tctctctaat ccccatctgt aaaactggga ttagagaaac tatctcaaag 180
ggtgtgataa aaattacttg acatattgtg acacctgtca cacagtgagt gctcaatacg 240
ttggttcttt gcccactatg gtccaaatcg aatgtaccag tccaaagtcc ctccttcaaa 300
caaaacgttc caagatgatc cctgccccca cgtcttcaaa gccggaactg acaccttcct 360
atcccaaatg cttcctaatc aaagagtctt tcaggggctc aaatcattct ctgccttgca 420
cttgaaaagg ctcatgtccc cgtggggact gtgagtccca atgggcggga ccctggtctg 480
atttctgtgc cattcctaga cattgtggcc cgaatgtatt taataaatac ccttttgact 540
gaacaaataa gtaaatgaaa gcttctggga aagtaagaga ggctggtaag gctctcatca 600
ccttaggggc ttcactcaca ttcataagag acctcgtggg gaaactaagg ttcagggatt 660
gcagaggcgt attgggggcg caggggggcgg gagcaagaca aatgggcggg gcttaccgtg 720
ggggtggggc ttacctgcta gactcatgta gattggctgg cgggagcgga agtgattcag 780

agcgccccca gagcagttct gctcttcgca ctgcagtacg cagtcgcggt acaccggctc 840
acggtcgccc tgggagccgc tcgccagcgc cgctgcccca gctagcagga ccaaccgcgc 900
cgccaggccg gccatccttt ctccctggct cgccgccggg ggaggagctt aggagtatga 960
agcttccact tccggagtaa ccggaagttc ctgtgttctt tattctactc tccgctgaag 1020
tccacacagt ttaaattaaa gttcccggat ttttgtgggc gcctgccccg cccctcgtcc 1080
ccctgctgtg tccatatatc gaggcgatag ggttaaggga aggcggacgc ctgatgggtt 1140
aatgagcaaa ctgaagtgtt ttccatgatc ttttttgagg tagggctgtt tactgtcacc 1200
acccctgtcg gattttactt cctaaacgta cctgtaacta tccacttctc tccatctctt 1260
ctggcaccac cctggttaaa gacaccatca tgtgtcgcca agacagccgc agtagcttct 1320
taatggctct ccctgcctct acttttgcct cttccaacct gcgctccatt ttgaaaaatt 1380
aaaatttgcc catatcactt ttttttcttt aaaattattt actggctccc aattaccttg 1440
ggtaaaatac agtctccaca aaccctgcct gatttggccc ctgtccactg gtctccctca 1500
ctcccttgct ccagacccgc ttcagagggc tatgtccctc aagcttcctg actgcctggc 1560
ctggtctgaa tcactcactc ttcttttttc ttctagtcgc aattgaagta ccacctcccg 1620
agggtgattg cttccccatg cggggtagaa cctttgctgt cctgttcacc actctacctc 1680
cagcacagaa tttggcttat ggtaggcgct aactgcgttt gtttgttctt ctgtttaatg 1740
aatgaacagc atacatcaac ataagaactt gacaaatcca gggctgtaaa atcatcagta 1800
tggttctgca ctgagatcgg agagaagtaa tatttctagg aaaattagga accctgggaa 1860
caggacgctt gctttagtat cctctccctg ctcacctccc ctgcactccc atcagcaccg 1920
acccacaccc aatctcatag aagccttgta gctaaggatc accctttctc ctccccccact 1980
ctcctcaccc cttgtcaact tttctttttc gtcctggggg ttggaatgag taagaagtag 2040
cctgggattc cattcactca cttaacaaac atttctgagt ccttagctct agcaccttgc 2100
taagcaaggc aaaatctcca ggaggcacca ttcacattgc attttctgtg aatggtgctc 2160
tggggagcag cattcacatt gccttttctg tgaatggcaa attcttccag ttaaatataa 2220
catgaatagt gtcccctgga gttgaccacc caactgatac tgactgagaa gctgaaatga 2280
acaaaacaac cccttagccc tccaggagct gaccggaaat ccagtgctaa tactactttg 2340
catcttacag attagttctt ttacaatact gttttttttt ctttttttcat ttcattttgt 2400

cctttctgtg actctgggat gagtcttttt atgaggatcc tcatataaag atggacattt 2460
aggattaaag aggatgaaat cctgacaaaa tagggagtct cccctttaga aaattcctaa 2520
gtaaggctgg gggtggtggc tcacgcctgt aatcccagca ctttgggagg ccgaggcgga 2580
cggatcacct gaggttagga gtttgagacc agcctgacca acatggagaa accccatctc 2640
tactaaaaat acaaaattag ttgggtgtgg tggtgcatgc ctgtaatccc agctactcag 2700
gaggctgagg caggagaatc gcttgaaccc agggaggcag aggttgtggt gagccaagat 2760
tgcgccatcg cactccagcc tgggcaacaa gagcgaaact caaaaaaaaa aaaaaaaaga 2820
aaaagaaaat tccaattttg aaggcctcat cctatattat gtcaaacata ctgaaatgca 2880
gtaacgcccc acattaaata agatttataa ataactatac atatatataa ttcaatctaa 2940
ttgctgttaa tagttgacat attgctacat ttatatacat ttagttaaaa aaaatttttt 3000
ttcccagaca gcctctcact ctttcaccta gactgaagtg cagtggcatg atcacgactc 3060
actgcaacct caacctccca gactcaagtg atccttccat ctcagcctcc tgagtagctg 3120
ggactgcagc atgcgccact atgccctgct aattttttta attttttgta gagacacggt 3180
cttgctatgt tgcctagact ggtctccaat tcctgggctc gagtgatcct cccgcctcaa 3240
cctcccaaag tgctgggatt acgggcgtga gccatgccac acggccataa aatattaatt 3300
ttcgcagctt tcttatattt tagaactaac aatggaaatt tgttcgggtc taaagtattt 3360
cagaggtcct tgaaaaccca tgcctacata cctgatggaa aaagcaatcc taggttaatg 3420
gtggaagtgg gagtagagac ttctgttctg ttgacttctt ggaagatggg gtactgtctc 3480
tctgggacag ctcttgagaa tttccctgcc agcacagccc cagataacaa tctctagatg 3540
gcgattacct ggcctctctt cccaactttc tagcctggag cccctagttc tcccctgagc 3600
ctccttagct tgtccttctt cctaacttgt atttggcttc agatgtgatc cacagtctga 3660
aaagtcacta attcattcct tcaactcagg cttattgagt cctcctgtgt atcagccatt 3720
gtactcatgg gggaaaaaaa agacaaagca tatgttaata gtagagtgtg ctggacaggc 3780
acagtggctc atgcctgtaa tcccagcact ttgggagggc gaggcaggtg gatcatctga 3840
ggtcaggagt tcgagaccag cctgacctaa catggagaaa ctcctgagat cgtgccattg 3900
cactccagcc tgggcaacaa gagcaaaact ccgtttcaaa aaaaaaaaaa aaagtatagt 3960
gtgctaaagg ctcaacggca agctgaccat gttcttagat caaaattggt agagagtcta 4020

caatgtgggt tccttattca tcaaatgttt attaagttta ccatgtgcaa gtctctggga 4080
acagagtgat gaacaaggca ctgtactttt catggtcaga ggagggaaac aggccataaa 4140
caagtgtcaa acaaaagact gaagccaggt gcggtggctc acatctgtaa tcccagcact 4200
gtgggaggcc aaggcaggcg gatcatgaga tcaggagatc gagaccatcc tagccaacat 4260
ggtgaaaccc catctctact aaaaatacaa aaaaattagc tgggcatggt ggcacgtgcc 4320
tgtaatccca gctactccgg aagctgaggc aggagaattg cttgaaccag ggagttggag 4380
gttgcagtga gcctggatta tgccactgca ctccagcctg gtgacagagc gagactccat 4440
ctacattaaa aaaaaaaata tatatatata tatatacaca cacacacaca cacacacaca 4500
catacccctct aacccaggaa tttcactcct aggtatacct acataagctc cagtatacct 4560
aaacaagtgc aaatttgttt aagtacagtt atttgtggta gcattagtca ttgttttcaa 4620
tagcaagaag aaaaaggaaa caactaaatg tccatcaata gggaatgaat tatattaatg 4680
gagggagagc catacaatgg aaggctgaac agaaattaat aggaatgggg cagatttgta 4740
atgtactagc atggtaaaac cttcatgata gatatagata tagatataga tatagatata 4800
gatatatata catatacata tacatataca tatacatata tatatatata tatatatatc 4860
tcttgtgtct cagcctcccg agtagctggg attacaggtg tgtgccacca catccggcta 4920
atttttgtat tttttagtag agacagggct tcaccatgtt ggtaaggctg tcttgaactc 4980
ccgacctcag gtgatccacc tgtctcagcc tcccaaagtg ctgggattat aggcatgagc 5040
catcacacct ggccaaatat ttttgataag tatcaagtgc acagtgcaga acaaaatatg 5100
tgtgtgtgta tgcatgtgta tgtacaccta tacacttata tacagtaccc catgtgaaga 5160
aaaataaggg tacgtgttat gcgcgtagta ttatggttgt tatttttgag aatatatcta 5220
gaaagataaa aaagaaagtg gaaatagttc ttgcctctgg tgggaagtgg gactatgtgc 5280
ctgatcaata gggaagtaag gaacactttt tttttttttt tttaaacgga gtttttgctc 5340
ttgttaccca ggttggagtg caatggcgcg atcttagctc actgcaacct ctgcctccca 5400
ggttcaagcg attctgctgc ctcagcctcc tgagtagctg ggattatagg catgcgcctc 5460
cacgcctggc taattttgta tttttagtaa agatggggtt tctccatgtt ggtcaggctg 5520
gtcttgaact ccccacctca ggtgatccgt ccgcctcagc ctcccaaagt gctaggatta 5580
caggcgtgag ccaccgtgcc tggccaggaa cgctttttat tttgtacct ttaaaagtgt 5640

gtaccgtctg tgtatataat cagttaaaaa caaagaaaag ctgagtgtgg tggctcatgc 5700
ctgtaatccc agcccttaag gaggccgagg ccggcggcag atcacctgag gtcaggagtt 5760
caagaccggc ctgaccaaaa cggtgaaaac tcatctctac aaaaacataa aaattagcca 5820
ggcatgatgg caagtgcctg taatcccagc tggttgggag gctgaggtgg gagacttgct 5880
tgaacctagg aggcagagat tgcagtgagc caagactgta ccactgcact ccagcctggg 5940
caacagagca agtctctgtc tcaaaacaaa aacaaaaaca caaagaaaaa atgtaaaaca 6000
atttcatgca gtagcaagca tcgagttaaa tacagttgac ccttgaacaa cacaggtttg 6060
aattgcacgg gtccatttat actcacattt cttccacctc tgccaccccc aaaatagcaa 6120
gaccaacccc atctcttttc ctttctcttc cccctcctca gcctactcaa tgtgaagatg 6180
atgaggatga aaacctttgt gatgatccac ttccacttaa tgaatggtaa atatgttttt 6240
tcttacttat gattttctta gtagcatttt cttttctcta gcttccttta ttgtaaaaat 6300
acagtatata acacatatca catacaaaat gtgtgtaaat ggactgtttg ctattgataa 6360
gtattctggt aaacagtaga ctattagttt tttttgtttt gtgacaaggt ctccctctgt 6420
cgcccagcct ggaatgccgt ggtgtgatca tggctcactg cagccaaaaa cttctgggct 6480
aaagcaatcc tctactaaaa atacaaaaat tagccaggca tggtggtgcg cttctgtaat 6540
cccagctact caggaggctg aggcaggaga attgcttgaa cccgggaggc agaggttgca 6600
gtgagctgag attgcaccgt tgcattccag cctggacaac agagcgagac tccatctcga 6660
aaataaaata ataataataa taataataat aataataata ataatagggc tgggtgtggt 6720
ggctcatgcc tgtaatccca gcactttggg aggccaaggt ggacagatca cctgaggtca 6780
ggagtctcaa ttaaaaaata aataggccgg gcacagtggc tcatgcccat aatcccagca 6840
ctttgggagg ccgaggtggg cagatcacct gaggtcagga gtttgagacc agcctggcca 6900
acacggagaa acgctgcctc tatcaaaaat acaaaaatta gctggatgtg gtggtgcatg 6960
ctataatccc agtaatacca gctactcgga aggctgaggc aggagaatca ctcgaatccg 7020
ggacacggag gttgcagtga gccgacatca tgccactgcg ctccagcctg ggtgacagtg 7080
agactctgtc tcagaaaaaa aaaaaaaaaa aaaaaaaaaa aaaaaaaata tatatatata 7140
tatatatata tatatatata tatatatatg tgtgtatata tatatatata cacatatata 7200
tgtgtatata tatatacaca cacacatata tatgtgtata tataaaataa aataaataat 7260

aataaaacat ttactttggc tgctgttgct gcggggagaa ttgcagggtg tcaaaagtag 7320
cactggtgga ggggtagtga tcaaagtctg gtgctttagc ccaaaggaga aatgatagag 7380
actcagacta gctggtgatg gaggtagaat aagcataaat gtatcaaaaa gaggagttga 7440
tagatcttaa agaatgattg gatttgaagg gcaaaggaag agaagaatca accaggtggg 7500
ttcagtgaat gaaaccatca gaaacgaatt gtcccctgaa atcaagactt tgtgattgcc 7560
atagttgtat gcttctcaaa ggttcctcgt ctcctcttcc ttggaccaaa agtcagaggc 7620
aagaatgccc tcattcatac cccagtggtc tatacctcca gcagcaagtc gagtgagcaa 7680
gtgatgtcct gaaaggccca gtggatcagt ggaatgaagc gggcaggaag acttagtgct 7740
cctgaaacaa ggaatccaga atccaggaga aggatggctc agtggggctt tcaagggaca 7800
agtatggggg ttgaaggggt cactgtccct ataccaaatc cgaaaatatt gtgaccagga 7860
accattctgt ccaactcttc tatttcaggt ggcaaagcaa agctatattc aagaccacat 7920
gcaaagctac tccctgagca aagagtcaca gataaaacgg gggcaccagt agaatggcca 7980
ggacaaacgc agtgcagcac agagactcag accctggcag ccatgcctgc gcaggcagtg 8040
atgagagtga catgtactgt tgtggacatg cacaaaagtg aggtgagtcg caggacagaa 8100
gagtgctttt tgtttcagca gagcagcctg gggagagata aaagctactc ctggggcctg 8160
ggcctgcatt cctgagatgt gggtaagagg ggcccagggt cagagtgtct ggcaagcttg 8220
gctctgcccc tttgctgtcc tggagactag ggctaatcct gggctcaggg agtggcctcc 8280
ccatggttag gatacaagtg ctcatcaagg gccaccccta ggaaggacca attttcctat 8340
cagaagcttc taagttatcc tcctttggcc caaagggaca cctcaagcct actctgagga 8400
actctttcca atgaactaat tcctacagtc acttccccag caacctgtgc ctcagcctca 8460
aggcactgtg gggtaggcct cagtttgtgg cctggacatc ggactgtgga ccagacgact 8520
cctcccgatt tctgtttgtt ttcagtcctc tgaccccaag ctggctggtg aagtaggtag 8580
agggaggaga ctttggtgca tgcatacaca cacacacaca cacacacaca cacacacaca 8640
cacacacaca cacacacacg tctcctgtgc cccccagtct ccatggctgg tcaatgattg 8700
actggcattt cacaggccgc tggttgcagc cccagcctgt tgacttagag gtcaccctcg 8760
gaagctagag ccctgtcctg cctcttcagt gtcagtggtc actccactgc ccacaggctg 8820
gggtcttggg caaaacacac gcatctgccc tgatctgagt ttgctgccct ctgtcccgca 8880

gtcagcccca ctctgttccc actccctctc cccagccccc tagctagacc cctctcacca 8940
gcaccccttt cccttccctg agggtccccc tcgctgtctt tgtccctcag acatcctctt 9000
tcctgggctc tcctgccagg ccctgctgga gggacagtta aggaggaaat cgaatcagca 9060
gcgcccaccc ctgcccccct tcctctcctc ttgtcagaca ccagacgagg tttttttcctc 9120
tggcttccca gctctgaatg ggctcattct ttttcagagg ctcggcccct ctcgagcctc 9180
ctccccaggg cgtgagttct gaccccagct cctcccccca tccccactcc agcccccctct 9240
ccagcttgct ccaccctctc taccgcccac cgggactggg cattgtctgc cagtccgggt 9300
ttcttcctgg gatttgggat gcagagagga tgggtttgct tgggcggggg ggtggagagt 9360
gaagggggga agcaggatct ttgtagaggg agggacctac agttacctgg acttctttcc 9420
tctgtctccc ctcttggtac ccttgactgg ggctcttgag ggtaatgggt gaagccaaat 9480
ctgccatggc tcagttccca gctcagctct gtgaccttgg gaaagttcct ttagctcgtg 9540
gaatctcaag gctcaaggtt cctcttctgc aaaatgggga atgataacac ctgcctcctc 9600
tggagtcttg gggactcagt gttctgagga acgtggctgt aggtcagagt ggcacagagt 9660
agggtccaat gaagcatggc gtccacagta gctttcctga ctggactaac ctttccggac 9720
acaacagcag ggcaggggtg gggcctgggg agaaaggaca cctctaaccc tgatcctaac 9780
atcccgatgg cctctaaggc tgcctgcaca ctcatccagg tgcaagccct ccaaggtgtg 9840
gtgtgatgaa ccagtgactc ctggagccag gtcagcgcat cctcttcccg cagggctgta 9900
agctgcagga ctgagaggca ggttgaccag gtcctgggct ggatgatggg gtgagagtaa 9960
ggggtcagtt ttgatacatg cccaactttt ctctctagcc ctaagacatc ctgggcaaat 10020
tgcttacctc agttcccctg atcctcaccc taaccctaac accagctcaa gagaaaatag 10080
ggatattgat ggccatccag aagggctgct gtgttccata cacagcaata tttctcgaat 10140
gtttgtgaca gcggtccaag gaataagtta attttacatt atcactctgg atacctgtac 10200
aaaactccac cttatcctta ctatatgaat gtgctagggt tgttttttttg ttttgttttt 10260
ttttttttt tttgagacag agtttcgctc ttgttgccca ggctggagta caatggcgcg 10320
atcttggctc accgcaacct ccgcttccca ggttcaagcg attcacctgc ctcagccttc 10380
ccgagtagct gggattacag gcatgcgcca ccatgcccgg ctaatttgt gtttttagta 10440
gagacagggt ttctccatgt tggtcaggct ggtaccaaac tcccgacctc aggtgatcca 10500

cctgccttgg cctcccaaag tgctgcaatt acaggcatga gccaccgcac ccagccgtgc 10560
tagggtcttt ttctgttcaa ttcctttctc tctcttgctc tctttctttc tttcaatgga 10620
gtcttactct gtcacccagg ctggagtgca gtggcaagat ctcagctcac tgcaacctct 10680
gccctctgag ttcaagcaat tctcctgcct cagcctcccg agtagctggg attacaggtg 10740
cctgccacca cacctagtta atttttgtac ttttagtaga gatggggttt tgtcatgttg 10800
gccaggctgg tctcgaactc ctgacctcgt gatctgcctg tcttggcctc ccaaagtgct 10860
gggattacag gcatgagccg ccatactcgg ccaactttgt attactttct taaagagagt 10920
ttcccaaatt atataagctt caggccccac aaaacctaga tctgccccag tataactaaa 10980
tctgggacca tttattgagc aattattatg tgccaagtat tgcgctgagt gcttccagag 11040
cattatctcc tttaacccca gcatagtatg tcagatgctg ttttacagat gagccaactg 11100
agaccagaga tgctcagtca cttgcccaag gtgacatgac tgatatggaa tagagtcaag 11160
attttttttt tttttttga cacggagtct cactctgtct cccaggctgg agtgcagagg 11220
cgcaatctca gctcactgca agctctgcct cccaggttca cgccattctc ctgcctcagc 11280
ctcctgagta gctgggacta caggcacccg ccaccacacc tggctaattt tttgtatttt 11340
tagcagagac agggtttcac cgtgttagcc aggatggtct cgatctcctg acctcgtgat 11400
ctgcctgcct cggcctccca aagtgctgga attacaggtg tgagccaccg cgactggcca 11460
gattcaagat ttgaacccag gtcctcttgg tcccagaggc ccctgtttct caactcccta 11520
ggatggcata gcaacctgtc ccacaagagg tgcctgcttt aagtgtgctc agcacatgga 11580
agcaagttta gaaatgcaag tgtatacctg taaagaggtg tgggagatgg gggggaggga 11640
agagagaaag agatgctggt gtccttcatt ctccagtccc tgataggtgc ctttgatccc 11700
ttcttgacca gtatagctgc attcttggct ggggcattcc aactagaact gccaaattta 11760
gcacataaaa ataaggaggc ccagttaaat ttgaatttca gataaacaat gaataatttg 11820
ttagtataaa tatgtcccat gcaatatctt gttgaaatta aaaaaaaaaa aaaaagtctt 11880
ccttccatcc ccacccctac cactaggcct aaggaatagg gtcaggggct ccaaatagaa 11940
tgtggttgag aagtggaatt aagcaggcta atagaaggca aggggcaaag aagaaacctt 12000
gaatgcattg ggtgctgggt gcctccttaa ataagcaaga agggtgcatt ttgaagaatt 12060
gagatagaag tctttttggg ctgggtgcag ttgctcgtgg ttgtaattcc agcactttgg 12120

gaggctgagg cgggaggatc acctgaggtt gggagttcaa gaccagcctc accaacgtgg 12180
agaaaccctg tctttactaa aaatacaaaa aattagctgg tcatggtggc acatgcctgt 12240
aatcccagct gctcgggagg ctgaggcagg agaatcactt gaaccaggga ggcagaggtt 12300
gtggtgagca gagatcgcgc cattgctctc cagcctgggc aacaagagca aaagttcgtt 12360
taaaaaaaaa aaaaagtcct ttcgatgtga ctgtctcctc ccaaatttgt agaccctctt 12420
aagatcatgc ttttcagata cttcaaagat tccagaagat atgccccggg ggtcctggaa 12480
gccacaaggt aaacacaaca catccccctc cttgactatc aattttacta gaggatgtgg 12540
tgggaaaacc attatttgat attaaaacaa ataggcttgg gatggagtag gatgcaagct 12600
ccccaggaaa gtttaagata aaacctgaga cttaaaaggg tgttaagagt ggcagcctag 12660
ggaatttatc ccggactccg ggggaggggg cagagtcacc agcctctgca tttagggatt 12720
ctccgaggaa aagtgtgaga acggctgcag gcaacccagg cgtcccggcg ctaggaggga 12780
cgcacccagg cctgcgcgaa gagagggaga aagtgaagct gggagttgcc actcccagac 12840
ttgttggaat gcagttggag ggggcgagct gggagcgcgc ttgctcccaa tcacaggaga 12900
aggaggaggt ggaggaggag ggctgcttga ggaagtataa gaatgaagtt gtgaagctga 12960
gattcccctc cattgggacc ggagaaacca ggggagcccc ccgggcagcc gcgcgcccct 13020
tcccacgggg ccctttactg cgccgcgcgc ccggccccca cccctcgcag caccccgcgc 13080
cccgcgccct cccagccggg tccagccgga gccatggggc cggagccgca gtgagcacca 13140
tggagctggc ggccttgtgc cgctgggggc tcctcctcgc cctcttgccc cccggagccg 13200
cgagcaccca aggtgggtct ggtgtgggga ggggacggag cagcggcggg accctgccct 13260
gtggatgccc cgccgaggtc ccgcggccgg cggggccaga ggggcccgga cgagctctcc 13320
tatcccgaag ttgtggacag tcgagacgct cagggcagcc gggccctggg gccctcgggc 13380
gggagggggc agttacacgg cagcggctcg agatggccca tccaagagac tggcgctttc 13440
caggctccga ggggctccgg gaacttgtca aagaagttct ctgaaattgt tcagaaagtt 13500
ttcccgcaaa gggtgtattg cgtagagcgc gcgcgcgcgt ttcccccctt cttgagcccc 13560
ctcaagcttt ctcaaagcct ttccagttgg cagcctccgc ctccggactg gcctgggctg 13620
gattccttgg gggggtcctc tgccctgccc ctcctccagc ccctccccgc tccccttcag 13680
acgattttgg tttggttgct cctgcttctg gcggggtcgg gtgtgtgtgt gtgtggtgga 13740

gtggagggtg gcatagcaac ctgtcccaac cagagccggg gaggaaaggg tggcccggag 13800
ggtggcctct tgctggggtc tgggttgggg gcgggggaga cgtttgcttt gaacagattc 13860
ttggggccag cttagggact gtgctctgtg acttttggag cgcgtggacc atggaggggt 13920
gggggtgggt ttcttggggt gtaaagtggg agagttccca gagaaggaag ctaagaaata 13980
aggccagatg ggagcctagg gagggctgcg ttgttctgct gccttttcct tggtgctgtg 14040
cgtggggaag ggtgagtggg ggcagtgtgt atcctgaccc atctgtccac ctgtgtgcat 14100
taatcataaa agctaacata tagcctgggc caggtatact ctgccaggaa ctgtttgtgg 14160
tgttttgcat gcattctcct ttaatcctag aacaccccta tagtggaagt tctgccagca 14220
ttctggactg agtagcagtc cagaggttga gtagcagcta gtaagtggtg gggtcaagat 14280
gggaccccag gcagtgcgac ccccaaccat gcattcgaaa tcgctatatg gatgagtgca 14340
cctggagcaa tgagggacac tgctccctga gtcactgggc tgcaggggag acaaaatgaa 14400
agtgttctgg gagtcgtggg tggtctccat aggtcagagg gtctggggag ggagtgggtg 14460
tcatcgtggc tgtgtgttgc ccgaggggcc ctctgtgagt gagtgcatgg ccgtgttatc 14520
tctgcaggtc tacgccaggg tgttcctcag ttgtgtggtc tttgtatttg tgtgtctggg 14580
ctttgtgttg ccaaacagca gtctctctgc tgacttgggg acacaggctg aactctgtcc 14640
tctgcaggaa ctcccttaag gtgctgggcc agatctgcca taaacagagg gaggtagcct 14700
tctatggcca cgccttcttg ctgaggaaga aggttcctct cttccaggga gtacatcctt 14760
gccctccctg tttcccagac aagcatcttc acctctcatc ttctgatgag aagggtgagg 14820
ccatactgag ctgtcaggct gagctgctgc ccttcctcac cttgggctgg gagttgatca 14880
gggaatggca gttgctgcag agctggattt gagggctggg ttctctggat ggggcctcct 14940
catgtcctca cccctcaacc tgcactattg attgtgttgt gcaggagtta gttaaaaagt 15000
cattgcacag cctgggcaac aaggcaaaac tctgtacaaa aaatacaaaa attagttgga 15060
tgtgattaca cgtgcctgta gtcccagcta ctccggaggc tgaggcagga ggatcacctg 15120
agcccaggaa gttgaggctt gcagtgagct gtgattgcaa atgctctcca gcctgggtga 15180
cagtgtgaga ctccgtttca gaaaaaaagt ataccaccca gctgcctcca gcacccagat 15240
tttacccaag gggtgaggtc tggggcagga atgtggggga aggggaggcc tagggggagc 15300
cccagagggg tcaggatttt tctgaaatcc tttcttagag gtatgggttt tacaaattgc 15360

agcaaataca tccttttaat cttgcagaac tccttcatat tttaattcca gtatgattct 15420
tccaacagcc tcctctcttt actatacttg gggaaagtac tcattttatt tgtcaagaaa 15480
aaaacaattg aaaagatagg gatcaaatgt aaaaagaaaa aatacgtggc attccaaagt 15540
caaacacaaa gcatgtttaa ttttctcgtg gtttgggatt acccatattc ctgctgtatg 15600
aacctgtctt gtcttaactt ttaagaaatg tacggtgtac ttcctatatg ctaggttttt 15660
atccatgctt tcatttaatc tctgtgacag tcctgtgaag taggtgcaca gatgagaaaa 15720
tggaagttca gagaaatgaa gcaacttatc caaggctccc agctacccag taatgtccag 15780
ggaattttg gactctgaag aggaggcatt aagaggtggt tagagtctta ttccagccaa 15840
caataatggg ttgaacaaag ccttaggggc aggcaggtgg ccagatggga ggagaagcgc 15900
tcctcttgtt caggcgaatg acctttccat ccacttctct aggctgtaga aagtggagct 15960
gagctggggg ccctgaggtt ccctcttgac ttcagagtcc tctcccttcc tgtccagcca 16020
atgcctgtct tccttttggg ccctaccagc atgacagggg gctgcgggca ggaggggaca 16080
gaggccacgt tgacacacag ggctgtgggt gagagagaca gctgaagtgt cagcgtgagg 16140
ggccagtgtg gggctgcggc tgggagggct ggggtggggc ccagggtagt tgtgcctgtc 16200
cttgggtgat ggaatgatct ggaaagagat tccttccctg ccctccacct gtgagaagcc 16260
cctctagagt gacatctcca tcttatgttt ggccacccat cctccccctg ggaagagagc 16320
cgaggtgggg taagggatgt gtactctttc aaggagtggg agaattattc tagcgaatgt 16380
ttgtgttgtc ccagttctgt ttacaaagcc tcgtcatgtt tacagatggc tgcgcaattc 16440
attacctcat ttaactctca tgtacctcct ctgagggagt aagagctgtt acagccaagt 16500
ttaggtcagt aaatattcac caagttgcag gtactgcagg gcatagagat gaatccgatt 16560
tagcttctgc cctggaggtc tgggaacttg ctcaagatca ctcagtgagc agctgagcta 16620
gggttctcaa ctaaagaccc tgggcccagg ccctggtctg atgtcaggcc tgatacacca 16680
ggtgtttgtg gtcggggaat cccagtgtca cttgaatggg ctgtgacatt atgggtctgg 16740
gagagctgag ctttggggac acaggtcatt ttactgtagt attcatggaa accaagggaa 16800
gtattggctt ttctgctgtg agcaagagga gcagctgggg ctgcaagctg gtggggagga 16860
gagaacccac ctgagagaaa cctcaggact ggggtcaagt cctgaccacc agagtccaga 16920
gagacatgaa ggactgtgac cagctctgag cagagagatg gattccatga cctcaactgg 16980

tcccttttgt tcggagactc gtgactggac ttcattcatc cactcattca ttcattcact 17040
cagcagacac ttatctagcg ctccctgtgg ctggtcctgc ctcatactgt ctttgctctg 17100
gagaattgga ggttggggtt cctgaggggc agggtcctgg agacaaggac actcctgggt 17160
agaattagga cctacccccc aggaaatcaa cggggaccag gtgccgtggc tcacacctgt 17220
aatcccagca ctttgggagg ccgagacggg cggatcacaa ggtcagcagt tcaggaccag 17280
cctggccaac atggtgaaac ccgcctcaac taaaaataca aaaattagcc aggtgtggtg 17340
tcaggcaccc gtaatcccag ctactgagga ggctgaggca ggagaattgc ttgaacccgg 17400
gaggcagagg ttgcagtgag ccgagattgc gccactgcac tccagcctgg cgacagggcg 17460
agactccatc tcaaaaaaag aaaaccaatg ggacagggca gatatgggga caatggtaag 17520
gagatgggag agtgggaggg aggtgtcagg aagaccttct tgacttcatg taggctggtg 17580
ggggtgttag ccagcaagcc tccagttccc tgggaaccgt tctcagggta ccaattttac 17640
cacctgtctg caaacacttt aagattctta atcagactca aattggccac aaatcaggta 17700
aacaaactca ctagtggggt ggggctacca cccgttctga ccctccagcc caacccagcc 17760
cagccaccct gccctccgta gagcctgtgg tgtttatcgg tggcattggg agaattagtg 17820
tgtatttatg ttggcgtggg gtgtggggtg gatttgtgtg tgtgcagtta ggcctagtgg 17880
aaggaatgtg ggatctgaag gcaggccagc ctgagttcca gtcctgcctg ttgctcacaa 17940
gctttatgag gcgagagcta acccctgcca gcctcagttg tcttctttgc aagatggagg 18000
ttgcagcccc agtctctgga gcatgttatg cagatccacc gagagtgcct gccaggcaca 18060
cagtaggtgc tcagctcagt tactgtggcg gcccccactc cccattgttg ttgttttcct 18120
attgcctggc ggccacagct ggtatccctt gaaaagggct acagggggtg gagtcggacc 18180
ctgccccagc cctgtggaga ccctgggctt gggccagggc ctggggtctg ggcctgcaga 18240
cagctgtgtc tataaagcag ctgaagggct gaggccgggg gaggtcctgg cagcagggcg 18300
ttattttggg cctggcctgc cacccccagc tcctgtttct cttgggagtc tgttggggga 18360
ggaagtgtgg ggaagaggag ggggtgcaag tgggtgaggc atggagtggg gaggcctccc 18420
tcagggacat ggacccttga gttctatttc tgttcctccc tcctgttcct ccctctttgt 18480
ccttatctgc ctagagaggt gggaatagag gccattctga gtatcactag gagaccacca 18540
gtttgtggcc actggccact ggcccaggca gggaacctgg gggcttgccc taccagcctc 18600

tcccagcaat ctgaaggcag ggggtacctc gtattacccc ctaggatttg accttaggct 18660
ccaacttgct gggagagcag tgcctctggt gtcagacccc aagccagccc ttgtgctgtc 18720
cctgaatctg catgtagcct gtgggaggcg gagcagtgac cggcaggaat tctgggcagc 18780
tcaggcacct gtgggcctga gggtgccctc tgcccccacc cttccgatct cctgggcaag 18840
acacgccagg tgattcatct caccagagca gaaaaacaag ttcaactggg cactttaatc 18900
tcccctcact ggcaggcctg gtgtgagctg ctaccccggc gcccctcacc aggggtgctt 18960
tacctcctct agtattcctg accttagtgg gcatttctgg tctcagggat accaggctgg 19020
ggtccaagtg ggccaggtgt ggcagttcag ccctatgccc catggctgat ggctcgcgct 19080
gggcaggtat gcagggctga cgtagtgcct ttgtggcagc agtttcgtgg cacacattct 19140
gccagctggt tctggagtct tgccctgagg aggtggccag ggtgagggtg ccagcgcagg 19200
aacctttggc gcatgcttca ccctggcctg ggatctgcag cctgggtcca gatgcccaca 19260
actggaatct gacgctcctt ttctcttcat gggggactcc cagaggtctc tgcaatgacc 19320
agagccccgg ttgtcccatg cctcagctgc aactccagct gaccctcctt ccccactctc 19380
tgggtggcat tacgggggtg tggatccctt gccaagaggt tggcatgtgg gtgtgctgga 19440
atggcatagg gagaatgcac cgagtttgtt tgcttgggag aggggcaggg ggtatccaga 19500
agattcatga ttcgtcatcg cctctcttgg gggattttta ccccttttgcc ctgagttgtg 19560
cctttgggac aaaggaagcc tttctttgcc agccaacacc ctgtactggc gggcgagctc 19620
cccagggctg gcacgctggg gcagcctctg aatgcacagg gtgggcctag tcagaagaag 19680
cctttcccct gaaatccctc tacttcccaa gcacgcaagc tttctcctgc tgttaaacct 19740
gcagtgtgca agggacatgg gcggaggggt ccttcagtca ggcttctccc tgtctgaggt 19800
ggcatgactt ggagtgagtt tggatggggt ggccaggtct gagaaggtcc cccgccagtg 19860
tcctctgacc catctgctct ctcctgccag tgtgcaccgg cacagacatg aagctgcggc 19920
tccctgccag tcccgagacc cacctggaca tgctccgcca cctctaccag ggctgccagg 19980
tggtgcaggg aaacctggaa ctcacctacc tgcccaccaa tgccagcctg tccttcctgc 20040
aggtgaggcc cgtgggcaac ccagccaggc cctgcctcca gctgggctga gccctctgtt 20100
tacaggtggg tggcagaaga aggtgccctg cccttctgtt tcctctcttg ttgtggtttc 20160
tcaaccagga agtcctttct aacatctaac ccccattcat tttactgcag aatcagttga 20220

ctctctctat aacgtggctg gccgaggtca tgtctggatg ggatgcgtct gtgtttccgc 20280
taaatcttgt gctctcttgc cagcatgatc atgtcccctg tccacctgct ccagccacta 20340
tccctctccc acttacagca gaagaaaggg ctggtgagaa aggtggatta caggcccact 20400
tctgccactg acgagcccta tgaatgtggc ctacaccccc ttagcttcac tgggtctcag 20460
tttccctatc tgtatattgg gagcagttgt gaagctcaga agagaaatgt ctgtgaaaag 20520
gttatgaaca ggagggagag tggaaaccaa cctgctggat cgtgtccaca gaccctggaa 20580
tggggccaca tgcttggttt gtcaaattgc agacgccggc cgggtgcgat ggctcatgcc 20640
tgtaatccca gcactttggg aggccgaggc ggacagatca cttgaggtcg ggagttcgag 20700
accagcctga ccaacatgga gaaaccccgt ctctactgaa aatacaaaat tagccaggca 20760
tggtggcaca tgcctataat cccagctact tgggaaggct gaggcaggag aatcacttga 20820
acctgggaga cggaggttgt ggtgagccta gatcgtgcca ttgtactcca gcctgggcaa 20880
caagagtgaa actccgtctc aaaaaaaaaa aatttgcaga cgccatccca tccaggcctt 20940
tgctttcact gatgaagaaa ctgagataca gagagggcag ggcacctgtt cggagtttat 21000
gaaatgcccc cccaccatta tctttcttga tcatataaga atctggtgag gcaaggtagg 21060
gcgtgatctt tatctctatt ttatcgtttt atttaagcgg gaacaggact gctcagtggc 21120
tgggggcctt gcccaagatc tccaagtact ggggaacccc agggaggccc tggggggtgg 21180
cagtgttcct atttcagccc cactctgctt ccccctccca ggatatccag gaggtgcagg 21240
gctacgtgct catcgctcac aaccaagtga ggcaggtccc actgcagagg ctgcggattg 21300
tgcgaggcac ccagctcttt gaggacaact atgccctggc cgtgctagac aatggagacc 21360
cgctgaacaa taccacccct gtcacagggg cctccccagg aggcctgcgg gagctgcagc 21420
ttcgaagcct cacaggtggc cttcaccgtc attgaaacct tctcttggtt attcagagct 21480
gaccagggcc actgctaacc agggggaggc tttgtgtgca ttagaaatgg tgtcccttct 21540
gggcagacgc aggcagagcc cgggaagacg ccctcagaag attggaaaaa gattcccctt 21600
cttcctggga agttgtagct tgcgtcagca catataattc aatcgtgaga atgcaggctg 21660
ggtttttgcc cccacttggc tgagtgaagt gtacagtgaa caacctatgt aactatttgc 21720
tggccctgga gccgactctg ccccagagtc tgggtgccag gtgctttgcc cgcatggccc 21780
atttcagtca cgctgcagtc ctgtcaggaa aaaatcagtg ttattctcat tctacatatg 21840

agaaaactga ggcttgcaga tataagggcc aaaagttaca cagctagtga gtgatggggc 21900
tgagtttcag actccacagt ctcttaacca ccaagcagca tgcccagagt agaggtgaga 21960
aggaaggaga gagctgcggt ccacatgagc atctggacct agcatggaca actcactcct 22020
ccctggctct cgctttgttc ttgttgcggg tgtggtggtg gtgggactca aagacggtaa 22080
agatagcttt ctctcctccc tggggaatct gggggttgtt taaaaggcct gctcctcttt 22140
tagaaggcag gagggcccca agggaagcag aaggtgacag aaggggaaag ggtcctctga 22200
tcattgctca ccccacagag atcttgaaag gaggggtctt gatccagcgg aaccccccagc 22260
tctgctacca ggacacgatt ttgtggaagg acatcttcca caagaacaac cagctggctc 22320
tcacactgat agacaccaac cgctctcggg cctgtaagcc atgcccctcc ctgctgcctc 22380
ttctctcaga cagcctgacc ccagccgcaa actcccaact tacaacccag tgcctgcccg 22440
ccactgcccc agccgcctac accacccatt tcctccctct ctgtccctcc tgccatctcc 22500
ctgtgcctct tcatctctgg ggttctctgt cttgtctccc tctgcttata ggttgtgcct 22560
ctggtttggg ggcctctcag cctgtctggg tccctccctt gctgtgcagt tggcctcgtg 22620
gcctctgctg ctgtttgtgc ctctctctgt tactaacccg tcctctcgct gttagacatc 22680
tctctcactg cctgtctctg gttctgtcct caggccaccc ctgttctccg atgtgtaagg 22740
gctcccgctg ctggggagag agttctgagg attgtcagag ccgtgagtct cagggaggcc 22800
tggagtcagg gaaggggagg gctggggccg ggtggaatgc aggtgtcata caggtgacat 22860
gggaggggtg ggataacagg cttgggatgt ctcccctggg ccaggtagtc tccctagaag 22920
gtgatgctga tgagggtctg gtgcccaggg cgccactcag ccctcatcct gccctttgcc 22980
caacagtgac gcgcactgtc tgtgccggtg gctgtgcccg ctgcaagggg ccactgccca 23040
ctgactgctg ccatgagcag tgtgctgccg gctgcacggg ccccaagcac tctgactgcc 23100
tggtatgtgc ctctgctttg tgcccaatgt gctctacccc ccaggatgca aggggtgggc 23160
accctgcctg gtactgccct attgcccctg gcacaccagg gcaaaacagc acagtgaaag 23220
ccagccacct gtccccccag gcctgcctcc acttcaacca cagtggcatc tgtgagctgc 23280
actgcccagc cctggtcacc tacaacacag acacgtttga gtccatgccc aatcccgagg 23340
gccggtatac attcggcgcc agctgtgtga ctgcctgtcc ctgtgagtgc cagggagaaa 23400
cacagttttc tcattttggt ggggaggttt gtttctgtaa atgggagcat atggggagca 23460

ctgtctgcat cttgctttga gagctggtca tgacagttcc tgccgagctg ccttgttctt 23520
tcaacagctg tggagcaggt ggcagtaagg agaggcagct aagagcccag acttgggagc 23580
cagactgcct gggtttgaaa cccagctcta tcaattagta ggcacgtgac cctcttgctg 23640
tgcctcagtt tcctcatcag taaaatgggg gcaagaatag tcccaactgc ataagatggt 23700
tataacattt gaaagagtta atatttgtaa agctcttaga acggtgcctg gtatgtacta 23760
agtgctccta aatgttagct tttattctat agcctggtga ggtcagtttt acctttcgtt 23820
ttgtttttga gaccgaattt agttagctct atcgcagtgg cgcgatctcg gctcactgca 23880
acctccgcct cccaggttcg tgctattctc gtgtctcagc ctcctgagta gctgggatta 23940
caggcgccca ccaccatgcc tcgctaaatt ttgtattttt agtagagaca gggtttcacc 24000
acgttggcca gactggtctc gaactcctga cttcaggcga tccacctgcc tcggcctctg 24060
aaagtgctgg gattacaggc gtgagccact gcacccggac tttttttttt ttggcagagt 24120
ctcgctccat tgcccaggct ggagtgcagt ggtgcaattt tggctcactg caacctctgc 24180
cttccgcatt caagcaattc ttgtgcctca gactcttgag taggtggaac tacaggcatg 24240
caccaccatg gctgggtaat ttttgtattt ttagtagaga cggagtttca ctatgttggc 24300
caagctggtc tcgaactcct gacctcaagt gatccacccg ccttggtctc ccaaagtgct 24360
gggattacag gcatgagcca tcgtgcctgg cctagctcag ttttatttaa cagatcacct 24420
atttactgat gggcgtttat ggactgggct cagacctggg gaacctcttt cctcctctca 24480
caggaacagg agtgggcctt cagatcctgg ctgactgtgt tagggagagg acaaaatgta 24540
gagccagacc atttgggttc aaatcctcgc tcctccactc actagcacaa tgaccttgaa 24600
taatttacag aactctctgc tttggtctcc ctttttgcaa aatgggaatc tcacagtgct 24660
gatcccgtct ggttgttgtg aggggtaaat ggatgtcagg tgctgatgcg tggtagggca 24720
tttaagtatt ggttgatatt attcttcttg tgcctgggca cggtaatgct gctcatggtg 24780
gtgcacgaag ggccagggta tgtggctaca tgttcctgat ctccttagac aactaccttt 24840
ctacggacgt gggatcctgc accctcgtct gccccctgca caaccaagag gtgacagcag 24900
aggatggaac acagcggtgt gagaagtgca gcaagccctg tgcccgaggt acccactcac 24960
tgcccccgag gccagctgca gttcctgtcc ctctgcgcat gcagcctggc ccagcccacc 25020
ctgtcctatc cttcctcaga ccctcttggg acctagtctc tgccttctac tctctacccc 25080

tggcccccct cagccctaca agtgtcccta tatcccctgt cagtgtgggg aggggcccgg 25140
accctgatgc tcatgtggct gttgacctgt cccggtatga aggctgagac ggccccttcc 25200
ccacccaccc ccacctcctc agtgtgctat ggtctgggca tggagcactt gcgagaggtg 25260
agggcagtta ccagtgccaa tatccaggag tttgctggct gcaagaagat ctttgggagc 25320
ctggcatttc tgccggagag ctttgatggg taagagtggg cacgatgacc tgagacagtg 25380
tcagggcaga cagagtcctg aggatccaga tgtggcagca tctcttgggg atggcaggag 25440
acagaagtgg ggggatcaag aatgcaaaga aagcagatgg gagaccagag gagcagggcc 25500
tttggtgggt gggggtgatt atttttgtaa atgacatgct atccgtgaac aaggacttgt 25560
atggaggtca gaccatctag ataaagtaaa attccctttg agttcatagc agctttattc 25620
aaaatatccc caaattggaa ataactcaaa tgtgcatcac taggtgaagg aataaacaag 25680
tggcagtgta tccatttggt gaagttctac ttagcaacca aaggaaatga actaccgata 25740
caacataaat gaatctcaga aacattacat tgagcaaaag aagccagaga caagattcca 25800
tactgtctga tcccctttat gtgaggctct gaaccgaaaa aaccactctg tggtgggaga 25860
gatcagaacg gtggttgccc cagggtgggg ggcttcaaaa gggaggcaca caaggacatt 25920
tctggggtaa tagaaatgct ctgtatagtg attggggtag tggatacatg agcgaatcca 25980
tttgtcaaaa ctcatcaaac tgtgtgataa gagtctgtgc attttattta tttcatttta 26040
tttttgaga tagagtctca ctctgtcagc aggctggagt gcagtggtac gatcttggct 26100
cactgcaacc tctgcctcct ggattcaagc aattctcctg cctcagtctc ctgagtagct 26160
gggactacag gtgtgtgcca ccatgcccag ctaattttg tatttttaat agagatgggg 26220
tttcaccatg ttggcaagga tggtctcgat ctcttgacgt cgtgatccgc ccacctcagc 26280
ctcccaaagt gctgggatta caggcatgag ccaccacacc cggtgcattt tattgtatat 26340
aagttatact tcaataagaa atgaattggg gccaggcacg gtggctcacg cctgtaatcc 26400
cagcactttg ggaggccgag gcaggcagat cacttgaggt caggagttca agaccagcct 26460
ggccaacatg gtgaaacccc atctctacta aaaaatataa aaaattagcc aggcttcctg 26520
gcatgcgcct atcatcccag ctacttggga ggctgaggca ggagaattgc atgaactcgg 26580
gaggtggagg ttgtagtgag ctgagatttc gctattgcac tccagcctgg gcgacagagt 26640
gagaccctgt ctcaaaaaga aaaaaaaaaa aaagggtcag gcgccgtggt gcacacctgt 26700

aatcccagca ctttgggagg ctgaagcagg aagattgctt gagcccagga attcaagaac 26760
agcgtgggca acatagtgag atcccatctc tacaaaaaaa cacaaaaaat tagccgggca 26820
tggtggtacg cacctgtagt ctcagctact agggagactg aggtgggaga atcacctgag 26880
cctgggaggt ggaggttgca gtgggttgaa atcatgtcac tgtactccag cctgggtgac 26940
agaatgagac cctgtctcaa aaaaaaaaaa aaaaaaaaaa ttccctttca cacttccttt 27000
acctccactc ccctttccag agggggccat ggttaacagt gtgtgtgttc acctagaccg 27060
tttatgcatc tgtagacaca cacacagtga agtgtggttt tcgtcgtttt ggtggggagg 27120
ttggtttctg taaatgggaa catataggga gcactgtctg caccttgctt tgagagccgg 27180
tcatgacagt tcccattgaa ctgccttgtt ctttcaatag ctgcagagca ggtggcggca 27240
aggagaggca gctaagagcc cagacttggg agccagactg cctgggtttg aaacccggct 27300
ctaccactta ctaggcatgt gacccttgtg ctgtgcctca gtttcttcat ctgtaaagtg 27360
ggggcaagaa cagtcccaac ttcataagat ggttatacca ccatgcctgg ccagatgatt 27420
ataaagtttg aatgagttaa tatttgtaaa gctcttagaa cagtgcctgg cagatactag 27480
gtgctcctaa atgttggttt ttattatgtg gctgggtggc tcggggtttt atttaacagc 27540
tcccctattt actaatagac atttagatca tgttccattt tcactcttac aaacagttcc 27600
actttgtgtg tggctctggg aacatgggcc agtgtctccc taggccacat tcctagaaat 27660
aagatttctt ttcttttttt tttttttttg agacagagtc tcgctttatc gccaggctgg 27720
tgtgcagtag tgtgatctcg gctcactgca acctctgcct cccgggttca agtgattctc 27780
ctgcctcagc ctctcgagta actgggacta taggcgcgcg gcaccacacc cagctaattt 27840
ttgtatttgt agtagagatg gggtttcacc atgttggcca ggatggtctc catctcttga 27900
cttcgtgatc cgcccgcctc ggcctcccaa agtgctggga ttacaggcgt gagccactga 27960
gcccaggcag aaataagatt tctagatcaa aggatataaa tactgttttg atagatgttg 28020
ccgaactaag gcctgggctt tgaagcccag gatgggaaca gctgggctcg atgggcaaag 28080
ggtttgagtg aaggcattca tggtggggag tggctggcat ggccagtgct gggagtgatg 28140
tccaccctgt tcctggccct gctgactcct ctcctgaccc ctccagggac ccagcctcca 28200
acactgcccc gctccagcca gagcagctcc aagtgtttga gactctggaa gagatcacag 28260
gtgggctctg tctctgcatc ctgttctgca ggggctggga gtccttgtcc tgtccccact 28320

cctttaatct caccctctgc ctgcaggtta cctatacatc tcagcatggc cggacagcct 28380
gcctgacctc agcgtcttcc agaacctgca agtaatccgg ggacgaattc tgcacaagtg 28440
agcactgaga aagagggggc ctgatgggga ggagtcccag ggaggagtcc ctgtgggaag 28500
ctttgggcct gagggagtac tcctgtagca gtaacctttc catgaaagtc tgcagagtgt 28560
gctggggatg gaggaagatg agaatagcct ttgctgaccg ggaaggggtc cgtggtaagg 28620
tgcccacctt tctcccatag tggcgcctac tcgctgaccc tgcaagggct gggcatcagc 28680
tggctggggc tgcgctcact gagggaactg ggcagtggac tggccctcat ccaccataac 28740
acccacctct gcttcgtgca cacggtgccc tgggaccagc tctttcggaa cccgcaccaa 28800
gctctgctcc acactgccaa ccggccagag gacgagtgtg gtaagacagg gagcccagtg 28860
tgcgcactcc ccatctgcca gcacacagca gtgcccaggg ggccctggca gcagcgttct 28920
tggacttgtg cagactgccc gtctctgtgc acccttcttg actcagcaca gctctggctg 28980
gcttggcctc ttggcatggc ttctctagct gggtcctacc tgccttggca tccttccctc 29040
cccctctgtt tctgaaatct cagaactctt cctctcccta catcggcccc acctgtcccc 29100
acccctccag cccacagcca tgcccacagc cagttccctg gttcacttgg acctggggcc 29160
tcccctaaaa gtcccctgcg gtcccttcct cctcactgca gtgggcgagg gcctggcctg 29220
ccaccagctg tgcgcccgag ggcactgctg gggtccaggg cccacccagt gtgtcaactg 29280
cagccagttc cttcggggcc aggagtgcgt ggaggaatgc cgagtactgc aggggtatga 29340
ggggcggagg agagggtggc tggaggggtg catggggctc ctctcagacc ccctcaccac 29400
tgtcccttct ctcaggctcc ccagggagta tgtgaatgcc aggcactgtt tgccgtgcca 29460
ccctgagtgt cagccccaga atggctcagt gacctgtttt ggaccggtga gctgctggcg 29520
ggctcagagc tgggtggagg ggggcagcga gggggattgc cagggacttg gcaggatggc 29580
gagatgcagt agggtgtgct atctggtaaa atatccctgg agagggctca gcgctcagac 29640
ctgaacagca acagagtggc agaaaagggg cctgggggac actggggccc ttcagactat 29700
gaaaaggttc taaggaggtc tgtgttggtg gctgtgactg tggctgtgct agggtggtga 29760
gccctgtggg ctcaggcgtc agactacctg gattcagacc cagctcctgc ttccaacctt 29820
ggttttttat tcctaaaatg ggtattgtaa taatacctac cttgctgggg tgtggcaaga 29880
atgaaattaa acagggcttg gcacagtgaa gcacgggaaa ggctttctac agagcagtga 29940

ctgttgttac tcgctgttac accttaggta atgcgttttc ctctctgggt gcctcccatt 30000
ttctggctca agtccctgcc caggatcaag cttggaggag ggccccgagg gaggggccac 30060
agagactggg tgaagagcaa gggtgtttgt cccaggagca tggcgaaaat tgctgctggg 30120
tggccttggg aagcacaaag gggacccaac taagggcctg atcctactgc cctgggggtg 30180
tcagtgccag ccccccacaa atcttttctg cccccccccag gaggctgacc agtgtgtggc 30240
ctgtgcccac tataaggacc ctcccttctg cgtggcccgc tgccccagcg gtgtgaaacc 30300
tgacctctcc tacatgccca tctggaagtt tccagatgag gagggcgcat gccagccttg 30360
ccccatcaac tgcacccact cgtgagtcca acggtctttt ctgcagaaag gaggactttc 30420
ctttcagggg tctttctggg gctcttacta taaaagggga ccaactctcc ctttgtcata 30480
tcttgtttct gatgacaaaa ataacacatt gttaaaattg taaaattaaa acatgaaata 30540
taaattaatg ccctagcagt tctatcccca ctgttaataa tttgaaatat ttttcctcta 30600
gttatttttg tctgtgcaca ttctaatatg tatatataag ttaacatata ttaatattat 30660
tctccagtta tttttatctg tgcacatttt aacacacaca cacacacaca cacacacaca 30720
catatgtatt tttagacgga gtttcactct gtcgcccagg ctggagtgca gtagtacaat 30780
cttggctcac tgcagcctcc acctcctggg tttaagcaat tctcctgctt ccgcctcctg 30840
agtagctggg attacgggaa cgtgctacct tgcctggcta atttttgtat ttttagtaca 30900
taggatttca ccatgttggc caggctggtc tcgaacccct gacctcaggt gatctgccag 30960
cctcggtccc ccaaagtgtt gggattacag cggtgagcca ccatgcccag tcatatattt 31020
ctttttaaca aatagaatca tagatcatac atattgtttg caaattgctt tttctcactt 31080
tccagaacct tgaaatgttt ttccatgttc taacatggtg atctacctta ttcttttaat 31140
ttttcttatt tagttgtctt tacacatgaa acacatgaat acatccttgt gataaacatt 31200
ttcagtaaca taaaagtata aatgttacaa agccaacgtg ccctttcact caactccctg 31260
tccacccagt ctctcctgtc tgctgggaga accaccgcat tgacttgtgt gttcaccctt 31320
ccaggctctt ttctgcacac ttatatagac atactacatt tatattaggt cgagtcaaat 31380
aagattgctg tttgtgtaaa ccaaaaagtg tcaagagcct gggcgcagtg actcacacct 31440
gtaatcccag cactttggga ggctgaggca ggcagatcac ttgagatcag gagttcgaga 31500
ccaatctggc caacatagcg agaccccgtc tctactaaaa atacaaaaac tagccaggtg 31560

tggtgatgct gttctgcact ttgctttccc cccgacttga ggtatccttt cttgtgagta 31620
cagacggatc taccaccttt attttttttt taattactca acctgtaaca tggatgtaat 31680
ttcactttgt ttttgaggga tattgagctt gtttccctgt ttttgcagtt tattgcaatt 31740
gagctccaca cacaagtgag ccctcttttg tatgcccccct agtgggaata cagtgctggc 31800
aatgtttatc acaaggatat attcatgcat ttcaatttaa agacaactaa atgagaaaaa 31860
ttaaaagaat atggatccag gctgggcatg gtggctcacg cctgtaatcc cagcactttg 31920
ggaggccgag gcaggcagat cacctgaggt caggagttca agaccagcct ggccaacatg 31980
gcaaaacccc gtctctacta aaaatacaaa aattagccag gcgtggtggt gggcgcctgt 32040
aatcccagct atttgagagg ttgagacagg agaattgctt gaacctgggc agcggaggtt 32100
gcagtgagac gagattgcac cagtgcactc caacctgggc aacacagtgc aactccttct 32160
caagaaaaaa aagaaaaaaa aaaagaatat gggtccagat ccatatggat cctagatcca 32220
gatcacggtg ttagaacatg gaaaaacatt gcaagattct gctaagtgaa aaaagcattt 32280
gcaaacagta tgtacagtct atattcagag gaggaactgc tgggtcatag atgatatttc 32340
ataggtattg ccaaaccgtt ctctggagaa gtggtatggg tttaccctgg gattcttcta 32400
tggagggaat agttgagctc ccgggcttgc tcttctgggt gcccctcccc gcttcctatc 32460
caccacaagg agctgcaggg gagcggggca tgccggttcc ttggctggag aaggagtctc 32520
cttgtgaggt ggtagaagga gcactgacgg ccttgagccc agtttctgcc tttgtcaaat 32580
ggggataatg acccagccac acccctccca gggttgttgt gaggctggaa aggtggttcc 32640
caagagggtg gttcccagaa ttgttgatga gactgtttct cctgcagctg tgtggacctg 32700
gatgacaagg gctgccccgc cgagcagaga gccaggttgg cctggacccc aggatgtacc 32760
cttcattgcc cttcactccc ccactggatg ctgggtggtc actgctgtag ggaggggacc 32820
ccctgacata tgtcccttcc cacccactct tccactgtgg aacctcctgt cattttccac 32880
ttcaccaagt gacagaggac ctgctcagat gctgagggga ggggactgca aggaaagatg 32940
gctaggaaac ccagtccctc cacaccctag agtaacttga tgccttgtga gggacacagg 33000
caaagttcaa ttccttggaa gtcaagggag actgagaaga gtacagctgc agcactgagg 33060
gagtgatgaa ttcttaactg gggatggtgg gaggcttcga gtgggaggtg gcatttgagc 33120
taggctttga gagaggagca ggtattgcac ttgcatttag gtagaaagca ttggggtgca 33180

aggtgacact ggaggggggag gcatcaggaa atccaggatg tcttcaaagt tctggtgtcg 33240
ggggctgttg agtaagcaca ggaataaggg ggtcaagtta gagtcagggt ggggtctgac 33300
ctggatgcca taggacctga tccccaagcc acagggtggg acttgactgg gcagtgggga 33360
cctttggaaa ggactttggg gagaaaaaca gactggagtc tgtcttaggc gatcatcggt 33420
ccgtgaaatg agcatgtgtt acaggcttgg tatgtaccag accctgtgct aagcaagggg 33480
gtatggagag gagagggtga caagaatatt ggatcaacac ccgggagctc catctatccc 33540
aggatgcact atcttttttt tattttttttg agacggagtc tcactctgcc tgcaggctgg 33600
agtgcagtgg ctccatctcg gttcactgca acctctgcct cctgggttca agcgcttctt 33660
gtgcctcagc ctcccaagta gctgggatta caggcacatg ccaccacacc cagctaattt 33720
ttgtattttt agtagagacg gggtttcacc atgttggcca ggatggtctc gatctcttga 33780
cctcaagatc cgcccacctt ggcctcccaa agtgctggga ttacagacat gagccaccgt 33840
gcccagccag atacgctatc tttttattga gtgattgaga cagggtcttg ctctcttgtc 33900
cagtcttgaa tgtggtggtg taatcacagg ctcactgcag ccttgacctc ctgggctcaa 33960
gttacccttc tgcagtagct gggactatag gagcgtgcca ccacgcctgg gtaatttaaa 34020
aaattttttt tgtatagaca gggtctcact atgttgcccg agctggtctc aaactcgtgg 34080
gctcaagtga tcctccagtt ttggcctccc aaaatgttgg gatcacagga gtgagccacc 34140
actcctggcg atgagccaag tctttttttt tttttttttt ttttgatatg gagtcttgct 34200
ctgttgccca ggctggagtg caatgacacg atcttggctc actgcaacct ctgcctccca 34260
ggttcaagca gttcaagcaa tcctcctgtc tcagcccccc agtagctggg attacaggca 34320
tgcgctacca cgtccggcta atttttgtat ttttagtaga gatgaggttt tgccatgttg 34380
gccaggctgg tcttgaactg ctgacctcag gtgatccacc tgcctcggcc tcccaaagtg 34440
ctgggattac aggtgtgagc catcgtgcct ggcggagccg agtcttaaaa gatgaccctg 34500
tggagaaatg gtggtccagg ctgaagggac agcctatgca aacactggga ggtgtggaaa 34560
atcatgacct gtgggtggaa attttggcta gaacatcaaa atcatcaggt gtacattcct 34620
gtacccatgc agcagtcaga atctctgggg gtggggcccc aaaattgtat gcatacagac 34680
tgtgtgctga tttgtgatat tacttaggat tttttgactt tacaatggtg gaaaagcaat 34740
aatatacatt cagtataaac cgtactttga atacccatac agccattctg tttttcactt 34800

ttatttttat ttatttattt atttattatt tattttgaga tgtcattttg ctgttgttac 34860
ccaggctgga gtgcaatggc gcagtcttgg ctcaccgcaa cctccacctc tcaggttcaa 34920
acgattctcc tgcttcagcc tccagagtgg ctgggattac aggcaggcac caccacaccc 34980
ggctaatttt gtatttttag tagagacggg gtttctccat gttagtcagg ctggtctcga 35040
actcgagagc tcaggtgatc tgcccatctc agcctcaagc caccatgccc agccctactt 35100
tcagtattca ataaattaca tagccaggca ccgtggctca cacctgtaat cccagcactt 35160
taggaggcca aggtgggagg atcctttgag gccagaagct cgagaccagc ctgggcaaca 35220
tagtgagacc ccatttctac aaaaaataaa aaaactagct gagtgtggtg gcgtgtgtct 35280
gtagtcccag ctacttgggc agctgaggtg gaaagactgc ttgagcccag aggtcagggc 35340
tgcagtgggc catgatctca ccactgcact cagcctgggc aacacagcaa ggccctgtct 35400
caaaaataaa taaataaata acacaaactt atttaacagt ttactataaa ataggctttg 35460
tgtcagatga ttctgcccaa ctgtaagctg ctggcagtgt aaatgttctg agcacgtgta 35520
agccaggcta ggtgtcttaa atgcattttc agtttcaact tagaattggt ttatcaggac 35580
gtagcccctt ggtgttgagg ggcatgtgta ttaacagtct ccttagtgac ttttttttt 35640
ttgagatgga gtcttgcact ggccgtagtg cagtggcaca atctcagctc actgcaacct 35700
cttgtctccc gggttcaagc gattctcctg cctcagtctc ccaagtagct gggattacag 35760
gcacccacac cacgcccagc taatttttgt gtgtgtgtat ttttagtaga gacggggtt 35820
tcactatgtt ggccaggctg gtctcgaact cctgaccttg tgatctgccc acctcagact 35880
ctcaaagtgc taggattcca ggcatgagcc accgcgccca gagtccttag tgatttttac 35940
accatgaatt gttgaagccc taagccagag ccaagggcaa gagtatagag aatctggaga 36000
tgcggagagg gttctgattg cctacaagga gtttggactt tattgtggag gcagcgggga 36060
gccaaggcag gttttagagt aggagagggt ccaagcctgt gggtcaccct tccgacttcc 36120
ctttccgaat gccaaacacc ttcatgtccc ccgtgggccc cctttgtccc tcccacccca 36180
aactagccct caatccctga ccctggcttc cgcccccagc cctctgacgt ccatcatctc 36240
tgcggtggtt ggcattctgc tggtcgtggt cttgggggtg gtctttggga tcctcatcaa 36300
gcgacggcag cagaagatcc ggaagtacac gatgcggaga ctgctgcagg aaacggaggt 36360
gaggcggggt gaagtcctcc cagcccgcgt ggggtctgca ccggcccccg gcactgaccc 36420

accaccccct caccccagct ggtggagccg ctgacaccta gcggagcgat gcccaaccag 36480
gcgcagatgc ggatcctgaa agagacggag ctgaggaagg tgaaggtgct tggatctggc 36540
gcttttggca cagtctacaa ggtcagggcc aggtcctggg gtgggcggcc ccagaggatg 36600
ggggcggtgc ctggaggggt gtggtcggca gttctgatgg gaggggcaag agctggaggc 36660
agtgtttggg ggagggcagt tacagcggag aagggagcgg ggccaagccc tagggtggtg 36720
aaggatgttt ggaggacaag taatgatctc ctggaaggca ggtaggatcc agcccacgct 36780
cttctcactc atatcctcct ctttctgccc agggcatctg gatccctgat ggggagaatg 36840
tgaaaattcc agtggccatc aaagtgttga gggaaaacac atcccccaaa gccaacaaag 36900
aaatcttaga cgtaagcccc tccaccctct cctgctagga ggacaggaag gaccccatgg 36960
ctgcaggtct gggctctggt ctctcttcat tggggtttgg ggagatatga ctcccgcaaa 37020
cctagactat ttttttggag acggagtctt gctctgtcac ccaggctgga gtgcagtggc 37080
gttatctcgg ctcactgcaa cctccacctc ctggactcaa gcgattttca tgcctcaggc 37140
tcctgagtag ctgggattac aagcgcccgc taattttttt tttttttttg agacagagtc 37200
tcgctctgtc acccaggcta gagtgaaatg gtgcggtctc agctcagcct cccaggttaa 37260
agcgattctt ctccctcagt ctcctgagta gctgggatta caggcgcgag ccaccacgcc 37320
cggctaattt ttgtattttt agtagagatg ggatttcacc atgttggcca ggttggtgtc 37380
aaactcctga cctcatgatc cgcccgcctc ggcctcccaa agtgctggga ttacaggtgt 37440
gagccaccgt gcccggccta atctttgtat ttttagtaga gacagggttt caccatgttg 37500
tccaggctgg tactttgagc cttcacaggc tgtgggccat ggctgtggtt tgtgatggtt 37560
gggaggctgt gtggtgtttg ggggtgtgtg gtctcccata ccctctcagc gtacccttgt 37620
ccccaggaag catacgtgat ggctggtgtg ggctccccat atgtctcccg ccttctgggc 37680
atctgcctga catccacggt gcagctggtg acacagctta tgccctatgg ctgcctctta 37740
gaccatgtcc gggaaaaccg cggacgcctg ggctcccagg acctgctgaa ctggtgtatg 37800
cagattgcca aggtatgcac ctgggctctt tgcaggtctc tccggagcaa acccctatgt 37860
ccacaagggg ctaggatggg gactcttgct gggcatgtgg ccaggcccag gccctcccag 37920
aaggtctaca tgggtgcttc ccattccagg ggatgagcta cctggaggat gtgcggctcg 37980
tacacaggga cttggccgct cggaacgtgc tggtcaagag tcccaaccat gtcaaaatta 38040

cagacttcgg gctggctcgg ctgctggaca ttgacgagac agagtaccat gcagatgggg 38100
gcaaggttag gtgaaggacc aaggagcaga ggaggctggg tggagtggtg tctagcccat 38160
gggagaactc tgagtggcca cctccccaca acacacagtt ggaggacttc ctcttctgcc 38220
ctcccaggtg cccatcaagt ggatggcgct ggagtccatt ctccgccggc ggttcaccca 38280
ccagagtgat gtgtggagtt atggtgtgtg atggggggtg ttgggagggg tgggtgagga 38340
gccatggctg gagggaggat gagagctggg atggggagaa ttacggggcc acctcagcat 38400
gtgaagggag ggaaggggct gcctgtgccc caccttgcag ggtctgtgca cttcccagga 38460
ttagggaaag accgggtagg gtctgtctcc tggcatcaca tctccccctg ctacctgcca 38520
tgatgctaga ctcctgagca gaacctctgg ctcagtacac taaagctccc tctggccctc 38580
ccactcctga ccctgtctct gccttaggtg tgactgtgtg ggagctgatg acttttgggg 38640
ccaaacctta cgatgggatc ccagcccggg agatccctga cctgctggaa aagggggagc 38700
ggctgcccca gccccccatc tgcaccattg atgtctacat gatcatggtc aaatgtgcgt 38760
ggctgagctg tgctggctgc ctggaggagg gtgggaggtc ctgggtggag gagcccacaa 38820
ggggcatgaa aggggaccag gatgtatgta gacccaggag ccctagtatg ttaggagcct 38880
caaaaccttc ttgtatccct tttacagtca aagtccaaag ccactcttga ggaacactct 38940
tgtacaaaat taagctgggc acagtggctc atgcctgtaa tcccagtact tttggaggct 39000
gaggtgggag gatcccttga agccaggagt tcaagaccag cctgggcaac atagtgagat 39060
cctatctcta caaaaaataa aaaaattatc tgggtgtggt ggtgtgtgcc agtagtccca 39120
gctactcagg agaggctgag gcaggaagat cacttgagcc tagtttaagg ttgcagtaag 39180
ctatgattgc accactgaaa tccagcctgg gtgacagagc gaaacctcat ctcaaaaaaa 39240
taaaaaagca aacaaaaaga aaaaaaaaat taaaagggaa actagaagag atgccaaagg 39300
ttctggctga agaccccaga gtctggtgct acttctctac cacctgaggg ctttgggctg 39360
tcccttggga ctgtctagac cagactggag gggagtgggg aggggagagg cagcaagcac 39420
acagggcctg ggactagcat gctgacctcc ctcctgcccc aggttggatg attgactctg 39480
aatgtcggcc aagattccgg gagttggtgt ctgaattctc ccgcatggcc agggaccccc 39540
agcgctttgt ggtcatccag gtactgggcc tctgtgcccc atccctgcct gtggctaaga 39600
gcaccctcct gcagagggtg ggaaggagag atgagtccag tatgccaggc ccctcacgga 39660

aggctgcatg ctgggctggg gaggggccac catcctgcct ctccttcctc cacagaatga 39720
ggacttgggc ccagccagtc ccttggacag caccttctac cgctcactgc tggaggacga 39780
tgacatgggg gacctggtgg atgctgagga gtatctggta ccccagcagg gcttcttctg 39840
tccagaccct gccccgggcg ctgggggcat ggtccaccac aggcaccgca gctcatctac 39900
cagggtcagt gccctcggtc acactgtgtg gctgtctgct tacctccccc aaccccggtg 39960
gactagggtc cctttctctg atgttccctc aactgtcacc tctcaaggaa accccattat 40020
ccctacaaaa aattcttact gccttccaac ccctgtgacc ccattctctc cacggtgact 40080
gtgtcatacc ccaaaggtga cctctgtttt tctcctgtga ccctgtcacc ttccatggag 40140
tccccatccc agatccgtga gtgaccccca tcatgacttt ctttcttgtc cccagagtgg 40200
cggtggggac ctgacactag ggctggagcc ctctgaagag gaggccccca ggtctccact 40260
ggcaccctcc gaaggggctg gctccgatgt atttgatggt gacctgggaa tgggggcagc 40320
caaggggctg caaagcctcc ccacacatga ccccagccct ctacagcggt acagtgagga 40380
ccccacagta cccctgccct ctgagactga tggctacgtt gccccctga cctgcagccc 40440
ccagcctggt atggagtcca gtctaagcag agagactgat gggcagggga ggtgggacct 40500
tcagcccagg gtccactgtg ggggcagagg gagtggcaga gacaccgggg ttccttcccc 40560
taatgggtca ccttctcttg acctttcaga atatgtgaac cagccagatg ttcggcccca 40620
gcccccttcg ccccgagagg gccctctgcc tgctgcccga cctgctggtg ccactctgga 40680
aaggcccaag actctctccc cagggaagaa tggggtcgtc aaagacgttt ttgcctttgg 40740
gggtgccgtg gagaaccccg agtacttgac accccaggga ggagctgccc ctcagcccca 40800
ccctcctcct gccttcagcc cagccttcga caacctctat tactgggacc aggacccacc 40860
agagcggggg gctccaccca gcaccttcaa agggacacct acggcagaga acccagagta 40920
cctgggtctg gacgtgccag tgtgaaccag aaggccaagt ccgcagaagc cctgatgtgt 40980
cctcagggag cagggaaggc ctgacttctg ctggcatcaa gaggtgggag ggccctccga 41040
ccacttccag gggaacctgc catgccagga acctgtccta aggaaccttc cttcctgctt 41100
gagttcccag atggctggaa ggggtccagc ctcgttggaa gaggaacagc actggggagt 41160
ctttgtggat tctgaggccc tgcccaatga gactctaggg tccagtggat gccacagccc 41220
agcttggccc tttccttcca gatcctgggt actgaaagcc ttagggaagc tggcctgaga 41280

ggggaagcgg ccctaaggga gtgtctaaga acaaaagcga cccattcaga gactgtccct 41340
gaaacctagt actgcccccc atgaggaagg aacagcaatg gtgtcagtat ccaggctttg 41400
tacagagtgc ttttctgttt agtttttact ttttttgttt tgttttttta aagatgaaat 41460
aaagacccag ggggagaatg ggtgttgtat ggggaggcaa gtgtgggggg tccttctcca 41520
cacccacttt gtccatttgc aaatatattt tggaaaacag ctaggcaccg gcctatgtct 41580
gggggtggct ctgtgccatc ccttcctgct caccttacac tcaattcctc ttttcctgga 41640
gggagtggct gggaatcttc agaaagcttg gatgaggagc cagacatcca gttctccagc 41700
ttcagggttg ggggagatcg ggaagctgga ttcagatctg agagcccttt gacgaccaga 41760
tcattcttat ttagaacgaa ctaattccta aggccactca ccagaatggg gttatttcct 41820
ccttgtgagt gaggagagtg gtgggtaggg tgggggaaag acgaggcagg gtctactgtg 41880
ggactcattt tccttgcctt ggctatgcca gaggagctca ctgacctaag gagtagctgt 41940
ccagaaagcc cacccagcca gcacagccac cctggcggtg caaaggctgg gctgaccgga 42000
cttctgtgcc ccccacattc ccagactgga cgtataaaaa cacacactag ttagcattag 42060
tgtttgtagc gccactttac tgccaatagc tgacattgcc ctgggttagg ggagaataaa 42120
taaaatctgt ggcatcagac aggtattacc gaggcgaaga gtggactggg ctttcgtggg 42180
cacttaccct gggaaggggg tatgaggtgg ctggagaagt gttcatggag agtgtctctc 42240
tcctgccccc aaggccacgg aatcttctat tccttctttg tacccaaagg gcaaagtgga 42300
ggccagggtc tctttgctaa ggagctaagt aggggaaaga ggcaggggga gctcccagca 42360
ggaccaaagg gagaccaagg tttggacccc agaacagagc aggaacccag agtcctgtgc 42420
agtcacagga tgacgcaggg aggacggctg ttggtgatct tttctagggt ttctccatta 42480
ctggctcttc ggatggcctc aatgagctag aggagtggaa tgacaggatg atgcactgtt 42540
ggggtagggt gaccaagagg tcctcccaac gctgtaaata ctcacatctt tctcataggg 42600
aaagccccca ttctccagct tggagaacac cagctgtcca tttatctcta tctcaaaggc 42660
acctggtaag aaatcaacag ataaatggta cactgagaac ataggcagaa gtcaggagtt 42720
cctccaaagg ggtcccccgc cttcaagtcc cccatcaatc ccactggaga cccatttgta 42780
gctccttaag caattcccca accctgggtc aactccaggg aacctgaggg aggcctcgcc 42840
tgtgtgcccc tcgcaacact caataaagga ctccctcccc tccaacccta cacgattgca 42900

gtctaatagg tttttcccca caggcctctg tgacaccccc tgacctatgg aagggaactc 42960
atgttggccg gacattttac catgtgccag gcactgcggg cactttacat aaagcagcca 43020
atgtcagaac tagaaagcgg gagagctggg attcagagca cagactgacc cagcaggtgt 43080
tctgcgagcc tcacctgtgc ccccgaggcg cgactcgatc tcgatgcccg gatactgctc 43140
cttcacagca ctggccagct ccaggtaggt cgcctcgaag ccgcagggtt cactggggag 43200
tcaagagatg gggctgggct ggggattcgg gggtggggcc tcccgtggac ccacctccgt 43260
ccggcccgcg ggaccagggt gcgggtcctc cagccggggc cgctcaccag tactccacca 43320
cgatgcggac cccactgccc ggctcgacct cctcgggagg gggcgctacg gacgtctgcc 43380
ccggctcccc gctcatcgcg gccggctccg ctcgggcccc tgcttccggg tgtgacgcga 43440
accgcgggca cgtgacgggg cggggcctct ggagcgggga ggggccgagg gtggcgttcg 43500
ggcgcccccct gcgggtctcc gccactgcta cgttttgacc tcgtagggca gctccggagc 43560
tcgctccccg ggcaggggct gggtccggtc cagaccctcc ttccctggcc cggtctcccg 43620
gagtgccggg cttccccact ccccactccc ggtgtccgct ggacttcatg gcttcattta 43680
ctctaatgga ctatcttaga ctgcaaagcg cttttcagtt tacaaaagaa agagaaagtg 43740
gtggggcaag catcctgggg atgggggtgg gtgtggctga tgcatgaaat agttgacatt 43800
ttctcgctaa catgccgatg gcattttttt tttttttttt tttgagacgg agtttcgctc 43860
ttgttgccca ggctggagtg caatggcgcg atctcggttc actgcaacct ctgcctcccg 43920
ggttcaagcg attcttctgc ctcagcttcc cgagtagctg ggactacagg cgcgtgccac 43980
cgcacccggt taattttgta ttcttagtag agacagggtt tcatcatgtt ggtcaggctg 44040
gtctcgaact cttgacctca ggtgatccac cagcctcggc ctcctaaagt gctggaatta 44100
cagacatgag ccaccgcgcc tggcccagtt ttttgtttgt ttgtttgttt gttttagtag 44160
agacagggtt tcaccatgtt cgccagactg gtctcgaact cctgacctca aatgatctgc 44220
ctgccttggc ctcccaaagt gctgggatta caggcgtgag ccaccacgcc cggtgctgat 44280
ggcatttgga accaccctgt agttacatac acaagtgttt ctgatcccaa agctctaaac 44340
tgctttttcc agtcttttgc agctgcttct tgcctgcact ggaaaacagt agatgctctt 44400
caggctctgt gcccccaaca gtttctgcgc atcccttctc atccagcctc tcactggggg 44460
gctggcgtca ttattcccat tttgcagctc tggaaatgga gtctgcacaa gtcctcgctg 44520

cctttccct gcctacagta ccactatggt atcctaacac gccacccact ctagtgtccc 44580
tttaggctat ttatttattt atttatttat tttgagatgg agtctcactc tgtcacccaa 44640
gcttgagtgc agtggcgcga tcttggctca ctgcaacctc cacctcctgg gttcaggcga 44700
ttctcctgcc tcagtctccc tagtagctga gatcataggt gccagccacc acgcccagct 44760
aacttttgta ttactattat tattattttt ttgagacaga gtcttgctct gttggccagg 44820
ctggagtgca atggtgcgat ctcggcttac tgcaacctct gccttccggc ctcaagtgag 44880
tctcctgtct cagcctcctg agcagctggg attacaggtg cccaccacca agccagctaa 44940
tttttctatt tttagtacag atggggtttc accatgttgg ccaggctggt ctcgaactcc 45000
tgaagttgtg atctgcccat cttggcctcc caaagtgctg ggattacagg tgtgaaccac 45060
tgcacccggg ctcatttttt tttttttaa accaggcagg tcgtagtggc tcacacctgt 45120
aatcccagcg ttttgggagg ccaaggtggg cggatcactt gacgccagga gttcaagacc 45180
agcctggcca actcgacaaa accctgtctc tactaaaaat acaaaaatta gctgggtgtg 45240
gtggcgcacc agctactcca gaggctgagg caggagaatc gcttgaacct gggaggtgga 45300
ggttgcagtg agctgagatc tcaccactgc actccagcct gggtgacaga gtgagactgt 45360
gttccaaaaa aaaaagaaaa gaaaagaaaa gaaagaaaat gcagtgattt gtactgttgc 45420
ctaaccatca ctacaatcca gttacagaat gtttccgtca acctgataag tccttcccac 45480
ctgtttctgg tcaatccagc tcccaattcc agtcctgtgc aaccactgat ctgctttctg 45540
tctctaactt tgccttttct ggatgtttca tataaacaga ataatacact atgtggcctt 45600
ttgcttctgg ttctttcatt caatatgttt tttgaaatgc gttcatcctg tagcgtctag 45660
agtagtttgt tccttttcat tgctgagtcg tattccactg tgtggttatg ccacattttt 45720
ttcttaattc accagctgat ggatatttag ggttccagtt ttttttgtct tttttgaata 45780
atgctgctac aaatgtttag gtgtctttgt ataggtgtgt gttttcattt ctcttgggta 45840
gattcctaag ggtagaactg ctgggtcctg tgtttaattt atgattatct tttttttttt 45900
tgagacaggg tctcgctctg ttgcccacgc tggaatgcag cagtgcaaac atggctcact 45960
gcagcctcga cctgctaaac tcaagctatc ctcttgcctc agcctcctga atagctggga 46020
cgacagacac gcaccaccat gcccagctaa tttttttttg tattttttgt agagacaagg 46080
ttttgccatt ttgcccaggc tagcatttaa cattttaaga aatggccaga ctgtttttca 46140

aagttttccc accagcaata tatgaggatt tgtctctcca tatcctcacc aatacttgtt 46200
ttctgtctct ttgattataa ccatcctagt ggacatgaca tggtatgtaa ctgtggtttt 46260
aattctcatt tatctaatga ctatgtcgtg tatcttttca tgtagttatt agccattcat 46320
atacatatta tttgaagaaa catctattca agtcttttgc ccatttttat ttgtctcttt 46380
attattgatt tgtaaaagtt ctttatatat tttggatacc aaccaaaata tatacaacca 46440
tatatacaac catatatagt tgtatatgat ttgcaactat tttctcccag tcttcggcta 46500
gtttttttc tttttcttt ctttctttct ttttttgaga caggggtctc actgtgtcac 46560
ccaggctgga gtgcagtggc acaatcttgg ctcacttcaa cctccacttc ctgggttcaa 46620
gagattcttg tgcctcagcc tcccaagcag ctgaaattac aggcgtgcac caccatagcc 46680
cagataattt tttgtgtttt tagtagcaat ggccaggctg ttttcgctat gttggcaagg 46740
ctggtctgga actctggcct caagtgatct ggctacctca gcctcccaaa gtgctgggat 46800
cacaggcatg agccacttca tctggcttca tttttcttt cttttctttg agacagtgtc 46860
tttctctgtc acccagactg gagcgcagta gcacaatcac agctcactgc agccccaact 46920
tctcaggctc aagtgattgt cacacctcag ccccaccaag tagctaggac tataggcccg 46980
caccaccatg cctggataga ttttatatct tttgtagaga tgaggtcttg ccaggttgcc 47040
taggctggtc tcaaactcct gggctcaagc gattcacctg cctcagcctc ccaaaatgct 47100
gggattacag gtatgagcca ccatgcctga cccttcttt ttttttttt ttttttttt 47160
ttttttcgta tggagttttg ctcttcttgc ccaggctgga gtgcaatggc accatcttgg 47220
ctcactgcaa cttccgcctc ctgggtttaa gcgattctcc tgcctcagac tcccgagtag 47280
ctgggattac aggcatgcac caccatgtct ggctaatttt gtattttttt ttttttttt 47340
ttttttttt tagtagagat ggggtttctc catgttggcc aggctggtct tgaactcccg 47400
acctcaggtg acccatccgc ctcggcctcc caaagtgctg ggattacagg tgtgagccac 47460
tgcgcgcggc ctcctggcct cttattaatg gtggcttttg aagtgcaaaa attttaaatt 47520
ttgacaaagt ctaacatcaa ttttttctc ttgtcagttg tctttttgct gttatattca 47580
agttctctgc ctaacccaag gtcatgaaga ctatctccca tggtttcttc tagaagtttt 47640
atggttttag ctcttacatt caggtatctg atctatttca agtttttttt ttcttatgca 47700
tcctgtgagc aaaagatcta aattcacctt ttggcatgtg gctatccaat tgatcacagc 47760

agcatttatt gaatgcctat cctttcctcc atgaaattgt cttggcacct tgtaaaatca 47820
attgaccaga aatgtaaggg tttctacttg gactctggat tctgttccat tgacctataa 47880
acgcattctt atttattatt attattattt tcccccgaga cggagtcttg ctctgtcacc 47940
ctggctggag tgcagtggca cgatctcagc tcactgcaac ccccgcctcc tgggttcaag 48000
caattctcct gcctcagcct cctgtgtagc tggaattata ggtgcacacc accatgccca 48060
gctaattttt gcatttttag cagaggcggg gtttcaccat gttggccagg ctggtctcga 48120
actcctgacc tcatgatcca cctgcctcgg cctctcaaag tgttgggatt acaggtgtta 48180
gccaccgtac ctggccttat agttgttgtt gtttttaaat tcagatacaa cttatacaca 48240
gtaaaattca tcctcttgag tgtacctttc tttttttttt cttttcttga gatggagtct 48300
cgctcttgtc tcccaggctg gagtgcaatg gctcgaactc agcacactga aacctccgtc 48360
tcgtgggttc aagagattct cctgcttcag cctcctgagt agctgagatt acaggcgtac 48420
gccaccacgc ccggctaatt tttgtatttt tagtagagac ggggttttgc catgttggtc 48480
aggctggtct tgaactcctg acctcgtgat ccacctgcct cagcctccca aagtcctgga 48540
attacagggg tgaaccacgc acccagcctt gagtgtgcct ttctatgagt cctaagaaac 48600
acacacattt gtgcatccaa ccccaaaacc aagatacagt atggtcccat cacctctcac 48660
aagtccctgt gccccctact gtggacccct ctccctcctc cccaacccca gacctggttt 48720
ttgttcacac agttccaccc gctccagaaa gccatataag ttgattccta cagcactccg 48780
ccctctgact gtggctttgt ttccatccca tccttcaata tccaggatga tctttccaaa 48840
atgtaaatgt gccctctcct ctgtgcttct acttaagtct aaatacaact tgcagggtct 48900
ggtctttgac cgctactcct gcccatctgg acaaacagga tggatggctc tcaaacctgg 48960
tttgcagaac agccacctga gggcttgtca ataccacacc ttcctgggtc ttagccctag 49020
agactgagat tcagttgctg gagaggagcc caagaatcta ttttccacac aaggttaggg 49080
aggtctagag gccacatttg cagaagcaca gcttcctctc tccacacctt ccacacctgt 49140
ccaggttcac ccacgtggcc tctgcttcct cccttctcct ccaaccccac tctctgtctc 49200
cgcatgctgc atctcctcct gcccagagct ctggcattga tttcctcact ggggaaagtg 49260
gcctccttcc ccaattccca tccaccgatt ccaaccccct tcccatgtca gccagcttct 49320
cctctgctca gctgtcacct ccactgagaa gtctttcctg actccctatg tgagtcattt 49380

tcccagcata gttgtctcct gtcatttctt atttgtgtga ttctttggtt aacatcaatc 49440
ttcctgattg gctgtgtgag gatgggcggg gtctgttttt gttgtttacg cctgtatccc 49500
cagcacaaaa catggggctg gcatatggta ggtactaaac agatagttga ataaatgaat 49560
gagtgaattg tctactctgc ctttgcacaa gctgctccat caccaaagag accttgcctc 49620
ctcccccact tctttgctgg gcccactcat accggttttc ctaggaaaac tctggagaaa 49680
attgaggcag gagccactca ttcgtgcccc ttccagggtg gacctcttat caccctaagg 49740
attgtttgtt ttcctgtcac ccacatcaag ctgggtggtg gccgcagcct ttgatctcca 49800
cctcctaacc cctctcccaa gtaccctgtg tttgcccagc actgaatgag acagctaaag 49860
tgatccctga gagaactaaa gtgttccctc aggccgggcg tggtggctta tgcctgtaat 49920
cctaacactt tgggaggccg aggtgggcag atcacctgag gtcaggagtt caagaccaac 49980
ttggttaaca tggagaaacc ccatctttac taaaaataca aaaattagct gggtgtggtg 50040
gcaggcgcct gtaatcccag ctactccgga ggctgaggta ggagaattgc ttgaacctgg 50100
gaggcagagt ttgcagtgcg ccaagatggc accactacac tccagcctgg gtgacagagc 50160
gggactctgg ctcaaaaaaa aataaaataa agtgatccct ctaagaggaa tccccagggg 50220
cccaggccag agctttctcc aggtcaccat ggctcctccc actgtgccac cctgcctaac 50280
cccctcgatt ttaaaggaga agccaagact ctggactttg ctcagaaggt gggggggcggg 50340
ggagggaggg cttaccctgg gtacctctga ggggccaggg cgcagccacc acggtaaggg 50400
tgactgcaga tcgaggggca ggccatgttg ctggacacca ccctggctgg cgtccctctc 50460
ggcagggtgc tctttgcccc atggggtggg atccagagct gcagacaggc ccccaggctt 50520
ggccaatgaa cagaccaggt tcggggaggg tgttggaaaa gagtggatgg ggtggttccc 50580
cttaccttgc agcccccagg ccctcccccc tccctcccag gtggtcggga ctcttgatct 50640
tcgctcgtgg tactgtctgt tcggctgtct tccccgcctc tccccaggca cctgcatcct 50700
cccttggcac ctgctgccag gctaggaagg gcaaaaacaa tcccagttgg cgtagtcagg 50760
gagtctccgc cctcctccca ggtttcctcc tcccaggcgc ctcccctgga cccgcccccca 50820
tctgcccaag ataattttag tttccttggg cctggaatct ggacacacag ggctcccccc 50880
cgcctctgac ttctctgtcc gaagtcggga caccctccta ccacctgtag agaagcggga 50940
gtggatctga aataaaatcc aggaatctgg gggttcctag acggagccag acttcggaac 51000

gggtgtcctg ctactcctgc tggggctcct ccaggtaagg ggaccgagat cggtctcaga 51060
cgacagcctg ccaggcaacc cccagccgca ccagggcccc aggctgtgcc agtctccggc 51120
tgaactggcc ggcggcccag gtgggaggat tggagccacc tggctctccc cctcccccgc 51180
tcggtgatgt caggccaggg gcgggggctg gagggggaca ggtagttaag ggcctggcgt 51240
ctccctccct gaagacgtgg tcccagccgg gtgtcctgac gctcggggtt caggtaagaa 51300
cccaagcggg catttatcct ttgggggcag gcacggccgg tctggaggcc ggtagggaaa 51360
ggctgggtct cctgatgggt tgcttggcct cccacttttc tcttttctgt ccatttccca 51420
ccctgccccc tcctccccac ccgccctctt ttcgtcccca tcctggtttc cagcgctcct 51480
tgttccctcc ctgtacacct ccctgcaggc agcagctgag aatgggaagg aagacctgat 51540
tctctcctcc gacttggctt tcccgctgaa actagcccct ccccgcacac ccggggagag 51600
ccgccctcac tctctggctc gcccagttta ccccagtttg ggtttcccca agtctctaag 51660
acagacgtct ctgcgggctg cggggagatg tggggagggc cccctccact ttggagggca 51720
gtgaaggaga gggatcctct aaattgtcga ggcttcatct ctccagattg tatgcccttc 51780
tcagcaacac cgcctccggc cctccgatgg gaaagtggag gccgggtgag acggggtccc 51840
tcctggcttc agcatgaaat gggcaggaag gctggagggg tgtatgaggg gctctgcagt 51900
gcggtggggc ctggggagag tggggtgggg gatagcagat ggtttcttct gcttgatgag 51960
gctagggaga gacccaggtt cccggtctgg gtctccctgg cagattgagg acagagaatg 52020
aatgcatctg ccggcaaggt ggaacgcccc ccaccccgcc ccccaactag ccgcaccgcc 52080
ccccaggaat gccctctttt ggtccgccgg tcttggactc tgctctcccc tctcctgata 52140
tcctactgta tgcttggtgg ggggacctca tggaggcggg cagcctgtaa tgattaactg 52200
cagagacact ccagcattct tcccgaatta aactttaaaa gagctgcccc gccttctccc 52260
attgggtatt tttcctgagt gagagtgggc ggggcctcag agtcctgggt cctcccttat 52320
actaggagtc atccaggggt ttctcacctt ctcacctggg ccctgcaccc taaaaaatta 52380
taagcctctc ccgtgggggc ggggcgtgga gtactgggcg gacgggggct ctcccgccac 52440
atccaggccc ctggcccagg cctcagtccc gcaagccctt ccccctgctc tgggaagcag 52500
agttgttttc tgttggccct gaacaccggg ccccgccacc tgcccttagg gccagccagg 52560
tggggcgtga ttcaggaagt aaacaaggag gttgggagga cgggaacgcg ggtggggctg 52620

gaaccactgg gggaggaggc agctgggaag ccacggacgg agttacctcg ttgaggggac 52680
aaactgattt tgggggtcgg tgtttccaac cgaagcttta ccggggcctc agtttgaaaa 52740
ctccagatct ctaagctgcc aggcccacag ccaggacccc ttcctcctcc ctgctcaccc 52800
cattggtctt gacgattcca cccgcctcca gccttagaag cttaagggca cggctggggg 52860
gctctgaagg caaaccccag ccttggactg gccctctctg atctctgagg ccaggctcta 52920
atgtgatttg aatctacttc taaccccttc caagcactgc cctcccgaat tctctgctcc 52980
tctccccacc ccactgttgg tctgtgattt cgaggcaggc gtggccccct gcagcctgga 53040
atgaagtcac tggggctgtt tggagaccgg ggctgtttgg aggttagact ggggtgggag 53100
tgggggattg tgttctggta ttgaggtgct ggtgtctctt gctagcgggt gatgggtgtg 53160
tcgtgcccct cctggcagga ccctgagccc cctccctggc ctttcatcct gtctgtcttc 53220
ttgagtgttt tccctttcca ctcctgctcc tgtggtaccc ctctggccgg gacacccagg 53280
agaatgtgtc aggaggaact tcatccacac agggcagggg ctctgagcga tgctttgggt 53340
ggggaggggc accgccggga tgctggcttc tctccaaatg ggcctctgaa tccaggccgg 53400
gcattgcctc tctccttccc aggcgaggga ctccctggcg aggaggtggt ttgggactgg 53460
gggtgagagc agatcgtagg tcccacctgg cctggagcta ggccgagggt ggggaggagg 53520
aggtgatgtc aggagcctca ggcccacatc ctcctccccc atcctgttcc cagccccgtg 53580
cctcccccat tccgctctgg ctagcagtgg gttggggagt tgggggaagg gtggaggcag 53640
ggctgttttt gtttctccta agccaggaag ttgtgtggat gagtcaaggt tgaatggaga 53700
gccctaggca gtgttgtccc gcccgccctg actcacctgt cccccaaatc ccctgatgcc 53760
ctcactgaag ggtcaggtgc ctgctccctc ctgaggggtc aatcccaggt gccccaggtg 53820
gcctcatagt actggcccag tgtttggaga acaggctggg ggctgggatt gcaggttctc 53880
ccaggtctca gcccggccaa aggggtctgc tgggttcctg cggagaaggt gccagcccca 53940
cctccacctc tagtgctcgt ctttgcctgc tgtcctgtct ttacttgtct tgagtgtctg 54000
tgcagcccct ataagtgata agacatgggt gagattggcc ttgcctgggg gtaccaggtg 54060
ccctcagaca aatgggtgga taggacaggt gcacagtaat ggatgatgcg gcccccgcac 54120
agtgctgaga gagagtcatt cagtctgaat gtggaaggcc aggaaggctt ctcagaggag 54180
gtggctctag cactggggct tcaggaaaga gttgggcagg aggatggcct ggggagggta 54240

cgcaaggttt gggggctgct ggggccagga cccctacccc aggccctgct ctcccccttg 54300
cactgcagcg gagcccgctc tcctgccttt ctgactccca tccttcccca cctccttctc 54360
ccctctgcat cactcatcac ttgggcaggc caaacctcgc caagtatgaa caggctctgg 54420
caactgggtc tggcaacaaa gtgctggaaa aacccggggc acagcccagc gcagtgagtg 54480
ccccacacgt ggaggcaaag gagtgggcct ttgccctgac cctgagggct gagcctgaga 54540
gtctgtgggt tgtgaaatct gttgtgacca ccaggcttgt ggtgggtgga ccctcagctc 54600
ttcctgtgag caactttgtg ctaaagaaag agcactggag ccgggcgtgg tggcttacgc 54660
ctgtaatccc agcactttgg gaggccaagg cgggcggatc aggagttcaa gaccagcctg 54720
gccaacatgg tgaaaccctg tctctactaa aaatacaaaa attagacggg cgtggtggtg 54780
ggcacctgta atcccagtta ctcgggaggt taaggcacga gaattacttg aacccaggag 54840
gtggaggttg cagtgagtgg agatgacgcc gctgcaaaaa aaaaaaaaaa aaaggagaaa 54900
aaaaacagaa agagcactgg acttggagtc taaacacagg ttctactccc gactcacctg 54960
tgactgaggg caagaatgct ttcttctcag agactcggtg tccccttttg taaaatgagg 55020
ctcattctgt ccttccccaa gggcttcagg gaaactccag ttagagcagt gaggtgctag 55080
gtaaactctg aggggcaccc taaccgccgt gtgaggtcag gaggtctgaa ttctctcatc 55140
ccctctcccc aggacaaggg cacacaactg gttccgttaa gcccctctct tgctcagacg 55200
ccatggagct ggatctgtct ccacctcatc ttagcagctc tccggaagac ctttgcccag 55260
cccctgggac ccctcctggg actccccggc cccctgatac ccctctgcct gaggaggtaa 55320
agaggtccca gcctctcctc atcccaacca ccggcaggta cgatggggcg tggggcttgg 55380
gggaggtcag tgctggataa tacacagagg cttgcaggcc actgctccct tccccacacc 55440
tctctccctt tttcttcttg ccacaggaaa cttcgagagg aggagaggcg tgccacctcc 55500
ctcccctcta tccccaaccc cttccctgag ctctgcagtc ctccctcaca gagcccaatt 55560
ctcgggggcc cctccagtgc aagggggctg ctcccccgcg atgccagccg cccccatgtg 55620
agttgtccct cagaagggaa gggagggatg cacgggttct gggtctgtgg gagatacaca 55680
gccgcttcca tggaggcagg ggatcttggt taggagtccc tgagggtcta gcaggtgcgg 55740
aaagggaatg aatcaccctt tgcctcccct caagtcgtgt gcaattcctg gggcgcaggt 55800
agtaaaggtg tacagtgagg atggggcctg caggtctgtg gaggtggcag caggtgccac 55860

agctcgccac gtgtgtgaaa tgctggtgca gcgagctcac gccttgagcg acgagacctg 55920
ggggctggtg gagtgccacc cccacctagc actgggtaag tcaggtgcat ggaactatcc 55980
gggctgggag atgatgcctt gcatcttggg ctaggcatgt ggctcatcca ggagatctgg 56040
ttgatctcca ataacccctg ctttttgccc ttgtccccag agcggggttt ggaggaccac 56100
gagtccgtgg tggaagtgca ggctgcctgg cccgtgggcg gagatagccg cttcgtcttc 56160
cggaaaaact tcgccaagta cgaactgttc aagagctccc cagtgagtgc atgagggctg 56220
tctgggcgct gggatgccct gatcctcaac ctggatgctg gagccctgat ccctgacact 56280
tgtctaccca cagcactccc tgttcccaga aaaaatggtc tccagctgtc tcgatgcaca 56340
cactggtata tcccatgaag acctcatcca ggtgggggga ccccccattt cactgcagat 56400
tcacgactcc ccagcattgg ccagtgcttc tccaccctta agtcctgtgc ctcccctcta 56460
tgttgtagaa agagccaaat cacagtcctg tgtgactggg gacagtcact ttccctgcct 56520
gagcatcagt ttcttctatt aaatgggggc gagaaatgca tgtggagcat ttccttgtaa 56580
aaacctgagg gtgggctggg cacggtggct catgcctata atcccagcac tttgggaggc 56640
tgaggcggga ggattactta agcctagaag tttgagagtt tgagaccagc ctgggcaaca 56700
taatgagacc tcgtctctcc aaaaaaaaaa aaaaaaaaaa aaaaaaggcc aggaatggtg 56760
gcatgagcct gtagtcccag gtgcttggga ggctgaggtg ggaggatcac ttgagctcgg 56820
gaggtcgagg ttgcagtgag ctgtgatcgt gccaccgcac tagcatgaga ccctgtctca 56880
aaaagaaaaa gaaaaagaga aacatcctgg tggtagaggg gaagggagga ggtcagacct 56940
gtcactctcc tctgctctcc tctggctcag aacttcctga atgctggcag ctttcctgag 57000
atccagggct ttctgcagct gcggggttca ggacggaagc tttggaaacg ctttttctgc 57060
ttcttgcgcc gatctggcct ctattactcc accaagggca cctctaaggt aaggtcttga 57120
gggtaccagc cccagcccct ccagtccctg gtccttttag aagttgcccc ttctctgctg 57180
gaacctctga gcccttctcc ccctgggccc cccaggccag ccacctccag tttaccatct 57240
ctccctacat ccttgcctag ctcacctgcc cagggaggta gcaggagaaa agatgatctt 57300
agtttaagtc ctggctctac ttctatttgc tgtgtgaccc tgggtattcc cctgcccctc 57360
tctggtcctg aaatctcccc acctggcttg tggggggagg taatagtggg cgggatctac 57420
ctgtaccaag tcctgctcac tcatgctgct taggatccga ggcacctgca gtacgtggca 57480

gatgtgaacg agtccaacgt gtacgtggtg acgcagggcc gcaagctcta cgggatgccc 57540
actgacttcg gtttctgtgt caaggtgaag acctggccag gcctggcccc tggcctgggg 57600
aagcaggaac tgctcaggcc cctggatcct gcccggggct tctgagcccc aattcagaca 57660
cctagactcc tctccctgca ccctggcctg ctggaaactc ctggattcag ctctgctatg 57720
tggagcaggg gcagacatgt ggtcctaaag gcagatatgg gaccagtcaa tttcctctct 57780
ctgcagccca acaagcttcg aaatggccac aaggggcttc ggatcttctg cagtgaagat 57840
gagcagagcc gcacctgctg gctggctgcc ttccgcctct tcaaggtgag accctgggag 57900
tggcatgggg ggctggcctg gccagaggga tccccagctc tgccctcagg aagtctcagg 57960
aatgaggagg gcatcacagc cttgctctct gataaccccc agtccaagcc tgaagttata 58020
ggaagtgccc atcgaaggca gaaacacagc cctggtctgg gcaagtcctg gtctgagggg 58080
ggcgtcacag ccacgccccc aggacctctc gacctcaagc tctctttctc tccccacccc 58140
cagtacgggg tgcagctgta caagaattac cagcaggcac agtctcgcca tctgcatcca 58200
tcttgtttgg gctccccacc cttggtgagt gtgcccaagg ggatgggagg gtgggtatgc 58260
aggccctgtc ttacgggtac ctgggccctg ttctgacctc tctcctctcc ttccatctcc 58320
agagaagtgc ctcagataat accctggtgg ccatggactt ctctggccat gctgggcgtg 58380
tcattgagaa cccccgggag gctctgagtg tggccctgga ggaggcccag gcctggaggg 58440
tgaggcctgc tgtgtgtgtg tgtgtttgtg ctggggaccc actctctggg tgggatccct 58500
gaaataggag ggaggaagag agggcggggg gaggcccctg gctgggaaga agtgctctac 58560
cttcctgagg tgctgggtaa tgcccccaag cacgccccga ctctcccgta tctcccactg 58620
ctccacagaa gaagacaaac caccgcctca gcctgcccat gccagcctcc ggcacgagcc 58680
tcagtgcagg tgggtgacgg ccccgagtcc tggggcgggg gctgcctcaa cttctctttg 58740
tattcccagt ggggagtaga tggtataggg gtcccctccc caaagtgacc gcccatgtcc 58800
ttccccacca cagccatcca ccgcacccaa ctctggttcc acgggcgcat ttcccgtgag 58860
gagagccagc ggcttattgg acagcagggc ttggtagacg ggtaaggggc agggccgggc 58920
aacagaccca gggataagag agactggggt ccaggtggca gccatggtcc ttgggtagta 58980
atgctgcccc atctcctgtc ttctggcagc ctgttcctgg tccgggagag tcagcggaac 59040
ccccagggct ttgtcctctc tttgtgccac ctgcagaaag tgaagcatta tctcatcctg 59100

ccggtgagct tccctgcgtc cccggagtcc tgcaatgaga cacaggactc ccagcaacct 59160
gtcctcctca ccaggcccct ccagaggctc cctggccccc agtgcgtctc ccttttccct 59220
gcacaagaag tgggaggctg agtgcggtgg ctcacacctg taatcccagc acttaggacg 59280
gccaaggtgg gagaatggct tgagcccagg agttcgagac cagcctgggc aacacaggga 59340
gaccccatct ctacaaataa tttaaaaatg agccaggcat ggtggtgcac acctgtagtc 59400
cagctactca ggaggctgag gtgggagcat tgcttgagcc cagagggtca aggctgcagt 59460
gagccatggt ggcaccacca cactccagcc tggatgacac agtgagaaaa ctgtctcaaa 59520
aaaaaaaaaa gaaagaaaaa gaaaaagaaa aaagaaaaga ataaaaggaa atggtggggc 59580
cctggcccag gagggagctt cccaagcctc gggcccctcc ctgaacttcc acccccttta 59640
ctgtacccca gagcgaggag gagggccgcc tgtacttcag catggatgat ggccagaccc 59700
gcttcactga cctgctgcag ctcgtggagt tccaccagct gaaccgcggc atcctgccgt 59760
gcttgctgcg ccattgctgc acgcgggtgg ccctctgacc aggccgtgga ctggctcatg 59820
cctcagcccg ccttcaggct gcccgccgcc cctccaccca tccagtggac tctggggcgc 59880
ggccacaggg gacgggatga ggagcgggag ggttccgcca ctccagtttt ctcctctgct 59940
tctttgcctc cctcagatag aaaacagccc ccactccagt ccactcctga cccctctcct 60000
caagggaagg ccttgggtgg ccccctctcc ttctcctagc tctggaggtg ctgctctagg 60060
gcagggaatt atgggagaag tgggggcagc ccaggcggtt tcacgcccca cactttgtac 60120
agaccgagag gccagttgat ctgctctgtt ttatactagt gacaataaag attatttttt 60180
gatacaccta tgagttctgt ctggcaaggc ctggctggct gaatcaagaa gggaaccaga 60240
gctggacgtg gtggctcatg cctgtaatcc cagcactttg ggaggccaag gtaggagaat 60300
tgcttgagtc caggagtttg agaccagcct gggcaacatg gcaagaccct gtccctacaa 60360
aaaataaaaa aatgagccgg gcatggtggt gtgcacctgt agtcccagct tctcaggagg 60420
ctgaggtggg aggatccctt gttccttgag cctgggatgt caaggttgca gtgaactgag 60480
attgtgccgc tgcactcagc ctgagtgaca gagtgagacc ctgtctggaa aaaaaaagag 60540
gggagacctg gagaggtggg cacctgtgga ggccttggtg gaagtgtcaa atggatcgag 60600
gccatgtctc agcctgcctg gatgttcctc aagggcagga gtggttatct gagagtctcc 60660
agtgcccacc atgcagcttg acacatagta ggcgcccagt cattgctaat taagtaagtg 60720

aatagacaag agaccatcat cccagagaga ttttctgaca gtctaagtct agagaggtaa 60780
ttaacagggc ctgggagttg gagatgagtc cgacagcatg cttgtcctcc gctagtcctg 60840
gactagctga cggatagttg gccccagcat gcacacagtt atgcatccag ccccaccacc 60900
aagacacaga atggcctcat cacccccaac aagtccctgt gccccctact atcagccatg 60960
ctccctccta cccaaactga aacctccttt ctgtcctagt tctgcccctt ccagaaagcc 61020
atataaatgg agcctgctag cattcagccc tctgcatctg gcttccatct ccaacacatc 61080
cttcagcacc cagagtgatc ttttctcaaa tataaattca ccactcccgg cagggcactg 61140
tggctcacac ctgtaatccc agcactttgg gaggccgagg cgggcggatc acgaggtcag 61200
gagtttgaga ccagcctggc caacatggca aaaccccatc tctactaaaa atacaaaaat 61260
tagctgggca tggtggcggg cacctgtaat cccagctact agggaggctg aggcaggaga 61320
atagcttgaa ccggggaggc agaggttgca gtgagccaag atcatgccat tgcactccag 61380
cctgggcaac aagagcaaga ctcagtctta aaaaaaaaga cagatgtctg gcctctgccc 61440
actgctcatg ccagtctatg tttttgtctt agaggttttt ttgttttgtt ttttgagatg 61500
gagtctcact ctgttgcgca agctggaatg cagtggcacg atctcagctc actacaacct 61560
ccttctcctg ggttcaagcg attctcttgc ctcatcctcc caagtaactg ggattacagg 61620
cgcttgccac cacgcctggc taattttttg tatttgtggt agagacgggg tttcaccatg 61680
ttggccagcc tggccttgaa ctcctgacct caagtgatcc actcaccttg gcctcccaaa 61740
gtgctgggat tacacaccca agccactgcg cctggccttg tctaagagtt ttttagtttg 61800
agctcttacc tgatgggagc tctttaataa atatgtcaga tcgactgcat ctgaacttgc 61860
tgcttaactc aggagacaga ccctcctgca gacctgtgaa ccccaactag tcattgtttt 61920
tgtcaaaaca ccaaacctga atctgattcc ttggattcaa caactagttt acgggaaata 61980
caagggaaac aatgacaaaa atccagattg ggaaattcta cagaacagat aacccagttt 62040
cttcttcaac agcaacaaaa ttaccagaaa agatgggcaa tctctagatt tcaagaaatt 62100
tagacatata tcaaccaaat gatgtgttga cctttgattc aaatgcaaac taactgtaaa 62160
aagcatttat gagcaattgg gggaaatttg aattctgatc agatattcga tgatattaag 62220
ggattatggc taactttcta aggtgtaata atggtattgc agttacggtt ttttctaaat 62280
ctttatcttt aagaaattca ttatgaagta tttaaaggtg aaatcacatg atgtctcagc 62340

tttgtttta ttccttttct ttttttttt tccaactttt attttagatt cagggagtac 62400
atgtgcaggt ttattacctg ggtatattga gtaatgctga agctttgttt ttaaaataac 62460
ccagaggtgg ccaggcacag tgggtcatgc ctgtaatccc agtactttgg gagctgaggt 62520
gggaggagtt tgagaccagc ctgggcaaca tagtgaggcc ctctctacaa aaataaaaaa 62580
taaaaaatta gccaggtgtg gtgacatgca tctgtggtcc cagctacctg ggaggctggg 62640
gtgaaaggat cgtttgagcc caggaagtca aggctggtgt gagccatgat tgcatctctg 62700
cactccagcc tgggccacag aacatgacct tgtctcaaaa aataaaaaga aaaaaatgaa 62760
atccagaggt taaaggagag ggtgtgggca tagtcatggt gtggccatga gttgacactg 62820
gctgttgata ttggttgaag cttggtgaca ggtactggct atgtggggat ttgttaaatt 62880
atcctctcca cctttgtata tgcttggata ttctattata tattgatata ttttaatgga 62940
gagcccctc tcaaatcttt gggactgact gggcaggagg tggtgagacg ctgttttgta 63000
ggtgaggaaa ctgacggccc agtgggcagc ctggccccag atcacagtgg gagagccaag 63060
attccaaccc aggtctgagg accaggtctg ggcatgttct gcttcccctg tctggctgac 63120
gtccaggtgg cattcctcag ccacagtgac agcaatagtg gcctactggg gtcctccctt 63180
agagagcccc tcccttccct gcagccttgg tctctggcct ggagggttgt cctgaggtga 63240
ccttgagact gtgcagcctc tgcaatcagc ccctgggctg ggtctggcag ccctggcagc 63300
cactcccgac caaggcaccc ccatccttgg ctaactccag gctgggaagc tgggaccagg 63360
tccagcagac agaggtgggc agagaggtct catgcacacc ccagcctggg acctcctggg 63420
aatctgctgg aagaacttac tgcagcccaa gggcaggaag ggtggcagat tttgcttctt 63480
tttagcagga ctggaggcag gacccagtgc aacctgctgc ctgccacctg ccacggttcc 63540
cagagccatc cccaccacag gctgtggctc gagcaccgaa cagggcagtg tcagctgtgt 63600
ggctggccat gggtgcccag ggccacctcc gaggtgtggg tgccagagtt ccaggctgga 63660
gtgagagtaa aggggtcaag atgagtcacc tcttcagcat gtccaggggt tcagactgta 63720
aaaaaagagg acccagtgtc ccttcacagc ctgaggcact gggcagagac aaagcctctc 63780
tgtcaatcag ctaattggag actgtgagac agatgtcctt cccactttac aaatgggaaa 63840
ctgaggctca gggaaagaca gggactcacc tgcgacaggg gtcgcagagg gagttcggga 63900
cagaggccag actcagccca ctggacaggt ggtgaaatgc agccctgccc tgctctagcc 63960

tttgttctct gccccctccc ttctcagtgc tggccacagt gagggaccta ggctgccgag 64020
agcccttaga gggggcagca tttggccctg atctggccac ctgagaccag cagcagccca 64080
ttcttggtga gtgtctgaga tttctggctt tttggagacg tagctgctaa gagtttgggg 64140
gcacagggac cctctcaggg ccagctcaca gtggaacccc tgggtaagat cccccagctc 64200
tgaggatgtg ggaaggatgc tgctctacct cagagactgc agcatgacct acagtggtca 64260
gacagcagaa aggactgcca aggacggctg cagtgctaga tcggggaggc ttgtggttct 64320
gagaaaatct gggctggtgg gaggggaagc aggggcttct ctgggggaga gggtgggggc 64380
tgggacctca gatactgtct tttcaaaatt gatttattgg gtttcattct aaaattatac 64440
gtacttatta aaaacatttc agaaaaaaac ttgagggcaa aggggaaatt cctcatcatc 64500
tttccatgca gaggaggacc cagtactgag agagggtgac agggcccaca tctggggagc 64560
tgacgcgaag tgctgcctgc catgcatcac acgttccagc ttcccgggac cagcgtggcc 64620
ggtgggagcc gggtgccagg gcacattgct atccttggca gcaccatggg cctttagggt 64680
aggggttctg tggagggtgt ggctacaatg ttggcctccc tgcctgctca agtgtggagg 64740
ggagggagga ctgctgccaa cgctctggac tctgggatgg gggtgggcat gattccaggg 64800
gagcccacct ccctgggcgg ggcatccaga caaagccctg gccagttctg caacatccaa 64860
gttcacgggt ggtggacagg cccaagggtt caacatcagg tcagggaccc tgcctggctc 64920
cttccttggg ccccagtgcc aggcaccctg atgagggatg aagagaggaa ggacaggaca 64980
cagacagggg agcccaaagc cacttggaag gattctttct tccaaactat ccacataagc 65040
agttttctaa ctccttccca gagcccaagg gttctggcag gggcttgagg ggcaggttgg 65100
ggtggtgatg ggggatgcat tgcctttcta tctggaactt gagccttatg gtatattcag 65160
gaaaaaggct tgaaccagtg aagtactgat ctggtccaac agatagaaag agggcccaga 65220
gaggtcaggg cttggtccaa ctcgcacagt ggtggggcgt ctgcagactg ctttcctgtc 65280
ctgtgctgtt ggcagaacct cagctttggg acttgagggg gagtatgcag tggcaattag 65340
gacaaacgac tctgagctct cagttgagtt gggggaggag agaaaagaag gagttagatc 65400
ctgggggagt tggatgttgc agactggact ccagagcctg ggggtgtggg cgatcctggg 65460
gtgtggggag ggcgggctga ggctgctcca caagcgttct cgaagttcac catacaagaa 65520
ggggcggtgt ggggaaatgg ggatgaggcc tgaaattcaa tcccatgtgg gttgagttat 65580

gatgtcatct gacaccctct cacacggctg catctacccc aaggaagggg caagtttttc 65640
tttgatgggc acctgggggg gctgtttagc tggagttttt tttgtttcgc tttttttgtt 65700
tttgtgtttt ttttgagaca agtttcgctc tgtggcccag gctggagtgc aatggtgtga 65760
tctcggttta ccgcaacctc cacctcctgg gttcaagcaa ttctcctgcc tcagcctccc 65820
aagtagctgg gattacagac atgcaccacc acgcccagca attttgtatt tttagtagag 65880
atggggttta tccacgttgg tgaagctcgt ctcgaactcc cgacttcatg tgatccgccc 65940
gcctcagcct cccaaagtgc tgggattaca ggtgtgagcc accgcgccgg gcctggagct 66000
gcatttttgt tgtaagcact ttgttcttac ttggattagg tttattttag ggtggtttgg 66060
ggcagactga gggagattgg aaggcagaaa ccaccccctc aggacaacca cgtggatctc 66120
cttagggtct gggtagtgga actgggccct gtgaccttgg agttggaggg gaagctgtaa 66180
gcagggatgg aacgggccac ccgcaccctc acacagctgt gaccaacagg cacggtcatc 66240
cgcattcacc tgcccaactc ccatgccctg agtcctgtcc tccagggcat tcctactggc 66300
actaccccaa tttcccaaaa gattccaggg atctctctct cgctctctct tttttttttt 66360
ttacagtctc actctgttgc ccaggctgga gtgcagtggc tgatctcagc tcactgcaac 66420
ctccaccccc tgggttcaag cgattttcct gcctcagcct cttgagtagt ttgggattac 66480
aggcgtgcgc caccacgccc agctaatttt tgtattttta gtagacgggg ttttgccatg 66540
ttggccagac tgatctcaaa ctcctgacct caagtgatcc tcccgctttg gcctcccaag 66600
ggagggttcc ttcctcccag gctgaggcac cctgccccac acatctactg gaggaactta 66660
gtgttggggc cagggggtgg ctgggtaagg ctggatccca cacttgtcag gccaggttgg 66720
ggtaggggag gatacacggg ggacccgtct ctcagccgtt ctgggccggt gcctgaggcg 66780
atctgccccg ccgcccgccc ttccgccacc acccaagtcg catgcgcgcg ccggggccga 66840
cgggcgccag gctccaccga gccaccctcc gtcgtggcgg cccctcgtgc ctctgcggag 66900
ggaaggccgc cggcttcgcc agggggcgcg cgtcggggtc gcggctcttt gaactcggcc 66960
ccacgagggg acgcgccatc ggtttcgtgg cttgcgtggc gccgcagcaa cgagccggtc 67020
gccccggggc gttgcttagt ccgggcctcg ctgcgagtct gtgggcgagg cgggaggtgc 67080
ttccttggcc aggcgggtgg aggaaggtgt gggcgccgca tcccagcgtc gcggagcctc 67140
caggcgcggg caggcgagac tggcgtgcag cgcgacggcc tagtcccggc gtcccaccgt 67200

cccccactcc cgcaggcgcc gagccgattc agccgcgcgg ctgcagtgac acccagcggc 67260
cgtcgcggga agtgcggtcg ccgcggccag gcctccgggg ctcctgtctc tcgaggttcc 67320
cacccccggtt acgcgcgggc accccggagg agagcggccg cgcgctgcag atgcccgcgg 67380
gggcacttcc cctgcccctc ccccgtttcc tccccgcagg cgcccgagcg ccgggagatc 67440
ctgcgcagcg gcagtgcccg gaagggcggg gcggggtgca gcagcggcgc tgggctgtga 67500
cccgggcagt ttcacttctg gatcctgctc gggctgtgat aggacagcgc gggggcagcg 67560
ggggaacacg cgggcacgcc gagatccggc gctcgggacc ccgatgaggg gaggcctttc 67620
actcctgggg tgtcacgcga cccacgcttt cctccatgtc cactcccacc ccccatgggt 67680
tccactttgc tgtcccccga gctgccgctc ctccccagca gacaccgtct ccgccggggc 67740
tgccgacccc cggagaggcc ccgagcggcg tgggcggcgg gaggagggag ctgtgaagcc 67800
gcaggcaggg ggttaggctg cgggctgctg agacgccgag ctgtttctca gaagtgcagc 67860
tgcccctccc ctcacacccc cctcactccc accgcgcgcc gcacccctgc cccctctccg 67920
ccggcctccc tttcctctcc caggcaccct agccgggaat ctggggtctg ggtccacgca 67980
gaattaagag gagaacctga gaaagggcgg gggccggggg ttcagaccca gcctcagcgc 68040
gcgttattcc gagcctccta taattccaca ggtgagggga tcctgtcccc taccccctggg 68100
gtccaggatt ggaatttgat agaacagtta ggaaaatgac atcttgagag agtcagggcc 68160
cggccactat tactctagct ccagcgtcca gtgaggagag gagagagagg gaagacctgg 68220
agcccggctg gtgcagtgag gttgaggcct gtcttaggag taccagctgg aaggcccttc 68280
agagatccct gactctgggg aaaatcaacg cccagaaggg gaagtcaagt gtctgggctc 68340
atcccgagag ggagagggct gggattggaa ccaggactcc aaatagttgc gggagtctgc 68400
gactgtcttt tgcttgggac ttcagagctt tgctggattc actgactcag cctgctttcc 68460
ctcaacacac acaaacacac acacacgaga cacgtgcacg cgcgcacaca cacagataca 68520
cagacacaca cagacagaca tgcaaggaga gagacaggca cacatggaga cagacacgca 68580
cacgcaaaga gacacacagc tccccaggag tctagcgggg cagctgaggt ctgtgagtga 68640
gacattctca gtgagacact gagaatgcaa ttccagtggg ggtgggatgg ggattggcgg 68700
gtggaaggac aagcccgggg gaggaggtgg catcgccccc ctagttccca tccaccacca 68760
cccccagtgc tgtggtttat ttgcctaggc agctcttctg ggccaggggg ccagggtact 68820

ggggtggaga ggcgaggtga tgggaaaggc tgttctgggg cttcctgccc cctcactcct 68880
ggggaagggg ggtagctgcc atcacccgca ggcctttcca ctgcagccct ttccttgggt 68940
atgtgtgtta ttctggggga agatctgtta tttgcggaga agggaggcag ctgcctactt 69000
cacaggtcaa gacagagtta aaaacaaaac cacagcaaat tcaacacccc gtgtccttca 69060
gggactttcc atgacacctt ccttcccccc tctcccctaa gccagggagc acctccccag 69120
agggcctccc caccccaggt gcagtggctt ttggctttta tgcaaacaac tgtctgatcc 69180
cggaatgctc cacagcccga actgtggcct gggccgaatg tgcccctcca ccatcccttg 69240
ggccagtgtt tctggtcttc ctggagttgt gacctccact cttcctcaga ccctggattc 69300
tgagggacag gagatgaggg caggaagcta ggtcctttcc ccctgcctgc cctcgggtgg 69360
cagttgcctg caggtggagg gtttgggcac agcaacctaa gtgaatggtc cggggttttg 69420
agtgatgggg gaggggaaga aggagggtgc aggtgtgggg tggcctccac tgccaggccc 69480
accaccctct agggcattga attagctccc aagatcagga aggaaaactc ttctcaggct 69540
gggagtccat tcaaccccaa cgccagtgtg gacctccggt cctgctcact ggcaaagggg 69600
gtcaggatct ccctgtctcc ctctcttctg cctgagctgc ttctgctttg gggagggatt 69660
tggggatggg gaaggccata taatcttctc tccagggctg aaaaccagct actgaggggc 69720
cgtttttgaa tgtattcagt gctaaaatgc aaaggcaggg tgccatgcca ttcctttctc 69780
ctcctcagct tgagcaagac tgggaaacct gtaggagacc ctcagccagt ttgaacattt 69840
tggagtgctg atgggaggga gatgatgctg atcatggagc cagaggacgc tggtgccagc 69900
ctagccccac atttgttagt cttaaaactt cagacaatct ttctcatgcc tcttccacag 69960
aagacggaaa atggagggtg ctttagatga ggctgagcag aaggccagac tctctctcta 70020
ctttctctaa cgttttctaa gagagaacac aatgatgatg gaaagaaagc agcctcatcc 70080
ctatgccatt ctcctaggac ttttccccct aacaccttcg gtttgggtct ccaggtatct 70140
aaaaactgta tttaaatatg gtcgagttta aggactgcaa ctcaagtttc ccaatgtgga 70200
tgtctgtgag cccccacaca tgaaaaagtc agaacttgac tgtattttaa gaagaatctc 70260
tgttgaggga ggaaggcaga gcagtgactt ggaatagggg tgcaccatgc tgaatgtgct 70320
gatcatgcta agtccaggtc ctcaacctcc tccagtttcc ttagtgaact aaggaccagt 70380
ggaaccagga accctatctt gtgggttaca ggggccagca gcaggatcac agtggtaggc 70440

tcagcaaggc gtcaggtgag aacctttatt tcagtgataa ttcatttttc ttgataactg 70500
gactttaaga aatagagggg caaaattaag aaagtattcc ttggggcggg atgaaaccct 70560
tctacatcct gacaccagca gacacaaatc ataagtgata aggatgcaca aggactaatt 70620
tcaaacatac caggattttt ttatttttca gtgtaaaaat caaacatgat aaacctagaa 70680
aactatcagc agggctattt cttacagggt tccagcaagg gttagcattg ttacatttca 70740
gaagctgact ttctttaaac catctttaca gaggagtaaa cttcacagtt ccacacatgg 70800
ctgggctcca ggtagaactc cataggagtg acgagggaaa gtcaccactg ggcagggagg 70860
gcatttctga gaaatgcaga tgagaggaga ggctgtgagc acaggctgtg tcaaaaccca 70920
gggcaagggc tccctcccgc cttccctccc agtgggaagg ccaccctgag ccccaaacca 70980
cattctgttc cttcctcgtc attctgcaga caatggtcat ccacagacca cacgtgtggt 71040
ggctttggca accagaaatt taaaataagc tatggttttt ccagtagcca aaatgatcct 71100
gcaccaaagc tcatagactg agaacctgag catgcaaaac cacagtctgg gtgaagggat 71160
gtctgctttg taaatgacct gctaattctt tgcaacccac agtaatttgg tttctgtgaa 71220
cccacagaag caggcccacc aaaaagggcc ttgtctgcta gcctggagta tacatgagtc 71280
actggcggtg ggatcagtca ttttttaggc tgccccattt tcctaacatg ttaaaatgtg 71340
tgttctcagt cttttcaaga gaggaagaag caaagcggca cttacagagt gtgagataga 71400
cacagatctg tggcgaggga ttggggaagg tgggtggcat cttgggactc tctccaggct 71460
ttctggagtg gggtcagtgg agagatgaac agtgagaaac atttgaaata actcaagttt 71520
tggagcagcg gggggtgggg tactaatggc tgaggactca cagacagtga gggagcaaag 71580
cgctttgact tccctgggtg cgacaccatg aagtgtctat agcacgtggt tagctcacgc 71640
tggttccaag ggtcagcaga tcctctgccc ttgtgcatta aaacaacatg aaggaaactg 71700
ttagagatca gttacctcca taattcattt gtaaaggaat tgctattatg atctagtatg 71760
atcttgggat atttttcatg tctttaaatt taggacacgg tacattttcc actgaaaatt 71820
tacaatcatc caacataatg cacaccaccc ctcaaaggta acattagctc atatacaaaa 71880
tcatggaaaa cctcacaaca tccttggaag gtagatattg ttatatcctt acaaaaattt 71940
aatacaccca ttaacattcc tatttcaaat agctcaatcg atatcaacac ataagacagc 72000
ccacaattgt gatacagtat taagaagaaa ctcaggccag ccgcggtgcc tcacgcctgt 72060

aaccccaaca ctttgggagg ctgaggtggg cagattactt gagcccagga gttcaggacc 72120
agcctgggca acatggtgag accctgtctc tacaaaaagt acaaaaatta gctgggtgtg 72180
gtgacccacg cctgtagtct cagctagttg ggaggctaag acgggaggat ggctcgttgc 72240
ttgagcccag gaggttgagg cggcaagtaa gccttgattg tgccactgca ctcagcctgg 72300
ggaaacagag cgagactttg tctcaaaaag aaaaaaaaaa agaggaaact tgtatccaat 72360
ttattcaaac tgcaaaaccc tgtgtatctt cataacaatg gtggggcaaa gactatctac 72420
agctggggca agggagaggc acagtcttgt gttactttac aagctctcag aagtttaaag 72480
cccacaattg tccttcaggc tcttctgagt ccatggtcct aaagggtcag gagtcctgaa 72540
ccctggctta ttctcagaac cattcagtcg tgctgccttg gagaacagag tccttttcct 72600
gaggtggccc aggtccagct gctggtttca gatcaaaatt gacctccttc atcggcactt 72660
atatctcaaa tcattctcct tgtctgaact ctggatatag aggtttttttg aactatactt 72720
tctgtgtgaa cacaaaaata gcttcccatt ctctaaaaga tgaggtatca ctatatatcg 72780
ggtttttttc ttctgttcct ggtgctaact tttaggacaa agtttcacca ctccctttc 72840
tcttcttgag ctcacaagtc ccgatgctga ctctttgagt cctcagatag ccaggctctt 72900
tcacatcttg agtgtcccag agtttccaaa tctctgctag agtccaacat acctcttctc 72960
agtggggtac tctttaaagt gaggacttaa acatttccat tgtggtctta tcacctggga 73020
ttatatgtaa cagtactatt tttttcctta aaacattctt ttttttttt tttttgagat 73080
ggagtctcac tctgttgtcc aggctggatt gcaatggcgc gatctcggct cactgcaacc 73140
tccacctccc aggttcaagc aattctcctg cgtcagcctc ccgagtagct gggattacag 73200
gtgtgcacca ccacacccag ctaatttttg tatttttttt ttcgagatgg agtctcactc 73260
tgtcacccag gctggagtgc aatggcacga tctcggctca ccacaacctc ctcctcctgg 73320
gttcaagcga ttctcctgcc tcagcctcct aagtagctgg gattacaggc atgtgccacc 73380
acgcctggct aattttgtat ttttagtaga gacagggttt ctccatgttg gtcaggctgg 73440
tctcgaactc ccaaactcag gtgatccgcc cgccttggcc tcccaaagtg ctgggattac 73500
aggcgtgagc caccgcacct gaccctcctt aaaacattct aaacctcaca ttcagtaggg 73560
cttggggagg tgggggtggt acaggaaata ggagaataat agtgatacag atgctacctg 73620
gaaaaacttt gttctgtctg ttcctcacga aacaaagccg aaaagaaaaa ctttatgtgg 73680

atgtgagatt tgatagtaac acagtgtttc acctgagcaa aattttgtgt ttttaaacat 73740
ttctgtttgc caaagaaatc tgaggttgtg gctgggcgca gtggctcttg cctgtagttc 73800
cagcactttg ggaggctgag gtgggcagat tgcttgagcc caggattttg agactagcct 73860
gggcagtatg gcgaaacgcc atctctgcag aaaaatacaa aaaaaattag ctgtgcatgg 73920
tggtgtgcct atagtcccag ctacttggga ggctgaggta ggaggattgt ttgagcctag 73980
gagatcaaaa tctgaggtca ttttatatct ggccgatttt gagtatgggc ctaacaaaat 74040
gttaagcact gcatttaaaa ggtgagaaat tttgtcactg acaaagtcag aagctgtgtc 74100
aggaacagct cacagtgggt cataaagcat aagattagct ggcttatctg tgccaaggcg 74160
aatgtctttt tgggagaata acttgaggaa actaaagact gatgagttca ttttctctct 74220
ttttttttc tggtggtagt ggtgggggct cacgggagag acacttctat ttagttttct 74280
ttccgtagga agtaccgatg caacacttca ggtagtgaca gaaaacattt ggagagctgg 74340
gcattgtttg tgcatgcctg taatcccagc tacttgggag gctgaggcag gattgcttga 74400
gcacaggagt ttgagtctgg cctaggcaac atagtgagac cctggtcctc taaaatatac 74460
atacatatat atacatatac aaaaaaaata aaaataaatt aaaaaattaa aaaacacctg 74520
gagcactggt tctgtttctt ttccaccctc agcccagttc tgtcaccttg gctgcaagga 74580
aactttgcct gggttttttt gtttgtttgt ttgtttttgt tttagacaga gtttcgctct 74640
tgttgccgag gctggagtgc agtggtgcca tctcggctca ccgcaacctc cgcctcccgg 74700
gttcaagcga ttctcctgcc tcagcctcct gagtagctgg gattacaggc atgtgccatc 74760
acgcccggct aattttgtat ttttagtaga gatgggggtt tctccatgtt ggtcaggcag 74820
gtctcgaact cccaacctca ggtgatctgc ctgccccagc ctcccaaagt gctggcatta 74880
caggcgtgag ctatcgcgcc cggccctttg catgggtttg gttgttcctc agaccgtggc 74940
ctcctgggtg tatggtggaa agcccgcctg ataagaggct ggtgggaaat gaaggagagg 75000
ggcctacaag gctcttgttc agagggagtc tgcatgagaa agtacaggta ctatctgaag 75060
ctgctccagg gcttcagtgc ccagcagtgg cagatgcaag tcctgactct gcccttctgc 75120
taagattctc cttcaccctc acctccccag ccctggcaag gggtttgtta attagtacag 75180
cataaggggt atgtacagaa ggcagcgttt atctgtgtga tggtaaccac tgcatatctg 75240
gttcaacttc aagacttaag gatgcagagt ttccacacat aaattttttc tggaattgat 75300

gggccccaat ctaggctcag aatgccacaa agaaagtgcc tttgtgagtg gtcaccttac 75360
tcagaaaact aacctcagaa gaatagaaat catgcagcaa attcattagt cacaggaaaa 75420
aggaggatca tatctataaa aagagtcccc aggctggtgg aatgggcctt ggtttcacaa 75480
tcttgagatg caaaaggtgg tctcaggctg ggtgcggtag ctcatgcctg taatcccagc 75540
actgtgggag gccaaggcgg gcagatcacc tgaggtcagg agtttgagac tggcctggcc 75600
aacatggtga aaccttgtct ctactaaaaa tacaaaaatt agccgggctt ggtggtgagc 75660
acctgtaatc ccagctactt gggaggctga ggcaggagaa ttgcttgaac ccgggaggcg 75720
gaggttgccg tgagccgaga tcgcgccact gcactccagc ctgggcaaca gagcgagaga 75780
ctgtctcaaa acaaaagaaa acaaaaaaag ttggtctcct ctgcaaagac ttgaacacta 75840
atctggtgga aaggcttcct ttccctcgtt ttctcttcta ctaatccctg cttccaaatt 75900
ctctttctct tgtcccttga agaaccaaca ggatgtgcga tatttcaaga acgtaatgag 75960
caactcctct ttcacaaagg gaataagcaa tctggcatag cttggagtct agtattacgg 76020
aaatacagtg atatactgaa gactacacac caaaatacat ccattccttt agctgaagtc 76080
tcaaacatgt tgggactgtt gccatggaat ttcataaaac ctttttttcct gagacagacg 76140
ccatgccaca aagaaggata agccatttgt gcagtctgag gtggaacatt ttaattactg 76200
gcagcacata gacgagttga gttggagccg aatgcataag ccccttagaa ataaaacact 76260
aatctaccag gaatttggga ttcttggctc tggtagaaat gttacaaatt aagctgactc 76320
ttaatttgta aataggctcc atgaagttgt ccctggaagc agaatttatt ttccctaaca 76380
taatttctta aaaagtaaaa aaaaaaagaa ggctttggca aacagctgca tgtgccaatt 76440
tgtgtgtatg ctttgtggca agatcaaaat acctttctgt tttttgttcc tctccaccac 76500
cttcttcaca ttcacaactc agtggttaaa ggaaaaactt aacacaccat tttcatacca 76560
aaggaatgtg aaacatgtat tcatataaat actcttctgc ttaatatcca tacattagga 76620
ggtttaaaaa ataccttgtg gttactgcat aatcacatat gaattaaagc tagaagggac 76680
ctaagagaac attgaaatct aattcctatt ttatggatga ggaaactgag ttttttttgt 76740
ttttgtttgt ttgtttttgt tttgttttgt ttgagacagg gtctcactct gtcgcccaga 76800
ctggagtgca gtagtgtgat ctccactcac cgcaacctca acctcccagg cttaagcgat 76860
ttttctgcct cagcctccgg agtagctggg attacaggca tgtgccacta ctgcccagct 76920

aatttttgta tttttagtag agacagggtt tcaccatgtg ggtcaggctg gtctcgaact 76980
cctgacctca aatgatgcgc ctgcctcggc ttcccaaagt gttgggatta caggtgtgag 77040
ccaccgagcc tggccggaaa ctgagtttta agagttaaat aaggctgggt gtggtggctc 77100
acacctgtaa tcccagcact ttaggaggct gaggcgggct gattgcttga gcccaggagt 77160
tcaagaccag catgggcaac atagtgagac ccctatctct acaaaaagaa caaaatgtag 77220
ccagacatgg tggtgtgcac ttgttgtccc agtactcaag aggctgaggt gggaggatca 77280
cctgagcctg gggaggtcga ggctgcactg agccatgatt gcaccactgc actccagcct 77340
gggcaacaga gtgagatcct gtctcaaaaa aaaaaaaaaa aaaaaagtta aacgatttct 77400
caaagtcaca tagctaatta gccagcattt tgtgacctcc tattccagtg ctaattttac 77460
catgataaca gcactcctcc tgatagtatg tacatattat gagtgacaag gatgacttat 77520
acatctgcag aaccgtgatc agactttcag gaattttcat ggaatacagc agttccattt 77580
aaatgggaaa aaaagggccg aagcctgcct gatcaaggag aacctctcaa tagaggtgtc 77640
cctcctcacc ccattccgga gctcaactcc gatctaaaca cacatgccat ctatctccaa 77700
ttcctctcct gtggggaggg ggaggctgag ccatactttt tggacttttt ttgtggaaaa 77760
tatcattggc aaatttgctt tgggtctgta tcaccaattc tccatgaact ctttcaagaa 77820
gttttaggta gctgtttttt gtttgtttgt tttctgttgg tgctgccaaa caatttcccc 77880
ctttataccc ttgctctgcc aaccttgacc aaaggaaagc gatgtcatca tccacagtgg 77940
ataaatttct ggagtttcga tgatggtcta tatttaaaac catgagttct tctggcatag 78000
attttcttct atagacaact ggtttagccc aaagcagctc agcttttcac tggagatggg 78060
aaggtaaagg acagaagcag aagtgccatc agatgacatt ttttaaaaat gttgtgatag 78120
gaaatggatc tgatgcttgg agaatggggt cttcagttac tcgtaacaga tacttatttt 78180
atggctgttc ttttacttac tcttagactg tcatttagat tagggagatt agatcttaac 78240
ccttcagcct gtgaagggca gaagggtgag gcttcccagc aaggtcagct ggtcagctct 78300
tcctctttgt ggatttgcat gtatgactga aaggcttttc cacgtgtggt tgatatatct 78360
cagaaagaat gtttcatata gcacatctcc gggaccactc attccactgc tgtagaagat 78420
aatgaccaaa taccttttg gcttttaaat tccataaaat tcaagtccct gatgggaaaa 78480
caaaggtttc acaaaagtaa taatatgcta gacctgcgaa taatttctga aggaagacag 78540

tgtttccctg gctacccctg tgaacacagc taaaaatggt atgaaaagaa gtttggaact 78600
gagtatgttt tgagagcaat ctgttaggcg aggtcattgg tttttagaaa cgatgctgaa 78660
tacataggag caatccctga gaccagatat tcacttcagc agggctctgt gttctcctct 78720
ggctatgtga gacgagaact cataccgatc atggcttcga tatccacaca tgttacactc 78780
gaaagggtca cggaagccgt ggcagcccat gtgaatcgtg aacatcacat agtccaggaa 78840
gaggacgcgg cagtggtcac accgatacac atccatcacc tccccttcct tgttgatcac 78900
tttgacggag tctcttgggc agatgggcgg gggcttgagg agttcgtaag agcggggaac 78960
ctccttcaga agtggcatcc cattgcgggc ccgagacagg accatgtgat tttgctgata 79020
gatgtgattc tggcgttctt catggttgct gtcagtgtcc gtggagtcgt ggccactatt 79080
gttgggagag aggcctctct cagaaggcac gctcttctct ggaaggtgga tgcttttctt 79140
ttccagctct tgaggggcac cgtttgacat ctcagcccgg gtgagggcta tgggatacat 79200
gctgctgata actggaacca tctccgaggt gggagcaggc ggtgtctgga ccaaggggcg 79260
cagggcttcg gcgccaagat agctgatggc gttattgatg gcttggtcca tcatgcgggt 79320
ctgtatgagc tcactctctt tctcatacat gtaacttgaa ttatagttga catcaaagca 79380
gtggcgcttc tcacctggaa caagtgacag aaagggttac aaagggaaca ctgccaaggc 79440
agagagtttg taaatatttt ctagagacct caaaaaggga ggagagttaa gttgcctgct 79500
gctcaagaag accaagcctg gcagaatggt ttcccatggt gctgtctcag gggacactgg 79560
tcggcagcaa cgtgtttgga tggttaaaat aaagccagca ggcagaagag aggggcagag 79620
atgtggttca gttgaggcca catggtaagg tatgcccact gtgcatgtta gaggagagcc 79680
ctcaagaaag gagggaaaga agcaggacac taggatgggg ctagagggac aagggagcca 79740
ccagcccaca ggagcctggg acttctgcta tagttcatga tctcttaaga cacagtcggc 79800
tcccaactct tattttgctt ttgcttgtta ttttctgctt gcaatattag gtagccaggg 79860
ggacagaagt gctgcaaaga gtggggacac tcggtgcctc acctcggcag ctttttaaat 79920
agctacagag atagagaaaa tagcccccca gactccaact ggctactccc caaactcacc 79980
cgtcctggct gactgagact gaagtgaaag agcttcaaat gtagaaagtt tgagaaaagt 80040
tttcttgaga agagatcatg gtctttgtcc ctttcctaac gtgtgtttct cttcctttgc 80100
ttggcaagtg tatctctgat acacatgtga tcattagggc ctatctcaca cttaactgaa 80160

aaaatatact tgtggtcacc ctctcctact tctcatgtca aactgctgag aagttttgaa 80220
aacagagttt agccaccttc agagtgtgtg ggtgttgtgt gttagaaaac agaggctctc 80280
tggtcacagg gaaggcaggg atggggactg cagtgagggc agatgtgctc agtggtgagt 80340
ggtggggaga ggggagagaa agcgaggaag tggagccgag tgctggaggt gaggctgagg 80400
tcactgttga gcctctccat tctgtcagct tggatcctgg agggatttgc taaaccatat 80460
ccttctataa tgtttcctag tcatgttgag attccaaaat ttgtcactgc cctacagtaa 80520
agggttcagc aatgaaaata aagaagaaga agaaaaaatg ccatgagaaa acaaaaggat 80580
tggaagaacc agttcctgtt cttgaggtgt ctccaaccta gtggatgaga aataagacct 80640
atatatatat agaactcgag aaaaagtata agttaaaagt tggccgggcg cggtggctca 80700
ggcctgtaat cctagcactt tgggaggctg aggagggagg atcacttgag cccaggagtt 80760
cgagaccagc ccgggcaaca tggaggaacc ttgtctctac aaaaaaatac aaaaattagc 80820
caggcatctg tagtcccagc tactcaggag gctgaggtgg gaggatcacc tgagcctggg 80880
aggcagagat tgtagtgagc tgagatggca ccactgcact ccagcctggg tggcagagta 80940
agaccttgtc tcaaaaaaaa aagttgtaaa aggactgctt tgctctatgg aggtctttgc 81000
ttttattcta aatgcccaca tttgaggcat ttaggcaggg aagtaacgtg ttctttaaaa 81060
agaataataa ggtaacatac aggcatcggt gagacaattt ggtataaatt gtatatttag 81120
gcattaaggg aatgcagaaa cttcagaagt acttggggaa gggaaagcat tcttggaaaa 81180
tctaaatttt gagaagggtc ttgaactgga agaatttaga ttttcagaga agcgggagag 81240
gacatcctag ttgaggggga gtgcaggaat gaaggcagag aggtgggact acacgggacg 81300
tgtgcagaaa gcgagtctgg ctgaaacgcg atcaagaagt atgaaataca gtatttctca 81360
ggcttcactc ttccttcaca ttctgcaata tccacacacc agggcagtgt ggtggttgag 81420
agaataaact ctggtgtcag atgggctggg tttgatgctg gctccattgg tcaccagttt 81480
tgtgaccttg ggcaagtcac ttaacctatg ttccgtgtcc tcatgtaaag tgaagataat 81540
tgtatgtacc tcacagggtc actggaaagc acttagaaca gggcctgcca catggaaaac 81600
acttggtaaa tgcttgggtt tttttcctct gtttgatttg gttcacattt tctttctttt 81660
tttcttttc taaaataaat gtggttttta aggaaacttt atatcactaa cataaacgga 81720
aaaccagaat cactttccac aagtagaagg taactatgaa aaataaagac aatgaaacag 81780

agcatttcat taaattttga tcggatacta ctgtgagtac ttggagacta agatcccttc 81840
cccctttgtt aaaaacagag attaccaagt gtgagattag tgttaaagac aactggcatc 81900
aaattaagtc taaatttttc tccttagtat gatccaggag actgaaagag aactggaaag 81960
aaaataatta tcctgctgtg tggacccagg tttttaaatg ctccatgtgc ttgctggcaa 82020
tttgcatcct acatttgggg aaacactgaa aaaagataat gcatggtagg accgaatggt 82080
gggcattccg aatggcccac tgtagggtgt gaatccgaac caagagccaa ggctacctgc 82140
ccttgagaaa gacaggtttg tggttaacgt aacagcaatg tgtaaggcag accagaacag 82200
tgagaaattg gagccagaag aaccagctaa aagatctatt cccatgagat gaatttgtct 82260
ttgcatcccc acaagcagct gaccttaagg ccaacctgat agtttccaga gaaatacctg 82320
agagagaaaa aaaatatgca aacgaaaata ccctgtcttg gaaactcaat gagaagcaac 82380
agcagagtag accttggcca aagtaatcaa tgttttttat tttcagtttc cagattggat 82440
atatctttgg tcaaggccat ctggaagact gagaacatga gagccagcta aggtcacaga 82500
cattcattcc ctagaatgtg acacagaaat gatgaacttc ggtttcttgg ccttttttaag 82560
gttattggtt attttccact gtggccacct ctattcatct ccctaaatac caggggtcct 82620
tgagaaactt tttctctttt cacccaacag agaggctcca gaaggtttct gggtggggta 82680
ggcacatcac aaactctgat tttatccttt ctcctaagag tttgctttac ttaacaaggc 82740
ctacaatttc acaatctata ccacctgcag catatgtcag aaaacagctg agtgctaaaa 82800
ttgtaaaagg caaaacatta agaggaagat aaacccctgg atgagtggaa atatgtgact 82860
gaattctgta gttaacacag accattgttc attgttaagg agatggggca gcctcagatg 82920
ttcacgtgca tgcaacttgc aatcatcaga tcccctcgtc tctgagttac ggtaacttga 82980
gcagtgcaga tgattatcag tcacctgtag acacgatgac tcaatgtgta tgaaacccag 83040
aaagggtaaa ctacagtaat tgaaaagcca acagctcacg tttctttagt aacacactca 83100
taaaaaaggt tagtcaaacc acaaaagtgt aactgcttcc aaacctgcac ggagcatctg 83160
gtccagcttc tagtcaaaat cacaccacgc ctttgaatga ttcatgatac cagcttcagg 83220
ctgaattaga ggtcttcttc caacaaaaat acacacggca acagcaagtc ctaagtttag 83280
aaactaaggc tgcctggttg ggccattccc tacctgtggt tctttttaaa ggtgtcaatg 83340
tcttatatct tggctttgtt aactctaaac taagaaacca cctgacctga actgatgcaa 83400

tgtggtgagg acatatggtt gttggcttct aagtgccaac ttggaagtac agaatcacag 83460
gctagagcta aaagggccct taactgagca agtggattca gaactctgtc tgcagggttg 83520
agggggaaga cccaccaccc taatatgttt acttctggtc tttatatttc acagtacctt 83580
gcactgtgcc ttatatacaa taatcccaat aatgataagt actatttatt gagcccctgc 83640
tgtatccacc gtgccatcct agatactttg catactgtct cattttacca gcccattagg 83700
ggttgatact tttttttttt ttttgagaca gagtcttgct ctgtcaccca ggatggagtg 83760
caatggtacg atctcgactc actgcaacct ccgtctcctg ggttcaagtg attctcttgc 83820
ctcagcctcc caagtgtctg ggactacagg cacgtgccac tatgcctggc taatttttgt 83880
atgatacctt ttaaggtatg aattatcatc tcttagatga agaacctaaa gcacaaacag 83940
tttgattcac tttccaagat tgcccagttg gtgaacagag aaccaggatg taatccaaga 84000
gcccacatat cccagtatat gtttgagtgt taactgatgt ttcctggctg ctaccttgcc 84060
catggctgca cagccccacc accagcagtg acttcaggtt ttctgctgtg atggttccca 84120
tagcagaggg aaagggggct tagtggcagc aactgctttt tccacatgga ccagcacatg 84180
cactcaagaa gtatatgctg tgcagagttg ttatgatatc tataaagtgc ttagcagtgc 84240
caggcatgtg taagcactct attcccatta tttgcttagc accatggtta ggtgatttga 84300
ggaatacaac aaaattgcaa tccactagag gagatgcagc atgtacatca tttcatgaca 84360
aatcagtatc cttaatgcta catggtagaa caaaacatag agtagccaag agagtggaaa 84420
gagaaaactg aattacttct caaatatgcc acacttaata tggggcaaag attcatctat 84480
tctttaaagt ctccttgaaa tcagaggtgg tggtagtgac ggtggtggag acagtcatat 84540
ctataataag aagcagacta gtaaactcgc ctggagtgca gagggaggat ggagcagaga 84600
gagaacacag gggtgtggga atgtggacaa tgtgtgcttc tttttatttt ttatttttga 84660
gacaaggtct cactctgttg ctcaggtggg attgcagtgg cgtgatcaca gctcactgca 84720
gcctcaacct cctgggctca agtgatactc ccacctcagc cccctaagta gctgggacca 84780
caggcccatg ccaccacacc tggctaggtt tttgattttt tttttttttt cagagacaga 84840
gtttcactat gttgcttggg ccggtctcaa attcctgagc tcaagtgatc ctcccacctt 84900
ggtctcctaa agtgctggga ttacaggcat gaggcactgt gcctggccta caatgtgtat 84960
tttaagggca gatgtgaggg aggccattca gacaagagtg agatattaca cagaagaggg 85020

aggaggagtt cattgtggct gaagtaaagg actggttggc tgaaatgata aaattagaag 85080
aggtaagttg aagacagatt gtggagggct cagaaggcct gactgaatgg tttagatttg 85140
attccactgg gagccattga aaggtttttt tgaagagggg ggcaaaatga tcgaaccttt 85200
agaaaaataa cagaaatcca atatttagag tcaccatgat ggggtaaggt gaaatattca 85260
ggtcttaagt cttaaagttc ttcaagtcac ttaaatgtgt ttgctgctgc gtcatataat 85320
ttcccacaag tgttatccct cactcttact atcagcacac atccaaaagc aaatgaacaa 85380
aaaaggcagg aaaatagaat atccaaccat catcatggct ctgatctcaa cctaagtgaa 85440
actaacactt cttgcaggag gtggaaaaca gaggggagcc agagaacaag aagaaacagg 85500
aaggaggaaa gaacagagag agctgtgggg gtggcactgg ggaaagctga gctggagacc 85560
acatttggtt gctgcagaag tctttgtcct ctgtgagaca ctgggaagtg ctgtgtggat 85620
gtcatctgtt ctcatggagt caccagggaa gactgttctt ttctaaagag aatcacccttt 85680
gagggagaac aattgccctt cttcaagggt ttttttcctc ctatcaattt tctttttgt 85740
ttttgaaaca gagtcttgct ctgttgccca agctggtgcg atctcggctt actgcaactt 85800
ctgcttcctg ggttcaagtg attcttgtgc ctcagcctcc caaatagttg ggattacagg 85860
cacccaccac cacacctggc taatttttgt atttttagta gagatagggt ttcaccatat 85920
tggcccggct ggtcttgaac tcctgacctc aagtgatcca cctgcttagg actcccaaag 85980
tggggaatta caggtgtgag ccactgcacc tggcctctcc caccaacttt caatgaccag 86040
actccttgct gtccaaagcc ccgcgcatga catgcactgc tcttggtcac ctcctgggga 86100
aggaagggcc tggactgcag tgccttatag gccgaggtct ggcgctgctc tgtcacctgc 86160
cagctgtgtc actccacctc tctgatcctc cttccttcca tggtaaaaga gcctcactgg 86220
tggttcctaa tagcccaaaa atactatgac attgtaatgc ttacaggatt atggacatca 86280
ggcctttggg aaggggttgg aaaaatactt ttttttctct tagaaacagg tattaaaact 86340
gcttcctgtt gctgttaacc aaagcaggca atttctcatt agttacttca cagggagatc 86400
cgatggtttg ttgaagtgtc aaaacctcat taacatgttt accacaggtg agtgagatct 86460
ttttttcatg ttcttttcgt tatgaaaact tcctttaata aagtaataca taaaactttt 86520
aaaataagga gatgctatgg taaccacttt ggtctgaata acatctgttt ttttgggaaa 86580
gaataaaaat caggagaaat atgcagaaat gtagtcattt ttcttctgca tccaagagag 86640

aaatccattt tgagccaagt atttgaggat ggcctagttt gaagtgtagt caaacaagtt 86700
tataacagga gaacaagatt cagagaaagg aaatccccta aaggacagta tgtgcaaagg 86760
aaggtcttct tgtcctttga aaagaacaac ttaaacagaa ttattctgac aatgtaaaga 86820
tggtgattcc tgatggacgt gtgtccattt tttttttcc catttgaaac aaagacatac 86880
aaaatttctt ggagaattag aaaaaaaaat atttttctcc aacttgagag catgtttgtg 86940
tctcaaaata catttagact tgatccaatc tgtccctctt ccccagttca tctctaatgg 87000
atgcaaaatg ctccctactg gtaattatcg ccagcccgcc tgagatagca gcgggctgac 87060
ctgatggtct attttcacct ttaccctcac agttcaagag gtcagggaag tcaaggctgg 87120
tctgagtgct gtgcaactgt cagcaaggat taaagggaat gactcactct tccttcacac 87180
cttacccatt gccattaccc tggcagctga atcttcaaaa aacagtgcct tggagaggaa 87240
atataggaga aaggagagaa gaggagaaga gaagggagga gaggagggaa gaggagggga 87300
gggaacaaga ggacaagaga ggagaggtga ggtgaggcga ggcaaggcga ggggagatgg 87360
agagaaaaca gtagccccat gttttacatc acttcatctc atacactgca cagtgtcaca 87420
aataatgttc ctttctccct ccgttgacat tactattttc tatcacaaag gttgattcaa 87480
ttaaataatt taaggggttt cttttatttt taatttttaa aaacctttca aacaaaagtt 87540
taggatataa gttaatgcaa gaagattaga acagtgccca aaacaagaat accatatata 87600
tgacaggaac tcttaacctt ctgtatgatg aatgaatgga caaaaaagtg agtgaacaag 87660
tagtactgcc ctaagtaaaa cacatgaatg tcgggctaat gcgactgtgt tttagattct 87720
cagatttata ccatcttcct aaaacaaaaa agactctaaa aacctaggat atggtttata 87780
gcataaacac cacatctgta tagccttgag aaaaaggcta tctgaagtta cgcacatggg 87840
ctttctaaac acacagtggt atcaataatt gctgcctttt atttcttttc taattaatct 87900
cacagacttg tacatgggag gagacttcct ggtcacttca tcacctattc agtctcctca 87960
tttcacagat gagtaaagag aggcccagaa aggttctgtg attggtctaa acacacagtt 88020
aagaaatgac agagctagga tcagaacctg ggtcttttag cttctaatcc agtactcttc 88080
ttattgcact acaacacaac ttacgaaaaa aaaaaaaaat tcccccagtt attctgggcc 88140
ctgaagccct gaagttgcta cctcaatcaa gagctaactc tactaggttt ctgattccag 88200
cacagagaga atgtgatggt tcagaagatg agagaaatca acacctacaa gatcagcatc 88260

tctcacaagt ctggaagtag tgaagtgaaa atcctctggt tgcaattgta gcaaacaaac 88320
gaaatagatc cccgaggcaa caggcaaaat ggcatgaatt tgcacatatg aaacatgcta 88380
tatttttgaa cccatgaaat accaaagcaa cttttattat attaagtagg gctttagttc 88440
aactataggt ttagggcaac cgaaaatact agggactgtc tctcaaattt tagttgtctt 88500
acaccaaaat gcattgacca caactggatt gaaattaagt cacagctggg cgcagtggct 88560
cacgcctgta atcccagcac tttgggaggc caaggcgggt ggatcacctg aggtcgggag 88620
ttcaagacca gcctgaccaa catggagaaa ccctgtctct actaaaaata caaaattagc 88680
caggcatggt ggtgcatgcc tgtaatccca gctactcagg aggctgaggc aggaaaatcg 88740
cttgaaccca ggaggcagag gttgcggtga gccgagatcg cgccattgca ccccagcctg 88800
ggcgacaaga gtgaaactct gtctcaaaaa aaaaaaaaaa agaaagaaag aaagaaatga 88860
agtcgcttta catttttttc ttgcccagct ctccctatga tgtccctcct cttgtcggct 88920
tcagccattt ctagagtcaa agctttctcc agaccagcct gggcaacatg aggagactct 88980
atctctacaa aaagtttaaa aattagctga gcctggtggt gcgtgcccat agttccagct 89040
acttgggagg ctggggtagg aggatttctt gagcccaggt gtttgaggct gtgattgcac 89100
cacagcacgc cagcctaggc agtacagtga gaccccgtct caaaacaaac caacaaacaa 89160
aacccataac tttctccaaa ctaaaataca ctcagaataa taaggcaagc tttcttttgt 89220
actccagtca gaacctcttc acataagtaa agcccaaaag atatatggga atatttgtag 89280
acaatgtctt aaactcacca tttgtttact gtgcctaaac tctaaatgga agtcactaca 89340
tgctcccctg acatgtctca ccaaggggag ggaggacagc aaggaggagg ggatctccct 89400
aaactgaaat ataccaactt ttctctgaca caatttgttc ttaaagtgac taaaagtgca 89460
caaaaacctt tcagctagtc aaaataaaat aagtaggcag aaataccaaa ttaggtaatt 89520
ttgaaatata tgggttcttt caatatacct ctataattca catagactgt ttgcttgtca 89580
aaacattatc tataccagaa tcaaatttag tcagatttaa aaaccaggca ggcaacttat 89640
tatctgtaag agctattgag aagaataaat acaaaaggac tgaaataaaa aatcaacagc 89700
ggcaagcagc tttggaaaaa gaggctgaga caggtttcta aaatattact gatgttgagg 89760
gaggtctatt ttcatgtgat aactattttt gttcaatatc accaactttc aatttatagc 89820
aaaactgata cattcaattt cagctgtcta cttcacaaat acaaatctat ttcaaatatc 89880

atggtcacat ttatgtaaac atctgggaga gttcagcaaa atgtctctgc catttggaag 89940
tcctaacact tcaatgaaca taattatttc atacccatct cattctgctg tattttttga 90000
caatgtgaaa accaatggca taaaaatgaa ctccagtaag acaaatgatt aaatcaaacc 90060
ttgttattta aatctatgtt tgcccaacca ctttccctta aagccaattc tcaaattcaa 90120
gaaaatatgg caatatgttt tagagcatga aaaaagtttg agcatgctaa cgaaccaact 90180
tgtaaaatat aattacaaaa gattacaaaa atgctatctt gaacatctta ctggtataaa 90240
ttgcataaac tctctcatgc aacatatgtc attcagagta aagaggaagg aacaggattt 90300
taaacatcac atccatttag gaaattggat atcaactact cccatctcct gtcatgatga 90360
tgctttagtt aatatctttt attctatttt ccagagctat cagaatgaac catgaagaat 90420
aaaagtacac aatgaaaaac taattatttt aacagaggtt aagctaggaa aggccttgtg 90480
tagcaagcat cctatgttat ttcattcaaa ctatctgcta acaacagcag gaaaaaggtt 90540
tgtattctta ccaatgaatt tctgaggcat tgagcttttt cgttttgcca cattgcttgc 90600
taatctgtcc agtacgagag ctctttcact tcccatctct gctttgatgt gtcttgcctc 90660
cgcacttgct agtttggaaa aatgtaaaaa gaagagagaa aagaacacat tggtacatgg 90720
ggagaaaagg aaataaactg gaaggagaag tgtccgaagt tggatgcaca ctctattgtt 90780
gttacctgtt ataactgctc agattcttgc ctggggtacc tgctgtggtc agtgaagccg 90840
acaccagggc acattcaaat cttgcagtct tatgccgaga tgggaaagct cgacccatcc 90900
ccaaccagta ccttcattag caagaggaag tattaaataa acagcctgga cgtggttggt 90960
tgcaaaaaga tagatagatg gagatctaac ccccgatcag ccttctttct gattctgagt 91020
aactgcgtgt gacacctgca gactgatata attcttaaca actttgaaaa aaaagcatgg 91080
tgccccagat ttactgctct gtttatgtta acagcacttt tgaaaatagg tacactcaca 91140
cagatagcaa gacatctact cagtgatttg tgtattgaaa ttctgagtgg tttctttctt 91200
tttcttttc ttttttttt tcagcacgct cctctcagtt cctatctttc atattctgtt 91260
tctctgtgct ctgtattatc tgtttcatat gctcttcaca tttcaaaata gtctcaatta 91320
aaatttccct aagcaacact tctgccttga aattaaagaa caatttgtat aatgcatctg 91380
tattgaattt ctaataaaaa tgtgaataaa aactgcctat gggctgggtg cggtggctca 91440
tgcctgtaat cccagcactt tgggaggtgg aggtgggcag atcacctgag gtcggagttc 91500

gagaccagcc tgaccaactt ggagaaaccc tgtctctact aaaaatacaa aattagcctg 91560
gtgtgatggt gcatgcctgt aatcccagct actcgggagg ctgaggcagg agaatcactt 91620
gaacctggga ggcagaggtt gcagtgagcc gagatggcgc cattgcactc ctgcctgggc 91680
aacaagggca aaattccgtc tcaaaaaaca aagaaacaaa caaaacacaa aaaaacctgc 91740
ctatgaatgt gggcccatcc acaaatagac ctgtgagtgg tgtaggcctg tggttctcag 91800
acctggctgc acattagact cacctgaata gcaggtaaga aatacctatg cccaggcccc 91860
ccttcagatc aattaaatca ggatttctgg tataggggcc taggcaagaa tattttcaaa 91920
gctccccaga tgattttaaa atgcagccag aattgagaac tactagaaga gtatataaat 91980
agtggcaata tctaatcaac taaaatagta aactgtaatg aatataattt acctcaattc 92040
agcaggcagt gcccaaattt atgtctaaag ttcaaagaag gccaggcacg gtggctcacg 92100
tctataatcc cagcactttg caaggccgag gcgggcagat cacctgaggt cgggagttca 92160
agaccagcct gaccaacatg gagaaacccc atctctacta aaaatataaa taattagctg 92220
ggtgtggtgg cgaatgcctg taatcttagc tctcgggagg ctgaggccgg agaatcgctt 92280
gaacccagga ggcagaggtt gtggtgagcc gagattgagc cattgcactc cagcctgggc 92340
aataagagca aaactccatc tccaaaaaat aaataaatta attaaataaa tttcaaagag 92400
ataccagctt ctctaaatac aaggcttgac attttaacac atgatgctag cctgaaccag 92460
ggatcagcaa acttttgtct gtaaagggcc agacagtaaa ttttaaaaat ttgccagaca 92520
catatagtct ctatgttata ttctttgttt tttgtttttt ttttttctgt tccacggcct 92580
tttaaaaacg taaaaatccc tgtacacaaa caggccactg gctgcacttg cctagagcgc 92640
cacagtttgc cctcacctgc cctgggcaaa catattgtga tgctgtaaac atttttctct 92700
gttttctcat aaacacttcg ggagatccaa actaaaatga actatccata catttctatt 92760
atgtctaagg aagaataaat tgaaattagt ttaagggatt agagtaaaaa gcaagtagtg 92820
gctggcacag tggctcaccc ctgtaatccc agtgctttgg gagactgagg caggaagatt 92880
gcttgaggcc aggagttgag accaacctgg gcaatatagc aagatcctgt tcctacaaaa 92940
agtaatttta aaaaattagc tgggcatgtt ggcatgcgcc tgtagtccta gctacttggg 93000
agactgaggt gggagggttc cttgagccca ggggtttgag gttacggtga actattaagg 93060
taactgcaaa gaccacaatt acttttgcac caacctatta gtgccagtgc actccagcct 93120

```
gcgtgacaga gtgagactct gtctaaaaaa aaaaaaaaaa gccccaaagt attaaatatt     93180
tctaatttta ttagtggcct taaaaaactg tatgttagtc attacacaat acattataaa     93240
gctcagtttt cattatttag ttaatttatt tacttatttt tgaaacaggg tctcaactct     93300
gttacccagg gtggagtgca gtggcatgat cacagctcac tgcaggctca acctcctggg     93360
ctcaagtaat ccttctacct caggttccaa agttgctggg actacagtct aaagccacca     93420
tgcctggcta attaaaaaaa aaaattatta gtctcaatat attgctcagt ctggtctcca     93480
actcttggcc tcaggcagtc ctcccacttc agcctcccaa agtgttggga ttacaggcat     93540
gagccactgc actcagccaa agctcagctt tttttttttt cctggaataa cataaaatct     93600
ggctctgcca ggtgtggtgg cactagccca tagtcccagc tactcaggag gctgaggtga     93660
gaggatggct tgagcctagg agttcaagtt cagcttgggc aacacagtga gatcccatct     93720
cttaaaaaga aaaagaaatc cagctccaaa aatgaaaaag aagaggatga taataatgtg     93780
ataataatga caatatttac tagacagtga gtttcttgtc aaaaataatt ctaggctcta     93840
aaggaataaa tcatagcctt cagtgaagct attaacaact atttgtgtaa atgaaaacag     93900
taagggtcag aatagggggc ctgagtggct tttgggctag tgcaaaagta ctcgactgac     93960
tctggctgac cagcagttgt aaataacggg cacttcagcc acaagacact gctattgagt     94020
gaagagggtc aaattctgtc cagcacaggg ataggtttaa atgaccaaac aggaaattat     94080
ttatatatat tatttccttt cacacaaaac caccaccaga tgtggcacta cccaaagccc     94140
caagagcaga gagatgaaag acaaagcttg gaatttgcag taagttcctg gggctgttag     94200
gagacttttt tcttagaatc                                                 94220
```

<210> 2
<211> 94121
<212> DNA
<213> Artificial Sequence

<220>
<223> nucleotide sequence of human genome 17q12 region based on NCBI Build 37.1 (August, 2009)

<400> 2

```
caagcttgtg gtcaaacagg ccagaactgg aaactccact ttactacata ctggctgtgt        60
gaccttgggc agggcattta atctctctaa tccccatctg taaaactggg attagagaaa       120
```

```
ctatctcaaa gggtgtgata aaaattactt gacatattgt gacacctgtc acacagtgag    180
tgctcaatac gttggttctt tgcccactat ggtccaaatc gaatgtacca gtccaaagtc    240
cctccttcaa acaaaacgtt ccaagatgat ccctgccccc acgtcttcaa agccggaact    300
gacaccttcc tatcccaaat gcttcctaat caaagagtct ttcaggggct caaatcattc    360
tctgccttgc acttgaaaag gctcatgtcc ccgtggggac tgtgagtccc aatgggcggg    420
accctggtct gatttctgtg ccattcctag acattgtggc ccgaatgtat ttaataaata    480
cccttttgac tgaacaaata agtaaatgaa agcttctggg aaagtaagag aggctggtaa    540
ggctctcatc accttagggg cttcactcac attcataaga gacctcgtgg ggaaactaag    600
gttcagggat tgcagaggcg tattgggggc gcagggggcg ggagcaagac aaatgggcgg    660
ggcttaccgt gggggtgggg cttacctgct agactcatgt agattggctg gcgggagcgg    720
aagtgattca gagcgccccc agagcagttc tgctcttcgc actgcagtac gcagtcgcgg    780
tacaccggct cacggtcgcc ctgggagccg ctcgccagcg ccgctgcccc agctagcagg    840
accaaccgcg ccgccaggcc ggccatcctt tctccctggc tcgccgccgg gggaggagct    900
taggagtatg aagcttccac ttccggagta accggaagtt cctgtgttct ttattctact    960
ctccgctgaa gtccacacag tttaaattaa agttcccgga tttttgtggg cgcctgcccc   1020
gcccctcgtc cccctgctgt gtccatatat cgaggcgata gggttaaggg aaggcggacg   1080
cctgatgggt taatgagcaa actgaagtgt tttccatgat ctttttgag gtagggctgt   1140
ttactgtcac cacccctgtc ggattttact tcctaaacgt acctgtaact atccacttct   1200
ctccatctct tctggcacca ccctggttaa agacaccatc atgtgtcgcc aagacagccg   1260
cagtagcttc ttaatggctc tccctgcctc tacttttgcc tcttccaacc tgcgctccat   1320
tttgaaaaat taaaatttgc ccatatcact ttttttttct taaaattatt tactggctcc   1380
caattacctt gggtaaaata cagtctccac aaaccctgcc tgatttggcc cctgtccact   1440
ggtctccctc actcccttgc tccagacccg cttcagaggg ctatgtccct caagcttcct   1500
gactgcctgg cctggtctga atcactcact cttctttttt cttctagtcg caattgaagt   1560
accacctccc gagggtgatt gcttccccat gcggggtaga acctttgctg tcctgttcac   1620
cactctacct ccagcacaga atttggctta tggtaggcgc taactgcgtt tgtttgttct   1680
tctgtttaat gaatgaacag catacatcaa cataagaact tgacaaatcc agggctgtaa   1740
```

```
aatcatcagt atggttctgc actgagatcg gagagaagta atatttctag gaaaattagg    1800
aaccctggga acaggacgct tgctttagta tcctctccct gctcacctcc cctgcactcc    1860
catcagcacc gacccacacc caatctcata gaagccttgt agctaaggat cacccttttct    1920
cctccccac tctcctcacc ccttgtcaac tttctttt cgtcctgggg gttggaatga    1980
gtaagaagta gcctgggatt ccattcactc acttaacaaa catttctgag tccttagctc    2040
tagcaccttg ctaagcaagg caaaatctcc aggaggcacc attcacattg cattttctgt    2100
gaatggtgct ctggggagca gcattcacat tgccttttct gtgaatggca aattcttcca    2160
gttaaatata acatgaatag tgtcccctgg agttgaccac ccaactgata ctgactgaga    2220
agctgaaatg aacaaaacaa ccccttagcc ctccaggagc tgaccggaaa tccagtgcta    2280
atactacttt gcatcttaca gattagttct tttacaatac tgtttttttt tcttttttca    2340
tttcattttg tcctttctgt gactctggga tgagtctttt tatgaggatc ctcatataaa    2400
gatggacatt taggattaaa gaggatgaaa tcctgacaaa atagggagtc tcccctttag    2460
aaaattccta agtaaggctg ggggtggtgg ctcacgcctg taatcccagc actttgggag    2520
gccgaggcgg acggatcacc tgaggttagg agtttgagac cagcctgacc aacatggaga    2580
aaccccatct ctactaaaaa tacaaaatta gttgggtgtg gtggtgcatg cctgtaatcc    2640
cagctactca ggaggctgag gcaggagaat cgcttgaacc cagggaggca gaggttgtgg    2700
tgagccaaga ttgcgccatc gcactccagc ctgggcaaca agagcgaaac tcaaaaaaaa    2760
aaaaaaaaag aaaaagaaaa ttccaatttt gaaggcctca tcctatatta tgtcaaacat    2820
actgaaatgc agtaacgccc cacattaaat aagatttata aataactata catatatata    2880
attcaatcta attgctgtta atagttgaca tattgctaca tttatataca tttagttaaa    2940
aaaaattttt tttcccagac agcctctcac tctttcacct agactgaagt gcagtggcat    3000
gatcacgact cactgcaacc tcaacctccc agactcaagt gatccttcca tctcagcctc    3060
ctgagtagct gggactgcag catgcgccac tatgccctgc taattttttt aatttttgt    3120
agagacacgg tcttgctatg ttgcctagac tggtctccaa ttcctgggct cgagtgatcc    3180
tcccgcctca acctcccaaa gtgctgggat tacgggcgtg agccatgcca cacggccata    3240
aaatattaat tttcgcagct ttcttatatt ttagaactaa caatggaaat ttgttcgggt    3300
ctaaagtatt tcagaggtcc ttgaaaaccc atgcctacat acctgatgga aaaagcaatc    3360
```

```
ctaggttaat ggtggaagtg ggagtagaga cttctgttct gttgacttct tggaagatgg    3420
ggtactgtct ctctgggaca gctcttgaga atttccctgc cagcacagcc ccagataaca    3480
atctctagat ggcgattacc tggcctctct tcccaacttt ctagcctgga gcccctagtt    3540
ctcccctgag cctccttagc ttgtccttct tcctaacttg tatttggctt cagatgtgat    3600
ccacagtctg aaaagtcact aattcattcc ttcaactcag gcttattgag tcctcctgtg    3660
tatcagccat tgtactcatg gggaaaaaa aagacaaagc atatgttaat agtagagtgt    3720
gctggacagg cacagtggct catgcctgta atcccagcac tttgggaggg cgaggcaggt    3780
ggatcatctg aggtcaggag ttcgagacca gcctgaccta acatggagaa actcctgaga    3840
tcgtgccatt gcactccagc ctgggcaaca agagcaaaac tccgtttcaa aaaaaaaaaa    3900
aaaagtatag tgtgctaaag gctcaacggc aagctgacca tgttcttaga tcaaaattgg    3960
tagagagtct acaatgtggg ttccttattc atcaaatgtt tattaagttt accatgtgca    4020
agtctctggg aacagagtga tgaacaaggc actgtacttt tcatggtcag aggagggaaa    4080
caggccataa acaagtgtca aacaaaagac tgaagccagg tgcggtggct cacatctgta    4140
atcccagcac tgtgggaggc caaggcaggc ggatcatgag atcaggagat cgagaccatc    4200
ctagccaaca tggtgaaacc ccatctctac taaaaataca aaaaaattag ctgggcatgg    4260
tggcacgtgc ctgtaatccc agctactccg gaagctgagg caggagaatt gcttgaacca    4320
gggagttgga ggttgcagtg agcctggatt atgccactgc actccagcct ggtgacagag    4380
cgagactcca tctacattaa aaaaaaaaat atatatatat atatatacac acacacacac    4440
acacacacac acataccctc taacccagga atttcactcc taggtatacc tacataagct    4500
ccagtatacc taaacaagtg caaatttgtt taagtacagt tatttgtggt agcattagtc    4560
attgttttca atagcaagaa gaaaaaggaa acaactaaat gtccatcaat agggaatgaa    4620
ttatattaat ggagggagag ccatacaatg gaaggctgaa cagaaattaa taggaatggg    4680
gcagatttgt aatgtactag catggtaaaa ccttcatgat agatatagat atagatatag    4740
atatagatat agatatatat acatatacat atacatatac atatacatat atatatatat    4800
atatatatat ctcttgtgtc tcagcctccc gagtagctgg gattacaggt gtgtgccacc    4860
acatccggct aatttttgta tttttagta gagacagggc ttcaccatgt tggtaaggct    4920
gtcttgaact cccgacctca ggtgatccac ctgtctcagc ctcccaaagt gctgggatta    4980
```

taggcatgag ccatcacacc tggccaaata tttttgataa gtatcaagtg cacagtgcag 5040
aacaaaatat gtgtgtgtgt atgcatgtgt atgtacacct atacacttat atacagtacc 5100
ccatgtgaag aaaaataagg gtacgtgtta tgcgcgtagt attatggttg ttatttttga 5160
gaatatatct agaaagataa aaaagaaagt ggaaatagtt cttgcctctg gtgggaagtg 5220
ggactatgtg cctgatcaat agggaagtaa ggaacacttt tttttttttt ttttaaacgg 5280
agtttttgct cttgttaccc aggttggagt gcaatggcgc gatcttagct cactgcaacc 5340
tctgcctccc aggttcaagc gattctgctg cctcagcctc ctgagtagct gggattatag 5400
gcatgcgcct ccacgcctgg ctaattttgt atttttagta aagatggggt ttctccatgt 5460
tggtcaggct ggtcttgaac tccccacctc aggtgatccg tccgcctcag cctcccaaag 5520
tgctaggatt acaggcgtga gccaccgtgc ctggccagga acgcttttta tttttgtacc 5580
tttaaaagtg tgtaccgtct gtgtatataa tcagttaaaa acaaagaaaa gctgagtgtg 5640
gtggctcatg cctgtaatcc cagcccttaa ggaggccgag gccggcggca gatcacctga 5700
ggtcaggagt tcaagaccgg cctgaccaaa acggtgaaaa ctcatctcta caaaaacata 5760
aaaattagcc aggcatgatg gcaagtgcct gtaatcccag ctggttggga ggctgaggtg 5820
ggagacttgc ttgaacctag gaggcagaga ttgcagtgag ccaagactgt accactgcac 5880
tccagcctgg gcaacagagc aagtctctgt ctcaaaacaa aaacaaaaac acaaagaaaa 5940
aatgtaaaac aatttcatgc agtagcaagc atcgagttaa atacagttga cccttgaaca 6000
acacaggttt gaattgcacg ggtccattta tactcacatt tcttccacct ctgccacccc 6060
caaaatagca agaccaaccc catctctttt cctttctctt ccccctcctc agcctactca 6120
atgtgaagat gatgaggatg aaaacctttg tgatgatcca cttccactta atgaatggta 6180
aatatgtttt ttcttactta tgattttctt agtagcattt tcttttctct agcttccttt 6240
attgtaaaaa tacagtatat aacacatatc acatacaaaa tgtgtgtaaa tggactgttt 6300
gctattgata agtattctgg taaacagtag actattagtt tttttgtttt tgtgacaagg 6360
tctccctctg tcgcccagcc tggaatgccg tggtgtgatc atggctcact gcagccaaaa 6420
acttctgggc taaagcaatc ctctactaaa aatacaaaaa ttagccaggc atggtggtgc 6480
gcttctgtaa tcccagctac tcaggaggct gaggcaggag aattgcttga acccgggagg 6540
cagaggttgc agtgagctga gattgcaccg ttgcattcca gcctggacaa cagagcgaga 6600

ctccatctcg aaaataaaat aataataata ataataataa taataataat aataataggg 6660
ctgggtgtgg tggctcatgc ctgtaatccc agcactttgg gaggccaagg tggacagatc 6720
acctgaggtc aggagtctca attaaaaaat aaataggccg ggcacagtgg ctcatgccca 6780
taatcccagc actttgggag gccgaggtgg gcagatcacc tgaggtcagg agtttgagac 6840
cagcctggcc aacacggaga aacgctgcct ctatcaaaaa tacaaaaatt agctggatgt 6900
ggtggtgcat gctataatcc cagtaatacc agctactcgg aaggctgagg caggagaatc 6960
actcgaatcc gggacacgga ggttgcagtg agccgacatc atgccactgc gctccagcct 7020
gggtgacagt gagactctgt ctcagaaaaa aaaaaaaaaa aaaaaaaaaa aaaaaaaaat 7080
atatatatat atatatatat atatatatat atatatatat gtgtgtatat atatatatat 7140
acacatatat atgtgtatat atatatacac acacacatat atatgtgtat atataaaata 7200
aaataaataa taataaaaca tttactttgg ctgctgttgc tgcggggaga attgcagggt 7260
gtcaaaagta gcactggtgg aggggtagtg atcaaagtct ggtgctttag cccaaaggag 7320
aaatgataga gactcagact agctggtgat ggaggtagaa taagcataaa tgtatcaaaa 7380
agaggagttg atagatctta aagaatgatt ggatttgaag ggcaaaggaa gagaagaatc 7440
aaccaggtgg gttcagtgaa tgaaaccatc agaaacgaat tgtcccctga aatcaagact 7500
ttgtgattgc catagttgta tgcttctcaa aggttcctcg tctcctcttc cttggaccaa 7560
aagtcagagg caagaatgcc ctcattcata ccccagtggt ctatacctcc agcagcaagt 7620
cgagtgagca agtgatgtcc tgaaaggccc agtggatcag tggaatgaag cgggcaggaa 7680
gacttagtgc tcctgaaaca aggaatccag aatccaggag aaggatggct cagtggggct 7740
ttcaagggac aagtatgggg gttgaagggg tcactgtccc tataccaaat ccgaaaatat 7800
tgtgaccagg aaccattctg tccaactctt ctatttcagg tggcaaagca aagctatatt 7860
caagaccaca tgcaaagcta ctccctgagc aaagagtcac agataaaacg gggcaccag 7920
tagaatggcc aggacaaacg cagtgcagca cagagactca gaccctggca gccatgcctg 7980
cgcaggcagt gatgagagtg acatgtactg ttgtggacat gcacaaaagt gaggtgagtc 8040
gcaggacaga agagtgcttt ttgtttcagc agagcagcct ggggagagat aaaagctact 8100
cctggggcct gggcctgcat tcctgagatg tgggtaagag gggcccaggg tcagagtgtc 8160
tggcaagctt ggctctgccc ctttgctgtc ctggagacta gggctaatcc tgggctcagg 8220

gagtggcctc cccatggtta ggatacaagt gctcatcaag ggccacccct aggaaggacc 8280
aattttccta tcagaagctt ctaagttatc ctcctttggc ccaaagggac acctcaagcc 8340
tactctgagg aactctttcc aatgaactaa ttcctacagt cacttcccca gcaacctgtg 8400
cctcagcctc aaggcactgt ggggtaggcc tcagtttgtg gcctggacat cggactgtgg 8460
accagacgac tcctcccgat ttctgtttgt tttcagtcct ctgaccccaa gctggctggt 8520
gaagtaggta gagggaggag actttggtgc atgcatacac acacacacac acacacacac 8580
acacacacac acacacacac acacacacac gtctcctgtg ccccccagtc tccatggctg 8640
gtcaatgatt gactggcatt tcacaggccg ctggttgcag ccccagcctg ttgacttaga 8700
ggtcaccctc ggaagctaga gccctgtcct gcctcttcag tgtcagtggt cactccactg 8760
cccacaggct ggggtcttgg gcaaaacaca cgcatctgcc ctgatctgag tttgctgccc 8820
tctgtcccgc agtcagcccc actctgttcc cactccctct ccccagcccc ctagctagac 8880
ccctctcacc agcacccctt tcccttccct gagggtcccc ctcgctgtct ttgtccctca 8940
gacatcctct ttcctgggct ctcctgccag gccctgctgg agggacagtt aaggaggaaa 9000
tcgaatcagc agcgcccacc cctgcccccc ttcctctcct cttgtcagac accagacgag 9060
gtttttttcct ctggcttccc agctctgaat gggctcattc tttttcagag gctcggcccc 9120
tctcgagcct cctccccagg gcgtgagttc tgaccccagc tcctccccc atccccactc 9180
cagcccccctc tccagcttgc tccaccctct ctaccgccca ccgggactgg gcattgtctg 9240
ccagtccggg tttcttcctg ggatttggga tgcagagagg atgggtttgc ttgggcgggg 9300
gggtggagag tgaagggggg aagcaggatc tttgtagagg gagggaccta cagttacctg 9360
gacttctttc ctctgtctcc cctcttggta cccttgactg gggctcttga gggtaatggg 9420
tgaagccaaa tctgccatgg ctcagttccc agctcagctc tgtgaccttg ggaaagttcc 9480
tttagctcgt ggaatctcaa ggctcaaggt tcctcttctg caaaatgggg aatgataaca 9540
cctgcctcct ctggagtctt ggggactcag tgttctgagg aacgtggctg taggtcagag 9600
tggcacagag tagggtccaa tgaagcatgg cgtccacagt agctttcctg actggactaa 9660
cctttccgga cacaacagca gggcaggggt ggggcctggg gagaaaggac acctctaacc 9720
ctgatcctaa catcccgatg gcctctaagg ctgcctgcac actcatccag gtgcaagccc 9780
tccaaggtgt ggtgtgatga accagtgact cctggagcca ggtcagcgca tcctcttccc 9840

gcagggctgt aagctgcagg actgagaggc aggttgacca ggtcctgggc tggatgatgg 9900
ggtgagagta aggggtcagt tttgatacat gcccaacttt tctctctagc cctaagacat 9960
cctgggcaaa ttgcttacct cagttcccct gatcctcacc ctaaccctaa caccagctca 10020
agagaaaata gggatattga tggccatcca gaagggctgc tgtgttccat acacagcaat 10080
atttctcgaa tgtttgtgac agcggtccaa ggaataagtt aattttacat tatcactctg 10140
gatacctgta caaaactcca ccttatcctt actatatgaa tgtgctaggg ttgttttttt 10200
gttttgtttt tttttttttt ttttgagaca gagtttcgct cttgttgccc aggctggagt 10260
acaatggcgc gatcttggct caccgcaacc tccgcttccc aggttcaagc gattcacctg 10320
cctcagcctt cccgagtagc tgggattaca ggcatgcgcc accatgcccg gctaattttg 10380
tgtttttagt agagacaggg tttctccatg ttggtcaggc tggtaccaaa ctcccgacct 10440
caggtgatcc acctgccttg gcctcccaaa gtgctgcaat tacaggcatg agccaccgca 10500
cccagccgtg ctagggtctt tttctgttca attcctttct ctctcttgct ctctttcttt 10560
ctttcaatgg agtcttactc tgtcacccag gctggagtgc agtggcaaga tctcagctca 10620
ctgcaacctc tgccctctga gttcaagcaa ttctcctgcc tcagcctccc gagtagctgg 10680
gattacaggt gcctgccacc acacctagtt aatttttgta cttttagtag agatggggtt 10740
ttgtcatgtt ggccaggctg gtctcgaact cctgacctcg tgatctgcct gtcttggcct 10800
cccaaagtgc tgggattaca ggcatgagcc gccatactcg gccaactttg tattactttc 10860
ttaaagagag tttcccaaat tatataagct tcaggcccca caaaacctag atctgcccca 10920
gtataactaa atctgggacc atttattgag caattattat gtgccaagta ttgcgctgag 10980
tgcttccaga gcattatctc ctttaacccc agcatagtat gtcagatgct gttttacaga 11040
tgagccaact gagaccagag atgctcagtc acttgcccaa ggtgacatga ctgatatgga 11100
atagagtcaa gatttttttt tttttttttg acacggagtc tcactctgtc tcccaggctg 11160
gagtgcagag gcgcaatctc agctcactgc aagctctgcc tcccaggttc acgccattct 11220
cctgcctcag cctcctgagt agctgggact acaggcaccc gccaccacac ctggctaatt 11280
ttttgtattt ttagcagaga cagggtttca ccgtgttagc caggatggtc tcgatctcct 11340
gacctcgtga tctgcctgcc tcggcctccc aaagtgctgg aattacaggt gtgagccacc 11400
gcgactggcc agattcaaga tttgaaccca ggtcctcttg gtcccagagg cccctgtttc 11460

```
tcaactccct aggatggcat agcaacctgt cccacaagag gtgcctgctt taagtgtgct      11520
cagcacatgg aagcaagttt agaaatgcaa gtgtatacct gtaaagaggt gtgggagatg      11580
gggggaggg aagagagaaa gagatgctgg tgtccttcat tctccagtcc ctgataggtg       11640
cctttgatcc cttcttgacc agtatagctg cattcttggc tggggcattc caactagaac      11700
tgccaaattt agcacataaa aataaggagg cccagttaaa tttgaatttc agataaacaa      11760
tgaataattt gttagtataa atatgtccca tgcaatatct tgttgaaatt aaaaaaaaaa      11820
aaaaaagtct tccttccatc cccaccccta ccactaggcc taaggaatag ggtcaggggc      11880
tccaaataga atgtggttga gaagtggaat taagcaggct aatagaaggc aaggggcaaa      11940
gaagaaacct tgaatgcatt gggtgctggg tgcctcctta aataagcaag aagggtgcat      12000
tttgaagaat tgagatagaa gtctttttgg gctgggtgca gttgctcgtg gttgtaattc      12060
cagcactttg ggaggctgag gcgggaggat cacctgaggt tgggagttca agaccagcct      12120
caccaacgtg gagaaaccct gtctttacta aaaatacaaa aaattagctg gtcatggtgg      12180
cacatgcctg taatcccagc tgctcgggag gctgaggcag gagaatcact tgaaccaggg      12240
aggcagaggt tgtggtgagc agagatcgcg ccattgctct ccagcctggg caacaagagc      12300
aaaagttcgt ttaaaaaaaa aaaaaagtcc tttcgatgtg actgtctcct cccaaatttg      12360
tagaccctct taagatcatg cttttcagat acttcaaaga ttccagaaga tatgccccgg      12420
gggtcctgga agccacaagg taaacacaac acatcccccct ccttgactat caattttact     12480
agaggatgtg gtgggaaaac cattatttga tattaaaaca aataggcttg ggatggagta      12540
ggatgcaagc tccccaggaa agtttaagat aaaacctgag acttaaaagg gtgttaagag      12600
tggcagccta gggaatttat cccggactcc gggggagggg gcagagtcac cagcctctgc      12660
atttagggat tctccgagga aaagtgtgag aacggctgca ggcaacccag gcgtcccggc      12720
gctaggaggg acgcacccag gcctgcgcga agagagggag aaagtgaagc tgggagttgc      12780
cactcccaga cttgttggaa tgcagttgga gggggcgagc tgggagcgcg cttgctccca      12840
atcacaggag aaggaggagg tggaggagga gggctgcttg aggaagtata agaatgaagt      12900
tgtgaagctg agattcccct ccattgggac cggagaaacc aggggagccc cccgggcagc      12960
cgcgcgcccc ttcccacggg gccctttact gcgccgcgcg cccggccccc acccctcgca      13020
gcacccccgcg ccccgcgccc tcccagccgg gtccagccgg agccatgggg ccggagccgc     13080
```

agtgagcacc atggagctgg cggccttgtg ccgctggggg ctcctcctcg ccctcttgcc 13140
ccccggagcc gcgagcaccc aaggtgggtc tggtgtgggg aggggacgga gcagcggcgg 13200
gaccctgccc tgtggatgcc ccgccgaggt cccgcggccg gcggggccag aggggcccgg 13260
acgagctctc ctatcccgaa gttgtggaca gtcgagacgc tcagggcagc cgggccctgg 13320
ggccctcggg cgggaggggg cagttacacg gcagcggctc gagatggccc atccaagaga 13380
ctggcgcttt ccaggctccg aggggctccg ggaacttgtc aaagaagttc tctgaaattg 13440
ttcagaaagt tttcccgcaa agggtgtatt gcgtagagcg cgcgcgcgcg tttcccccct 13500
tcttgagccc cctcaagctt tctcaaagcc tttccagttg gcagcctccg cctccggact 13560
ggcctgggct ggattccttg ggggggtcct ctgccctgcc cctcctccag cccctccccg 13620
ctccccttca gacgattttg gtttggttgc tcctgcttct ggcggggtcg ggtgtgtgtg 13680
tgtgtggtgg agtggagggt ggcatagcaa cctgtcccaa ccagagccgg ggaggaaagg 13740
gtggcccgga gggtggcctc ttgctggggt ctgggttggg ggcgggggag acgtttgctt 13800
tgaacagatt cttggggcca gcttagggac tgtgctctgt gacttttgga gcgcgtggac 13860
catggagggg tgggggtggg tttcttgggg tgtaaagtgg gagagttccc agagaaggaa 13920
gctaagaaat aaggccagat gggagcctag ggagggctgc gttgttctgc tgccttttcc 13980
ttggtgctgt gcgtggggaa gggtgagtgg gggcagtgtg tatcctgacc catctgtcca 14040
cctgtgtgca ttaatcataa aagctaacat atagcctggg ccaggtatac tctgccagga 14100
actgtttgtg gtgttttgca tgcattctcc tttaatccta gaacacccct atagtggaag 14160
ttctgccagc attctggact gagtagcagt ccagaggttg agtagcagct agtaagtggt 14220
ggggtcaaga tgggacccca ggcagtgcga cccccaacca tgcattcgaa atcgctatat 14280
ggatgagtgc acctggagca atgagggaca ctgctccctg agtcactggg ctgcagggga 14340
gacaaaatga aagtgttctg ggagtcgtgg gtggtctcca taggtcagag ggtctgggga 14400
gggagtgggt gtcatcgtgg ctgtgtgttg cccgaggggc cctctgtgag tgagtgcatg 14460
gccgtgttat ctctgcaggt ctacgccagg gtgttcctca gttgtgtggt ctttgtattt 14520
gtgtgtctgg gctttgtgtt gccaaacagc agtctctctg ctgacttggg gacacaggct 14580
gaactctgtc ctctgcagga actcccttaa ggtgctgggc cagatctgcc ataaacagag 14640
ggaggtagcc ttctatggcc acgccttctt gctgaggaag aaggttcctc tcttccaggg 14700

agtacatcct tgccctccct gtttcccaga caagcatctt cacctctcat cttctgatga 14760
gaagggtgag gccatactga gctgtcaggc tgagctgctg cccttcctca ccttgggctg 14820
ggagttgatc agggaatggc agttgctgca gagctggatt tgagggctgg gttctctgga 14880
tggggcctcc tcatgtcctc acccctcaac ctgcactatt gattgtgttg tgcaggagtt 14940
agttaaaaag tcattgcaca gcctgggcaa caaggcaaaa ctctgtacaa aaaatacaaa 15000
aattagttgg atgtgattac acgtgcctgt agtcccagct actccggagg ctgaggcagg 15060
aggatcacct gagcccagga agttgaggct tgcagtgagc tgtgattgca aatgctctcc 15120
agcctgggtg acagtgtgag actccgtttc agaaaaaaag tataccaccc agctgcctcc 15180
agcacccaga ttttacccaa ggggtgaggt ctggggcagg aatgtggggg aaggggaggc 15240
ctagggggag ccccagaggg gtcaggattt ttctgaaatc ctttcttaga ggtatgggtt 15300
ttacaaattg cagcaaatac atccttttaa tcttgcagaa ctccttcata ttttaattcc 15360
agtatgattc ttccaacagc ctcctctctt tactatactt ggggaaagta ctcattttat 15420
ttgtcaagaa aaaaacaatt gaaaagatag ggatcaaatg taaaaagaaa aaatacgtgg 15480
cattccaaag tcaaacacaa agcatgttta attttctcgt ggtttgggat tacccatatt 15540
cctgctgtat gaacctgtct tgtcttaact tttaagaaat gtacggtgta cttcctatat 15600
gctaggtttt tatccatgct ttcatttaat ctctgtgaca gtcctgtgaa gtaggtgcac 15660
agatgagaaa atggaagttc agagaaatga agcaacttat ccaaggctcc cagctaccca 15720
gtaatgtcca gggaattttt ggactctgaa gaggaggcat taagaggtgg ttagagtctt 15780
attccagcca acaataatgg gttgaacaaa gccttagggg caggcaggtg gccagatggg 15840
aggagaagcg ctcctcttgt tcaggcgaat gacctttcca tccacttctc taggctgtag 15900
aaagtggagc tgagctgggg gccctgaggt tccctcttga cttcagagtc ctctcccttc 15960
ctgtccagcc aatgcctgtc ttccttttgg gccctaccag catgacaggg ggctgcgggc 16020
aggaggggac agaggccacg ttgacacaca gggctgtggg tgagagagac agctgaagtg 16080
tcagcgtgag gggccagtgt ggggctgcgg ctgggagggc tggggtgggg cccagggtag 16140
ttgtgcctgt ccttgggtga tggaatgatc tggaaagaga ttccttccct gccctccacc 16200
tgtgagaagc ccctctagag tgacatctcc atcttatgtt tggccaccca tcctccccct 16260
gggaagagag ccgaggtggg gtaagggatg tgtactcttt caaggagtgg gagaattatt 16320

ctagcgaatg tttgtgttgt cccagttctg tttacaaagc ctcgtcatgt ttacagatgg 16380
ctgcgcaatt cattacctca tttaactctc atgtacctcc tctgagggag taagagctgt 16440
tacagccaag tttaggtcag taaatattca ccaagttgca ggtactgcag ggcatagaga 16500
tgaatccgat ttagcttctg ccctggaggt ctgggaactt gctcaagatc actcagtgag 16560
cagctgagct agggttctca actaaagacc ctgggcccag gccctggtct gatgtcaggc 16620
ctgatacacc aggtgtttgt ggtcggggaa tcccagtgtc acttgaatgg gctgtgacat 16680
tatgggtctg ggagagctga gctttgggga cacaggtcat tttactgtag tattcatgga 16740
aaccaaggga agtattggct tttctgctgt gagcaagagg agcagctggg gctgcaagct 16800
ggtggggagg agagaaccca cctgagagaa acctcaggac tggggtcaag tcctgaccac 16860
cagagtccag agagacatga aggactgtga ccagctctga gcagagagat ggattccatg 16920
acctcaactg gtccctttg ttcggagact cgtgactgga cttcattcat ccactcattc 16980
attcattcac tcagcagaca cttatctagc gctccctgtg gctggtcctg cctcatactg 17040
tctttgctct ggagaattgg aggttggggt tcctgagggg cagggtcctg gagacaagga 17100
cactcctggg tagaattagg acctaccccc caggaaatca acggggacca ggtgccgtgg 17160
ctcacacctg taatcccagc actttgggag gccgagacgg gcggatcaca aggtcagcag 17220
ttcaggacca gcctggccaa catggtgaaa cccgcctcaa ctaaaaatac aaaaattagc 17280
caggtgtggt gtcaggcacc cgtaatccca gctactgagg aggctgaggc aggagaattg 17340
cttgaacccg ggaggcagag gttgcagtga gccgagattg cgccactgca ctccagcctg 17400
gcgacagggc gagactccat ctcaaaaaaa gaaaaccaat gggacagggc agatatgggg 17460
acaatggtaa ggagatggga gagtgggagg gaggtgtcag gaagaccttc ttgacttcat 17520
gtaggctggt gggggtgtta gccagcaagc ctccagttcc ctgggaaccg ttctcagggt 17580
accaatttta ccacctgtct gcaaacactt taagattctt aatcagactc aaattggcca 17640
caaatcaggt aaacaaactc actagtgggg tggggctacc acccgttctg accctccagc 17700
ccaacccagc ccagccaccc tgccctccgt agagcctgtg gtgtttatcg gtggcattgg 17760
gagaattagt gtgtatttat gttggcgtgg ggtgtggggt ggatttgtgt gtgtgcagtt 17820
aggcctagtg gaaggaatgt gggatctgaa ggcaggccag cctgagttcc agtcctgcct 17880
gttgctcaca agctttatga ggcgagagct aacccctgcc agcctcagtt gtcttctttg 17940

caagatggag gttgcagccc cagtctctgg agcatgttat gcagatccac cgagagtgcc 18000
tgccaggcac acagtaggtg ctcagctcag ttactgtggc ggcccccact ccccattgtt 18060
gttgttttcc tattgcctgg cggccacagc tggtatccct tgaaaagggc tacagggggt 18120
ggagtcggac cctgccccag ccctgtggag accctgggct tgggccaggg cctggggtct 18180
gggcctgcag acagctgtgt ctataaagca gctgaagggc tgaggccggg ggaggtcctg 18240
gcagcagggc gttattttgg gcctggcctg ccaccccag ctcctgtttc tcttgggagt 18300
ctgttggggg aggaagtgtg gggaagagga gggggtgcaa gtgggtgagg catggagtgg 18360
ggaggcctcc ctcagggaca tggacccttg agttctattt ctgttcctcc ctcctgttcc 18420
tccctctttg tccttatctg cctagagagg tgggaataga ggccattctg agtatcacta 18480
ggagaccacc agtttgtggc cactggccac tggcccaggc agggaacctg ggggcttgcc 18540
ctaccagcct ctcccagcaa tctgaaggca gggggtacct cgtattaccc cctaggattt 18600
gaccttaggc tccaacttgc tgggagagca gtgcctctgg tgtcagaccc caagccagcc 18660
cttgtgctgt ccctgaatct gcatgtagcc tgtgggaggc ggagcagtga ccggcaggaa 18720
ttctgggcag ctcaggcacc tgtgggcctg agggtgccct ctgcccccac ccttccgatc 18780
tcctgggcaa gacacgccag gtgattcatc tcaccagagc agaaaaacaa gttcaactgg 18840
gcactttaat ctcccctcac tggcaggcct ggtgtgagct gctaccccgg cgcccctcac 18900
caggggtgct ttacctcctc tagtattcct gaccttagtg ggcatttctg gtctcaggga 18960
taccaggctg gggtccaagt gggccaggtg tggcagttca gccctatgcc ccatggctga 19020
tggctcgcgc tgggcaggta tgcagggctg acgtagtgcc tttgtggcag cagtttcgtg 19080
gcacacattc tgccagctgg ttctggagtc ttgccctgag gaggtggcca gggtgagggt 19140
gccagcgcag gaacctttgg cgcatgcttc accctggcct gggatctgca gcctgggtcc 19200
agatgcccac aactggaatc tgacgctcct tttctcttca tgggggactc ccagaggtct 19260
ctgcaatgac cagagccccg gttgtcccat gcctcagctg caactccagc tgaccctcct 19320
tccccactct ctgggtggca ttacgggggt gtggatccct tgccaagagg ttggcatgtg 19380
ggtgtgctgg aatggcatag ggagaatgca ccgagtttgt ttgcttggga gaggggcagg 19440
gggtatccag aagattcatg attcgtcatc gcctctcttg ggggattttt accccttgc 19500
cctgagttgt gcctttggga caaaggaagc ctttctttgc cagccaacac cctgtactgg 19560

cgggcgagct ccccagggct ggcacgctgg ggcagcctct gaatgcacag ggtgggccta 19620
gtcagaagaa gcctttcccc tgaaatccct ctacttccca agcacgcaag ctttctcctg 19680
ctgttaaacc tgcagtgtgc aagggacatg ggcggagggg tccttcagtc aggcttctcc 19740
ctgtctgagg tggcatgact tggagtgagt ttggatgggg tggccaggtc tgagaaggtc 19800
ccccgccagt gtcctctgac ccatctgctc tctcctgcca gtgtgcaccg gcacagacat 19860
gaagctgcgg ctccctgcca gtcccgagac ccacctggac atgctccgcc acctctacca 19920
gggctgccag gtggtgcagg gaaacctgga actcacctac ctgcccacca atgccagcct 19980
gtccttcctg caggtgaggc ccgtgggcaa cccagccagg ccctgcctcc agctgggctg 20040
agccctctgt ttacaggtgg gtggcagaag aaggtgccct gcccttctgt ttcctctctt 20100
gttgtggttt ctcaaccagg aagtcctttc taacatctaa cccccattca ttttactgca 20160
gaatcagttg actctctcta taacgtggct ggccgaggtc atgtctggat gggatgcgtc 20220
tgtgtttccg ctaaatcttg tgctctcttg ccagcatgat catgtcccct gtccacctgc 20280
tccagccact atccctctcc cacttacagc agaagaaagg gctggtgaga aaggtggatt 20340
acaggcccac ttctgccact gacgagccct atgaatgtgg cctacacccc cttagcttca 20400
ctgggtctca gtttccctat ctgtatattg ggagcagttg tgaagctcag aagagaaatg 20460
tctgtgaaaa ggttatgaac aggagggaga gtggaaacca acctgctgga tcgtgtccac 20520
agaccctgga atggggccac atgcttggtt tgtcaaattg cagacgccgg ccgggtgcga 20580
tggctcatgc ctgtaatccc agcactttgg gaggccgagg cggacagatc acttgaggtc 20640
gggagttcga gaccagcctg accaacatgg agaaaccccg tctctactga aaatacaaaa 20700
ttagccaggc atggtggcac atgcctataa tcccagctac ttgggaaggc tgaggcagga 20760
gaatcacttg aacctgggag acggaggttg tggtgagcct agatcgtgcc attgtactcc 20820
agcctgggca acaagagtga aactccgtct caaaaaaaaa aaatttgcag acgccatccc 20880
atccaggcct ttgctttcac tgatgaagaa actgagatac agagagggca gggcacctgt 20940
tcggagttta tgaaatgccc ccccaccatt atctttcttg atcatataag aatctggtga 21000
ggcaaggtag ggcgtgatct ttatctctat tttatcgttt tatttaagcg ggaacaggac 21060
tgctcagtgg ctgggggcct tgcccaagat ctccaagtac tggggaaccc cagggaggcc 21120
ctgggggtg gcagtgttcc tatttcagcc ccactctgct tccccctccc aggatatcca 21180

ggaggtgcag ggctacgtgc tcatcgctca caaccaagtg aggcaggtcc cactgcagag 21240
gctgcggatt gtgcgaggca cccagctctt tgaggacaac tatgccctgg ccgtgctaga 21300
caatggagac ccgctgaaca ataccacccc tgtcacaggg gcctccccag gaggcctgcg 21360
ggagctgcag cttcgaagcc tcacaggtgg ccttcaccgt cattgaaacc ttctcttggt 21420
tattcagagc tgaccagggc cactgctaac caggggagg ctttgtgtgc attagaaatg 21480
gtgtcccttc tgggcagacg caggcagagc ccgggaagac gccctcagaa gattggaaaa 21540
agattcccct tcttcctggg aagttgtagc ttgcgtcagc acatataatt caatcgtgag 21600
aatgcaggct gggtttttgc ccccacttgg ctgagtgaag tgtacagtga acaacctatg 21660
taactatttg ctggccctgg agccgactct gccccagagt ctgggtgcca ggtgctttgc 21720
ccgcatggcc catttcagtc acgctgcagt cctgtcagga aaaaatcagt gttattctca 21780
ttctacatat gagaaaactg aggcttgcag atataagggc caaaagttac acagctagtg 21840
agtgatgggg ctgagtttca gactccacag tctcttaacc accaagcagc atgcccagag 21900
tagaggtgag aaggaaggag agagctgcgg tccacatgag catctggacc tagcatggac 21960
aactcactcc tccctggctc tcgctttgtt cttgttgcgg gtgtggtggt ggtgggactc 22020
aaagacggta aagatagctt tctctcctcc ctggggaatc tgggggttgt ttaaaaggcc 22080
tgctcctctt ttagaaggca ggagggcccc aagggaagca gaaggtgaca gaaggggaaa 22140
gggtcctctg atcattgctc accccacaga gatcttgaaa ggaggggtct tgatccagcg 22200
gaaccccccag ctctgctacc aggacacgat tttgtggaag gacatcttcc acaagaacaa 22260
ccagctggct ctcacactga tagacaccaa ccgctctcgg gcctgtaagc catgcccctc 22320
cctgctgcct cttctctcag acagcctgac cccagccgca aactcccaac ttacaaccca 22380
gtgcctgccc gccactgccc cagccgccta caccacccat ttcctccctc tctgtccctc 22440
ctgccatctc cctgtgcctc ttcatctctg gggttctctg tcttgtctcc ctctgcttat 22500
aggttgtgcc tctggtttgg gggcctctca gcctgtctgg gtccctccct tgctgtgcag 22560
ttggcctcgt ggcctctgct gctgtttgtg cctctctctg ttactaaccc gtcctctcgc 22620
tgttagacat ctctctcact gcctgtctct ggttctgtcc tcaggccacc cctgttctcc 22680
gatgtgtaag ggctcccgct gctggggaga gagttctgag gattgtcaga gccgtgagtc 22740
tcagggaggc ctggagtcag ggaaggggag ggctggggcc gggtggaatg caggtgtcat 22800

acaggtgaca tgggaggggt gggataacag gcttgggatg tctcccctgg gccaggtagt 22860
ctccctagaa ggtgatgctg atgagggtct ggtgcccagg gcgccactca gccctcatcc 22920
tgccctttgc ccaacagtga cgcgcactgt ctgtgccggt ggctgtgccc gctgcaaggg 22980
gccactgccc actgactgct gccatgagca gtgtgctgcc ggctgcacgg gccccaagca 23040
ctctgactgc ctggtatgtg cctctgcttt gtgcccaatg tgctctaccc cccaggatgc 23100
aaggggtggg caccctgcct ggtactgccc tattgcccct ggcacaccag ggcaaaacag 23160
cacagtgaaa gccagccacc tgtcccccca ggcctgcctc cacttcaacc acagtggcat 23220
ctgtgagctg cactgcccag ccctggtcac ctacaacaca gacacgtttg agtccatgcc 23280
caatcccgag ggccggtata cattcggcgc cagctgtgtg actgcctgtc cctgtgagtg 23340
ccagggagaa acacagtttt ctcattttgg tggggaggtt tgtttctgta aatgggagca 23400
tatggggagc actgtctgca tcttgctttg agagctggtc atgacagttc ctgccgagct 23460
gccttgttct ttcaacagct gtggagcagg tggcagtaag gagaggcagc taagagccca 23520
gacttgggag ccagactgcc tgggtttgaa acccagctct atcaattagt aggcacgtga 23580
ccctcttgct gtgcctcagt ttcctcatca gtaaaatggg ggcaagaata gtcccaactg 23640
cataagatgg ttataacatt tgaaagagtt aatatttgta aagctcttag aacggtgcct 23700
ggtatgtact aagtgctcct aaatgttagc ttttattcta tagcctggtg aggtcagttt 23760
tacctttcgt tttgtttttg agaccgaatt tagttagctc tatcgcagtg gcgcgatctc 23820
ggctcactgc aacctccgcc tcccaggttc gtgctattct cgtgtctcag cctcctgagt 23880
agctgggatt acaggcgccc accaccatgc ctcgctaaat tttgtatttt tagtagagac 23940
agggtttcac cacgttggcc agactggtct cgaactcctg acttcaggcg atccacctgc 24000
ctcggcctct gaaagtgctg ggattacagg cgtgagccac tgcacccgga cttttttttt 24060
tttggcagag tctcgctcca ttgcccaggc tggagtgcag tggtgcaatt ttggctcact 24120
gcaacctctg ccttccgcat tcaagcaatt cttgtgcctc agactcttga gtaggtggaa 24180
ctacaggcat gcaccaccat ggctgggtaa tttttgtatt tttagtagag acggagtttc 24240
actatgttgg ccaagctggt ctcgaactcc tgacctcaag tgatccaccc gccttggtct 24300
cccaaagtgc tgggattaca ggcatgagcc atcgtgcctg gcctagctca gttttattta 24360
acagatcacc tatttactga tgggcgttta tggactgggc tcagacctgg ggaacctctt 24420

tcctcctctc acaggaacag gagtgggcct tcagatcctg gctgactgtg ttagggagag 24480
gacaaaatgt agagccagac catttgggtt caaatcctcg ctcctccact cactagcaca 24540
atgaccttga ataatttaca gaactctctg ctttggtctc ccttttgca aaatgggaat 24600
ctcacagtgc tgatcccgtc tggttgttgt gaggggtaaa tggatgtcag gtgctgatgc 24660
gtggtagggc atttaagtat tggttgatat tattcttctt gtgcctgggc acggtaatgc 24720
tgctcatggt ggtgcacgaa gggccagggt atgtggctac atgttcctga tctccttaga 24780
caactacctt tctacggacg tgggatcctg caccctcgtc tgccccctgc acaaccaaga 24840
ggtgacagca gaggatggaa cacagcggtg tgagaagtgc agcaagccct gtgcccgagg 24900
tacccactca ctgcccccga ggccagctgc agttcctgtc cctctgcgca tgcagcctgg 24960
cccagcccac cctgtcctat ccttcctcag accctcttgg gacctagtct ctgccttcta 25020
ctctctaccc ctggcccccc tcagccctac aagtgtccct atatcccctg tcagtgtggg 25080
gaggggcccg gaccctgatg ctcatgtggc tgttgacctg tcccggtatg aaggctgaga 25140
cggccccttc cccacccacc cccacctcct cagtgtgcta tggtctgggc atggagcact 25200
tgcgagaggt gagggcagtt accagtgcca atatccagga gtttgctggc tgcaagaaga 25260
tctttgggag cctggcattt ctgccggaga gctttgatgg gtaagagtgg gcacgatgac 25320
ctgagacagt gtcagggcag acagagtcct gaggatccag atgtggcagc atctcttggg 25380
gatggcagga gacagaagtg gggggatcaa gaatgcaaag aaagcagatg ggagaccaga 25440
ggagcagggc ctttggtggg tgggggtgat tatttttgta aatgacatgc tatccgtgaa 25500
caaggacttg tatggaggtc agaccatcta gataaagtaa aattcccttt gagttcatag 25560
cagctttatt caaaatatcc ccaaattgga aataactcaa atgtgcatca ctaggtgaag 25620
gaataaacaa gtggcagtgt atccatttgg tgaagttcta cttagcaacc aaaggaaatg 25680
aactaccgat acaacataaa tgaatctcag aaacattaca ttgagcaaaa gaagccagag 25740
acaagattcc atactgtctg atcccctttta tgtgaggctc tgaaccgaaa aaaccactct 25800
gtggtgggag agatcagaac ggtggttgcc ccagggtggg gggcttcaaa agggaggcac 25860
acaaggacat ttctggggta atagaaatgc tctgtatagt gattggggta gtggatacat 25920
gagcgaatcc atttgtcaaa actcatcaaa ctgtgtgata agagtctgtg cattttattt 25980
atttcatttt attttttgag atagagtctc actctgtcag caggctggag tgcagtggta 26040

cgatcttggc tcactgcaac ctctgcctcc tggattcaag caattctcct gcctcagtct 26100
cctgagtagc tgggactaca ggtgtgtgcc accatgccca gctaattttt gtatttttaa 26160
tagagatggg gtttcaccat gttggcaagg atggtctcga tctcttgacg tcgtgatccg 26220
cccacctcag cctcccaaag tgctgggatt acaggcatga gccaccacac ccggtgcatt 26280
ttattgtata taagttatac ttcaataaga aatgaattgg ggccaggcac ggtggctcac 26340
gcctgtaatc ccagcacttt gggaggccga ggcaggcaga tcacttgagg tcaggagttc 26400
aagaccagcc tggccaacat ggtgaaaccc catctctact aaaaaatata aaaaattagc 26460
caggcttcct ggcatgcgcc tatcatccca gctacttggg aggctgaggc aggagaattg 26520
catgaactcg ggaggtggag gttgtagtga gctgagattt cgctattgca ctccagcctg 26580
ggcgacagag tgagaccctg tctcaaaaag aaaaaaaaaa aaaagggtca ggcgccgtgg 26640
tgcacacctg taatcccagc actttgggag gctgaagcag gaagattgct tgagcccagg 26700
aattcaagaa cagcgtgggc aacatagtga gatcccatct ctacaaaaaa acacaaaaaa 26760
ttagccgggc atggtggtac gcacctgtag tctcagctac tagggagact gaggtgggag 26820
aatcacctga gcctgggagg tggaggttgc agtgggttga aatcatgtca ctgtactcca 26880
gcctgggtga cagaatgaga ccctgtctca aaaaaaaaaa aaaaaaaaaa attccctttc 26940
acacttcctt tacctccact cccctttcca gaggggggcca tggttaacag tgtgtgtgtt 27000
cacctagacc gtttatgcat ctgtagacac acacacagtg aagtgtggtt ttcgtcgttt 27060
tggtggggag gttggtttct gtaaatggga acatataggg agcactgtct gcaccttgct 27120
ttgagagccg gtcatgacag ttcccattga actgccttgt tctttcaata gctgcagagc 27180
aggtggcggc aaggagaggc agctaagagc ccagacttgg gagccagact gcctgggttt 27240
gaaacccggc tctaccactt actaggcatg tgacccttgt gctgtgcctc agtttcttca 27300
tctgtaaagt gggggcaaga acagtcccaa cttcataaga tggttatacc accatgcctg 27360
gccagatgat tataaagttt gaatgagtta atatttgtaa agctcttaga acagtgcctg 27420
gcagatacta ggtgctccta aatgttggtt tttattatgt ggctgggtgg ctcggggttt 27480
tatttaacag ctcccctatt tactaataga catttagatc atgttccatt ttcactctta 27540
caaacagttc cactttgtgt gtggctctgg gaacatgggc cagtgtctcc ctaggccaca 27600
ttcctagaaa taagatttct tttcttttt ttttttttt gagacagagt ctcgctttat 27660

cgccaggctg gtgtgcagta gtgtgatctc ggctcactgc aacctctgcc tcccgggttc 27720
aagtgattct cctgcctcag cctctcgagt aactgggact ataggcgcgc ggcaccacac 27780
ccagctaatt tttgtatttg tagtagagat ggggtttcac catgttggcc aggatggtct 27840
ccatctcttg acttcgtgat ccgcccgcct cggcctccca aagtgctggg attacaggcg 27900
tgagccactg agcccaggca gaaataagat ttctagatca aaggatataa atactgtttt 27960
gatagatgtt gccgaactaa ggcctgggct ttgaagccca ggatgggaac agctgggctc 28020
gatgggcaaa gggtttgagt gaaggcattc atggtgggga gtggctggca tggccagtgc 28080
tgggagtgat gtccaccctg ttcctggccc tgctgactcc tctcctgacc cctccaggga 28140
cccagcctcc aacactgccc cgctccagcc agagcagctc caagtgtttg agactctgga 28200
agagatcaca ggtgggctct gtctctgcat cctgttctgc aggggctggg agtccttgtc 28260
ctgtccccac tcctttaatc tcaccctctg cctgcaggtt acctatacat ctcagcatgg 28320
ccggacagcc tgcctgacct cagcgtcttc cagaacctgc aagtaatccg gggacgaatt 28380
ctgcacaagt gagcactgag aaagaggggg cctgatgggg aggagtccca gggaggagtc 28440
cctgtgggaa gctttgggcc tgagggagta ctcctgtagc agtaaccttt ccatgaaagt 28500
ctgcagagtg tgctggggat ggaggaagat gagaatagcc tttgctgacc gggaaggggt 28560
ccgtggtaag gtgcccacct ttctcccata gtggcgccta ctcgctgacc ctgcaagggc 28620
tgggcatcag ctggctgggg ctgcgctcac tgagggaact gggcagtgga ctggccctca 28680
tccaccataa cacccacctc tgcttcgtgc acacggtgcc ctgggaccag ctctttcgga 28740
acccgcacca agctctgctc cacactgcca accggccaga ggacgagtgt ggtaagacag 28800
ggagcccagt gtgcgcactc cccatctgcc agcacacagc agtgcccagg gggccctggc 28860
agcagcgttc ttggacttgt gcagactgcc cgtctctgtg cacccttctt gactcagcac 28920
agctctggct ggcttggcct cttggcatgg cttctctagc tgggtcctac ctgccttggc 28980
atccttccct ccccctctgt ttctgaaatc tcagaactct tcctctccct acatcggccc 29040
cacctgtccc cacccctcca gcccacagcc atgcccacag ccagttccct ggttcacttg 29100
gacctggggc ctcccctaaa agtcccctgc ggtcccttcc tcctcactgc agtgggcgag 29160
ggcctggcct gccaccagct gtgcgcccga gggcactgct ggggtccagg gcccacccag 29220
tgtgtcaact gcagccagtt ccttcggggc caggagtgcg tggaggaatg ccgagtactg 29280

caggggtatg aggggcggag gagagggtgg ctggaggggt gcatggggct cctctcagac 29340
cccctcacca ctgtcccttc tctcaggctc cccagggagt atgtgaatgc caggcactgt 29400
ttgccgtgcc accctgagtg tcagccccag aatggctcag tgacctgttt tggaccggtg 29460
agctgctggc gggctcagag ctgggtggag gggggcagcg agggggattg ccagggactt 29520
ggcaggatgg cgagatgcag tagggtgtgc tatctggtaa aatatccctg gagagggctc 29580
agcgctcaga cctgaacagc aacagagtgg cagaaaaggg gcctggggga cactggggcc 29640
cttcagacta tgaaaaggtt ctaaggaggt ctgtgttggt ggctgtgact gtggctgtgc 29700
tagggtggtg agccctgtgg gctcaggcgt cagactacct ggattcagac ccagctcctg 29760
cttccaacct tggtttttta ttcctaaaat gggtattgta ataataccta ccttgctggg 29820
gtgtggcaag aatgaaatta aacagggctt ggcacagtga agcacgggaa aggctttcta 29880
cagagcagtg actgttgtta ctcgctgtta caccttaggt aatgcgtttt cctctctggg 29940
tgcctcccat tttctggctc aagtccctgc ccaggatcaa gcttggagga gggccccgag 30000
ggaggggcca cagagactgg gtgaagagca agggtgtttg tcccaggagc atggcgaaaa 30060
ttgctgctgg gtggccttgg gaagcacaaa ggggacccaa ctaagggcct gatcctactg 30120
ccctgggggt gtcagtgcca gccccccaca aatcttttct gcccccccca ggaggctgac 30180
cagtgtgtgg cctgtgccca ctataaggac cctcccttct gcgtggcccg ctgccccagc 30240
ggtgtgaaac ctgacctctc ctacatgccc atctggaagt ttccagatga ggagggcgca 30300
tgccagcctt gccccatcaa ctgcacccac tcgtgagtcc aacggtcttt tctgcagaaa 30360
ggaggacttt cctttcaggg gtctttctgg ggctcttact ataaaagggg accaactctc 30420
cctttgtcat atcttgtttc tgatgacaaa aataacacat tgttaaaatt gtaaaattaa 30480
aacatgaaat ataaattaat gccctagcag ttctatcccc actgttaata atttgaaata 30540
tttttcctct agttattttt gtctgtgcac attctaatat gtatatataa gttaacatat 30600
attaatatta ttctccagtt atttttatct gtgcacattt taacacacac acacacacac 30660
acacacacac acatatgtat ttttagacgg agtttcactc tgtcgcccag gctggagtgc 30720
agtagtacaa tcttggctca ctgcagcctc cacctcctgg gtttaagcaa ttctcctgct 30780
tccgcctcct gagtagctgg gattacggga acgtgctacc ttgcctggct aatttttgta 30840
tttttagtac ataggatttc accatgttgg ccaggctggt ctcgaacccc tgacctcagg 30900

tgatctgcca gcctcggtcc cccaaagtgt tgggattaca gcggtgagcc accatgccca 30960
gtcatatatt tctttttaac aaatagaatc atagatcata catattgttt gcaaattgct 31020
ttttctcact ttccagaacc ttgaaatgtt tttccatgtt ctaacatggt gatctacctt 31080
attcttttaa tttttcttat ttagttgtct ttacacatga aacacatgaa tacatccttg 31140
tgataaacat tttcagtaac ataaaagtat aaatgttaca aagccaacgt gccctttcac 31200
tcaactccct gtccacccag tctctcctgt ctgctgggag aaccaccgca ttgacttgtg 31260
tgttcaccct tccaggctct tttctgcaca cttatataga catactacat ttatattagg 31320
tcgagtcaaa taagattgct gtttgtgtaa accaaaaagt gtcaagagcc tgggcgcagt 31380
gactcacacc tgtaatccca gcactttggg aggctgaggc aggcagatca cttgagatca 31440
ggagttcgag accaatctgg ccaacatagc gagaccccgt ctctactaaa aatacaaaaa 31500
ctagccaggt gtggtgatgc tgttctgcac tttgctttcc ccccgacttg aggtatcctt 31560
tcttgtgagt acagacggat ctaccacctt tatttttttt ttaattactc aacctgtaac 31620
atggatgtaa tttcactttg tttttgaggg atattgagct tgtttccctg tttttgcagt 31680
ttattgcaat tgagctccac acacaagtga gccctctttt gtatgccccc tagtgggaat 31740
acagtgctgg caatgtttat cacaaggata tattcatgca tttcaattta aagacaacta 31800
aatgagaaaa attaaaagaa tatggatcca ggctgggcat ggtggctcac gcctgtaatc 31860
ccagcacttt gggaggccga ggcaggcaga tcacctgagg tcaggagttc aagaccagcc 31920
tggccaacat ggcaaaaccc cgtctctact aaaaatacaa aaattagcca ggcgtggtgg 31980
tgggcgcctg taatcccagc tatttgagag gttgagacag gagaattgct tgaacctggg 32040
cagcggaggt tgcagtgaga cgagattgca ccagtgcact ccaacctggg caacacagtg 32100
caactccttc tcaagaaaaa aaagaaaaaa aaaaagaata tgggtccaga tccatatgga 32160
tcctagatcc agatcacggt gttagaacat ggaaaaacat tgcaagattc tgctaagtga 32220
aaaaagcatt tgcaaacagt atgtacagtc tatattcaga ggaggaactg ctgggtcata 32280
gatgatattt cataggtatt gccaaaccgt tctctggaga agtggtatgg gtttaccctg 32340
ggattcttct atggagggaa tagttgagct cccgggcttg ctcttctggg tgcccctccc 32400
cgcttcctat ccaccacaag gagctgcagg ggagcggggc atgccggttc cttggctgga 32460
gaaggagtct ccttgtgagg tggtagaagg agcactgacg gccttgagcc cagtttctgc 32520

ctttgtcaaa tggggataat gacccagcca cacccctccc agggttgttg tgaggctgga 32580
aaggtggttc ccaagagggt ggttcccaga attgttgatg agactgtttc tcctgcagct 32640
gtgtggacct ggatgacaag ggctgccccg ccgagcagag agccaggttg gcctggaccc 32700
caggatgtac ccttcattgc ccttcactcc cccactggat gctgggtggt cactgctgta 32760
gggaggggac cccctgacat atgtcccttc ccacccactc ttccactgtg gaacctcctg 32820
tcattttcca cttcaccaag tgacagagga cctgctcaga tgctgagggg aggggactgc 32880
aaggaaagat ggctaggaaa cccagtccct ccacacccta gagtaacttg atgccttgtg 32940
agggacacag gcaaagttca attccttgga agtcaaggga gactgagaag agtacagctg 33000
cagcactgag ggagtgatga attcttaact ggggatggtg ggaggcttcg agtgggaggt 33060
ggcatttgag ctaggctttg agagaggagc aggtattgca cttgcattta ggtagaaagc 33120
attggggtgc aaggtgacac tggaggggga ggcatcagga aatccaggat gtcttcaaag 33180
ttctggtgtc gggggctgtt gagtaagcac aggaataagg gggtcaagtt agagtcaggg 33240
tggggtctga cctggatgcc ataggacctg atccccaagc cacagggtgg gacttgactg 33300
ggcagtgggg acctttggaa aggactttgg ggagaaaaac agactggagt ctgtcttagg 33360
cgatcatcgg tccgtgaaat gagcatgtgt tacaggcttg gtatgtacca gaccctgtgc 33420
taagcaaggg ggtatggaga ggagagggtg acaagaatat tggatcaaca cccgggagct 33480
ccatctatcc caggatgcac tatctttttt ttattttttt gagacggagt ctcactctgc 33540
ctgcaggctg gagtgcagtg gctccatctc ggttcactgc aacctctgcc tcctgggttc 33600
aagcgcttct tgtgcctcag cctcccaagt agctgggatt acaggcacat gccaccacac 33660
ccagctaatt tttgtatttt tagtagagac ggggtttcac catgttggcc aggatggtct 33720
cgatctcttg acctcaagat ccgcccacct tggcctccca aagtgctggg attacagaca 33780
tgagccaccg tgcccagcca gatacgctat ctttttattg agtgattgag acagggtctt 33840
gctctcttgt ccagtcttga atgtggtggt gtaatcacag gctcactgca gccttgacct 33900
cctgggctca agttaccctt ctgcagtagc tgggactata ggagcgtgcc accacgcctg 33960
ggtaatttaa aaaatttttt ttgtatagac agggtctcac tatgttgccc gagctggtct 34020
caaactcgtg ggctcaagtg atcctccagt tttggcctcc caaaatgttg ggatcacagg 34080
agtgagccac cactcctggc gatgagccaa gtcttttttt tttttttttt tttttgatat 34140

ggagtcttgc tctgttgccc aggctggagt gcaatgacac gatcttggct cactgcaacc 34200
tctgcctccc aggttcaagc agttcaagca atcctcctgt ctcagccccc cagtagctgg 34260
gattacaggc atgcgctacc acgtccggct aatttttgta tttttagtag agatgaggtt 34320
ttgccatgtt ggccaggctg gtcttgaact gctgacctca ggtgatccac ctgcctcggc 34380
ctcccaaagt gctgggatta caggtgtgag ccatcgtgcc tggcggagcc gagtcttaaa 34440
agatgaccct gtggagaaat ggtggtccag gctgaaggga cagcctatgc aaacactggg 34500
aggtgtggaa aatcatgacc tgtgggtgga aattttggct agaacatcaa aatcatcagg 34560
tgtacattcc tgtacccatg cagcagtcag aatctctggg ggtggggccc caaaattgta 34620
tgcatacaga ctgtgtgctg atttgtgata ttacttagga ttttttgact ttacaatggt 34680
ggaaaagcaa taatatacat tcagtataaa ccgtactttg aatacccata cagccattct 34740
gtttttcact tttattttta tttatttatt tatttattat ttattttgag atgtcatttt 34800
gctgttgtta cccaggctgg agtgcaatgg cgcagtcttg gctcaccgca acctccacct 34860
ctcaggttca aacgattctc ctgcttcagc ctccagagtg gctgggatta caggcaggca 34920
ccaccacacc cggctaattt tgtattttta gtagagacgg ggtttctcca tgttagtcag 34980
gctggtctcg aactcgagag ctcaggtgat ctgcccatct cagcctcaag ccaccatgcc 35040
cagccctact ttcagtattc aataaattac atagccaggc accgtggctc acacctgtaa 35100
tcccagcact ttaggaggcc aaggtgggag gatcctttga ggccagaagc tcgagaccag 35160
cctgggcaac atagtgagac cccatttcta caaaaaataa aaaaactagc tgagtgtggt 35220
ggcgtgtgtc tgtagtccca gctacttggg cagctgaggt ggaaagactg cttgagccca 35280
gaggtcaggg ctgcagtggg ccatgatctc accactgcac tcagcctggg caacacagca 35340
aggccctgtc tcaaaaataa ataaataaat aacacaaact tatttaacag tttactataa 35400
aataggcttt gtgtcagatg attctgccca actgtaagct gctggcagtg taaatgttct 35460
gagcacgtgt aagccaggct aggtgtctta aatgcatttt cagtttcaac ttagaattgg 35520
tttatcagga cgtagcccct tggtgttgag gggcatgtgt attaacagtc tccttagtga 35580
cttttttttt tttgagatgg agtcttgcac tggccgtagt gcagtggcac aatctcagct 35640
cactgcaacc tcttgtctcc cgggttcaag cgattctcct gcctcagtct cccaagtagc 35700
tgggattaca ggcacccaca ccacgcccag ctaatttttg tgtgtgtgta tttttagtag 35760

agacgggggt ttcactatgt tggccaggct ggtctcgaac tcctgacctt gtgatctgcc 35820
cacctcagac tctcaaagtg ctaggattcc aggcatgagc caccgcgccc agagtcctta 35880
gtgattttta caccatgaat tgttgaagcc ctaagccaga gccaagggca agagtataga 35940
gaatctggag atgcggagag ggttctgatt gcctacaagg agtttggact ttattgtgga 36000
ggcagcgggg agccaaggca ggttttagag taggagaggg tccaagcctg tgggtcaccc 36060
ttccgacttc cctttccgaa tgccaaacac cttcatgtcc cccgtgggcc ccctttgtcc 36120
ctcccacccc aaactagccc tcaatccctg accctggctt ccgcccccag ccctctgacg 36180
tccatcatct ctgcggtggt tggcattctg ctggtcgtgg tcttgggggt ggtctttggg 36240
atcctcatca agcgacggca gcagaagatc cggaagtaca cgatgcggag actgctgcag 36300
gaaacggagg tgaggcgggg tgaagtcctc ccagcccgcg tggggtctgc accggccccc 36360
ggcactgacc caccaccccc tcaccccagc tggtggagcc gctgacacct agcggagcga 36420
tgcccaacca ggcgcagatg cggatcctga aagagacgga gctgaggaag gtgaaggtgc 36480
ttggatctgg cgcttttggc acagtctaca aggtcagggc caggtcctgg ggtgggcggc 36540
cccagaggat gggggcggtg cctggagggg tgtggtcggc agttctgatg ggaggggcaa 36600
gagctggagg cagtgtttgg gggagggcag ttacagcgga gaagggagcg gggccaagcc 36660
ctagggtggt gaaggatgtt tggaggacaa gtaatgatct cctggaaggc aggtaggatc 36720
cagcccacgc tcttctcact catatcctcc tctttctgcc cagggcatct ggatccctga 36780
tggggagaat gtgaaaattc cagtggccat caaagtgttg agggaaaaca catcccccaa 36840
agccaacaaa gaaatcttag acgtaagccc ctccaccctc tcctgctagg aggacaggaa 36900
ggaccccatg gctgcaggtc tgggctctgg tctctcttca ttggggtttg gggagatatg 36960
actcccgcaa acctagacta ttttttttgga gacggagtct tgctctgtca cccaggctgg 37020
agtgcagtgg cgttatctcg gctcactgca acctccacct cctggactca agcgattttc 37080
atgcctcagg ctcctgagta gctgggatta caagcgcccg ctaattttttt tttttttttt 37140
gagacagagt ctcgctctgt cacccaggct agagtgaaat ggtgcggtct cagctcagcc 37200
tcccaggtta aagcgattct tctccctcag tctcctgagt agctgggatt acaggcgcga 37260
gccaccacgc ccggctaatt tttgtatttt tagtagagat gggatttcac catgttggcc 37320
aggttggtgt caaactcctg acctcatgat ccgcccgcct cggcctccca aagtgctggg 37380

attacaggtg tgagccaccg tgcccggcct aatctttgta tttttagtag agacagggtt 37440
tcaccatgtt gtccaggctg gtactttgag ccttcacagg ctgtgggcca tggctgtggt 37500
ttgtgatggt tgggaggctg tgtggtgttt gggggtgtgt ggtctcccat accctctcag 37560
cgtacccttg tccccaggaa gcatacgtga tggctggtgt gggctcccca tatgtctccc 37620
gccttctggg catctgcctg acatccacgg tgcagctggt gacacagctt atgccctatg 37680
gctgcctctt agaccatgtc cgggaaaacc gcggacgcct gggctcccag gacctgctga 37740
actggtgtat gcagattgcc aaggtatgca cctgggctct ttgcaggtct ctccggagca 37800
aacccctatg tccacaaggg gctaggatgg ggactcttgc tgggcatgtg gccaggccca 37860
ggccctccca gaaggtctac atgggtgctt cccattccag gggatgagct acctggagga 37920
tgtgcggctc gtacacaggg acttggccgc tcggaacgtg ctggtcaaga gtcccaacca 37980
tgtcaaaatt acagacttcg ggctggctcg gctgctggac attgacgaga cagagtacca 38040
tgcagatggg ggcaaggtta ggtgaaggac caaggagcag aggaggctgg gtggagtggt 38100
gtctagccca tgggagaact ctgagtggcc acctccccac aacacacagt tggaggactt 38160
cctcttctgc cctcccaggt gcccatcaag tggatggcgc tggagtccat tctccgccgg 38220
cggttcaccc accagagtga tgtgtggagt tatggtgtgt gatgggggt gttgggaggg 38280
gtgggtgagg agccatggct ggagggagga tgagagctgg gatggggaga attacggggc 38340
cacctcagca tgtgaaggga gggaaggggc tgcctgtgcc ccaccttgca gggtctgtgc 38400
acttcccagg attagggaaa gaccgggtag ggtctgtctc ctggcatcac atctccccct 38460
gctacctgcc atgatgctag actcctgagc agaacctctg gctcagtaca ctaaagctcc 38520
ctctggccct cccactcctg accctgtctc tgccttaggt gtgactgtgt gggagctgat 38580
gacttttggg gccaaacctt acgatgggat cccagcccgg gagatccctg acctgctgga 38640
aaagggggag cggctgcccc agccccccat ctgcaccatt gatgtctaca tgatcatggt 38700
caaatgtgcg tggctgagct gtgctggctg cctggaggag ggtgggaggt cctgggtgga 38760
ggagcccaca aggggcatga aaggggacca ggatgtatgt agacccagga gccctagtat 38820
gttaggagcc tcaaaacctt cttgtatccc ttttacagtc aaagtccaaa gccactcttg 38880
aggaacactc ttgtacaaaa ttaagctggg cacagtggct catgcctgta atcccagtac 38940
ttttggaggc tgaggtggga ggatcccttg aagccaggag ttcaagacca gcctgggcaa 39000

catagtgaga tcctatctct acaaaaaata aaaaaattat ctgggtgtgg tggtgtgtgc 39060
cagtagtccc agctactcag gagaggctga ggcaggaaga tcacttgagc ctagtttaag 39120
gttgcagtaa gctatgattg caccactgaa atccagcctg ggtgacagag cgaaacctca 39180
tctcaaaaaa ataaaaaagc aaacaaaaag aaaaaaaaaa ttaaaaggga aactagaaga 39240
gatgccaaag gttctggctg aagaccccag agtctggtgc tacttctcta ccacctgagg 39300
gctttgggct gtcccttggg actgtctaga ccagactgga gggggagtgg gaggggagag 39360
gcagcaagca cacagggcct gggactagca tgctgacctc cctcctgccc caggttggat 39420
gattgactct gaatgtcggc caagattccg ggagttggtg tctgaattct cccgcatggc 39480
cagggacccc cagcgctttg tggtcatcca ggtactgggc ctctgtgccc catccctgcc 39540
tgtggctaag agcaccctcc tgcagagggt gggaaggaga gatgagtcca gtatgccagg 39600
cccctcacgg aaggctgcat gctgggctgg ggaggggcca ccatcctgcc tctccttcct 39660
ccacagaatg aggacttggg cccagccagt cccttggaca gcaccttcta ccgctcactg 39720
ctggaggacg atgacatggg ggacctggtg gatgctgagg agtatctggt accccagcag 39780
ggcttcttct gtccagaccc tgccccgggc gctgggggca tggtccacca caggcaccgc 39840
agctcatcta ccagggtcag tgccctcggt cacactgtgt ggctgtctgc ttacctcccc 39900
caaccccggt ggactagggt ccctttctct gatgttccct caactgtcac ctctcaagga 39960
aaccccatta tccctacaaa aaattcttac tgccttccaa cccctgtgac cccattctct 40020
ccacggtgac tgtgtcatac cccaaaggtg acctctgttt ttctcctgtg accctgtcac 40080
cttccatgga gtccccatcc cagatccgtg agtgaccccc atcatgactt tctttcttgt 40140
ccccagagtg gcggtgggga cctgacacta gggctggagc cctctgaaga ggaggccccc 40200
aggtctccac tggcaccctc cgaaggggct ggctccgatg tatttgatgg tgacctggga 40260
atgggggcag ccaaggggct gcaaagcctc cccacacatg accccagccc tctacagcgg 40320
tacagtgagg accccacagt acccctgccc tctgagactg atggctacgt tgccccccctg 40380
acctgcagcc cccagcctgg tatggagtcc agtctaagca gagagactga tgggcagggg 40440
aggtgggacc ttcagcccag ggtccactgt gggggcagag ggagtggcag agacaccggg 40500
gttccttccc ctaatgggtc accttctctt gacctttcag aatatgtgaa ccagccagat 40560
gttcggcccc agcccccttc gccccgagag ggccctctgc ctgctgcccg acctgctggt 40620

gccactctgg aaaggcccaa gactctctcc ccagggaaga atggggtcgt caaagacgtt 40680
tttgcctttg ggggtgccgt ggagaacccc gagtacttga caccccaggg aggagctgcc 40740
cctcagcccc accctcctcc tgccttcagc ccagccttcg acaacctcta ttactgggac 40800
caggacccac cagagcgggg ggctccaccc agcaccttca aagggacacc tacggcagag 40860
aacccagagt acctgggtct ggacgtgcca gtgtgaacca gaaggccaag tccgcagaag 40920
ccctgatgtg tcctcaggga gcagggaagg cctgacttct gctggcatca agaggtggga 40980
gggccctccg accacttcca ggggaacctg ccatgccagg aacctgtcct aaggaacctt 41040
ccttcctgct tgagttccca gatggctgga aggggtccag cctcgttgga agaggaacag 41100
cactggggag tctttgtgga ttctgaggcc ctgcccaatg agactctagg gtccagtgga 41160
tgccacagcc cagcttggcc ctttccttcc agatcctggg tactgaaagc cttagggaag 41220
ctggcctgag aggggaagcg gccctaaggg agtgtctaag aacaaaagcg acccattcag 41280
agactgtccc tgaaacctag tactgccccc catgaggaag gaacagcaat ggtgtcagta 41340
tccaggcttt gtacagagtg cttttctgtt tagtttttac ttttttgtt ttgtttttt 41400
aaagatgaaa taaagaccca gggggagaat gggtgttgta tggggaggca agtgtggggg 41460
gtccttctcc acacccactt tgtccatttg caaatatatt ttggaaaaca gctaggcacc 41520
ggcctatgtc tgggggtggc tctgtgccat cccttcctgc tcaccttaca ctcaattcct 41580
cttttcctgg agggagtggc tgggaatctt cagaaagctt ggatgaggag ccagacatcc 41640
agttctccag cttcagggtt gggggagatc gggaagctgg attcagatct gagagccctt 41700
tgacgaccag atcattctta tttagaacga actaattcct aaggccactc accagaatgg 41760
ggttatttcc tccttgtgag tgaggagagt ggtgggtagg gtgggggaaa gacgaggcag 41820
ggtctactgt gggactcatt ttccttgcct tggctatgcc agaggagctc actgacctaa 41880
ggagtagctg tccagaaagc ccacccagcc agcacagcca ccctggcggt gcaaaggctg 41940
ggctgaccgg acttctgtgc cccccacatt cccagactgg acgtataaaa acacacacta 42000
gttagcatta gtgtttgtag cgccacttta ctgccaatag ctgacattgc cctgggttag 42060
gggagaataa ataaaatctg tggcatcaga caggtattac cgaggcgaag agtggactgg 42120
gctttcgtgg gcacttaccc tgggaagggg gtatgaggtg gctggagaag tgttcatgga 42180
gagtgtctct ctcctgcccc caaggccacg gaatcttcta ttccttcttt gtacccaaag 42240

ggcaaagtgg aggccagggt ctctttgcta aggagctaag taggggaaag aggcaggggg 42300
agctcccagc aggaccaaag ggagaccaag gtttggaccc cagaacagag caggaaccca 42360
gagtcctgtg cagtcacagg atgacgcagg gaggacggct gttggtgatc ttttctaggg 42420
tttctccatt actggctctt cggatggcct caatgagcta gaggagtgga atgacaggat 42480
gatgcactgt tggggtaggg tgaccaagag gtcctcccaa cgctgtaaat actcacatct 42540
ttctcatagg gaaagccccc attctccagc ttggagaaca ccagctgtcc atttatctct 42600
atctcaaagg cacctggtaa gaaatcaaca gataaatggt acactgagaa cataggcaga 42660
agtcaggagt tcctccaaag gggtcccccg ccttcaagtc ccccatcaat cccactggag 42720
acccatttgt agctccttaa gcaattcccc aaccctgggt caactccagg gaacctgagg 42780
gaggcctcgc ctgtgtgccc ctcgcaacac tcaataaagg actccctccc ctccaaccct 42840
acacgattgc agtctaatag gtttttcccc acaggcctct gtgacacccc ctgacctatg 42900
gaagggaact catgttggcc ggacatttta ccatgtgcca ggcactgcgg gcactttaca 42960
taaagcagcc aatgtcagaa ctagaaagcg ggagagctgg gattcagagc acagactgac 43020
ccagcaggtg ttctgcgagc ctcacctgtg cccccgaggc gcgactcgat ctcgatgccc 43080
ggatactgct ccttcacagc actggccagc tccaggtagg tcgcctcgaa gccgcagggt 43140
tcactgggga gtcaagagat ggggctgggc tggggattcg ggggtggggc ctcccgtgga 43200
cccacctccg tccggcccgc gggaccaggg tgcgggtcct ccagccgggg ccgctcacca 43260
gtactccacc acgatgcgga ccccactgcc cggctcgacc tcctcgggag ggggcgctac 43320
ggacgtctgc cccggctccc cgctcatcgc ggccggctcc gctcgggccc ctgcttccgg 43380
gtgtgacgcg aaccgcgggc acgtgacggg gcggggcctc tggagcgggg aggggccgag 43440
ggtggcgttc gggcgccccc tgcgggtctc cgccactgct acgttttgac ctcgtagggc 43500
agctccggag ctcgctcccc gggcaggggc tgggtccggt ccagaccctc cttccctggc 43560
ccggtctccc ggagtgccgg gcttccccac tccccactcc cggtgtccgc tggacttcat 43620
ggcttcattt actctaatgg actatcttag actgcaaagc gcttttcagt ttacaaaaga 43680
aagagaaagt ggtggggcaa gcatcctggg gatgggggtg ggtgtggctg atgcatgaaa 43740
tagttgacat tttctcgcta acatgccgat ggcatttttt tttttttttt ttttgagacg 43800
gagtttcgct cttgttgccc aggctggagt gcaatggcgc gatctcggtt cactgcaacc 43860

tctgcctccc gggttcaagc gattcttctg cctcagcttc ccgagtagct gggactacag 43920
gcgcgtgcca ccgcacccgg ttaattttgt attcttagta gagacagggt ttcatcatgt 43980
tggtcaggct ggtctcgaac tcttgacctc aggtgatcca ccagcctcgg cctcctaaag 44040
tgctggaatt acagacatga gccaccgcgc ctggcccagt tttttgtttg tttgtttgtt 44100
tgttttagta gagacagggt ttcaccatgt tcgccagact ggtctcgaac tcctgacctc 44160
aaatgatctg cctgccttgg cctcccaaag tgctgggatt acaggcgtga gccaccacgc 44220
ccggtgctga tggcatttgg aaccaccctg tagttacata cacaagtgtt tctgatccca 44280
aagctctaaa ctgcttttc cagtcttttg cagctgcttc ttgcctgcac tggaaaacag 44340
tagatgctct tcaggctctg tgccccccaac agtttctgcg catcccttct catccagcct 44400
ctcactgggg ggctggcgtc attattccca ttttgcagct ctggaaatgg agtctgcaca 44460
agtcctcgct gccttttccc tgcctacagt accactatgg tatcctaaca cgccacccac 44520
tctagtgtcc ctttaggcta tttatttatt tatttattta ttttgagatg gagtctcact 44580
ctgtcaccca agcttgagtg cagtggcgcg atcttggctc actgcaacct ccacctcctg 44640
ggttcaggcg attctcctgc ctcagtctcc ctagtagctg agatcatagg tgccagccac 44700
cacgcccagc taacttttgt attactatta ttattatttt tttgagacag agtcttgctc 44760
tgttggccag gctggagtgc aatggtgcga tctcggctta ctgcaacctc tgccttccgg 44820
cctcaagtga gtctcctgtc tcagcctcct gagcagctgg gattacaggt gcccaccacc 44880
aagccagcta atttttctat ttttagtaca gatggggttt caccatgttg gccaggctgg 44940
tctcgaactc ctgaagttgt gatctgccca tcttggcctc ccaaagtgct gggattacag 45000
gtgtgaacca ctgcacccgg gctcattttt ttttttttta aaccaggcag gtcgtagtgg 45060
ctcacacctg taatcccagc gttttgggag gccaaggtgg gcggatcact tgacgccagg 45120
agttcaagac cagcctggcc aactcgacaa aaccctgtct ctactaaaaa tacaaaaatt 45180
agctgggtgt ggtggcgcac cagctactcc agaggctgag gcaggagaat cgcttgaacc 45240
tgggaggtgg aggttgcagt gagctgagat ctcaccactg cactccagcc tgggtgacag 45300
agtgagactg tgttccaaaa aaaaaagaaa agaaaagaaa agaaagaaaa tgcagtgatt 45360
tgtactgttg cctaaccatc actacaatcc agttacagaa tgtttccgtc aacctgataa 45420
gtccttccca cctgtttctg gtcaatccag ctcccaattc cagtcctgtg caaccactga 45480

tctgctttct gtctctaact ttgccttttc tggatgtttc atataaacag aataatacac 45540
tatgtggcct tttgcttctg gttctttcat tcaatatgtt ttttgaaatg cgttcatcct 45600
gtagcgtcta gagtagtttg ttccttttca ttgctgagtc gtattccact gtgtggttat 45660
gccacatttt tttcttaatt caccagctga tggatattta gggttccagt tttttttgtc 45720
tttttttgaat aatgctgcta caaatgttta ggtgtctttg tataggtgtg tgttttcatt 45780
tctcttgggt agattcctaa gggtagaact gctgggtcct gtgtttaatt tatgattatc 45840
tttttttttt ttgagacagg gtctcgctct gttgcccacg ctggaatgca gcagtgcaaa 45900
catggctcac tgcagcctcg acctgctaaa ctcaagctat cctcttgcct cagcctcctg 45960
aatagctggg acgacagaca cgcaccacca tgcccagcta atttttttttt gtattttttg 46020
tagagacaag gttttgccat tttgcccagg ctagcattta acattttaag aaatggccag 46080
actgtttttc aaagttttcc caccagcaat atatgaggat ttgtctctcc atatcctcac 46140
caatacttgt tttctgtctc tttgattata accatcctag tggacatgac atggtatgta 46200
actgtggttt taattctcat tttatctaatg actatgtcgt gtatcttttc atgtagttat 46260
tagccattca tatacatatt atttgaagaa acatctattc aagtcttttg cccattttta 46320
tttgtctctt tattattgat ttgtaaaagt tctttatata ttttggatac caaccaaaat 46380
atatacaacc atatatacaa ccatatatag ttgtatatga tttgcaacta ttttctccca 46440
gtcttcggct agtttttttt ctttttcttt tctttctttc tttttttgag acaggggtct 46500
cactgtgtca cccaggctgg agtgcagtgg cacaatcttg gctcacttca acctccactt 46560
cctgggttca agagattctt gtgcctcagc ctcccaagca gctgaaatta caggcgtgca 46620
ccaccatagc ccagataatt ttttgtgttt ttagtagcaa tggccaggct gttttcgcta 46680
tgttggcaag gctggtctgg aactctggcc tcaagtgatc tggctacctc agcctcccaa 46740
agtgctggga tcacaggcat gagccacttc atctggcttc atttttttctt tcttttcttt 46800
gagacagtgt ctttctctgt cacccagact ggagcgcagt agcacaatca cagctcactg 46860
cagccccaac ttctcaggct caagtgattg tcacacctca gccccaccaa gtagctagga 46920
ctataggccc gcaccaccat gcctggatag attttatatc ttttgtagag atgaggtctt 46980
gccaggttgc ctaggctggt ctcaaactcc tgggctcaag cgattcacct gcctcagcct 47040
cccaaaatgc tgggattaca ggtatgagcc accatgcctg accccttctt tttttttttt 47100

tttttttttt tttttttcgt atggagtttt gctcttcttg cccaggctgg agtgcaatgg 47160
caccatcttg gctcactgca acttccgcct cctgggttta agcgattctc ctgcctcaga 47220
ctcccgagta gctgggatta caggcatgca ccaccatgtc tggctaattt tgtatttttt 47280
tttttttttt tttttttttt ttagtagaga tggggtttct ccatgttggc caggctggtc 47340
ttgaactccc gacctcaggt gacccatccg cctcggcctc ccaaagtgct gggattacag 47400
gtgtgagcca ctgcgcgcgg cctcctggcc tcttattaat ggtggctttt gaagtgcaaa 47460
aattttaaat tttgacaaag tctaacatca atttttttct cttgtcagtt gtctttttgc 47520
tgttatattc aagttctctg cctaacccaa ggtcatgaag actatctccc atggtttctt 47580
ctagaagttt tatggtttta gctcttacat tcaggtatct gatctatttc aagttttttt 47640
tttcttatgc atcctgtgag caaaagatct aaattcacct tttggcatgt ggctatccaa 47700
ttgatcacag cagcatttat tgaatgccta tcctttcctc catgaaattg tcttggcacc 47760
ttgtaaaatc aattgaccag aaatgtaagg gtttctactt ggactctgga ttctgttcca 47820
ttgacctata aacgcattct tatttattat tattattatt ttccccccgag acggagtctt 47880
gctctgtcac cctggctgga gtgcagtggc acgatctcag ctcactgcaa cccccgcctc 47940
ctgggttcaa gcaattctcc tgcctcagcc tcctgtgtag ctggaattat aggtgcacac 48000
caccatgccc agctaatttt tgcattttta gcagaggcgg ggtttcacca tgttggccag 48060
gctggtctcg aactcctgac ctcatgatcc acctgcctcg gcctctcaaa gtgttgggat 48120
tacaggtgtt agccaccgta cctggcctta tagttgttgt tgtttttaaa ttcagataca 48180
acttatacac agtaaaattc atcctcttga gtgtaccttt ctttttttttt tcttttcttg 48240
agatggagtc tcgctcttgt ctcccaggct ggagtgcaat ggctcgaact cagcacactg 48300
aaacctccgt ctcgtgggtt caagagattc tcctgcttca gcctcctgag tagctgagat 48360
tacaggcgta cgccaccacg cccggctaat ttttgtattt ttagtagaga cggggttttg 48420
ccatgttggt caggctggtc ttgaactcct gacctcgtga tccacctgcc tcagcctccc 48480
aaagtcctgg aattacaggg gtgaaccacg cacccagcct tgagtgtgcc tttctatgag 48540
tcctaagaaa cacacacatt tgtgcatcca accccaaaac caagatacag tatggtccca 48600
tcacctctca caagtccctg tgccccctac tgtggacccc tctccctcct ccccaacccc 48660
agacctggtt tttgttcaca cagttccacc cgctccagaa agccatataa gttgattcct 48720

acagcactcc gccctctgac tgtggctttg tttccatccc atccttcaat atccaggatg 48780
atctttccaa aatgtaaatg tgccctctcc tctgtgcttc tacttaagtc taaatacaac 48840
ttgcagggtc tggtctttga ccgctactcc tgcccatctg gacaaacagg atggatggct 48900
ctcaaacctg gtttgcagaa cagccacctg agggcttgtc aataccacac cttcctgggt 48960
cttagcccta gagactgaga ttcagttgct ggagaggagc ccaagaatct attttccaca 49020
caaggttagg gaggtctaga ggccacattt gcagaagcac agcttcctct ctccacacct 49080
tccacacctg tccaggttca cccacgtggc ctctgcttcc tcccttctcc tccaacccca 49140
ctctctgtct ccgcatgctg catctcctcc tgcccagagc tctggcattg atttcctcac 49200
tggggaaagt ggcctccttc cccaattccc atccaccgat tccaaccccc ttcccatgtc 49260
agccagcttc tcctctgctc agctgtcacc tccactgaga agtctttcct gactccctat 49320
gtgagtcatt ttcccagcat agttgtctcc tgtcatttct tatttgtgtg attctttggt 49380
taacatcaat cttcctgatt ggctgtgtga ggatgggcgg ggtctgtttt tgttgtttac 49440
gcctgtatcc ccagcacaaa acatggggct ggcatatggt aggtactaaa cagatagttg 49500
aataaatgaa tgagtgaatt gtctactctg cctttgcaca agctgctcca tcaccaaaga 49560
gaccttgcct cctcccccac ttctttgctg ggcccactca taccggtttt cctaggaaaa 49620
ctctggagaa aattgaggca ggagccactc attcgtgccc cttccagggt ggacctctta 49680
tcaccctaag gattgtttgt tttcctgtca cccacatcaa gctgggtggt ggccgcagcc 49740
tttgatctcc acctcctaac ccctctccca agtaccctgt gtttgcccag cactgaatga 49800
gacagctaaa gtgatccctg agagaactaa agtgttccct caggccgggc gtggtggctt 49860
atgcctgtaa tcctaacact ttgggaggcc gaggtgggca gatcacctga ggtcaggagt 49920
tcaagaccaa cttggttaac atggagaaac cccatcttta ctaaaaatac aaaaattagc 49980
tgggtgtggt ggcaggcgcc tgtaatccca gctactccgg aggctgaggt aggagaattg 50040
cttgaacctg ggaggcagag tttgcagtgc gccaagatgg caccactaca ctccagcctg 50100
ggtgacagag cgggactctg gctcaaaaaa aaataaaata aagtgatccc tctaagagga 50160
atccccaggg gcccaggcca gagctttctc caggtcacca tggctcctcc cactgtgcca 50220
ccctgcctaa ccccctcgat tttaaaggag aagccaagac tctggacttt gctcagaagg 50280
tggggggcgg gggagggagg gcttaccctg ggtacctctg aggggccagg gcgcagccac 50340

cacggtaagg gtgactgcag atcgaggggc aggccatgtt gctggacacc accctggctg 50400
gcgtccctct cggcagggtg ctctttgccc catggggtgg gatccagagc tgcagacagg 50460
cccccaggct tggccaatga acagaccagg ttcggggagg gtgttggaaa agagtggatg 50520
gggtggttcc ccttaccttg cagcccccag gccctccccc ctccctccca ggtggtcggg 50580
actcttgatc ttcgctcgtg gtactgtctg ttcggctgtc ttccccgcct ctccccaggc 50640
acctgcatcc tcccttggca cctgctgcca ggctaggaag ggcaaaaaca atcccagttg 50700
gcgtagtcag ggagtctccg ccctcctccc aggtttcctc ctcccaggcg cctcccctgg 50760
acccgccccc atctgcccaa gataatttta gtttccttgg gcctggaatc tggacacaca 50820
gggctccccc ccgcctctga cttctctgtc cgaagtcggg acaccctcct accacctgta 50880
gagaagcggg agtggatctg aaataaaatc caggaatctg ggggttccta gacggagcca 50940
gacttcggaa cgggtgtcct gctactcctg ctggggctcc tccaggtaag gggaccgaga 51000
tcggtctcag acgacagcct gccaggcaac ccccagccgc accagggccc caggctgtgc 51060
cagtctccgg ctgaactggc cggcggccca ggtgggagga ttggagccac ctggctctcc 51120
ccctcccccg ctcggtgatg tcaggccagg ggcgggggct ggaggggac aggtagttaa 51180
gggcctggcg tctccctccc tgaagacgtg gtcccagccg ggtgtcctga cgctcggggt 51240
tcaggtaaga acccaagcgg gcatttatcc tttgggggca ggcacggccg gtctggaggc 51300
cggtagggaa aggctgggtc tcctgatggg ttgcttggcc tcccactttt ctcttttctg 51360
tccatttccc accctgcccc ctcctcccca cccgccctct tttcgtcccc atcctggttt 51420
ccagcgctcc ttgttccctc cctgtacacc tccctgcagg cagcagctga gaatgggaag 51480
gaagacctga ttctctcctc cgacttggct ttcccgctga aactagcccc tccccgcaca 51540
cccggggaga gccgccctca ctctctggct cgcccagttt accccagttt gggtttcccc 51600
aagtctctaa gacagacgtc tctgcgggct gcggggagat gtggggaggg ccccctccac 51660
tttggagggc agtgaaggag agggatcctc taaattgtcg aggcttcatc tctccagatt 51720
gtatgccctt ctcagcaaca ccgcctccgg ccctccgatg ggaaagtgga ggccgggtga 51780
gacggggtcc ctcctggctt cagcatgaaa tgggcaggaa ggctggaggg gtgtatgagg 51840
ggctctgcag tgcggtgggg cctggggaga gtggggtggg ggatagcaga tggtttcttc 51900
tgcttgatga ggctagggag agacccaggt tcccggtctg ggtctccctg gcagattgag 51960

gacagagaat gaatgcatct gccggcaagg tggaacgccc cccaccccgc cccccaacta 52020
gccgcaccgc cccccaggaa tgccctcttt tggtccgccg gtcttggact ctgctctccc 52080
ctctcctgat atcctactgt atgcttggtg gggggacctc atggaggcgg gcagcctgta 52140
atgattaact gcagagacac tccagcattc ttcccgaatt aaactttaaa agagctgccc 52200
cgccttctcc cattgggtat ttttcctgag tgagagtggg cggggcctca gagtcctggg 52260
tcctccctta tactaggagt catccagggg tttctcacct tctcacctgg gccctgcacc 52320
ctaaaaaatt ataagcctct cccgtggggg cggggcgtgg agtactgggc ggacgggggc 52380
tctcccgcca catccaggcc cctggcccag gcctcagtcc cgcaagccct tccccctgct 52440
ctgggaagca gagttgtttt ctgttggccc tgaacaccgg gccccgccac ctgcccttag 52500
ggccagccag gtggggcgtg attcaggaag taaacaagga ggttgggagg acgggaacgc 52560
gggtggggct ggaaccactg ggggaggagg cagctgggaa gccacggacg gagttacctc 52620
gttgagggga caaactgatt ttgggggtcg gtgtttccaa ccgaagcttt accggggcct 52680
cagtttgaaa actccagatc tctaagctgc caggcccaca gccaggaccc cttcctcctc 52740
cctgctcacc ccattggtct tgacgattcc acccgcctcc agccttagaa gcttaagggc 52800
acggctgggg ggctctgaag gcaaacccca gccttggact ggccctctct gatctctgag 52860
gccaggctct aatgtgattt gaatctactt ctaacccctt ccaagcactg ccctcccgaa 52920
ttctctgctc ctctccccac cccactgttg gtctgtgatt tcgaggcagg cgtggccccc 52980
tgcagcctgg aatgaagtca ctggggctgt ttggagaccg gggctgtttg gaggttagac 53040
tggggtggga gtgggggatt gtgttctggt attgaggtgc tggtgtctct tgctagcggg 53100
tgatgggtgt gtcgtgcccc tcctggcagg accctgagcc ccctccctgg cctttcatcc 53160
tgtctgtctt cttgagtgtt ttccctttcc actcctgctc ctgtggtacc cctctggccg 53220
ggacacccag gagaatgtgt caggaggaac ttcatccaca cagggcaggg gctctgagcg 53280
atgctttggg tggggagggg caccgccggg atgctggctt ctctccaaat gggcctctga 53340
atccaggccg ggcattgcct ctctccttcc caggcgaggg actccctggc gaggaggtgg 53400
tttgggactg ggggtgagag cagatcgtag gtcccacctg gcctggagct aggccgaggg 53460
tggggaggag gaggtgatgt caggagcctc aggcccacat cctcctcccc catcctgttc 53520
ccagccccgt gcctccccca ttccgctctg gctagcagtg ggttggggag ttgggggaag 53580

ggtggaggca gggctgtttt tgtttctcct aagccaggaa gttgtgtgga tgagtcaagg 53640
ttgaatggag agccctaggc agtgttgtcc cgcccgccct gactcacctg tcccccaaat 53700
cccctgatgc cctcactgaa gggtcaggtg cctgctccct cctgaggggt caatcccagg 53760
tgccccaggt ggcctcatag tactggccca gtgtttggag aacaggctgg gggctgggat 53820
tgcaggttct cccaggtctc agcccggcca aaggggtctg ctgggttcct gcggagaagg 53880
tgccagcccc acctccacct ctagtgctcg tctttgcctg ctgtcctgtc tttacttgtc 53940
ttgagtgtct gtgcagcccc tataagtgat aagacatggg tgagattggc cttgcctggg 54000
ggtaccaggt gccctcagac aaatgggtgg ataggacagg tgcacagtaa tggatgatgc 54060
ggcccccgca cagtgctgag agagagtcat tcagtctgaa tgtggaaggc caggaaggct 54120
tctcagagga ggtggctcta gcactggggc ttcaggaaag agttgggcag gaggatggcc 54180
tggggagggt acgcaaggtt tgggggctgc tggggccagg acccctaccc caggccctgc 54240
tctccccctt gcactgcagc ggagcccgct ctcctgcctt tctgactccc atccttcccc 54300
acctccttct cccctctgca tcactcatca cttgggcagg ccaaacctcg ccaagtatga 54360
acaggctctg gcaactgggt ctggcaacaa agtgctggaa aaacccgggg cacagcccag 54420
cgcagtgagt gccccacacg tggaggcaaa ggagtgggcc tttgccctga ccctgagggc 54480
tgagcctgag agtctgtggg ttgtgaaatc tgttgtgacc accaggcttg tggtgggtgg 54540
accctcagct cttcctgtga gcaactttgt gctaaagaaa gagcactgga gccgggcgtg 54600
gtggcttacg cctgtaatcc cagcactttg ggaggccaag gcgggcggat caggagttca 54660
agaccagcct ggccaacatg gtgaaaccct gtctctacta aaaatacaaa aattagacgg 54720
gcgtggtggt gggcacctgt aatcccagtt actcgggagg ttaaggcacg agaattactt 54780
gaacccagga ggtggaggtt gcagtgagtg gagatgacgc cgctgcaaaa aaaaaaaaaa 54840
aaaaggagaa aaaaaacaga aagagcactg gacttggagt ctaaacacag gttctactcc 54900
cgactcacct gtgactgagg gcaagaatgc tttcttctca gagactcggt gtcccctttt 54960
gtaaaatgag gctcattctg tccttcccca agggcttcag ggaaactcca gttagagcag 55020
tgaggtgcta ggtaaactct gaggggcacc ctaaccgccg tgtgaggtca ggaggtctga 55080
attctctcat cccctctccc caggacaagg gcacacaact ggttccgtta agcccctctc 55140
ttgctcagac gccatggagc tggatctgtc tccacctcat cttagcagct ctccggaaga 55200

cctttgccca gcccctggga cccctcctgg gactccccgg ccccctgata cccctctgcc 55260
tgaggaggta aagaggtccc agcctctcct catcccaacc accggcaggt acgatggggc 55320
gtggggcttg gggaggtca gtgctggata atacacagag gcttgcaggc cactgctccc 55380
ttccccacac ctctctccct ttttcttctt gccacaggaa acttcgagag gaggagaggc 55440
gtgccacctc cctcccctct atccccaacc ccttccctga gctctgcagt cctccctcac 55500
agagcccaat tctcgggggc ccctccagtg caagggggct gctcccccgc gatgccagcc 55560
gcccccatgt gagttgtccc tcagaaggga agggagggat gcacgggttc tgggtctgtg 55620
ggagatacac agccgcttcc atggaggcag gggatcttgg ttaggagtcc ctgagggtct 55680
agcaggtgcg gaaagggaat gaatcaccct ttgcctcccc tcaagtcgtg tgcaattcct 55740
ggggcgcagg tagtaaaggt gtacagtgag gatggggcct gcaggtctgt ggaggtggca 55800
gcaggtgcca cagctcgcca cgtgtgtgaa atgctggtgc agcgagctca cgccttgagc 55860
gacgagacct gggggctggt ggagtgccac ccccacctag cactgggtaa gtcaggtgca 55920
tggaactatc cgggctggga gatgatgcct tgcatcttgg gctaggcatg tggctcatcc 55980
aggagatctg gttgatctcc aataacccct gctttttgcc cttgtcccca gagcggggtt 56040
tggaggacca cgagtccgtg gtggaagtgc aggctgcctg gcccgtgggc ggagatagcc 56100
gcttcgtctt ccggaaaaac ttcgccaagt acgaactgtt caagagctcc ccagtgagtg 56160
catgagggct gtctgggcgc tgggatgccc tgatcctcaa cctggatgct ggagccctga 56220
tccctgacac ttgtctaccc acagcactcc ctgttcccag aaaaaatggt ctccagctgt 56280
ctcgatgcac acactggtat atcccatgaa gacctcatcc aggtgggggg accccccatt 56340
tcactgcaga ttcacgactc cccagcattg gccagtgctt ctccacccct aagtcctgtg 56400
cctcccctct atgttgtaga aagagccaaa tcacagtcct gtgtgactgg ggacagtcac 56460
tttccctgcc tgagcatcag tttcttctat taaatggggg cgagaaatgc atgtggagca 56520
tttccttgta aaaacctgag ggtgggctgg gcacggtggc tcatgcctat aatcccagca 56580
ctttgggagg ctgaggcggg aggattactt aagcctagaa gtttgagagt ttgagaccag 56640
cctgggcaac ataatgagac ctcgtctctc caaaaaaaaa aaaaaaaaaa aaaaaaaggc 56700
caggaatggt ggcatgagcc tgtagtccca ggtgcttggg aggctgaggt gggaggatca 56760
cttgagctcg ggaggtcgag gttgcagtga gctgtgatcg tgccaccgca ctagcatgag 56820

accctgtctc aaaaagaaaa agaaaaagag aaacatcctg gtggtagagg ggaagggagg 56880
aggtcagacc tgtcactctc ctctgctctc ctctggctca gaacttcctg aatgctggca 56940
gctttcctga gatccagggc tttctgcagc tgcggggttc aggacggaag ctttggaaac 57000
gctttttctg cttcttgcgc cgatctggcc tctattactc caccaagggc acctctaagg 57060
taaggtcttg agggtaccag ccccagcccc tccagtccct ggtcctttta gaagttgccc 57120
cttctctgct ggaacctctg agcccttctc cccctgggcc ccccaggcca gccacctcca 57180
gtttaccatc tctccctaca tccttgccta gctcacctgc ccagggaggt agcaggagaa 57240
aagatgatct tagtttaagt cctggctcta cttctatttg ctgtgtgacc ctgggtattc 57300
ccctgcccct ctctggtcct gaaatctccc cacctggctt gtggggggag gtaatagtgg 57360
gcgggatcta cctgtaccaa gtcctgctca ctcatgctgc ttaggatccg aggcacctgc 57420
agtacgtggc agatgtgaac gagtccaacg tgtacgtggt gacgcagggc cgcaagctct 57480
acgggatgcc cactgacttc ggtttctgtg tcaaggtgaa gacctggcca ggcctggccc 57540
ctggcctggg gaagcaggaa ctgctcaggc ccctggatcc tgcccggggc ttctgagccc 57600
caattcagac acctagactc ctctccctgc accctggcct gctggaaact cctggattca 57660
gctctgctat gtggagcagg ggcagacatg tggtcctaaa ggcagatatg ggaccagtca 57720
atttcctctc tctgcagccc aacaagcttc gaaatggcca caaggggctt cggatcttct 57780
gcagtgaaga tgagcagagc cgcacctgct ggctggctgc cttccgcctc ttcaaggtga 57840
gaccctggga gtggcatggg gggctggcct ggccagaggg atccccagct ctgccctcag 57900
gaagtctcag gaatgaggag ggcatcacag ccttgctctc tgataacccc cagtccaagc 57960
ctgaagttat aggaagtgcc catcgaaggc agaaacacag ccctggtctg ggcaagtcct 58020
ggtctgaggg gggcgtcaca gccacgcccc caggacctct cgacctcaag ctctctttct 58080
ctccccaccc ccagtacggg gtgcagctgt acaagaatta ccagcaggca cagtctcgcc 58140
atctgcatcc atcttgtttg ggctccccac ccttggtgag tgtgcccaag gggatgggag 58200
ggtgggtatg caggccctgt cttacgggta cctgggccct gttctgacct ctctcctctc 58260
cttccatctc cagagaagtg cctcagataa taccctggtg gccatggact tctctggcca 58320
tgctgggcgt gtcattgaga acccccggga ggctctgagt gtggccctgg aggaggccca 58380
ggcctggagg gtgaggcctg ctgtgtgtgt gtgtgtttgt gctggggacc cactctctgg 58440

gtgggatccc tgaaatagga gggaggaaga gagggcgggg ggaggcccct ggctgggaag 58500
aagtgctcta ccttcctgag gtgctgggta atgcccccaa gcacgccccg actctcccgt 58560
atctcccact gctccacaga agaagacaaa ccaccgcctc agcctgccca tgccagcctc 58620
cggcacgagc ctcagtgcag gtgggtgacg gccccgagtc ctggggcggg ggctgcctca 58680
acttctcttt gtattcccag tggggagtag atggtatagg ggtcccctcc ccaaagtgac 58740
cgcccatgtc cttccccacc acagccatcc accgcaccca actctggttc cacgggcgca 58800
tttcccgtga ggagagccag cggcttattg gacagcaggg cttggtagac gggtaagggg 58860
cagggccggg caacagaccc agggataaga gagactgggg tccaggtggc agccatggtc 58920
cttgggtagt aatgctgccc catctcctgt cttctggcag cctgttcctg gtccgggaga 58980
gtcagcggaa cccccagggc tttgtcctct ctttgtgcca cctgcagaaa gtgaagcatt 59040
atctcatcct gccggtgagc ttccctgcgt ccccggagtc ctgcaatgag acacaggact 59100
cccagcaacc tgtcctcctc accaggcccc tccagaggct ccctggcccc cagtgcgtct 59160
cccttttccc tgcacaagaa gtgggaggct gagtgcggtg gctcacacct gtaatcccag 59220
cacttaggac ggccaaggtg ggagaatggc ttgagcccag gagttcgaga ccagcctggg 59280
caacacaggg agaccccatc tctacaaata atttaaaaat gagccaggca tggtggtgca 59340
cacctgtagt ccagctactc aggaggctga ggtgggagca ttgcttgagc ccagagggtc 59400
aaggctgcag tgagccatgg tggcaccacc acactccagc ctggatgaca cagtgagaaa 59460
actgtctcaa aaaaaaaaaa agaaagaaaa agaaaaagaa aaaagaaaag aataaaagga 59520
aatggtgggg ccctggccca ggagggagct tcccaagcct cgggcccctc cctgaacttc 59580
cacccccttt actgtacccc agagcgagga ggagggccgc ctgtacttca gcatggatga 59640
tggccagacc cgcttcactg acctgctgca gctcgtggag ttccaccagc tgaaccgcgg 59700
catcctgccg tgcttgctgc gccattgctg cacgcgggtg gccctctgac caggccgtgg 59760
actggctcat gcctcagccc gccttcaggc tgcccgccgc ccctccaccc atccagtgga 59820
ctctggggcg cggccacagg ggacgggatg aggagcggga gggttccgcc actccagttt 59880
tctcctctgc ttctttgcct ccctcagata gaaaacagcc cccactccag tccactcctg 59940
acccctctcc tcaagggaag gccttgggtg gccccctctc cttctcctag ctctggaggt 60000
gctgctctag ggcagggaat tatgggagaa gtgggggcag cccaggcggt ttcacgcccc 60060

acactttgta cagaccgaga ggccagttga tctgctctgt tttatactag tgacaataaa 60120
gattattttt tgatacacct atgagttctg tctggcaagg cctggctggc tgaatcaaga 60180
agggaaccag agctggacgt ggtggctcat gcctgtaatc ccagcacttt gggaggccaa 60240
ggtaggagaa ttgcttgagt ccaggagttt gagaccagcc tgggcaacat ggcaagaccc 60300
tgtccctaca aaaaataaaa aaatgagccg ggcatggtgg tgtgcacctg tagtcccagc 60360
ttctcaggag gctgaggtgg gaggatccct tgttccttga gcctgggatg tcaaggttgc 60420
agtgaactga gattgtgccg ctgcactcag cctgagtgac agagtgagac cctgtctgga 60480
aaaaaaaaga ggggagacct ggagaggtgg gcacctgtgg aggccttggt ggaagtgtca 60540
aatggatcga ggccatgtct cagcctgcct ggatgttcct caagggcagg agtggttatc 60600
tgagagtctc cagtgcccac catgcagctt gacacatagt aggcgcccag tcattgctaa 60660
ttaagtaagt gaatagacaa gagaccatca tcccagagag attttctgac agtctaagtc 60720
tagagaggta attaacaggg cctgggagtt ggagatgagt ccgacagcat gcttgtcctc 60780
cgctagtcct ggactagctg acggatagtt ggccccagca tgcacacagt tatgcatcca 60840
gccccaccac caagacacag aatggcctca tcacccccaa caagtccctg tgccccctac 60900
tatcagccat gctccctcct acccaaactg aaacctcctt tctgtcctag ttctgcccct 60960
tccagaaagc catataaatg gagcctgcta gcattcagcc ctctgcatct ggcttccatc 61020
tccaacacat ccttcagcac ccagagtgat cttttctcaa atataaattc accactcccg 61080
gcagggcact gtggctcaca cctgtaatcc cagcactttg ggaggccgag gcgggcggat 61140
cacgaggtca ggagtttgag accagcctgg ccaacatggc aaaaccccat ctctactaaa 61200
aatacaaaaa ttagctgggc atggtggcgg gcacctgtaa tcccagctac tagggaggct 61260
gaggcaggag aatagcttga accggggagg cagaggttgc agtgagccaa gatcatgcca 61320
ttgcactcca gcctgggcaa caagagcaag actcagtctt aaaaaaaaag acagatgtct 61380
ggcctctgcc cactgctcat gccagtctat gtttttgtct tagaggtttt tttgttttgt 61440
tttttgagat ggagtctcac tctgttgcgc aagctggaat gcagtggcac gatctcagct 61500
cactacaacc tccttctcct gggttcaagc gattctcttg cctcatcctc ccaagtaact 61560
gggattacag gcgcttgcca ccacgcctgg ctaatttttt gtatttgtgg tagagacggg 61620
gtttcaccat gttggccagc ctggccttga actcctgacc tcaagtgatc cactcacctt 61680

ggcctcccaa agtgctggga ttacacaccc aagccactgc gcctggcctt gtctaagagt 61740
tttttagttt gagctcttac ctgatgggag ctctttaata aatatgtcag atcgactgca 61800
tctgaacttg ctgcttaact caggagacag accctcctgc agacctgtga accccaacta 61860
gtcattgttt ttgtcaaaac accaaacctg aatctgattc cttggattca acaactagtt 61920
tacgggaaat acaagggaaa caatgacaaa aatccagatt gggaaattct acagaacaga 61980
taacccagtt tcttcttcaa cagcaacaaa attaccagaa aagatgggca atctctagat 62040
ttcaagaaat ttagacatat atcaaccaaa tgatgtgttg acctttgatt caaatgcaaa 62100
ctaactgtaa aaagcattta tgagcaattg ggggaaattt gaattctgat cagatattcg 62160
atgatattaa gggattatgg ctaactttct aaggtgtaat aatggtattg cagttacggt 62220
tttttctaaa tctttatctt taagaaattc attatgaagt atttaaaggt gaaatcacat 62280
gatgtctcag ctttgttttt attccttttc tttttttttt ttccaacttt tattttagat 62340
tcagggagta catgtgcagg tttattacct gggtatattg agtaatgctg aagctttgtt 62400
tttaaaataa cccagaggtg gccaggcaca gtgggtcatg cctgtaatcc cagtactttg 62460
ggagctgagg tgggaggagt ttgagaccag cctgggcaac atagtgaggc cctctctaca 62520
aaaataaaaa ataaaaaatt agccaggtgt ggtgacatgc atctgtggtc ccagctacct 62580
gggaggctgg ggtgaaagga tcgtttgagc ccaggaagtc aaggctggtg tgagccatga 62640
ttgcatctct gcactccagc ctgggccaca gaacatgacc ttgtctcaaa aaataaaaag 62700
aaaaaaatga aatccagagg ttaaaggaga gggtgtgggc atagtcatgg tgtggccatg 62760
agttgacact ggctgttgat attggttgaa gcttggtgac aggtactggc tatgtgggga 62820
tttgttaaat tatcctctcc acctttgtat atgcttggat attctattat atattgatat 62880
attttaatgg agagcccctt ctcaaatctt tgggactgac tgggcaggag gtggtgagac 62940
gctgttttgt aggtgaggaa actgacggcc cagtgggcag cctggcccca gatcacagtg 63000
ggagagccaa gattccaacc caggtctgag gaccaggtct gggcatgttc tgcttcccct 63060
gtctggctga cgtccaggtg gcattcctca gccacagtga cagcaatagt ggcctactgg 63120
ggtcctccct tagagagccc ctcccttccc tgcagccttg gtctctggcc tggagggttg 63180
tcctgaggtg accttgagac tgtgcagcct ctgcaatcag cccctgggct gggtctggca 63240
gccctggcag ccactcccga ccaaggcacc cccatccttg gctaactcca ggctgggaag 63300

ctgggaccag gtccagcaga cagaggtggg cagagaggtc tcatgcacac cccagcctgg 63360
gacctcctgg gaatctgctg gaagaactta ctgcagccca agggcaggaa gggtggcaga 63420
ttttgcttct ttttagcagg actggaggca ggacccagtg caacctgctg cctgccacct 63480
gccacggttc ccagagccat ccccaccaca ggctgtggct cgagcaccga acagggcagt 63540
gtcagctgtg tggctggcca tgggtgccca gggccacctc cgaggtgtgg gtgccagagt 63600
tccaggctgg agtgagagta aaggggtcaa gatgagtcac ctcttcagca tgtccagggg 63660
ttcagactgt aaaaaaagag gacccagtgt cccttcacag cctgaggcac tgggcagaga 63720
caaagcctct ctgtcaatca gctaattgga gactgtgaga cagatgtcct tcccacttta 63780
caaatgggaa actgaggctc agggaaagac agggactcac ctgcgacagg ggtcgcagag 63840
ggagttcggg acagaggcca gactcagccc actggacagg tggtgaaatg cagccctgcc 63900
ctgctctagc ctttgttctc tgccccctcc cttctcagtg ctggccacag tgagggacct 63960
aggctgccga gagcccttag agggggcagc atttggccct gatctggcca cctgagacca 64020
gcagcagccc attcttggtg agtgtctgag atttctggct ttttggagac gtagctgcta 64080
agagtttggg ggcacaggga ccctctcagg gccagctcac agtggaaccc ctgggtaaga 64140
tcccccagct ctgaggatgt gggaaggatg ctgctctacc tcagagactg cagcatgacc 64200
tacagtggtc agacagcaga aaggactgcc aaggacggct gcagtgctag atcggggagg 64260
cttgtggttc tgagaaaatc tgggctggtg ggaggggaag caggggcttc tctgggggag 64320
agggtggggg ctgggacctc agatactgtc ttttcaaaat tgatttattg ggtttcattc 64380
taaaattata cgtacttatt aaaaacattt cagaaaaaaa cttgagggca aaggggaaat 64440
tcctcatcat ctttccatgc agaggaggac ccagtactga gagagggtga cagggcccac 64500
atctggggag ctgacgcgaa gtgctgcctg ccatgcatca cacgttccag cttcccggga 64560
ccagcgtggc cggtgggagc cgggtgccag ggcacattgc tatccttggc agcaccatgg 64620
gcctttaggg taggggttct gtggagggtg tggctacaat gttggcctcc ctgcctgctc 64680
aagtgtggag gggagggagg actgctgcca acgctctgga ctctgggatg ggggtgggca 64740
tgattccagg ggagcccacc tccctgggcg gggcatccag acaaagccct ggccagttct 64800
gcaacatcca agttcacggg tggtggacag gcccaagggt tcaacatcag gtcagggacc 64860
ctgcctggct ccttccttgg gccccagtgc caggcaccct gatgagggat gaagagagga 64920

aggacaggac acagacaggg gagcccaaag ccacttggaa ggattctttc ttccaaacta 64980
tccacataag cagttttcta actccttccc agagcccaag ggttctggca ggggcttgag 65040
gggcaggttg gggtggtgat gggggatgca ttgcctttct atctggaact tgagccttat 65100
ggtatattca ggaaaaaggc ttgaaccagt gaagtactga tctggtccaa cagatagaaa 65160
gagggcccag agaggtcagg gcttggtcca actcgcacag tggtggggcg tctgcagact 65220
gctttcctgt cctgtgctgt tggcagaacc tcagctttgg gacttgaggg ggagtatgca 65280
gtggcaatta ggacaaacga ctctgagctc tcagttgagt tgggggagga gagaaaagaa 65340
ggagttagat cctgggggag ttggatgttg cagactggac tccagagcct gggggtgtgg 65400
gcgatcctgg ggtgtgggga gggcgggctg aggctgctcc acaagcgttc tcgaagttca 65460
ccatacaaga aggggcggtg tggggaaatg gggatgaggc ctgaaattca atcccatgtg 65520
ggttgagtta tgatgtcatc tgacaccctc tcacacggct gcatctaccc caaggaaggg 65580
gcaagttttt ctttgatggg cacctggggg ggctgtttag ctggagtttt ttttgtttcg 65640
ctttttttgt ttttgtgttt tttttgagac aagtttcgct ctgtggccca ggctggagtg 65700
caatggtgtg atctcggttt accgcaacct ccacctcctg ggttcaagca attctcctgc 65760
ctcagcctcc caagtagctg ggattacaga catgcaccac cacgcccagc aattttgtat 65820
ttttagtaga gatggggttt atccacgttg gtgaagctcg tctcgaactc ccgacttcat 65880
gtgatccgcc cgcctcagcc tcccaaagtg ctgggattac aggtgtgagc caccgcgccg 65940
ggcctggagc tgcatttttg ttgtaagcac tttgttctta cttggattag gtttatttta 66000
gggtggtttg gggcagactg agggagattg gaaggcagaa accacccccct caggacaacc 66060
acgtggatct ccttagggtc tgggtagtgg aactgggccc tgtgaccttg gagttggagg 66120
ggaagctgta agcagggatg gaacgggcca cccgcaccct cacacagctg tgaccaacag 66180
gcacggtcat ccgcattcac ctgcccaact cccatgccct gagtcctgtc ctccagggca 66240
ttcctactgg cactacccca atttcccaaa agattccagg gatctctctc tcgctctctc 66300
tttttttttt tttacagtct cactctgttg cccaggctgg agtgcagtgg ctgatctcag 66360
ctcactgcaa cctccacccc ctgggttcaa gcgattttcc tgcctcagcc tcttgagtag 66420
tttgggatta caggcgtgcg ccaccacgcc cagctaattt ttgtattttt agtagacggg 66480
gttttgccat gttggccaga ctgatctcaa actcctgacc tcaagtgatc ctcccgcttt 66540

ggcctcccaa gggagggttc cttcctccca ggctgaggca ccctgcccca cacatctact 66600
ggaggaactt agtgttgggg ccagggggtg gctgggtaag gctggatccc acacttgtca 66660
ggccaggttg gggtagggga ggatacacgg gggacccgtc tctcagccgt tctgggccgg 66720
tgcctgaggc gatctgcccc gccgcccgcc cttccgccac cacccaagtc gcatgcgcgc 66780
gccggggccg acgggcgcca ggctccaccg agccaccctc cgtcgtggcg gcccctcgtg 66840
cctctgcgga gggaaggccg ccggcttcgc cagggggcgc gcgtcggggt cgcggctctt 66900
tgaactcggc cccacgaggg gacgcgccat cggtttcgtg gcttgcgtgg cgccgcagca 66960
acgagccggt cgccccgggg cgttgcttag tccgggcctc gctgcgagtc tgtgggcgag 67020
gcgggaggtg cttccttggc caggcgggtg gaggaaggtg tgggcgccgc atcccagcgt 67080
cgcggagcct ccaggcgcgg gcaggcgaga ctggcgtgca gcgcgacggc ctagtcccgg 67140
cgtcccaccg tcccccactc ccgcaggcgc cgagccgatt cagccgcgcg gctgcagtga 67200
cacccagcgg ccgtcgcggg aagtgcggtc gccgcggcca ggcctccggg gctcctgtct 67260
ctcgaggttc ccaccccggt tacgcgcggg caccccggag gagagcggcc gcgcgctgca 67320
gatgcccgcg ggggcacttc ccctgcccct cccccgtttc ctccccgcag gcgcccgagc 67380
gccgggagat cctgcgcagc ggcagtgccc ggaagggcgg ggcggggtgc agcagcggcg 67440
ctgggctgtg acccgggcag tttcacttct ggatcctgct cgggctgtga taggacagcg 67500
cgggggcagc gggggaacac gcgggcacgc cgagatccgg cgctcgggac cccgatgagg 67560
ggaggccttt cactcctggg gtgtcacgcg acccacgctt tcctccatgt ccactcccac 67620
ccccatggg ttccactttg ctgtcccccg agctgccgct cctccccagc agacaccgtc 67680
tccgccgggg ctgccgaccc ccggagaggc cccgagcggc gtgggcggcg ggaggaggga 67740
gctgtgaagc cgcaggcagg gggttaggct gcgggctgct gagacgccga gctgtttctc 67800
agaagtgcag ctgcccctcc cctcacaccc ccctcactcc caccgcgcgc cgcacccctg 67860
ccccctctcc gccggcctcc ctttcctctc ccaggcaccc tagccgggaa tctggggtct 67920
gggtccacgc agaattaaga ggagaacctg agaaagggcg ggggccgggg gttcagaccc 67980
agcctcagcg cgcgttattc cgagcctcct ataattccac aggtgagggg atcctgtccc 68040
ctacccctgg ggtccaggat tggaatttga tagaacagtt aggaaaatga catcttgaga 68100
gagtcagggc ccggccacta ttactctagc tccagcgtcc agtgaggaga ggagagagag 68160

ggaagacctg gagcccggct ggtgcagtga ggttgaggcc tgtcttagga gtaccagctg 68220
gaaggccctt cagagatccc tgactctggg gaaaatcaac gcccagaagg ggaagtcaag 68280
tgtctgggct catcccgaga gggagagggc tgggattgga accaggactc caaatagttg 68340
cgggagtctg cgactgtctt ttgcttggga cttcagagct ttgctggatt cactgactca 68400
gcctgctttc cctcaacaca cacaaacaca cacacacgag acacgtgcac gcgcgcacac 68460
acacagatac acagacacac acagacagac atgcaaggag agagacaggc acacatggag 68520
acagacacgc acacgcaaag agacacacag ctccccagga gtctagcggg gcagctgagg 68580
tctgtgagtg agacattctc agtgagacac tgagaatgca attccagtgg gggtgggatg 68640
gggattgggc ggtggaagga caagcccggg ggaggaggtg gcatcgcccc cctagttccc 68700
atccaccacc acccccagtg ctgtggttta tttgcctagg cagctcttct gggccagggg 68760
gccagggtac tggggtggag aggcgaggtg atgggaaagg ctgttctggg gcttcctgcc 68820
ccctcactcc tggggaaggg gggtagctgc catcacccgc aggcctttcc actgcagccc 68880
tttccttggg tatgtgtgtt attctggggg aagatctgtt atttgcggag aagggaggca 68940
gctgcctact tcacaggtca agacagagtt aaaaacaaaa ccacagcaaa ttcaacaccc 69000
cgtgtccttc agggactttc catgacacct tccttccccc ctctccccta agccagggag 69060
cacctcccca gagggcctcc ccacccccagg tgcagtggct tttggctttt atgcaaacaa 69120
ctgtctgatc ccggaatgct ccacagcccg aactgtggcc tgggccgaat gtgcccctcc 69180
accatccctt gggccagtgt ttctggtctt cctggagttg tgacctccac tcttcctcag 69240
accctggatt ctgagggaca ggagatgagg gcaggaagct aggtcctttc cccctgcctg 69300
ccctcgggtg gcagttgcct gcaggtggag ggtttgggca cagcaaccta agtgaatggt 69360
ccggggtttt gagtgatggg ggagggggaag aaggagggtg caggtgtggg gtggcctcca 69420
ctgccaggcc caccaccctc tagggcattg aattagctcc caagatcagg aaggaaaact 69480
cttctcaggc tgggagtcca ttcaacccca acgccagtgt ggacctccgg tcctgctcac 69540
tggcaaaggg ggtcaggatc tccctgtctc cctctcttct gcctgagctg cttctgcttt 69600
ggggagggat ttggggatgg ggaaggccat ataatcttct ctccagggct gaaaaccagc 69660
tactgagggg ccgtttttga atgtattcag tgctaaaatg caaaggcagg gtgccatgcc 69720
attcctttct cctcctcagc ttgagcaaga ctgggaaacc tgtaggagac cctcagccag 69780

tttgaacatt ttggagtgct gatgggaggg agatgatgct gatcatggag ccagaggacg 69840
ctggtgccag cctagcccca catttgttag tcttaaaact tcagacaatc tttctcatgc 69900
ctcttccaca gaagacggaa aatggagggt gctttagatg aggctgagca gaaggccaga 69960
ctctctctct actttctcta acgttttcta agagagaaca caatgatgat ggaaagaaag 70020
cagcctcatc cctatgccat tctcctagga cttttccccc taacaccttc ggtttgggtc 70080
tccaggtatc taaaaactgt atttaaatat ggtcgagttt aaggactgca actcaagttt 70140
cccaatgtgg atgtctgtga gccccacac atgaaaaagt cagaacttga ctgtatttta 70200
agaagaatct ctgttgaggg aggaaggcag agcagtgact tggaataggg gtgcaccatg 70260
ctgaatgtgc tgatcatgct aagtccaggt cctcaacctc ctccagtttc cttagtgaac 70320
taaggaccag tggaaccagg aaccctatct tgtgggttac aggggccagc agcaggatca 70380
cagtggtagg ctcagcaagg cgtcaggtga gaacctttat ttcagtgata attcattttt 70440
cttgataact ggactttaag aaatagaggg gcaaaattaa gaaagtattc cttggggcgg 70500
gatgaaaccc ttctacatcc tgacaccagc agacacaaat cataagtgat aaggatgcac 70560
aaggactaat ttcaaacata ccaggatttt tttattttc agtgtaaaaa tcaaacatga 70620
taaacctaga aaactatcag cagggctatt tcttacaggg ttccagcaag ggttagcatt 70680
gttacatttc agaagctgac tttctttaaa ccatctttac agaggagtaa acttcacagt 70740
tccacacatg gctgggctcc aggtagaact ccataggagt gacgagggaa agtcaccact 70800
gggcagggag ggcatttctg agaaatgcag atgagaggag aggctgtgag cacaggctgt 70860
gtcaaaaccc agggcaaggg ctccctcccg ccttccctcc cagtgggaag gccaccctga 70920
gccccaaacc acattctgtt ccttcctcgt cattctgcag acaatggtca tccacagacc 70980
acacgtgtgg tggctttggc aaccagaaat ttaaaataag ctatggtttt tccagtagcc 71040
aaaatgatcc tgcaccaaag ctcatagact gagaacctga gcatgcaaaa ccacagtctg 71100
ggtgaaggga tgtctgcttt gtaaatgacc tgctaattct ttgcaaccca cagtaatttg 71160
gtttctgtga acccacagaa gcaggcccac caaaaagggc cttgtctgct agcctggagt 71220
atacatgagt cactggcggt gggatcagtc attttttagg ctgccccatt ttcctaacat 71280
gttaaaatgt gtgttctcag tcttttcaag agaggaagaa gcaaagcggc acttacagag 71340
tgtgagatag acacagatct gtggcgaggg attggggaag gtgggtggca tcttgggact 71400

ctctccaggc tttctggagt ggggtcagtg gagagatgaa cagtgagaaa catttgaaat 71460
aactcaagtt ttggagcagc ggggggtggg gtactaatgg ctgaggactc acagacagtg 71520
agggagcaaa gcgctttgac ttccctgggt gcgacaccat gaagtgtcta tagcacgtgg 71580
ttagctcacg ctggttccaa gggtcagcag atcctctgcc cttgtgcatt aaaacaacat 71640
gaaggaaact gttagagatc agttacctcc ataattcatt tgtaaaggaa ttgctattat 71700
gatctagtat gatcttggga tatttttcat gtctttaaat ttaggacacg gtacattttc 71760
cactgaaaat ttacaatcat ccaacataat gcacaccacc cctcaaaggt aacattagct 71820
catatacaaa atcatggaaa acctcacaac atccttggaa ggtagatatt gttatatcct 71880
tacaaaaatt taatacaccc attaacattc ctatttcaaa tagctcaatc gatatcaaca 71940
cataagacag cccacaattg tgatacagta ttaagaagaa actcaggcca gccgcggtgc 72000
ctcacgcctg taaccccaac actttgggag gctgaggtgg gcagattact tgagcccagg 72060
agttcaggac cagcctgggc aacatggtga gaccctgtct ctacaaaaag tacaaaaatt 72120
agctgggtgt ggtgacccac gcctgtagtc tcagctagtt gggaggctaa gacgggagga 72180
tggctcgttg cttgagccca ggaggttgag gcggcaagta agccttgatt gtgccactgc 72240
actcagcctg gggaaacaga gcgagacttt gtctcaaaaa gaaaaaaaaa aagaggaaac 72300
ttgtatccaa tttattcaaa ctgcaaaacc ctgtgtatct tcataacaat ggtggggcaa 72360
agactatcta cagctggggc aagggagagg cacagtcttg tgttacttta caagctctca 72420
gaagtttaaa gcccacaatt gtccttcagg ctcttctgag tccatggtcc taaagggtca 72480
ggagtcctga accctggctt attctcagaa ccattcagtc gtgctgcctt ggagaacaga 72540
gtccttttcc tgaggtggcc caggtccagc tgctggtttc agatcaaaat tgacctcctt 72600
catcggcact tatatctcaa atcattctcc ttgtctgaac tctggatata gaggtttttt 72660
gaactatact ttctgtgtga acacaaaaat agcttcccat tctctaaaag atgaggtatc 72720
actatatatc gggttttttt cttctgttcc tggtgctaac ttttaggaca aagtttcacc 72780
actccctttt ctcttcttga gctcacaagt cccgatgctg actctttgag tcctcagata 72840
gccaggctct ttcacatctt gagtgtccca gagtttccaa atctctgcta gagtccaaca 72900
tacctcttct cagtggggta ctctttaaag tgaggactta aacatttcca ttgtggtctt 72960
atcacctggg attatatgta acagtactat ttttttcctt aaaacattct ttttttttt 73020

tttttgaga tggagtctca ctctgttgtc caggctggat tgcaatggcg cgatctcggc 73080
tcactgcaac ctccacctcc caggttcaag caattctcct gcgtcagcct cccgagtagc 73140
tgggattaca ggtgtgcacc accacaccca gctaattttt gtattttttt tttcgagatg 73200
gagtctcact ctgtcaccca ggctggagtg caatggcacg atctcggctc accacaacct 73260
cctcctcctg ggttcaagcg attctcctgc ctcagcctcc taagtagctg ggattacagg 73320
catgtgccac cacgcctggc taattttgta tttttagtag agacagggtt tctccatgtt 73380
ggtcaggctg gtctcgaact cccaaactca ggtgatccgc ccgccttggc ctcccaaagt 73440
gctgggatta caggcgtgag ccaccgcacc tgaccctcct taaaacattc taaacctcac 73500
attcagtagg gcttggggag gtgggggtgg tacaggaaat aggagaataa tagtgataca 73560
gatgctacct ggaaaaactt tgttctgtct gttcctcacg aaacaaagcc gaaaagaaaa 73620
actttatgtg gatgtgagat ttgatagtaa cacagtgttt cacctgagca aaatttgtg 73680
tttttaaaca tttctgtttg ccaaagaaat ctgaggttgt ggctgggcgc agtggctctt 73740
gcctgtagtt ccagcacttt gggaggctga ggtgggcaga ttgcttgagc ccaggatttt 73800
gagactagcc tgggcagtat ggcgaaacgc catctctgca gaaaaataca aaaaaaatta 73860
gctgtgcatg gtggtgtgcc tatagtccca gctacttggg aggctgaggt aggaggattg 73920
tttgagccta ggagatcaaa atctgaggtc attttatatc tggccgattt tgagtatggg 73980
cctaacaaaa tgttaagcac tgcatttaaa aggtgagaaa ttttgtcact gacaaagtca 74040
gaagctgtgt caggaacagc tcacagtggg tcataaagca taagattagc tggcttatct 74100
gtgccaaggc gaatgtcttt ttgggagaat aacttgagga aactaaagac tgatgagttc 74160
attttctctc tttttttttt ctggtggtag tggtgggggc tcacgggaga gacacttcta 74220
tttagttttc tttccgtagg aagtaccgat gcaacacttc aggtagtgac agaaaacatt 74280
tggagagctg ggcattgttt gtgcatgcct gtaatcccag ctacttggga ggctgaggca 74340
ggattgcttg agcacaggag tttgagtctg gcctaggcaa catagtgaga ccctggtcct 74400
ctaaaatata catacatata tatacatata caaaaaaaat aaaaataaat taaaaaatta 74460
aaaaacacct ggagcactgg ttctgtttct tttccaccct cagcccagtt ctgtcacctt 74520
ggctgcaagg aaactttgcc tgggtttttt tgtttgtttg tttgtttttg ttttagacag 74580
agtttcgctc ttgttgccga ggctggagtg cagtggtgcc atctcggctc accgcaacct 74640

ccgcctcccg ggttcaagcg attctcctgc ctcagcctcc tgagtagctg ggattacagg 74700
catgtgccat cacgcccggc taattttgta tttttagtag agatggggt ttctccatgt 74760
tggtcaggca ggtctcgaac tcccaacctc aggtgatctg cctgccccag cctcccaaag 74820
tgctggcatt acaggcgtga gctatcgcgc ccggcccttt gcatgggttt ggttgttcct 74880
cagaccgtgg cctcctgggt gtatggtgga aagcccgcct gataagaggc tggtgggaaa 74940
tgaaggagag gggcctacaa ggctcttgtt cagagggagt ctgcatgaga aagtacaggt 75000
actatctgaa gctgctccag ggcttcagtg cccagcagtg gcagatgcaa gtcctgactc 75060
tgcccttctg ctaagattct ccttcaccct cacctcccca gccctggcaa ggggtttgtt 75120
aattagtaca gcataagggg tatgtacaga aggcagcgtt tatctgtgtg atggtaacca 75180
ctgcatatct ggttcaactt caagacttaa ggatgcagag tttccacaca taaatttttt 75240
ctggaattga tgggccccaa tctaggctca gaatgccaca aagaaagtgc ctttgtgagt 75300
ggtcacctta ctcagaaaac taacctcaga agaatagaaa tcatgcagca aattcattag 75360
tcacaggaaa aaggaggatc atatctataa aaagagtccc caggctggtg gaatgggcct 75420
tggtttcaca atcttgagat gcaaaaggtg gtctcaggct gggtgcggta gctcatgcct 75480
gtaatcccag cactgtggga ggccaaggcg ggcagatcac ctgaggtcag gagtttgaga 75540
ctggcctggc caacatggtg aaaccttgtc tctactaaaa atacaaaaat tagccgggct 75600
tggtggtgag cacctgtaat cccagctact tgggaggctg aggcaggaga attgcttgaa 75660
cccgggaggc ggaggttgcc gtgagccgag atcgcgccac tgcactccag cctgggcaac 75720
agagcgagag actgtctcaa aacaaaagaa aacaaaaaaa gttggtctcc tctgcaaaga 75780
cttgaacact aatctggtgg aaaggcttcc tttccctcgt tttctcttct actaatccct 75840
gcttccaaat tctctttctc ttgtcccttg aagaaccaac aggatgtgcg atatttcaag 75900
aacgtaatga gcaactcctc tttcacaaag ggaataagca atctggcata gcttggagtc 75960
tagtattacg gaaatacagt gatatactga agactacaca ccaaaataca tccattcctt 76020
tagctgaagt ctcaaacatg ttgggactgt tgccatggaa tttcataaaa ccttttttcc 76080
tgagacagac gccatgccac aaagaaggat aagccatttg tgcagtctga ggtggaacat 76140
tttaattact ggcagcacat agacgagttg agttggagcc gaatgcataa gccccttaga 76200
aataaaacac taatctacca ggaatttggg attcttggct ctggtagaaa tgttacaaat 76260

taagctgact cttaatttgt aaataggctc catgaagttg tccctggaag cagaatttat 76320
tttccctaac ataatttctt aaaaagtaaa aaaaaaaaga aggctttggc aaacagctgc 76380
atgtgccaat ttgtgtgtat gctttgtggc aagatcaaaa tacctttctg ttttttgttc 76440
ctctccacca ccttcttcac attcacaact cagtggttaa aggaaaaact taacacacca 76500
ttttcatacc aaaggaatgt gaaacatgta ttcatataaa tactcttctg cttaatatcc 76560
atacattagg aggtttaaaa aataccttgt ggttactgca taatcacata tgaattaaag 76620
ctagaaggga cctaagagaa cattgaaatc taattcctat tttatggatg aggaaactga 76680
gttttttttg tttttgtttg tttgtttttg ttttgttttg tttgagacag ggtctcactc 76740
tgtcgcccag actggagtgc agtagtgtga tctccactca ccgcaacctc aacctcccag 76800
gcttaagcga tttttctgcc tcagcctccg gagtagctgg gattacaggc atgtgccact 76860
actgcccagc taatttttgt atttttagta gagacagggt ttcaccatgt gggtcaggct 76920
ggtctcgaac tcctgacctc aaatgatgcg cctgcctcgg cttcccaaag tgttgggatt 76980
acaggtgtga gccaccgagc ctggccggaa actgagtttt aagagttaaa taaggctggg 77040
tgtggtggct cacacctgta atcccagcac tttaggaggc tgaggcgggc tgattgcttg 77100
agcccaggag ttcaagacca gcatgggcaa catagtgaga cccctatctc tacaaaaaga 77160
acaaaatgta gccagacatg gtggtgtgca cttgttgtcc cagtactcaa gaggctgagg 77220
tgggaggatc acctgagcct ggggaggtcg aggctgcact gagccatgat tgcaccactg 77280
cactccagcc tgggcaacag agtgagatcc tgtctcaaaa aaaaaaaaaa aaaaaaagtt 77340
aaacgatttc tcaaagtcac atagctaatt agccagcatt ttgtgacctc ctattccagt 77400
gctaatttta ccatgataac agcactcctc ctgatagtat gtacatatta tgagtgacaa 77460
ggatgactta tacatctgca gaaccgtgat cagactttca ggaattttca tggaatacag 77520
cagttccatt taaatgggaa aaaaagggcc gaagcctgcc tgatcaagga gaacctctca 77580
atagaggtgt ccctcctcac cccattccgg agctcaactc cgatctaaac acacatgcca 77640
tctatctcca attcctctcc tgtggggagg gggaggctga gccatacttt ttggactttt 77700
tttgtggaaa atatcattgg caaatttgct ttgggtctgt atcaccaatt ctccatgaac 77760
tctttcaaga agttttaggt agctgttttt tgtttgtttg ttttctgttg gtgctgccaa 77820
acaatttccc cctttatacc cttgctctgc caaccttgac caaaggaaag cgatgtcatc 77880

atccacagtg gataaatttc tggagtttcg atgatggtct atatttaaaa ccatgagttc 77940
ttctggcata gattttcttc tatagacaac tggtttagcc caaagcagct cagcttttca 78000
ctggagatgg gaaggtaaag gacagaagca gaagtgccat cagatgacat tttttaaaaa 78060
tgttgtgata ggaaatggat ctgatgcttg gagaatgggg tcttcagtta ctcgtaacag 78120
atacttattt tatggctgtt cttttactta ctcttagact gtcatttaga ttagggagat 78180
tagatcttaa cccttcagcc tgtgaagggc agaagggtga ggcttcccag caaggtcagc 78240
tggtcagctc ttcctctttg tggatttgca tgtatgactg aaaggctttt ccacgtgtgg 78300
ttgatatatc tcagaaagaa tgtttcatat agcacatctc cgggaccact cattccactg 78360
ctgtagaaga taatgaccaa ataccttttt ggcttttaaa ttccataaaa ttcaagtccc 78420
tgatgggaaa acaaaggttt cacaaaagta ataatatgct agacctgcga ataatttctg 78480
aaggaagaca gtgtttccct ggctacccct gtgaacacag ctaaaaatgg tatgaaaaga 78540
agtttggaac tgagtatgtt ttgagagcaa tctgttaggc gaggtcattg gtttttagaa 78600
acgatgctga atacatagga gcaatccctg agaccagata ttcacttcag cagggctctg 78660
tgttctcctc tggctatgtg agacgagaac tcataccgat catggcttcg atatccacac 78720
atgttacact cgaaagggtc acggaagccg tggcagccca tgtgaatcgt gaacatcaca 78780
tagtccagga agaggacgcg gcagtggtca caccgataca catccatcac ctccccttcc 78840
ttgttgatca ctttgacgga gtctcttggg cagatgggcg ggggcttgag gagttcgtaa 78900
gagcggggaa cctccttcag aagtggcatc ccattgcggg cccgagacag gaccatgtga 78960
ttttgctgat agatgtgatt ctggcgttct tcatggttgc tgtcagtgtc cgtggagtcg 79020
tggccactat tgttgggaga gaggcctctc tcagaaggca cgctcttctc tggaaggtgg 79080
atgcttttct tttccagctc ttgaggggca ccgtttgaca tctcagcccg ggtgagggct 79140
atgggataca tgctgctgat aactggaacc atctccgagg tgggagcagg cggtgtctgg 79200
accaaggggc gcagggcttc ggcgccaaga tagctgatgg cgttattgat ggcttggtcc 79260
atcatgcggg tctgtatgag ctcactctct ttctcataca tgtaacttga attatagttg 79320
acatcaaagc agtggcgctt ctcacctgga acaagtgaca gaaagggtta caaagggaac 79380
actgccaagg cagagagttt gtaaatattt tctagagacc tcaaaaaggg aggagagtta 79440
agttgcctgc tgctcaagaa gaccaagcct ggcagaatgg tttcccatgg tgctgtctca 79500

ggggacactg gtcggcagca acgtgtttgg atggttaaaa taaagccagc aggcagaaga 79560
gaggggcaga gatgtggttc agttgaggcc acatggtaag gtatgcccac tgtgcatgtt 79620
agaggagagc cctcaagaaa ggagggaaag aagcaggaca ctaggatggg gctagaggga 79680
caagggagcc accagcccac aggagcctgg gacttctgct atagttcatg atctcttaag 79740
acacagtcgg ctcccaactc ttattttgct tttgcttgtt attttctgct tgcaatatta 79800
ggtagccagg gggacagaag tgctgcaaag agtggggaca ctcggtgcct cacctcggca 79860
gctttttaaa tagctacaga gatagagaaa atagcccccc agactccaac tggctactcc 79920
ccaaactcac ccgtcctggc tgactgagac tgaagtgaaa gagcttcaaa tgtagaaagt 79980
ttgagaaaag ttttcttgag aagagatcat ggtctttgtc cctttcctaa cgtgtgtttc 80040
tcttcctttg cttggcaagt gtatctctga tacacatgtg atcattaggg cctatctcac 80100
acttaactga aaaaatatac ttgtggtcac cctctcctac ttctcatgtc aaactgctga 80160
gaagttttga aaacagagtt tagccacctt cagagtgtgt gggtgttgtg tgttagaaaa 80220
cagaggctct ctggtcacag ggaaggcagg gatggggact gcagtgaggg cagatgtgct 80280
cagtggtgag tggtggggag aggggagaga aagcgaggaa gtggagccga gtgctggagg 80340
tgaggctgag gtcactgttg agcctctcca ttctgtcagc ttggatcctg gagggatttg 80400
ctaaaccata tccttctata atgtttccta gtcatgttga gattccaaaa tttgtcactg 80460
ccctacagta aagggttcag caatgaaaat aaagaagaag aagaaaaaat gccatgagaa 80520
aacaaaagga ttggaagaac cagttcctgt tcttgaggtg tctccaacct agtggatgag 80580
aaataagacc tatatatata tagaactcga gaaaaagtat aagttaaaag ttggccgggc 80640
gcggtggctc aggcctgtaa tcctagcact ttgggaggct gaggagggag gatcacttga 80700
gcccaggagt tcgagaccag cccgggcaac atggaggaac cttgtctcta caaaaaaata 80760
caaaaattag ccaggcatct gtagtcccag ctactcagga ggctgaggtg ggaggatcac 80820
ctgagcctgg gaggcagaga ttgtagtgag ctgagatggc accactgcac tccagcctgg 80880
gtggcagagt aagaccttgt ctcaaaaaaa aaagttgtaa aaggactgct ttgctctatg 80940
gaggtctttg cttttattct aaatgcccac atttgaggca tttaggcagg gaagtaacgt 81000
gttctttaaa aagaataata aggtaacata caggcatcgg tgagacaatt tggtataaat 81060
tgtatattta ggcattaagg gaatgcagaa acttcagaag tacttgggga agggaaagca 81120

ttcttggaaa atctaaattt tgagaagggt cttgaactgg aagaatttag attttcagag 81180
aagcgggaga ggacatccta gttgaggggg agtgcaggaa tgaaggcaga gaggtgggac 81240
tacacgggac gtgtgcagaa agcgagtctg gctgaaacgc gatcaagaag tatgaaatac 81300
agtatttctc aggcttcact cttccttcac attctgcaat atccacacac cagggcagtg 81360
tggtggttga gagaataaac tctggtgtca gatgggctgg gtttgatgct ggctccattg 81420
gtcaccagtt ttgtgacctt gggcaagtca cttaacctat gttccgtgtc ctcatgtaaa 81480
gtgaagataa ttgtatgtac ctcacagggt cactggaaag cacttagaac agggcctgcc 81540
acatggaaaa cacttggtaa atgcttgggt ttttttcctc tgtttgattt ggttcacatt 81600
ttctttcttt ttttcttttt ctaaaataaa tgtggttttt aaggaaactt tatatcacta 81660
acataaacgg aaaaccagaa tcactttcca caagtagaag gtaactatga aaaataaaga 81720
caatgaaaca gagcatttca ttaaattttg atcggatact actgtgagta cttggagact 81780
aagatccctt cccccttttgt taaaaacaga gattaccaag tgtgagatta gtgttaaaga 81840
caactggcat caaattaagt ctaaattttt ctccttagta tgatccagga gactgaaaga 81900
gaactggaaa gaaaataatt atcctgctgt gtggacccag gtttttaaat gctccatgtg 81960
cttgctggca atttgcatcc tacatttggg gaaacactga aaaaagataa tgcatggtag 82020
gaccgaatgg tgggcattcc gaatggccca ctgtagggtg tgaatccgaa ccaagagcca 82080
aggctacctg cccttgagaa agacaggttt gtggttaacg taacagcaat gtgtaaggca 82140
gaccagaaca gtgagaaatt ggagccagaa gaaccagcta aaagatctat tcccatgaga 82200
tgaatttgtc tttgcatccc cacaagcagc tgaccttaag gccaacctga tagtttccag 82260
agaaatacct gagagagaaa aaaaatatgc aaacgaaaat accctgtctt ggaaactcaa 82320
tgagaagcaa cagcagagta gaccttggcc aaagtaatca atgtttttta ttttcagttt 82380
ccagattgga tatatctttg gtcaaggcca tctggaagac tgagaacatg agagccagct 82440
aaggtcacag acattcattc cctagaatgt gacacagaaa tgatgaactt cggtttcttg 82500
gcctttttaa ggttattggt tattttccac tgtggccacc tctattcatc tccctaaata 82560
ccaggggtcc ttgagaaact ttttctcttt tcacccaaca gagaggctcc agaaggtttc 82620
tgggtggggt aggcacatca caaactctga ttttatcctt tctcctaaga gtttgcttta 82680
cttaacaagg cctacaattt cacaatctat accacctgca gcatatgtca gaaaacagct 82740

gagtgctaaa attgtaaaag gcaaaacatt aagaggaaga taaacccctg gatgagtgga 82800
aatatgtgac tgaattctgt agttaacaca gaccattgtt cattgttaag gagatggggc 82860
agcctcagat gttcacgtgc atgcaacttg caatcatcag atcccctcgt ctctgagtta 82920
cggtaacttg agcagtgcag atgattatca gtcacctgta gacacgatga ctcaatgtgt 82980
atgaaaccca gaaagggtaa actacagtaa ttgaaaagcc aacagctcac gtttctttag 83040
taacacactc ataaaaaagg ttagtcaaac cacaaaagtg taactgcttc caaacctgca 83100
cggagcatct ggtccagctt ctagtcaaaa tcacaccacg cctttgaatg attcatgata 83160
ccagcttcag gctgaattag aggtcttctt ccaacaaaaa tacacacggc aacagcaagt 83220
cctaagttta gaaactaagg ctgcctggtt gggccattcc ctacctgtgg ttctttttaa 83280
aggtgtcaat gtcttatatc ttggctttgt taactctaaa ctaagaaacc acctgacctg 83340
aactgatgca atgtggtgag gacatatggt tgttggcttc taagtgccaa cttggaagta 83400
cagaatcaca ggctagagct aaaagggccc ttaactgagc aagtggattc agaactctgt 83460
ctgcagggtt gagggggaag acccaccacc ctaatatgtt tacttctggt ctttatattt 83520
cacagtacct tgcactgtgc cttatataca ataatcccaa taatgataag tactatttat 83580
tgagcccctg ctgtatccac cgtgccatcc tagatacttt gcatactgtc tcattttacc 83640
agcccattag gggttgatac tttttttttt tttttgagac agagtcttgc tctgtcaccc 83700
aggatggagt gcaatggtac gatctcgact cactgcaacc tccgtctcct gggttcaagt 83760
gattctcttg cctcagcctc ccaagtgtct gggactacag gcacgtgcca ctatgcctgg 83820
ctaatttttg tatgatacct tttaaggtat gaattatcat ctcttagatg aagaacctaa 83880
agcacaaaca gtttgattca ctttccaaga ttgcccagtt ggtgaacaga gaaccaggat 83940
gtaatccaag agcccacata tcccagtata tgtttgagtg ttaactgatg tttcctggct 84000
gctaccttgc ccatggctgc acagccccac caccagcagt gacttcaggt tttctgctgt 84060
gatggttccc atagcagagg gaaagggggc ttagtggcag caactgcttt ttccacatgg 84120
accagcacat gcactcaaga agtatatgct gtgcagagtt gttatgatat ctataaagtg 84180
cttagcagtg ccaggcatgt gtaagcactc tattcccatt atttgcttag caccatggtt 84240
aggtgatttg aggaatacaa caaaattgca atccactaga ggagatgcag catgtacatc 84300
atttcatgac aaatcagtat ccttaatgct acatggtaga acaaaacata gagtagccaa 84360

gagagtggaa agagaaaact gaattacttc tcaaatatgc cacacttaat atggggcaaa 84420
gattcatcta ttctttaaag tctccttgaa atcagaggtg gtggtagtga cggtggtgga 84480
gacagtcata tctataataa gaagcagact agtaaactcg cctggagtgc agagggagga 84540
tggagcagag agagaacaca ggggtgtggg aatgtggaca atgtgtgctt ctttttattt 84600
tttattttg agacaaggtc tcactctgtt gctcaggtgg gattgcagtg gcgtgatcac 84660
agctcactgc agcctcaacc tcctgggctc aagtgatact cccacctcag ccccctaagt 84720
agctgggacc acaggcccat gccaccacac ctggctaggt ttttgatttt tttttttttt 84780
tcagagacag agtttcacta tgttgcttgg gccggtctca aattcctgag ctcaagtgat 84840
cctcccacct tggtctccta aagtgctggg attacaggca tgaggcactg tgcctggcct 84900
acaatgtgta ttttaagggc agatgtgagg gaggccattc agacaagagt gagatattac 84960
acagaagagg gaggaggagt tcattgtggc tgaagtaaag gactggttgg ctgaaatgat 85020
aaaattagaa gaggtaagtt gaagacagat tgtggagggc tcagaaggcc tgactgaatg 85080
gtttagattt gattccactg ggagccattg aaaggttttt ttgaagaggg gggcaaaatg 85140
atcgaacctt tagaaaaata acagaaatcc aatatttaga gtcaccatga tggggtaagg 85200
tgaaatattc aggtcttaag tcttaaagtt cttcaagtca cttaaatgtg tttgctgctg 85260
cgtcatataa tttcccacaa gtgttatccc tcactcttac tatcagcaca catccaaaag 85320
caaatgaaca aaaaaggcag gaaaatagaa tatccaacca tcatcatggc tctgatctca 85380
acctaagtga aactaacact tcttgcagga ggtggaaaac agaggggagc cagagaacaa 85440
gaagaaacag gaaggaggaa agaacagaga gagctgtggg ggtggcactg gggaaagctg 85500
agctggagac cacatttggt tgctgcagaa gtctttgtcc tctgtgagac actgggaagt 85560
gctgtgtgga tgtcatctgt tctcatggag tcaccaggga agactgttct tttctaaaga 85620
gaatcaccct tgagggagaa caattgccct tcttcaaggg tttttttcct cctatcaatt 85680
ttcttttttg tttttgaaac agagtcttgc tctgttgccc aagctggtgc gatctcggct 85740
tactgcaact tctgcttcct gggttcaagt gattcttgtg cctcagcctc ccaaatagtt 85800
gggattacag gcacccacca ccacacctgg ctaatttttg tatttttagt agagataggg 85860
tttcaccata ttggcccggc tggtcttgaa ctcctgacct caagtgatcc acctgcttag 85920
gactcccaaa gtggggaatt acaggtgtga gccactgcac ctggcctctc ccaccaactt 85980

tcaatgacca gactccttgc tgtccaaagc cccgcgcatg acatgcactg ctcttggtca 86040
cctcctgggg aaggaagggc ctggactgca gtgccttata ggccgaggtc tggcgctgct 86100
ctgtcacctg ccagctgtgt cactccacct ctctgatcct ccttccttcc atggtaaaag 86160
agcctcactg gtggttccta atagcccaaa aatactatga cattgtaatg cttacaggat 86220
tatggacatc aggcctttgg gaaggggttg gaaaaatact tttttttctc ttagaaacag 86280
gtattaaaac tgcttcctgt tgctgttaac caaagcaggc aatttctcat tagttacttc 86340
acagggagat ccgatggttt gttgaagtgt caaaacctca ttaacatgtt taccacaggt 86400
gagtgagatc ttttttttcat gttcttttcg ttatgaaaac ttcctttaat aaagtaatac 86460
ataaaacttt taaaataagg agatgctatg gtaaccactt tggtctgaat aacatctgtt 86520
tttttgggaa agaataaaaa tcaggagaaa tatgcagaaa tgtagtcatt tttcttctgc 86580
atccaagaga gaaatccatt ttgagccaag tatttgagga tggcctagtt tgaagtgtag 86640
tcaaacaagt ttataacagg agaacaagat tcagagaaag gaaatcccct aaaggacagt 86700
atgtgcaaag gaaggtcttc ttgtcctttg aaaagaacaa cttaaacaga attattctga 86760
caatgtaaag atggtgattc ctgatggacg tgtgtccatt tttttttttc ccatttgaaa 86820
caaagacata caaaatttct tggagaatta gaaaaaaaaa tatttttctc caacttgaga 86880
gcatgtttgt gtctcaaaat acatttagac ttgatccaat ctgtccctct tccccagttc 86940
atctctaatg gatgcaaaat gctccctact ggtaattatc gccagcccgc ctgagatagc 87000
agcgggctga cctgatggtc tattttcacc tttaccctca cagttcaaga ggtcagggaa 87060
gtcaaggctg gtctgagtgc tgtgcaactg tcagcaagga ttaaagggaa tgactcactc 87120
ttccttcaca ccttacccat tgccattacc ctggcagctg aatcttcaaa aaacagtgcc 87180
ttggagagga aatataggag aaaggagaga agaggagaag agaagggagg agaggaggga 87240
agaggagggg agggaacaag aggacaagag aggagaggtg aggtgaggcg aggcaaggcg 87300
aggggagatg gagagaaaac agtagcccca tgttttacat cacttcatct catacactgc 87360
acagtgtcac aaataatgtt cctttctccc tccgttgaca ttactatttt ctatcacaaa 87420
ggttgattca attaaataat ttaaggggtt tcttttattt ttaattttta aaaacctttc 87480
aaacaaaagt ttaggatata agttaatgca agaagattag aacagtgccc aaaacaagaa 87540
taccatatat atgacaggaa ctcttaacct tctgtatgat gaatgaatgg acaaaaaagt 87600

gagtgaacaa gtagtactgc cctaagtaaa acacatgaat gtcgggctaa tgcgactgtg 87660
ttttagattc tcagatttat accatcttcc taaaacaaaa aagactctaa aaacctagga 87720
tatggtttat agcataaaca ccacatctgt atagccttga gaaaaaggct atctgaagtt 87780
acgcacatgg gctttctaaa cacacagtgg tatcaataat tgctgccttt tatttctttt 87840
ctaattaatc tcacagactt gtacatggga ggagacttcc tggtcacttc atcacctatt 87900
cagtctcctc atttcacaga tgagtaaaga gaggcccaga aaggttctgt gattggtcta 87960
aacacacagt taagaaatga cagagctagg atcagaacct gggtctttta gcttctaatc 88020
cagtactctt cttattgcac tacaacacaa cttacgaaaa aaaaaaaaaa ttcccccagt 88080
tattctgggc cctgaagccc tgaagttgct acctcaatca agagctaact ctactaggtt 88140
tctgattcca gcacagagag aatgtgatgg ttcagaagat gagagaaatc aacacctaca 88200
agatcagcat ctctcacaag tctggaagta gtgaagtgaa aatcctctgg ttgcaattgt 88260
agcaaacaaa cgaaatagat ccccgaggca acaggcaaaa tggcatgaat ttgcacatat 88320
gaaacatgct atatttttga acccatgaaa taccaaagca acttttatta tattaagtag 88380
ggctttagtt caactatagg tttagggcaa ccgaaaatac tagggactgt ctctcaaatt 88440
ttagttgtct tacaccaaaa tgcattgacc acaactggat tgaaattaag tcacagctgg 88500
gcgcagtggc tcacgcctgt aatcccagca ctttgggagg ccaaggcggg tggatcacct 88560
gaggtcggga gttcaagacc agcctgacca acatggagaa accctgtctc tactaaaaat 88620
acaaaattag ccaggcatgg tggtgcatgc ctgtaatccc agctactcag gaggctgagg 88680
caggaaaatc gcttgaaccc aggaggcaga ggttgcggtg agccgagatc gcgccattgc 88740
accccagcct gggcgacaag agtgaaactc tgtctcaaaa aaaaaaaaaa aagaaagaaa 88800
gaaagaaatg aagtcgcttt acattttttt cttgcccagc tctccctatg atgtccctcc 88860
tcttgtcggc ttcagccatt tctagagtca aagctttctc cagaccagcc tgggcaacat 88920
gaggagactc tatctctaca aaaagtttaa aaattagctg agcctggtgg tgcgtgccca 88980
tagttccagc tacttgggag gctggggtag gaggatttct tgagcccagg tgtttgaggc 89040
tgtgattgca ccacagcacg ccagcctagg cagtacagtg agaccccgtc tcaaaacaaa 89100
ccaacaaaca aaacccataa ctttctccaa actaaaatac actcagaata ataaggcaag 89160
ctttcttttg tactccagtc agaacctctt cacataagta aagcccaaaa gatatatggg 89220

aatatttgta gacaatgtct taaactcacc atttgtttac tgtgcctaaa ctctaaatgg 89280
aagtcactac atgctcccct gacatgtctc accaagggga gggaggacag caaggaggag 89340
gggatctccc taaactgaaa tataccaact tttctctgac acaatttgtt cttaaagtga 89400
ctaaaagtgc acaaaaacct ttcagctagt caaaataaaa taagtaggca gaaataccaa 89460
attaggtaat tttgaaatat atgggttctt tcaatatacc tctataattc acatagactg 89520
tttgcttgtc aaaacattat ctataccaga atcaaattta gtcagattta aaaaccaggc 89580
aggcaactta ttatctgtaa gagctattga gaagaataaa tacaaaagga ctgaaataaa 89640
aaatcaacag cggcaagcag ctttggaaaa agaggctgag acaggtttct aaaatattac 89700
tgatgttgag ggaggtctat tttcatgtga taactatttt tgttcaatat caccaacttt 89760
caatttatag caaaactgat acattcaatt tcagctgtct acttcacaaa tacaaatcta 89820
tttcaaatat catggtcaca tttatgtaaa catctgggag agttcagcaa aatgtctctg 89880
ccatttggaa gtcctaacac ttcaatgaac ataattattt catacccatc tcattctgct 89940
gtattttttg acaatgtgaa aaccaatggc ataaaaatga actccagtaa gacaaatgat 90000
taaatcaaac cttgttattt aaatctatgt ttgcccaacc actttccctt aaagccaatt 90060
ctcaaattca agaaaatatg gcaatatgtt ttagagcatg aaaaaagttt gagcatgcta 90120
acgaaccaac ttgtaaaata taattacaaa agattacaaa aatgctatct tgaacatctt 90180
actggtataa attgcataaa ctctctcatg caacatatgt cattcagagt aaagaggaag 90240
gaacaggatt ttaaacatca catccattta ggaaattgga tatcaactac tcccatctcc 90300
tgtcatgatg atgctttagt taatatcttt tattctattt tccagagcta tcagaatgaa 90360
ccatgaagaa taaaagtaca caatgaaaaa ctaattattt taacagaggt taagctagga 90420
aaggccttgt gtagcaagca tcctatgtta tttcattcaa actatctgct aacaacagca 90480
ggaaaaaggt ttgtattctt accaatgaat ttctgaggca ttgagctttt tcgttttgcc 90540
acattgcttg ctaatctgtc cagtacgaga gctctttcac ttcccatctc tgctttgatg 90600
tgtcttgcct ccgcacttgc tagtttggaa aaatgtaaaa agaagagaga aaagaacaca 90660
ttggtacatg gggagaaaag gaaataaact ggaaggagaa gtgtccgaag ttggatgcac 90720
actctattgt tgttacctgt tataactgct cagattcttg cctggggtac ctgctgtggt 90780
cagtgaagcc gacaccaggg cacattcaaa tcttgcagtc ttatgccgag atgggaaagc 90840

tcgacccatc cccaaccagt accttcatta gcaagaggaa gtattaaata aacagcctgg 90900
acgtggttgg ttgcaaaaag atagatagat ggagatctaa cccccgatca gccttctttc 90960
tgattctgag taactgcgtg tgacacctgc agactgatat aattcttaac aactttgaaa 91020
aaaaagcatg gtgccccaga tttactgctc tgtttatgtt aacagcactt ttgaaaatag 91080
gtacactcac acagatagca agacatctac tcagtgattt gtgtattgaa attctgagtg 91140
gtttctttct ttttcttttt cttttttttt ttcagcacgc tcctctcagt tcctatcttt 91200
catattctgt ttctctgtgc tctgtattat ctgtttcata tgctcttcac atttcaaaat 91260
agtctcaatt aaaatttccc taagcaacac ttctgccttg aaattaaaga acaatttgta 91320
taatgcatct gtattgaatt tctaataaaa atgtgaataa aaactgccta tgggctgggt 91380
gcggtggctc atgcctgtaa tcccagcact ttgggaggtg gaggtgggca gatcacctga 91440
ggtcggagtt cgagaccagc ctgaccaact tggagaaacc ctgtctctac taaaaataca 91500
aaattagcct ggtgtgatgg tgcatgcctg taatcccagc tactcgggag gctgaggcag 91560
gagaatcact tgaacctggg aggcagaggt tgcagtgagc cgagatggcg ccattgcact 91620
cctgcctggg caacaagggc aaaattccgt ctcaaaaaac aaagaaacaa acaaaacaca 91680
aaaaaacctg cctatgaatg tgggcccatc cacaaataga cctgtgagtg gtgtaggcct 91740
gtggttctca gacctggctg cacattagac tcacctgaat agcaggtaag aaatacctat 91800
gcccaggccc cccttcagat caattaaatc aggatttctg gtataggggc ctaggcaaga 91860
atattttcaa agctccccag atgattttaa aatgcagcca gaattgagaa ctactagaag 91920
agtatataaa tagtggcaat atctaatcaa ctaaaatagt aaactgtaat gaatataatt 91980
tacctcaatt cagcaggcag tgcccaaatt tatgtctaaa gttcaaagaa ggccaggcac 92040
ggtggctcac gtctataatc ccagcacttt gcaaggccga ggcgggcaga tcacctgagg 92100
tcgggagttc aagaccagcc tgaccaacat ggagaaaccc catctctact aaaaatataa 92160
ataattagct gggtgtggtg gcgaatgcct gtaatcttag ctctcgggag gctgaggccg 92220
gagaatcgct tgaacccagg aggcagaggt tgtggtgagc cgagattgag ccattgcact 92280
ccagcctggg caataagagc aaaactccat ctccaaaaaa taaataaatt aattaaataa 92340
atttcaaaga gataccagct tctctaaata caaggcttga cattttaaca catgatgcta 92400
gcctgaacca gggatcagca aacttttgtc tgtaaagggc cagacagtaa atttaaaaa 92460

tttgccagac acatatagtc tctatgttat attctttgtt ttttgttttt ttttttctg 92520
ttccacggcc ttttaaaaac gtaaaaatcc ctgtacacaa acaggccact ggctgcactt 92580
gcctagagcg ccacagtttg ccctcacctg ccctgggcaa acatattgtg atgctgtaaa 92640
catttttctc tgtttctca taaacacttc gggagatcca aactaaaatg aactatccat 92700
acatttctat tatgtctaag gaagaataaa ttgaaattag tttaagggat tagagtaaaa 92760
agcaagtagt ggctggcaca gtggctcacc cctgtaatcc cagtgctttg ggagactgag 92820
gcaggaagat tgcttgaggc caggagttga gaccaacctg ggcaatatag caagatcctg 92880
ttcctacaaa aagtaatttt aaaaaattag ctgggcatgt tggcatgcgc ctgtagtcct 92940
agctacttgg gagactgagg tgggagggtt ccttgagccc aggggtttga ggttacggtg 93000
aactattaag gtaactgcaa agaccacaat tacttttgca ccaacctatt agtgccagtg 93060
cactccagcc tgcgtgacag agtgagactc tgtctaaaaa aaaaaaaaaa agcccccaaag 93120
tattaaatat ttctaatttt attagtggcc ttaaaaaact gtatgttagt cattacacaa 93180
tacattataa agctcagttt tcattattta gttaatttat ttacttattt ttgaaacagg 93240
gtctcaactc tgttacccag ggtggagtgc agtggcatga tcacagctca ctgcaggctc 93300
aacctcctgg gctcaagtaa tccttctacc tcaggttcca aagttgctgg gactacagtc 93360
taaagccacc atgcctggct aattaaaaaa aaaaattatt agtctcaata tattgctcag 93420
tctggtctcc aactcttggc ctcaggcagt cctcccactt cagcctccca aagtgttggg 93480
attacaggca tgagccactg cactcagcca aagctcagct tttttttttt tcctggaata 93540
acataaaatc tggctctgcc aggtgtggtg gcactagccc atagtcccag ctactcagga 93600
ggctgaggtg agaggatggc ttgagcctag gagttcaagt tcagcttggg caacacagtg 93660
agatcccatc tcttaaaaag aaaaagaaat ccagctccaa aaatgaaaaa gaagaggatg 93720
ataataatgt gataataatg acaatattta ctagacagtg agtttcttgt caaaaataat 93780
tctaggctct aaaggaataa atcatagcct tcagtgaagc tattaacaac tatttgtgta 93840
aatgaaaaca gtaagggtca gaataggggg cctgagtggc ttttgggcta gtgcaaaagt 93900
actcgactga ctctggctga ccagcagttg taaataacgg gcacttcagc cacaagacac 93960
tgctattgag tgaagagggt caaattctgt ccagcacagg gataggttta aatgaccaaa 94020
caggaaatta tttatatata ttatttcctt tcacacaaaa ccaccaccag atgtggcact 94080

acccaaagcc ccaagagcag agagatgaaa gacaaagctt g 94121

<210> 3
<211> 20
<212> DNA
<213> Artificial Sequence

<220>
<223> T7 primer

<400> 3
taatacgact cactataggg 20

<210> 4
<211> 18
<212> DNA
<213> Artificial Sequence

<220>
<223> M13R primer

<400> 4
caggaaacag ctatgacc 18

## Description

### Field of the Invention

A diagnostic technology for a breast cancer, in particular, caused by Her-2 copy number variations is provided. Said diagnostic technology identifies the amplification due to Her-2 copy number variations, thereby allowing a correct, convenient and rapid diagnosis of breast cancer caused by the amplification due to Her-2 copy number variations. As such, said diagnostic technology is highly beneficial for customized treatments of breast cancer in the therapy using Her-2 antibody (e.g. Herceptin), and the like.

### Background Art

Breast cancer is a major gynecopathy, and thus, a number of diagnostic and treatments methods for the disease have been developed. However, many patients with breast cancer still die of or suffer from that disease.
Depending on the amplification of selective factors, there can be five subtypes of breast cancer, as shown in Table 1.

[Table 1]

| Type | Luminal A | Luminal B | Basal-like | Her2+/ER- | Unclassified |
|---|---|---|---|---|---|
| estrogen | + | + | - | - | - |
| progesterone | + | | - | - | - |
| Her-2 | - | + | - | + | - |
| Her-1 | | | + | | - |
| Cytokeratin 5/6 | | | + | | - |

Approximately 25% of the total breast cancer cases are due to a variation in gene copy
Approximately 25% of the total breast cancer cases are due to a variation in gene copy number in Her-2 (Human epidermal growth factor recaptor-2). As shown in Table 1, the two types of breast cancer are associated with Her-2 amplification. The relationship of the Her-2 gene with breast cancer is discussed in WO 2007/081613 A2, WO 2008/089577 A1, WO 2006/133460 A2, Journal of Pathology, vol. 216, no. 4, pages 399-407 and Breast Cancer Research and Treatment, vol. 118, no. 3, 24 January 2009, pages 481-498, respectively.
Treatment of breast can be carried out by 1) surgery 2) radiation therapy 3) chemotherapy, or 4) hormone therapy. Recently, attempts of customized therapies corresponding to types of cancer are being made for effective treatments. The most commonly-applied one is oral administration of a certain medication which specifically inhibits the amplification of Her-2 gene. Such approach excludes application of unnecessary treatments resulting from uncertainness of diagnosis, avoids undesirable side effects, and thus relives economic burden of patients.
A precise diagnosis should precede such customized treatments. While there are pre-existing technologies including IHC (Imunohistochemistry) and FISH (Fluorescent In Situ hybridization), those methods take much time and labor, with relatively low degree of preciseness. Further, they have critical deficits in that trained technicians should be used, and that diagnostic results cannot be quantified and are test to researcher's testive determination.
Therefore, development of technology is needed which diagnoses Her-2 gene amplification in a correct and prompt manner, such that a precisely customized therapy can be performed without application of other unnecessary treatments.

### Summary of Invention

To address the above-mentioned problems and needs in the existing technologies, it is aimed to provide a diagnostic technology for breast cancer caused by Her-2 copy number variations.
More specifically, an embodiment provides a composition for diagnosing breast cancer, comprising a marker specific to breast cancer caused by Her-2 copy number variation.
Another embodiment provides a use of a marker specific to breast cancer caused by Her-2 copy number variations for diagnosing breast cancer.
Another embodiment provides a microarray capable of detecting a marker specific to breast cancer caused by Her-2 copy number variation.

Another embodiment provides a DNA chip for detecting breast cancer, capable of detecting a marker specific to breast cancer caused by Her-2 copy number variation.

Another embodiment provides a method for detecting Her-2 copy number variations, using a marker specific to breast cancer caused by Her-2 copy number variation.

Still another embodiment provides a diagnostic method for breast cancer, using a marker specific to breast cancer caused by Her-2 copy number variation.

## Detailed Description of Invention

The present invention relates to diagnostic technology for breast cancer which is caused by amplified expression due to Her-2 copy number variation. That is, the present invention relates to a composition for diagnosing breast cancer containing a marker specific to breast cancer resulting from Her-2 copy number variations, a use of said marker for diagnosing breast, and a diagnostic technology for breast cancer using said marker. The present invention enables one to diagnose breast cancer resulting from Her-2 copy number variations in a correct and prompt manner, thereby allowing effective treatments customized for individual breast cancer patients.

The present inventors have examined BAC (Bacterial Artificial Chromosome) clones corresponding to markers including Her-2 gene, and have performed qualitative and quantitative analyses of aCGH (array-based comparative genomic hybridization) data using the cell lines of breast cancer patients which have been diagnosed as breast cancer by the conventional methods. The present inventors have thereby established a technology to assess Her-2 copy number variations, to complete the present invention.

In an embodiment, BAC (Bacterial Artificial Chromosome) clones including Her-2 gene are uniformly spotted on the slide, with a sample having normal Her-2 copy number and a sample suspected to have breast cancer being labeled with different fluorescent dyes, respectively. Ratios of the differences in fluorescence between the two samples can be quantified and can be used to exactly determine whether or not there is an amplification of Her-2 gene. Compared to other conventional methods such as IHC (Imunohistochemistry) and FISH (Fluorescent In Situ hybridization), the method according to the embodiment of the present invention saves test time by half at least, and can obtain an exact result at a significantly low cost.

More detailed description of the present invention is provided below.

An embodiment provides a composition for diagnosing breast cancer which comprises a marker capable of diagnosing breast cancer. Another embodiment provides a use of said marker for diagnosing breast cancer.

Said marker may be selected through the steps of:

(a) identifying genomic DNA fragments inserted into individual clones from the clones obtained from the genome library of a living organism;
(b) choosing a target chromosome region, to which chromosome variation is examined,
(c) among the genomic DNA fragments identified from step (a), selecting the genomic DNA fragments capable of detecting the target chromosome region.

Said breast cancer may be one caused by Her-2 copy number variations.

A nucleotide sequence constituting the marker comprises a single locus, and may comprise all or part of the target chromosome region, or complementary sequence thereto.

The marker specifically selected to examine the Her-2 copy number variations may be one or more selected from the group consisting of:

The genomic DNA fragment corresponding to SEQ ID No 2 of human genome chromosome region 17q12, as presented in Table 2; the complementary sequences to the genomic DNA fragments.

By using the marker, one can diagnose a breast cancer caused by amplification or reduction due to change in Her-2 gene copy number in a more rapid and correct manner.

The genomic DNA fragments at human genome chromosome region 17q12 are determined based on the human reference sequence (NCBI). According to the version of NCBI Build 36.1 (March, 2006) which is the most recent version at the filing date (2009.03.04) of the priority application of Korean Patent Application No. 10-2009-0018575, the location on the $17^{th}$ chromosome is indicated by the sequence from 35096879 to 35191098, which is 94220 bp as in SEQ ID NO. 1). According to the version of NCBI Build 37.1 (August, 2009) which is the most recent version at the filing date of the present application, the location on the $17^{th}$ chromosome is indicated by the sequence from 37843402 to 37937522, which is 94121 bp in size as in SEQ ID NO. 2. Table 2 summarizes the above.

[Table 2]

| Version | chromosomal region detected by FISH | Location on human chromosome | | Size (length, bp) | Reference sequence |
|---|---|---|---|---|---|
| | | Start | End | | |
| Ver. 36.1 | 17q12 | 35096879 | 35191098 | 94220 (SEQ NO. 1) | NCBI Build 36.1 (March, 2006) |
| Ver. 37.1 | 17q12 | 37843402 | 37937522 | 94121 (SEQ NO. 2) | NCBI Build 37.1 (August, 2009) |

Another embodiment of the present invention relates to a method for providing information necessary for breast cancer diagnosis, by using said marker or said probe, and a use of said marker or said probe for breast cancer diagnosis. The breast cancer may be one caused by Her-2 copy number variations.

More specifically, the method for providing information necessary for breast cancer diagnosis or the use for breast cancer diagnosis may include the steps of:

hybridizing said marker or said probe with a test DNA sample, and
measuring the degree of hybridization of the marker or the probe with the test DNA, to compare it to the degree of hybridization with reference (normal) DNA.

The method for providing information necessary for breast cancer diagnosis or the use for breast cancer diagnosis may be used to determine the occurrence of breast cancer caused by Her-2 copy number variations, by mixing and reacting a test DNA (labelled with a detectable label ('first label')) and a reference (normal) DNA (labelled with a detectable label('second label')) with the probe fixed to the microarray, at a 1:1 ratio, for example, and measuring hybridization rates for the test DNA and the normal DNA, respectively. When the test DNA's hybridization rate is identical to that of the normal DNA, it can be determined that there is no Her-2 copy number variations and that the test is not associated with breast cancer caused by Her-2 copy number variations. When the test DNA's hybridization rate is higher than that of the normal DNA, it can be determined that there is an increase in Her-2 copy number and that the test contracts breast cancer caused by Her-2 copy number variations.

the first and the second labels may be ones conventionally used for DNA labeling, and may be selected from the group consisting of radioisotopes, fluorescent compounds, light emitting chemicals, and enzymes. They may be the same with or different from each other. Said labeling-detection means may be any conventionally used one, and can be properly selected by those skilled in the art, corresponding to the label used. The test sample may be body fluid, cells, and/or tissues derived from mammals, preferably, mammals' mammary cells and/or tissues, and more preferably, cells and/or tissues derived from breast cancer. Said normal DNA is obtained from an individual with no variation in gene copy number, particularly, in Her-2 gene copy number.

According to the present invention, it is possible to simultaneously carry out the diagnosis of breast cancer and the investigation of biological and histological characteristics of individual breast cancer cells. Assessment of biological status of breast cancer for each tumor and tissue is of high importance in order to minimize patient pains by minimal invasive surgery and to provide customized treatments appropriate to individual patient's conditions. In the present invention, the type of breast cancer is correctly determined based on the information about DNA copy number variations measured by aCGH (Array-based comparative genomic hybridization), thereby allowing distinctions among different types of breast cancer.

In the present invention, DCNVs (DNA copy number variations) of the test DNA sample, which is hybridized with the probe, can be measured using any conventionally used methods. In a concrete embodiment, DCNVs is investigated by aCGH (cDNA, Oligonucleotide or BAC clone Array-based comparative genomic hybridization).

aCGH, which is a second generation CGH, can result in an excellent resolution, compared to other conventionally used CGH assays, and make it possible to perform a locus-by-locus analysis of high resolution for DCNVs (DNA copy number variations) with only a single time performance. The present invention, by using aCGH, establishes a novel and precise diagnosis of breast cancer caused by Her-2 copy number variations.

So far as known at present, there has been no reported diagnosing technology using aCGH assay for breast cancer caused by Her-2 copy number variations.

In a concrete embodiment, DCNVs (DNA copy number variations) are determined based on linear means of DNA copy number. In the present specification, 'linear means of DNA copy number' means a ratio of a test sample's DNA copy number (T) to a normal sample's DNA copy number (R), and is defined by the formula below.

$$\text{Linear mean of DNA copy number (T/R ratio mean)} = \frac{\text{Fluorescent signal in a test sample* (mean)}}{\text{Fluorescent signal in a reference sample** (mean)}}$$

In the above formula,

'*' refers to the fluorescent intensity of the fluorescent material used to detect the DNA copy number in a test sample. The fluorescent material may be any fluorescent substance that can be used for DNA detection purposes (e.g., Cy3, and the like). The fluorescent intensity may be measured using a conventionally used method in fluorescent signal detection.

'**' refers to the fluorescent intensity of the fluorescent material used to detect the DNA copy number in a normal sample. The fluorescent material may be any fluorescent substance that can be used for DNA detection purposes (e.g., Cy5). The fluorescent intensity may be measured using a conventionally used method in fluorescent signal detection.

In a concrete embodiment, when a T/R ratio mean is 1.5 or more (T/R≥1.5), the test sample can be diagnosed with breast cancer caused by Her-2 copy number variation. When a T/R ratio mean is 1.35 or more but less than 1.5, the test sample can be subject to re-examination as it is likely to develop to breast cancer caused by Her-2 copy number variation. The above cut-off means were obtained through various experiments on various cells from breast cancer caused by Her-2 copy number variation (see example 3).

As described above, the diagnosing method of the present invention for breast cancer caused by Her-2 copy number variation enables a prompt and correct diagnosis of breast cancer, compared to conventional methods, such as IHC, FISH, and the like. With the present invention, types of breast cancer can be easily identified.

## Brief Description of Drawings

Fig 1 is a schematic view briefly showing a manner by which aCGH method is performed, which was carried out in an embodiment of the present invention.

Fig 2 shows the structure of the BAC chip used in an embodiment of the present invention.

Fig 3 shows chromosomes and genes corresponding to each spot.

Fig 4 shows the results of the BAC chip analysis, IHC, and FISH for the test sample isolated from normal tissue (Normal B-Lymphocyte (46, XY), GM10851).

Fig 5 shows the results of the BAC chip analysis, IHC, and FISH for the test sample isolated from normal tissue (Normal B-Lymphocyte (46, XY), GM10851).

Fig 6 shows the results of the BAC chip analysis, IHC, and FISH for the test sample isolated from breast cancer tissue (MCF7).

Fig 7 shows the results of the BAC chip analysis, IHC, and FISH for the test sample isolated from breast cancer tissue (SK-BR-3).

Fig 8 shows the results of the BAC chip analysis, IHC, and FISH for the test sample isolated from breast cancer tissue (BT-474).

Fig 9 shows the results of the BAC chip analysis, IHC, and FISH for the test sample isolated from breast cancer tissue (MDA-MB-453).

Fig 10 shows the results of the BAC chip analysis, IHC, and FISH for the test sample isolated from breast cancer tissue (BT-20).

Fig 11 shows the results of the BAC chip analysis, IHC, and FISH for the test sample isolated from breast cancer tissue (MDA-MB-361).

Fig 12 shows the results of the BAC chip analysis, IHC, and FISH for the test sample isolated from breast cancer tissue (MDA-MB-175-VII).

Fig 13 shows the results of the BAC chip analysis, IHC, and FISH for the test sample isolated from breast cancer tissue (UACC-812).

Fig 14 is a graph showing Her-2 gene copy numbers for respective Her-2 related cells.
Fig 15 shows changes in the copy number of No 17 chromosome, to which Her-2 gene belongs.
Fig 16 is a graph showing the scores obtained from Figs. 14 and 15, as comparison ratios of the Her 2 copy numbers and the No.17 chromosome copy numbers, according to the FISH method.
Fig 17 shows the result for analyzing the clinical sample 1 using Her-2 gene (Clone 1) in Comparative Example.
Fig 18 shows the result for analyzing the clinical sample 2 using Her-2 gene (Clone 1) in Comparative Example.
Fig 19 shows the result for analyzing the clinical sample 3 using Her-2 gene (Clone 1) in Comparative Example.

## Examples

The present invention is further explained in more detail with reference to the following examples. These examples, however, should not be interpreted as limiting the scope of the present invention in any manner.

## Example 1: Preparation of BAC Clones

1-1. Construction of genomic library

A genome isolated from a Korean was treated with HindIII enzyme (New Englanf BioLabs) and PFGE (Pulsed-Field Gel Electrophoresis) to obtain DNA fragments of about 100Kb. DNA fragments of 100kb at average were ligated into BAC vectors, and transformation of *E. Coli* host cells (Invitrogen, DH10B) was performed. Here, *E. Coli cell* lines including individual DNA fragments are referred to as clones. From the construction of above genomic library, 96,768 clones in total were obtained.

1-1-1. Isolation of genome

A total genomic DNA was extracted from 20ml of Korean's semen, and electrophoresed on agarose gel, and then, the quality and quantity of the extracted DNA were analyzed.

1-1-2. Fragmentation and purification of genome

The genomic DNA was cleaved with BamHI (New Englanf BioLabs) and electrophoresed on PFG gel. DNA fragments were obtained by collecting the portions run at the 100 Kb region.

1-1-3. Transformation

The DNA fragments were inserted into the BAC vectors (pECBAC1, Friijter et al, 1997) at BamHI cleavage sites. The vectors were introduced into and transformed the host cells (Competent cell, E.coli DH10B BAC, Invitrogen). After incubation on LB agar plate, the clones for the respective DNA fragments were obtained. The clones were inoculated in a 96-well culture plate (clone/well) and incubated in the shaking incubator at 300rpm for 18 hr

1-1-4. Preparation of library cell stock solution

25μl of 65% glycerol were dividedly placed in a 384 well plate, and 25μl of the cells cultured from the 96 well format incubation blot were also placed therein (that is, the cells from the four 96 well format incubation blots are collectively placed in one 384 well plate). The 384 well plate was closed at the upper part and kept at -70°C.

1-2. End-sequencing

The 500bp end sequences from the both terminals of the 100Kb DNA fragments were analyzed, as specific primers of the BAC vectors. Information on the genomic DNAs inserted into the respective clones were identified and documented.

1-2-1. Isolation of BAC DNAs (Mini-prep.)

1 ml of 1 X LB was placed in a 96-well culture plate. 2μl of the library cell stock was inoculated thereto. At this point, the cells in the single 348 well-plate were distributed among four 96 well incubation blots. After incubating in the shake incubator at 37°C, at 300 rpm, for 18 hrs, 25μl of the test sample stock for respective wells were prepared. The remainder was centrifuged to obtain the cells. DNAs were collected using Montage mini-prep. kit (Millipore).

1-2-2. Sequence analysis

By T7 and M13R primers (T7: 5'-TAA-TAC-GAC-TCA-CTA-TAG-GG-3' (SEQ ID NO. 3) and M13R: 5'-CAG-GAA-ACA-GCT-ATG-ACC-3'(SEQ ID NO. 4)), the both terminals of the DNA fragments inserted into the respective clones were sequence-analyzed. The nucleotide sequences were identified by ABL 3700 automated sequence analyzer (Applied Biosystems).

1-3. Bioinformatics

The sequences analyzed above were compared to the whole human genomic sequence disclosed by Human Genome Project, in terms of homology, using bioinformatics technology. The analysis provided the locations of the BAC DNAs in the individual clones with respect to the genome, as well as their whole nucleotide sequences,.

1-3-1. Blast Search

Using the analyzed terminal sequences, Blast search was performed. By homology analysis, locations of the individual clones with respect to the genome were identified.

1-3-2. Selection of gene/marker

Among the genomic DNA fragments for respective clones obtained from the genomic library, the clones including the marker for cancer-related genes/ STS (Sequence-Tagged Site) were selected.

1-4. BAC Clone mapping by FISH

In order to more precisely identify the locations of the BAC clones in the genome, FISH was performed using as probes the genomic DNAs fragments included in the individual BAC clones. FISH, which uses the principle of DNA complementary bindings, identifies the location of a given probe in chromosome, by binding the probe-which specifically binds to a certain region on chromosome-directly to the chromosome fixed on the glass slide.

The identified locations of the genomic DNA fragments in chromosome, for the respective BAC clones, are as shown in Table 1 in Korean Patent No. 10-0776214.

**Example 2: Preparation of BAC chips**

2-1. Selection of clones to be used for the preparation of BAC chips.

For the purpose of diagnosing variation in human chromosome number and cancers (particularly, breast cancer), 1440 clones selected for each chromosome from the BAC library were hybridized. For each cancer-related gene, the selected clones were also hybridized (Table 1 in Korean Patent No. 10-0776214).

Among the above clones hybridized for respective chromosomes, the clones having relation to breast cancer attributable to Her-2 copy number were selected and are shown in Table 2-1 below.

[Table 2-1]

| Version | Chromosomal region detected by FIS H | Location on human chromosome | | Size (bp) | Reference sequence |
|---|---|---|---|---|---|
| | | Start | End | | |
| V er. 37.1 | 17q12 | 37843402 | 37937522 | 94121 (SEQ ID NO. 2) | NCBI Build 37.1 (August, 2009) |

2-2. Isolation of genomic DNA fragments from clones

2 μl stock solution of respective BAC clones were inoculated in 85 ml of 1X LB(DIFCO, Cat. No 244620) including 400μl of chloramphenicol solution (100% alcohol 100 ml + chloramphenicol 340 mg). After shaking incubation at 37 °C for 15 hrs at 250 rpm, the culture was centrifuged at 4 °C at 6,000rpm for 5 min to obtain pellets. DNAs were extracted using QUIAGEN-tip 100 (QIAGEN plasmid midi kit).

Of BAC DNA 1ml, 850μl were sonicated and were used as DNAs for slide blotting, and 3μl were used as

electrophoresis on agarose gel. 20μl of some randomly selected BAC DNAs were used to test Pulse field gel electrophoresis.

2-2-1. Agarose gel electrophoresis

In order to identify the isolated BAC DNAs, 3 μl of the BAC DNAs dissolved in distilled water were treated with Not I enzyme (New Englanf BioLabs) and were run on a 0.85% agarose gel. After examining the result of electrophoresis, the BAC DNAs including the genomic DNAs fragments were selected.

2-2-2. PFG electrophoresis (CHEFF 162-00133, BIO-RAD)

Among 48 of BAC clones, which is a unit of clones available from a single isolation, 6 clones were randomly selected and were PFG-electrophoresed. Another sets of 48 BAC clones were repeatedly re-isolated until all 6 clones were error-free.

2-3. Preparation for Spotting

2-3-1. Sonication

The isolated BAC DNA solution, as a result of restriction enzymes treatment and PFG electrophoresis, was centrifuged (12,000 rpm, 10 min). After collecting the supernatant 850 μl, sonication was performed under such conditions as below.

-Conditions for sonication-

Time: 5 sec, Temperature: 25°C, Pulse: 0.1/0.1

By conducting electrophoresis on a 1% agarose gel for all the 1440 BAC DNAs after sonication, it was examined whether sonication was properly performed Sonicated DNAs should be located at the region of 0.5Kb and 10Kb. The BAC DNAs solution was condensed/dried in SpeedVAC (SAVANT, AES2010-220) and was dissolved in distilled water 45 μl to test its viscosity. For samples with high viscosity, sonication was repeatedly performed.

2-3-2. Pooling by chromosome and preparation of spotting solution.

The isolated 1440 clones were placed in 15 ml tubes for respective chromosomes. The breast-cancer related genomic DNAs were also placed in a 15 ml tube.

After 45 μl of spotting solution (100% DMSO, 1.2 ug/μl nitrocellulose) was added, the clones were transferred into a 384-well plate(30μl per well). Each empty well was filed with 30μl of 50% DMSO.

The above prepared sample had been preserved at -70°C for a period longer than one month (at -20°C for one month or a shorter period). Before being used, the sample was thawed and spun down at 2000 rpm for one min.

2-4. Spotting

The genomic DNA fragments, which had been classified by chromosome number after being obtained from the 1440 clones, and the breast cancer related genomic DNA fragments in Table 2-1 were spotted on the microarray. In the present experiment, 4 subarrays as shown in Fig 3 were used, each subarray having 180 (12 x 15) spots. The identical genomic DNA fragments were horizontally spotted in triplicate. The left two subarrays account for the 1440 clones spotted for respective chromosomes, while the rightmost subarray account for the cancer related genes. In particular, Her-2 gene was spotted at spot 17 on the rightmost subarray. Table 3 indicates chromosome numbers and cancer genes spotted on each subarray

2-4-1. Preparation of slides

Glass slides of 25 mm x 75 mm x 1 mm were prepared. The slides were aminosilane-coated.

2-4-2. Spotting

Using GeneMachine MicroGrid100 (GeneAmchine) or Aushon 2470 Arrayer (Aushon biosystems), spotting was performed on the slides (5 - 10 pg per spot). With the nitrogen cylinder set at between 103.4kPa and 137.9 kPa, the range from 74.5kPa to 101.6kPa was maintained by the vacuum pump.

**Example 3: Diagnosis of breast cancer attributable to Her-2 copy number variations**

Test samples were obtained by culturing the cell lines received from ATCC (American Type Culture Collection Center) which over-express due to an increase in Her-2 copy number. Reactions were carried out with the BAC chip from Example 2. Other normal cells were provided by Coriell Institute for Medical Research and were tested in the same manner.

The Her-2 related cells provided are as indicated in Table 3.

[Table 3] Cells showing an increase in Her-2 copy number

| Cell name | Product number | IHC score | FISH | |
|---|---|---|---|---|
| | | | Her-2 Copy copy | FISH Ratio (Her-2/17chr) (R) |
| Normal B-Lymphocyte (46, XY) | GM10851 | 0 | C<4.0 | R<1.8 |
| Normal B-Lymphocyte (46, XX) | GM15510 | 0 | C<4.0 | R<1.8 |
| MCF7 | HTB-22 | 0 | C<4.0 | R<1.8 |
| SK-BR-3 | HTB-30 | +3 | C>6.0 | R>2.2 |
| BT-474 | HTB-20 | +3 | C>6.0 | R>2.2 |
| MDA-MB-453 | HTB-131 | +2 | C>6.0 | R>2.2 |
| BT-20 | HTB-19 | +1 | C<4.0 | R<1.8 |
| MDA-MB-361 | HTB-27 | +3 | C>6.0 | R>2.2 |
| MDA-MB-175-VII | HTB-25 | +1 | C<4.0 | R<1.8 |
| UACC-812 | CRL-1897 | +3 | C>6.0 | R>2.2 |

(Methods)

1. Isolation of genomic DNAs

500 ng of test DNAs were obtained from blood using commercially available Gentra Puregene cell Kit (Qiagen, Cat. No 158745). Reference samples were obtained from the reference cells (GM10851 and GM15510 from Coriell Institute; the other cells from ATCC) in the same manner.

2. Probe labeling

Test and reference samples were labeled with Cy3 (Cy3-dCTP) and Cy5 (Cy5-dCTP), respectively, by random priming. Then they were purified by the purification Kit (BioRnD)

1) Labeling

(1) 21μl (500ng) of the DNAs (test or reference) was placed in the tube, and 20ul of 2.5 X random reaction buffer was added thereto.
(2) The tube was boiled on the 100 °C heat block for 5 min, and then was immediately placed on the ice for 5 min.
(3) After spin-down, 5u of 2mM dNTP was added into each tube. To manufacture 2mM dNTP, ATP, dGTP, dTTP and dCTP (20ul : 20ul : 20ul : 10μl, 100mMd) were prepared, and TE 30ul were added thereto and mixed.
(4) Cy3 for the test sample and Cy5 for the reference sample were added (each 3ul).
(5) 1.2ul of klenow fragments were added per each.
(6) After pipette-mixing and spin-down, reactions were made in the 37 °C incubator.

2) Purification using spin colum (BioRnD, DNA purification kit)

(1) 250ul PB (binding buffer) was added to 50ul of the reaction solution above. Then it was spotted on the prepared spin column.
(2) The spin column was centrifuged at 13,200 rpm, for 1min 30 sec.
(3) 250ul PE (washing buffer) was added into the spin column.
(4) The spin column was centrifuged at 13,200 rpm, for 1min 30 sec.
(5) The solution collected in the 2ml tube was discarded.
(6)50ul EB (elution buffer) was added into the spin column and was allowed to stand at room temperature for 3 min.
(7) The spin column was centrifuged at 13,200 rpm, for 1min 30 sec.
(8) 30ul EB was added into the spin column and was allowed to stand at room temperature for 3 min.
(9) The spin column was centrifuged at 13,200 rpm, for 1min 30 sec.

3) Precipitation with ethanol

(1) 80ul of the test DNAs (Cy3-labeled), 80ul of the reference DNAs (Cy5-labeled), 30ul of Cot I DNA, 20ul of 3M sodium citrate, and 500ul of cool 100% ethanol were all mixed.
(2) The mixture was allowed to stand at -20 °C for 60 min.
(3) The mixture was centrifuged at 13,000rpm at 4 °C for 20 min.
(4) After centrifuging, purple precipitate was generated. The supernatant was discarded.
(5) 500ul of cool 70% ethanol was added, then the mixture was centrifuged at 13,000rpm at 4 °C for 10 min.
(6) The supernatant was discarded. After spin-down, the remaining ethanol was removed by the pipette.
(7) The mixture was left in the dark for 10 min.
(8) To the precipitates, 44ul of array hybridization solution and 4ul of yeast tRNA solution were added. When left in the dark for more than 30 min, the precipitates dissolved and developed a blue color. It was used as probe solution.

4. Hybridization reaction

- Pre-hybridization of BAC chip -

(1) With a diamond pen, the top and bottom of the BAC chip were marked.
(2) 10 uL of salmon sperm DNAs were added into 30 uL hybridization solution.
(3) The mixture was placed on the 70 °C heat block for 10 min for denaturation, and then was placed in the ice for 5 min.
(4) 40 uL of the mixture was loaded on the BAC chip and was stored in a humid slide box for 30 min.
(5) The BAC chip was soaked in triple-distilled water for 10 sec and washed two times.
(6) The BAC chip was washed with isopropanol and was centrifuged at 550rpm for 5 min. The slide was then dried.

- Hybridization -

(1) 44 ul of the probe-detection solution in purple was denatured for 15 min on the 70°C heat block, and was reacted with the BAC chip.
(2) The chip was left in the incubator at 37 °C for 1 hr.
(3) The hybridization solution was loaded.
(4) 20 X 40mm of cover glass was placed on the chip.
(5) The chip was placed in the slide box and was put on the shaker at about 5rpm. The chip was allowed to hybridize in the incubator at 37°C, at 100% humidity, for 48-72 hr.

- Washing-

(1) The BAC chip was washed for 15 min with pH7.3 solution containing 50% formamide and 2X SSC at 46°C.

(2) The chip was washed for 30 min with 46°C solution containing 2X SSC and 0.1 % SDS.

(3) The chip was washed for 15 min at room temperature, with 100 mL of mixture containing PN buffer (0.2M Na2HPO4(pH 9.0) and 0.2M NaH2PO4(pH 4.3), 100 mL of distilled water, and 200 μL of NP40).

(4) The chip was washed for 5 min at room temperature with 2X SSC.

(5) The chip was washed for 1 min at room temperature consecutively with 70%(v/v), 85%(v/v) and 100%(v/v) ethanol.

(6) The chip was centrifuged at 550rpm for 5 min. The slide was then dried.

4. Scanning and analysis

The BAC chip was inserted into the micraoarray chip analyzer (MAC VIEWER, 2nd grade, A661200) and scanned. The scanned images were obtained for the Cy3 and Cy5 filters, respectively. With the chip analysis program (Macrogen), in which information on the genomic DNA fragments spotted on the BAC chip was loaded, the above image results were analyzed.

1) Scanning

A. Preparation prior to using the microarray chip analyzer

The microarray chip analyzer was connected to a PCI-equipped PC. The recommend options for the PC are:

- IBM Pentium III, or more
- Windows NT 4.0 or Windows 2000
- 768 MB RAM or more
- Hard disk 30 GB or more
- Writable/rewritable CD-ROM

During scanning, the cy3 and cy5 channels were selected, with the exposure time of 1 sec. The scanning was performed after focusing, and the scanned images were saved under file names.

2) Analysis

The scanned files were operated on the MACviwer(Ver3.0), an analyzing software in which the BAC chip-related information is saved, in order to obtain a Linear Ratio chart.

The final experimental results for the BAC chip were the base T/R ratio means for each of the 1440 BAC clones-hybridized with respect to respective autosomes (Nos 1-22) and X, Y sex chromosomes-as well as the base T/R ratio means for each of the BAC clones hybridized with respect to Her2 gene (Fig 3, Fig 4, and Fig 5). A T/R ratio mean is a test sample's fluorescence intensity (Cy 3) divided by a reference's fluorescence intensity (Cy 5). A T/R ratio mean becomes 1.000 when the intensities of the two are the same, higher than 1.000 when the test sample's fluorescence intensity is stronger, and lower than 1.000 when the reference's fluorescence intensity is stronger.

For autosomes, the number of chromosomes is the same for the test and the reference, and they have the identical fluorescence intensities. Therefore, the average for each chromosome (chromo-mean) converges to 1.000. If there is an increase in gene amplification of certain chromosome or Her-2, the T/R ratio mean for Her-2 would increase proportionally to the copy number. The diagnosis of breast cancer caused by chromosome or Her-2 variations is made according to Table 4 below.

[Table 4] Criteria for diagnosing breast cancer by Her-2 copy number

| IHC level | Diagnosis by IHC | FISH | | Diagnosis by FISH | Linear ratio mean for Her-2 chip (aCGH) (Cut-off value: 1.500=T/R) | Diagnosis by Her-2 chip |
|---|---|---|---|---|---|---|
| | | FISH Ratio (Her-2/17chr) (R) | Her-2 Copy (C) | | | |
| 0 | Negative | R<1.8 | C<4.0 | Negative | T/R<1.350 | Negative |
| +1 | Negative | 1.8≤R≤2.2 | 4.0≤C≤6.0 | Re-examination required | 1.350≤T/R<1.500 | Re-examination required |
| +2, +3 | Positive | R>2.2 | C>6.0 | Positive | 1.500≤T/R | Positive |

The cut-off value for breast cancer attributable to Her-2 copy number is 1.500, meaning that values higher than 1.500 indicate presence of breast cancer. To improve the correctness of diagnosis, re-examination (re-diagnosis) is performed when values are 1.350 or more and less than 1.500. The experimental data to obtain the cut-off values is shown in Figs. 14 through 16.

Fig. 14 shows Her-2 copy numbers for various Her-2 related cells. It was confirmed that Her-2 copy numbers were increased (Her-2 copy number values being 1.500 or more) in the cells including MDA-MB-453, MDA-MB-361, SKBR3, BT-474, UACC-812 (ATCC (American Type Culture Collection)), and the like.

Fig. 15 shows changes in the copy number for No.17 chromosome, in which Her-2 gene is contained. It was confirmed that the copy number for No.17 chromosome remains unchanged despite an increase in Her-2 copy number. The copy number for No.17 chromosome falls in the normal range of the values of 1.000= M < 1.500

In Fig. 16, the values obtained from Figs. 14 and 15 were indicated by comparison ratios of the Her 2 copy numbers to the No. 17 chromosome copy numbers, according to the FISH principle. The ratios calculated tend to be associated with the Her-2 copy numbers.

Figs. 4 through 13 show the Linear T/R ratio means for 10 kinds of cells in Table 3.

Figs. 4 and 5 shows the results for the control group, in which the T/R ratio means are 1.002 and 1.101, respectively, indicating that Her-2 copy numbers are in the normal range.

In Fig. 6, the T/R ratio mean for Her-2 is 0.811, indicating that no breast cancer associated with Her-2 copy number exists.

In Fig. 7, the T/R ratio mean for Her-2 is 4.418, indicating that breast cancer associated with Her-2 copy number exists.

In Fig. 8, the T/R ratio mean for Her-2 is 6.925, indicating that breast cancer associated with Her-2 copy number exists.

In Fig. 9, the T/R ratio mean for Her-2 is 1.550, indicating that breast cancer associated with Her-2 copy number exists.

In Fig. 10, the T/R ratio mean for Her-2 is 0.923, indicating that no breast cancer associated with Her-2 copy number exists.

In Fig. 11, the T/R ratio mean for Her-2 is 2.855, indicating that breast cancer associated with Her-2 copy number exists.

In Fig. 12, the T/R ratio mean for Her-2 is 1.059, indicating that no breast cancer associated with Her-2 copy number exists.

In Fig. 13, the T/R ratio mean for Her-2 is 6.530, indicating that breast cancer associated with Her-2 copy number exists.

**Comparative Example: Diagnosis of breast cancer attributable to Her-2 copy number**

Four kinds of BAC clones (Macrogen)-including Her-2 gene (Clone 1), the clone according to the present invention of SEQ ID NO. 2 (Clone 2), and two clones of Her-2 adjacent genes (Clones 3 and 4)-were tested on the clinical samples 1 through 3, with regard to the efficiency of breast cancer diagnosis (measurement of T/R ratio means), in the same manner as in Experiment 3. Said clinical samples were provided by Seoul Asan Hospital, for which Her-2 gene amplification was confirmed by IHC (IHC +2). The above four kinds of BAC clones are shown in Table 5, and the results (T/R ratio means) are as shown in Table 6, and Figs 17 through 19.

[Table 5]

| No. | BAC Clone | Version | Location on Chromosome Identified by FISH | Location on Human Genome | | Size (bp) |
|---|---|---|---|---|---|---|
| | | | | Start | End | |
| 1 | Her-2 | Ver . 37.1 | 17q12 | 37844393 | 37884914 | 40522 |
| 2 | 2261(SEQ ID NO. 2) | Ver. 37.1 | 17q12 | 37843402 | 37937522 | 94221 |
| 3 | BAC234_C11 | Ver. 37.1 | 17q12 | 37735972 | 37838666 | 102695 |
| 4 | 7238 | Ver. 37.1 | 17q12~17q21.1 | 37937516 | 38081963 | 144448 |

[Table 6]

| No. | BAC Clone | clinical sample_1 | clinical sample_2 | clinical sample_3 |
|---|---|---|---|---|
| | | IHC +2 | IHC +2 | IHC +2 |
| 1 | Her-2 | 1.182 | 1.166 | 1.038 |
| 2 | 2261 | 1.619 | 1.514 | 1.689 |
| 3 | BAC234_C11 | 1.558 | 1.363 | 1.535 |
| 4 | 7238 | 1.093 | 1.127 | 1.025 |

The above clinical samples 1 through 3 were IHC 2+, i.e., breast cancer positive. When the Her-2 clone (Clone 1) or the clone of a Her-2 adjacent gene (Clones 3) was used, T/R ratio means were mixed and confused among the samples, making it difficult to precisely determine a breast cancer-positive sample. Further, the clone of another Her-2 adjacent gene (Clones 4) incorrectly turned out the values below 1.350 for all the three samples, i.e., all negative. By contrast, the Clone 2, according to the present invention, diagnosed cancer positive for all the three samples, with 100% correctness.

## Claims

**1.** A diagnosing composition for diagnosis of breast cancer comprising a genetic marker, wherein said marker is one or more selected from the group consisting of
the genomic DNA fragment corresponding to SEQ ID No: 2 from the human genome chromosome region 17q12, and the complementary sequence to the genomic DNA fragment.

**2.** A microarray for diagnosis of breast cancer to which a probe is fixed, wherein said probe has a single locus in chromosome and is one or more selected from the group consisting of

(a) the genomic DNA fragment corresponding to SEQ ID No: 2 from the human genome chromosome region 17q12; and
(b) the complementary sequence to the genomic DNA fragment.

**3.** The microarray for diagnosis of breast cancer according to Claim 2, wherein said probe is manufactured by inserting said genomic DNA fragment into a vector of one or more selected from the group consisting of BAC(Bacterial Artificial Chromosome), HAC(Human Artificial Chromosome), TAC(Transformation competent Artificial Chromosome), YAC (Yeast Artificial Chromosome), PAC(P1-derived Artificial Chromosome), P1 (phage), and cosmid.

**4.** The microarray for diagnosis of breast cancer according to Claim 3, wherein said probe is manufactured by inserting said genomic DNA fragment into BAC(Bacterial Artificial Chromosome).

**5.** The microarray for diagnosis of breast cancer according to Claim 2, wherein said probe is fixed at an amount from 2 to 100 pg per spot.

**6.** A kit for diagnosis of breast cancer comprising:

the composition for diagnosis of breast cancer according to Claim 1, or the microarray for diagnosis of breast cancer according to any of Claims 2 through 5, and
a label detection means.

**7.** The kit for diagnosis of breast cancer according to Claim 6, wherein said label is selected from the group consisting of radioisotopes, fluorescent compounds, light emitting chemicals, and enzymes.

**8.** A use of a genetic marker for diagnosis of breast cancer, wherein said marker is one

or more selected from the group consisting of; the genomic DNA fragment corresponding to SEQ ID No: 2 from the human genome chromosome region 17q12, and the complementary sequence to the genomic DNA fragment.

9. The use according to Claim 8, wherein the diagnosis of breast cancer is performed by the steps of:

hybridizing said marker with a test DNA sample, and
measuring the level of hybridization of said marker with the test DNA, and comparing the level of hybridization to that with a reference DNA,
wherein the reference DNA is a genome DNA with no variation in Her-2 gene copy number, and the level of hybridization with the test DNA is higher than that for the reference DNA, it is determined that there is an increase in Her-2 copy number and that the test sample has breast cancer.

10. The use according to Claim 9, wherein the levels of hybridization with said test DNA and said reference DNA are compared to each other by the following formula:

$$\text{Linear mean of DNA copy number (T/R ratio mean)} = \frac{\text{Fluorescent signal in a test sample* (mean)}}{\text{Fluorescent signal in a reference sample** (mean)}}$$

wherein when said T/R ratio mean is 1.5 or more, it is determined that the level of hybridization with the test DNA is higher than that with the reference DNA.

11. The use according to Claim 9, wherein said test sample is a mammary cell or tissue from mammals.

12. The use according to Claim 9, wherein said test sample is a cell or tissue from breast cancer.

13. The use according to Claim 9, wherein the levels of hybridization with said test DNA and said reference DNA are compared to each other by the following formula:

$$\text{Linear ratio mean of DNA copy number (T/R ratio mean)} = \frac{\text{Fluorescent signal in a test sample* (mean)}}{\text{Fluorescent signal in a reference sample** (mean)}}$$

wherein when said T/R ratio mean is 1.35 or more but less than 1.5, it is determined that said test is necessary to be subject to re-examination.

**Patentansprüche**

1. Diagnosezusammensetzung für die Diagnose von Brustkrebs, umfassend einen genetischen Marker, wobei es sich bei dem Marker um einen oder mehrere handelt, der bzw. die aus der Gruppe bestehend aus dem zu SEQ ID NO:2 korrespondierenden genomischen DNA-Fragment aus dem Chromosomenbereich 17q12 des menschlichen Genoms und der zu dem genomischen DNA-Fragment komplementären Sequenz ausgewählt ist bzw. sind.

2. Microarray für die Diagnose von Brustkrebs, an dem eine Sonde fixiert ist, wobei die Sonde einen einzigen Locus im Chromosom besitzt und es sich dabei um einen oder mehrere handelt, der bzw. die aus der Gruppe bestehend aus

(a) dem zu SEQ ID NO:2 korrespondierenden genomischen DNA-Fragment aus dem Chromosomenbereich 17q12 des menschlichen Genoms und

(b) der zu dem genomischen DNA-Fragment komplementären Sequenz ausgewählt ist bzw. sind.

**3.** Microarray für die Diagnose von Brustkrebs gemäß Anspruch 2, wobei die Sonde durch Einbringung des genomischen DNA-Fragments in einen Vektor eines oder mehrerer, ausgewählt aus der Gruppe bestehend aus BAC (Bacterial Artificial Chromosome), HAC (Human Artificial Chromosome), TAC (Transformation competent Artificial Chromosome), YAC (Yeast Artificial Chromosome), PAC (P1-derived Artificial Chromosome), P1 (Phage) und Cosmid, hergestellt wird.

**4.** Microarray für die Diagnose von Brustkrebs gemäß Anspruch 3, wobei die Sonde durch Einbringung des genomischen DNA-Fragments in BAC (Bacterial Artificial Chromosome) hergestellt wird.

**5.** Microarray für die Diagnose von Brustkrebs gemäß Anspruch 2, wobei die Sonde in einer Menge von 2 bis 100 pg pro Ort fixiert wird.

**6.** Kit für die Diagnose von Brustkrebs, umfassend:

die Zusammensetzung für die Diagnose von Brustkrebs gemäß Anspruch 1 oder das Microarray für die Diagnose von Brustkrebs gemäß einem der Ansprüche 2 bis 5 und
Markierungsnachweismittel.

**7.** Kit für die Diagnose von Brustkrebs gemäß Anspruch 6, wobei die Markierung aus der Gruppe bestehend aus Radioisotopen, fluoreszierenden Verbindungen, lichtemittierenden Chemikalien und Enzymen ausgewählt ist.

**8.** Verwendung eines genetischen Markers für die Diagnose von Brustkrebs, wobei es sich bei dem Marker um einen oder mehrere handelt, der bzw. die aus der Gruppe bestehend aus
dem zu SEQ ID NO:2 korrespondierenden genomischen DNA-Fragment aus dem Chromosomenbereich 17q12 des menschlichen Genoms und
der zu dem genomischen DNA-Fragment komplementären Sequenz ausgewählt ist bzw. sind.

**9.** Verwendung gemäß Anspruch 8, wobei die Diagnose von Brustkrebs durch folgende Schritte erfolgt:

das Hybridisieren des Markers mit einer Test-DNA-Probe und
das Messen des Hybridisierungsgrads des Markers bei der Test-DNA und das Vergleichen des Hybridisierungsgrads mit jenem bei einer Referenz-DNA,
wobei die Referenz-DNA eine Genom-DNA ohne Änderung der Her-2-Genkopiezahl ist und der Hybridisierungsgrad bei der Test-DNA höher als jener bei der Referenz-DNA ist und bestimmt wird, dass eine Erhöhung der Her-2-Kopiezahl vorliegt und dass die Prüfprobe Brustkrebs aufweist.

**10.** Verwendung gemäß Anspruch 9, wobei die Hybridisierungsgrade bei der Test-DNA und der Referenz-DNA durch folgende Formel miteinander verglichen werden:

$$\text{lineares Mittel der DNA-Kopiezahl (Mittelwert des T/R-Verhältnisses)} = \frac{\text{fluoreszierendes Signal in einer Prüfprobe* (Mittelwert)}}{\text{fluoreszierendes Signal in einer Vergleichsprobe** (Mittelwert)}}$$

wobei, wenn der Mittelwert des T/R-Verhältnisses 1,5 oder mehr beträgt, festgestellt wird, dass der Hybridisierungsgrad bei der Test-DNA höher als jener bei der Referenz-DNA ist.

**11.** Verwendung gemäß Anspruch 9, wobei die Prüfprobe eine Brustzelle oder Brustgewebe von Säugetieren ist.

**12.** Verwendung gemäß Anspruch 9, wobei die Prüfprobe eine Zelle oder Gewebe von Brustkrebs ist.

**13.** Verwendung gemäß Anspruch 9, wobei die Hybridisierungsgrade bei der Test-DNA und der Referenz-DNA durch folgende Formel miteinander verglichen werden:

$$\text{linearer Mittelwert des Verhältnisses der DNA-Kopiezahl (Mittelwert des T/R-Verhältnisses)} = \frac{\text{fluoreszierendes Signal in einer Prüfprobe* (Mittelwert)}}{\text{fluoreszierendes Signal in einer Vergleichsprobe ** (Mittelwert)}}$$

wobei, wenn der Mittelwert des T/R-Verhältnisses 1,35 oder mehr, jedoch weniger als 1,5 beträgt, festgestellt wird, dass es erforderlich ist, den Test einer Wiederholungsprüfung zu unterziehen.

**Revendications**

1. Composition de diagnostic pour le diagnostic du cancer du sein comprenant un marqueur génétique, où ledit marqueur est un ou plusieurs élément(s) choisi(s) parmi le groupe composé
du fragment d'ADN génomique correspondant à la séquence SEQ ID n°:2 provenant de la région chromosomique 17q12 du génome humain, et
de la séquence complémentaire au fragment d'ADN génomique.

2. Puce à ADN pour le diagnostic du cancer du sein à laquelle une sonde est fixée, où ladite sonde possède un seul locus dans un chromosome et est un ou plusieurs élément(s) choisi(s) parmi le groupe composé

   (a) du fragment d'ADN génomique correspondant à la séquence SEQ ID n°:2 provenant de la région chromosomique 17q12 du génome humain ; et
   (b) de la séquence complémentaire au fragment d'ADN génomique.

3. Puce à ADN pour le diagnostic du cancer du sein selon la revendication 2, dans laquelle ladite sonde est fabriquée en insérant ledit fragment d'ADN génomique dans un vecteur d'un ou plusieurs élément(s) choisi(s) parmi le groupe composé de BAC (chromosome artificiel bactérien), d'HAC (chromosome artificiel humain), de TAC (chromosome artificiel compétent pour la transformation), d'YAC (chromosome artificiel de levure), de PAC (chromosome artificiel dérivé du phage P1), de P1 (phage), et de cosmide.

4. Puce à ADN pour le diagnostic du cancer du sein selon la revendication 3, dans laquelle ladite sonde est fabriquée en insérant ledit fragment d'ADN génomique dans le chromosome BAC (chromosome artificiel bactérien).

5. Puce à ADN pour le diagnostic du cancer du sein selon la revendication 2, dans laquelle ladite sonde est fixée à une quantité comprise entre 2 à 100 pg par spot.

6. Kit pour le diagnostic du cancer du sein, comprenant :

   la composition pour le diagnostic du cancer du sein selon la revendication 1, ou la puce à ADN pour le diagnostic du cancer du sein selon l'une des revendications 2 à 5, et
   un moyen de détection de traceur.

7. Kit pour le diagnostic du cancer du sein selon la revendication 6, dans lequel ledit traceur est choisi parmi le groupe composé de radio-isotopes, de composés fluorescents, de produits chimiques électroluminescents, et d'enzymes.

8. Utilisation d'un marqueur génétique pour le diagnostic du cancer du sein, dans laquelle ledit marqueur est un ou plusieurs élément(s) choisi(s) parmi le groupe composé ;
du fragment d'ADN génomique correspondant à la séquence SEQ ID n°:2 provenant de la région chromosomique 17q12 du génome humain, et
de la séquence complémentaire au fragment d'ADN génomique.

**9.** Utilisation selon la revendication 8, dans laquelle le diagnostic du cancer du sein est effectué par les étapes qui consistent :

à hybrider ledit marqueur avec un échantillon d'ADN d'essai, et
à mesurer le niveau d'hybridation dudit marqueur avec l'ADN d'essai, et à comparer le niveau d'hybridation à celui avec un ADN de référence,
où l'ADN de référence est un ADN génomique qui ne présente aucune variation du nombre de copies du gène Her-2, et le niveau d'hybridation avec l'ADN d'essai est supérieur à celui pour l'ADN de référence, on détermine qu'il ya une augmentation du nombre de copies du gène Her-2 et que l'échantillon d'essai a un cancer du sein.

**10.** Utilisation selon la revendication 9, dans laquelle les niveaux d'hybridation avec ledit ADN d'essai et ledit ADN de référence sont comparés entre eux par la formule suivante:

$$\begin{array}{c}\textit{Moyenne linéaire du}\\ \textit{nombre de copies d'ADN}\\ \left(\textit{moyenne des rapports } \frac{T}{R}\right)\end{array} = \frac{\textit{Signal fluorescent dans un échantillon d'essai *(moyenne)}}{\textit{Signal fluorescent dans un échantillon de référence **(moyenne)}}$$

où lorsque ladite moyenne des rapports T/R est de 1,5 ou plus, on détermine que le niveau d'hybridation avec l'ADN d'essai est supérieur à celui avec l'ADN de référence.

**11.** Utilisation selon la revendication 9, dans laquelle ledit échantillon d'essai est un tissu mammaire ou une cellule mammaire de mammifères.

**12.** Utilisation selon la revendication 9, dans laquelle ledit échantillon d'essai est une cellule ou un tissu du cancer du sein.

**13.** Utilisation selon la revendication 9, dans laquelle les niveaux d'hybridation avec ledit ADN d'essai et ledit ADN de référence sont comparés entre eux par la formule suivante :

$$\begin{array}{c}\text{Moyenne des rapports linéaires}\\ \text{du nombre de copies d'ADN}\\ \left(\text{moyenne des rapports } \frac{T}{R}\right)\end{array} = \frac{\textit{Signal fluorescent dans un échantillon d'essai *(moyenne)}}{\textit{Signal fluorescent dans un échantillon de référence **(moyenne)}}$$

où lorsque ladite moyenne des rapports T/R est de 1,35 ou plus mais inférieure à 1,5, on détermine qu'il est nécessaire de réexaminer ledit essai.

Fig. 1

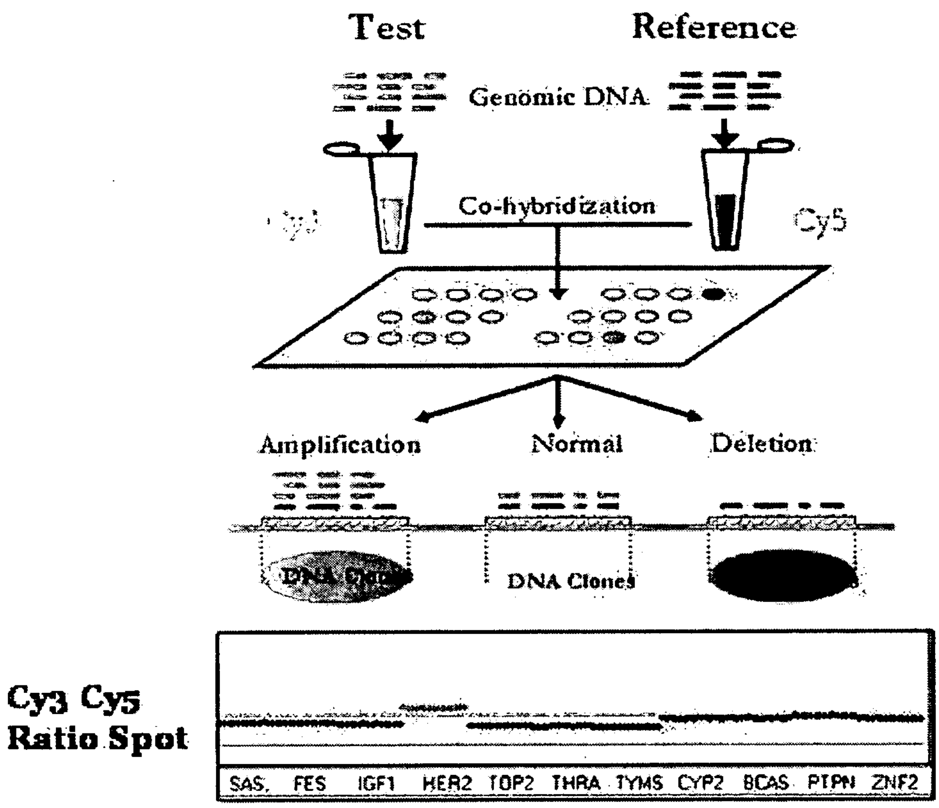
Test
Reference
Genomic DNA
Co-hybridization
Cy5
Amplification
Normal
Deletion
DNA Clones
Cy3 Cy5
Ratio Spot
SAS FES IGF1 HER2 TOP2 THRA TYMS CYP2 BCAS PTPN ZNF2

Fig. 2

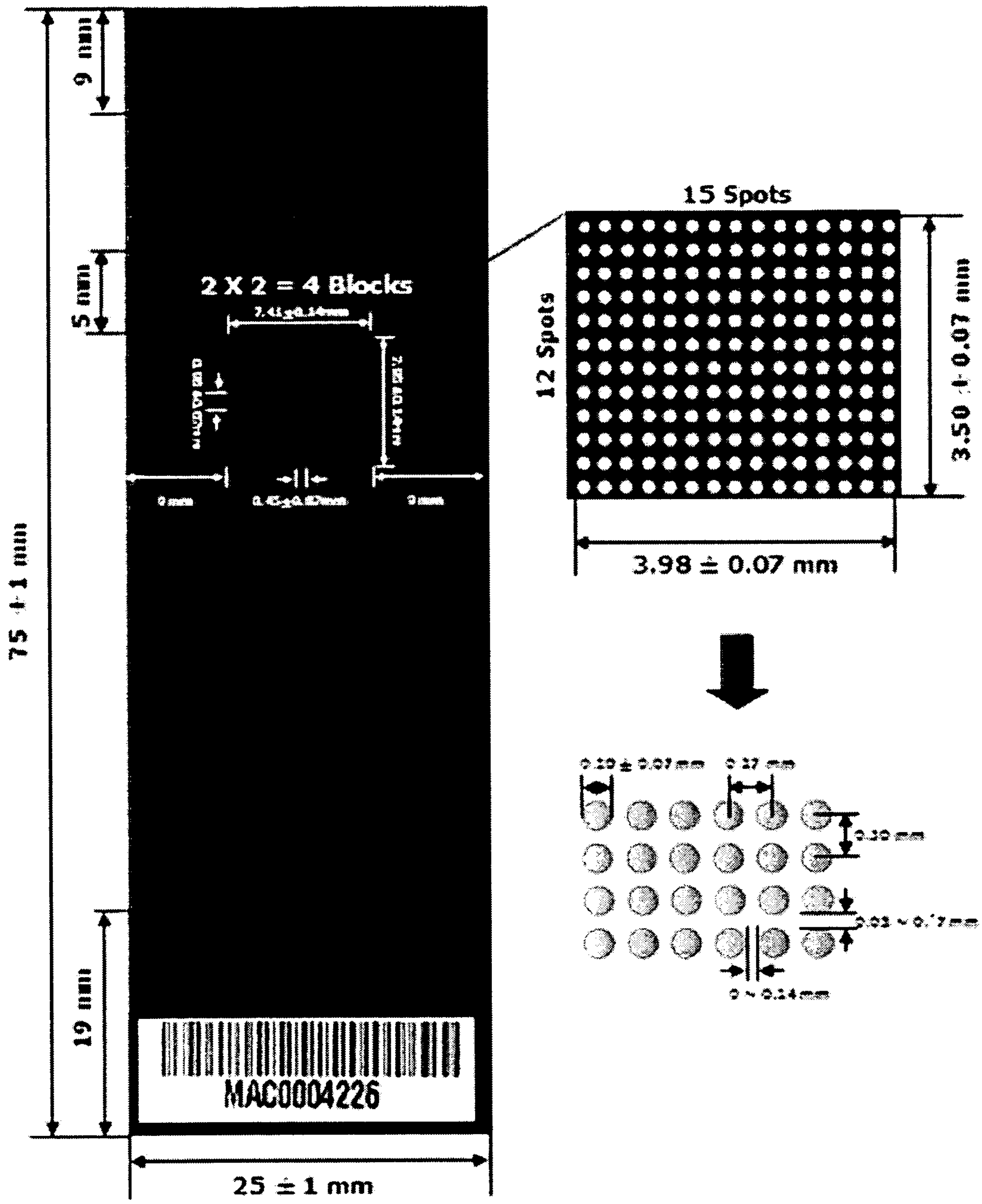

9 mm
5 mm
2 X 2 = 4 Blocks
19 mm
75 ± 1 mm
MAC0004226
25 ± 1 mm
15 Spots
12 Spots
3.50 ± 0.07 mm
3.98 ± 0.07 mm

Fig. 3

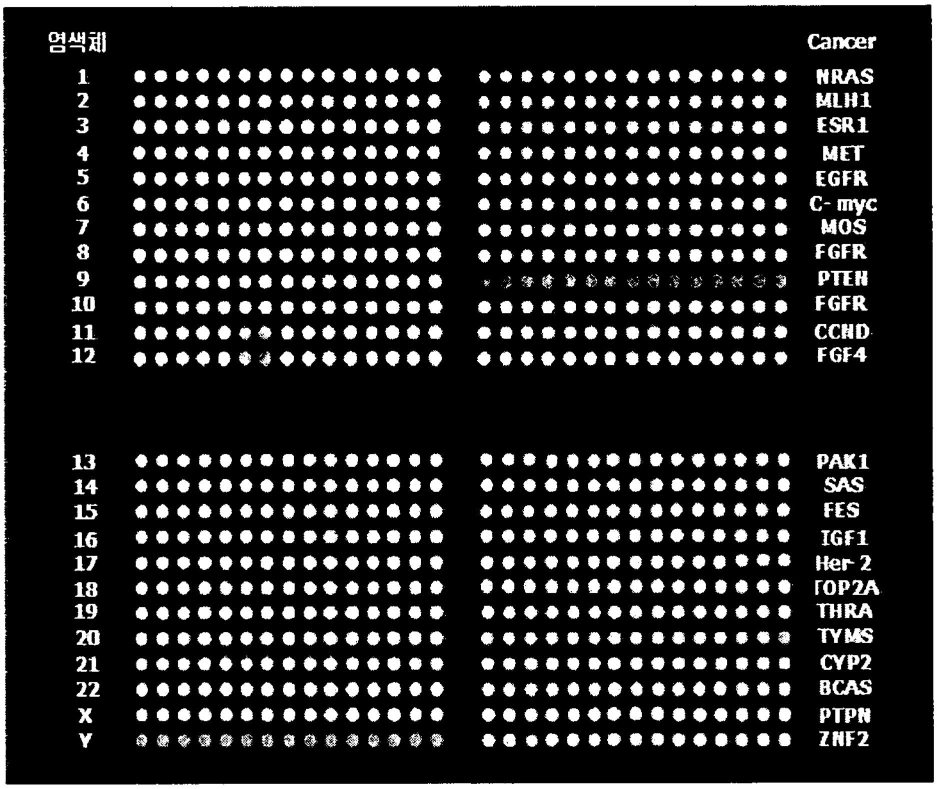
염색체
Cancer
1
NRAS
2
MLH1
3
ESR1
4
MET
5
EGFR
6
C-myc
7
MOS
8
FGFR
9
PTEN
10
FGFR
11
CCND
12
FGF4
13
PAK1
14
SAS
15
FES
16
IGF1
17
Her-2
18
TOP2A
19
THRA
20
TYMS
21
CYP2
22
BCAS
X
PTPN
Y
ZNF2

**Fig. 4**

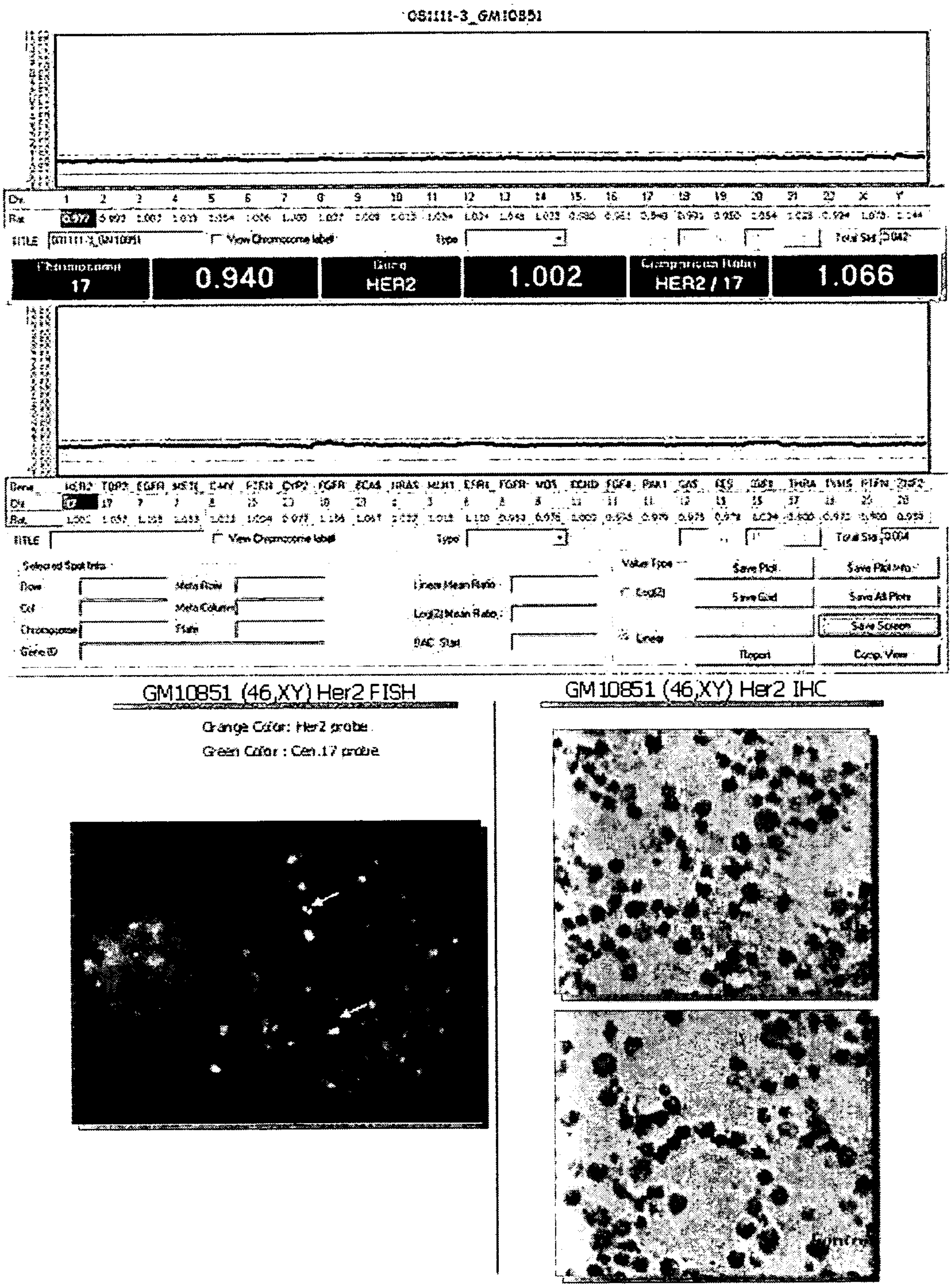
GS1111-3_GM10851
Chromosome
17
0.940
Gene
HER2
1.002
Comparison Ratio
HER2 / 17
1.066
GM10851 (46,XY) Her2 FISH
Orange Color: Her2 probe
Green Color : Cen.17 probe
GM10851 (46,XY) Her2 IHC

Fig. 5

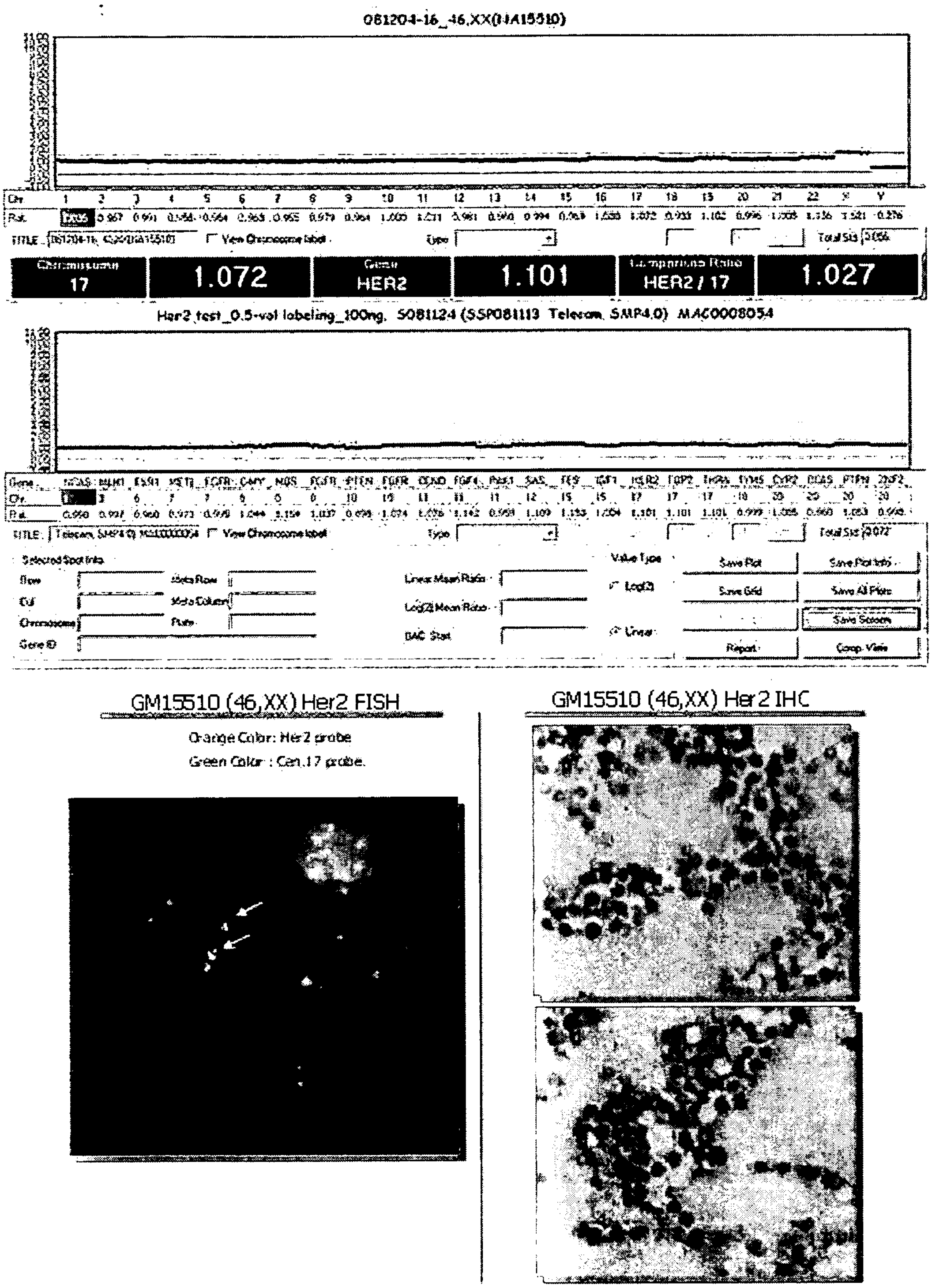

081204-16_46,XX(NA15510)
Chromosome 17
1.072
Gene HER2
1.101
Comparison Ratio HER2 / 17
1.027
Her2 test_0.5-vol labeling_100ng. SO81124 (SSP081113 Telecom, SMP4.0) MAC0008054
GM15510 (46,XX) Her2 FISH
Orange Color: Her2 probe
Green Color : Cen.17 probe.
GM15510 (46,XX) Her2 IHC

Fig. 6

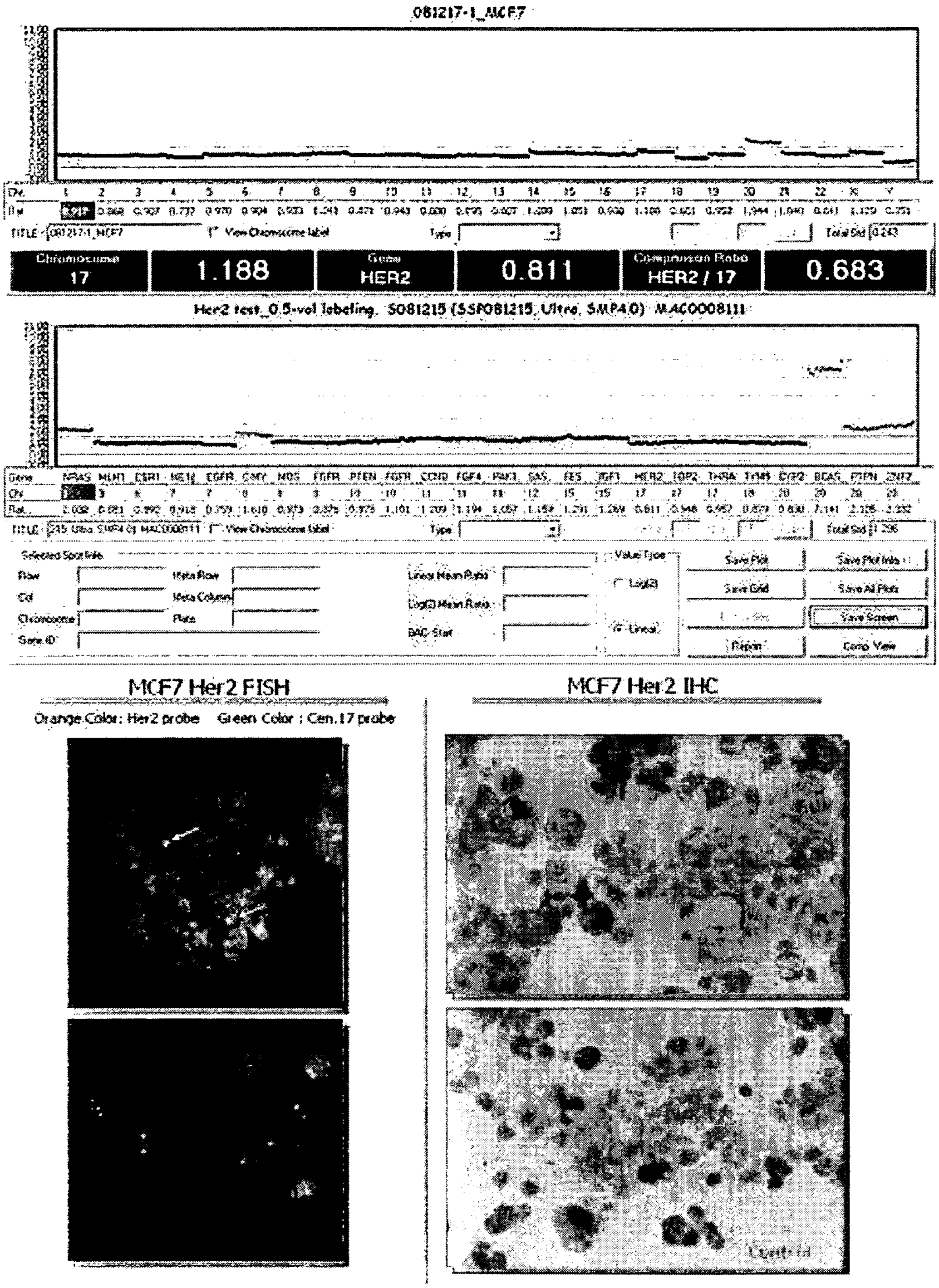
081217-1_MCF7
Chromosome 17
1.188
Gene HER2
0.811
Comparison Ratio HER2 / 17
0.683
MCF7 Her2 FISH
Orange Color: Her2 probe Green Color: Cen.17 probe
MCF7 Her2 IHC

Fig. 7

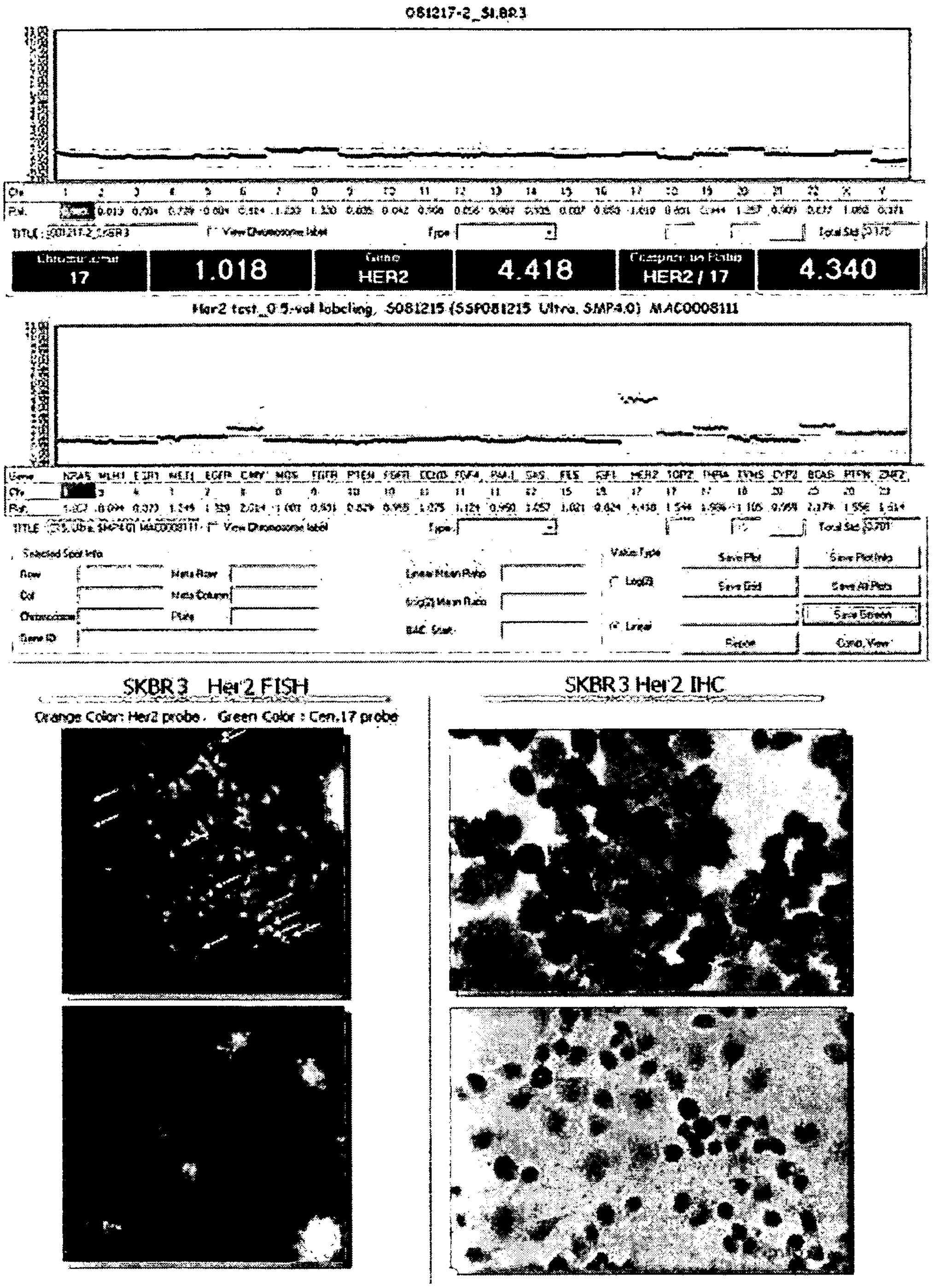
081217-2_SKBR3
17
1.018
HER2
4.418
HER2 / 17
4.340
SKBR3 Her2 FISH
Orange Color: Her2 probe , Green Color : Cen.17 probe
SKBR3 Her2 IHC

**Fig. 8**

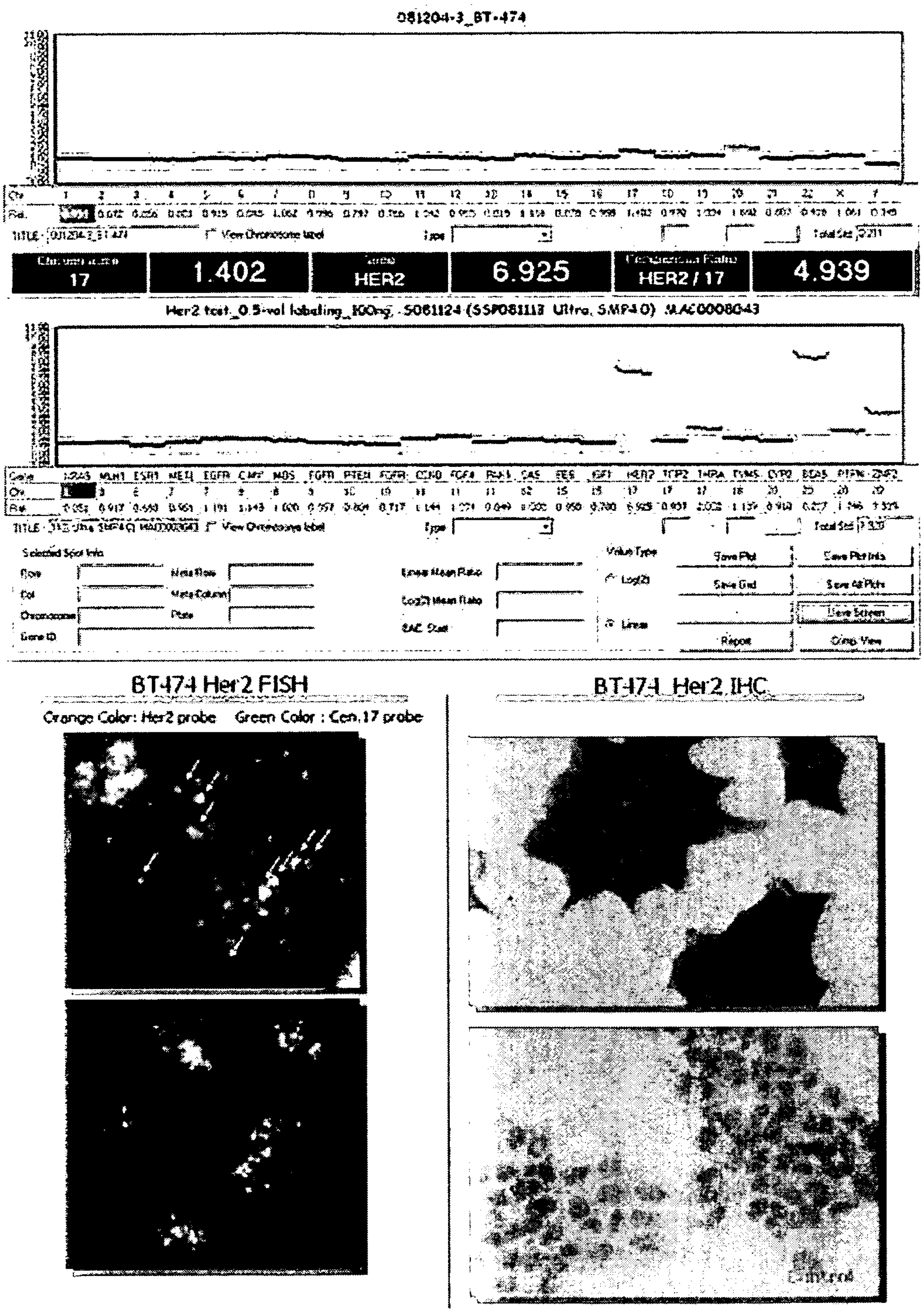
081204-3_BT-474
17
1.402
HER2
6.925
HER2 / 17
4.939
BT474 Her2 FISH
Orange Color: Her2 probe Green Color : Cen.17 probe
BT474 Her2 IHC

**Fig. 9**

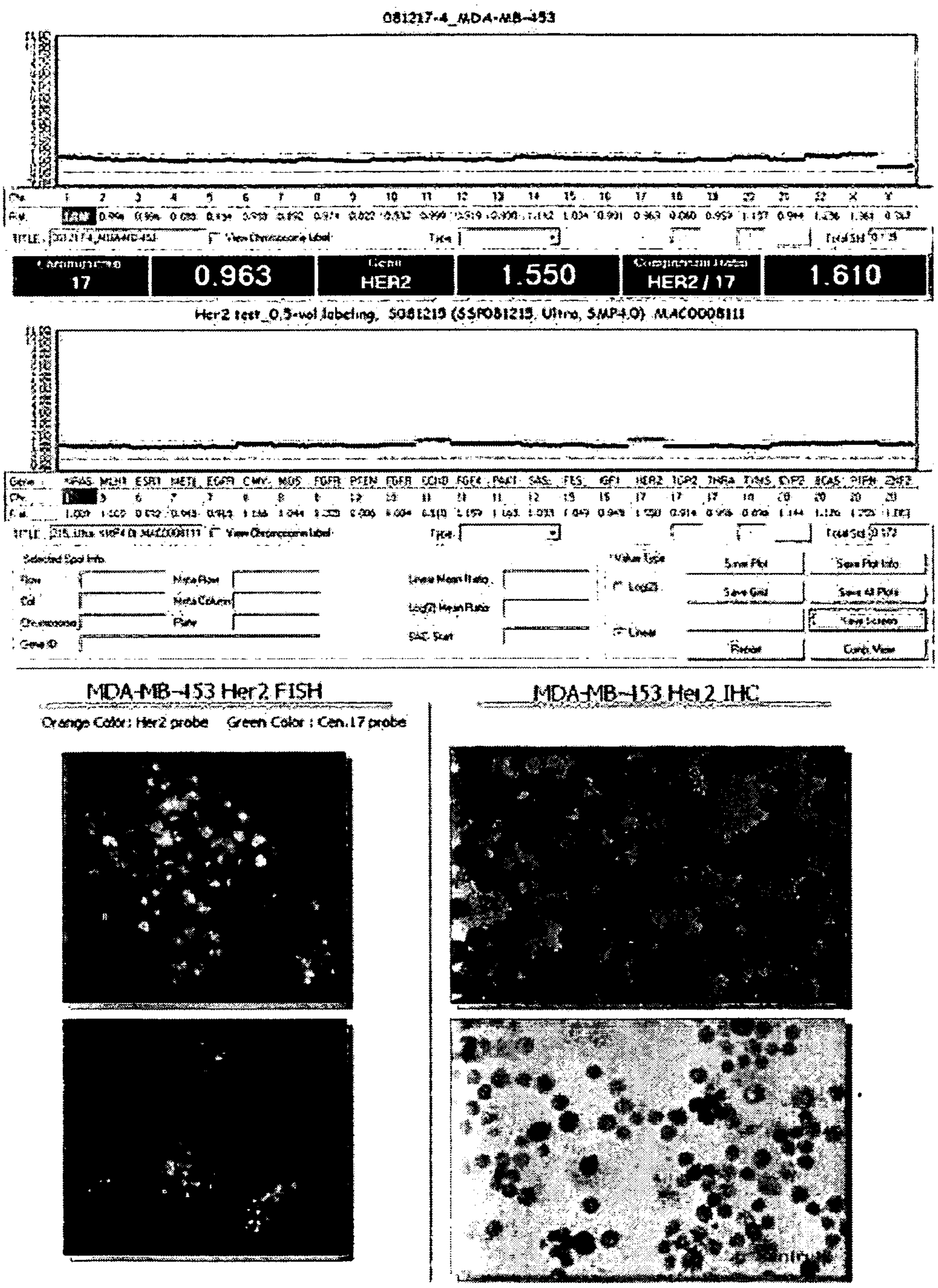
081217-4_MDA-MB-453
17
0.963
HER2
1.550
HER2 / 17
1.610
Her2 test_0.5-vol labeling, S081215 (SSF081215, Ultra, SMP4.0) MAC0008111
MDA-MB-453 Her2 FISH
Orange Color: Her2 probe Green Color : Cen.17 probe
MDA-MB-453 Her2 IHC

Fig. 10

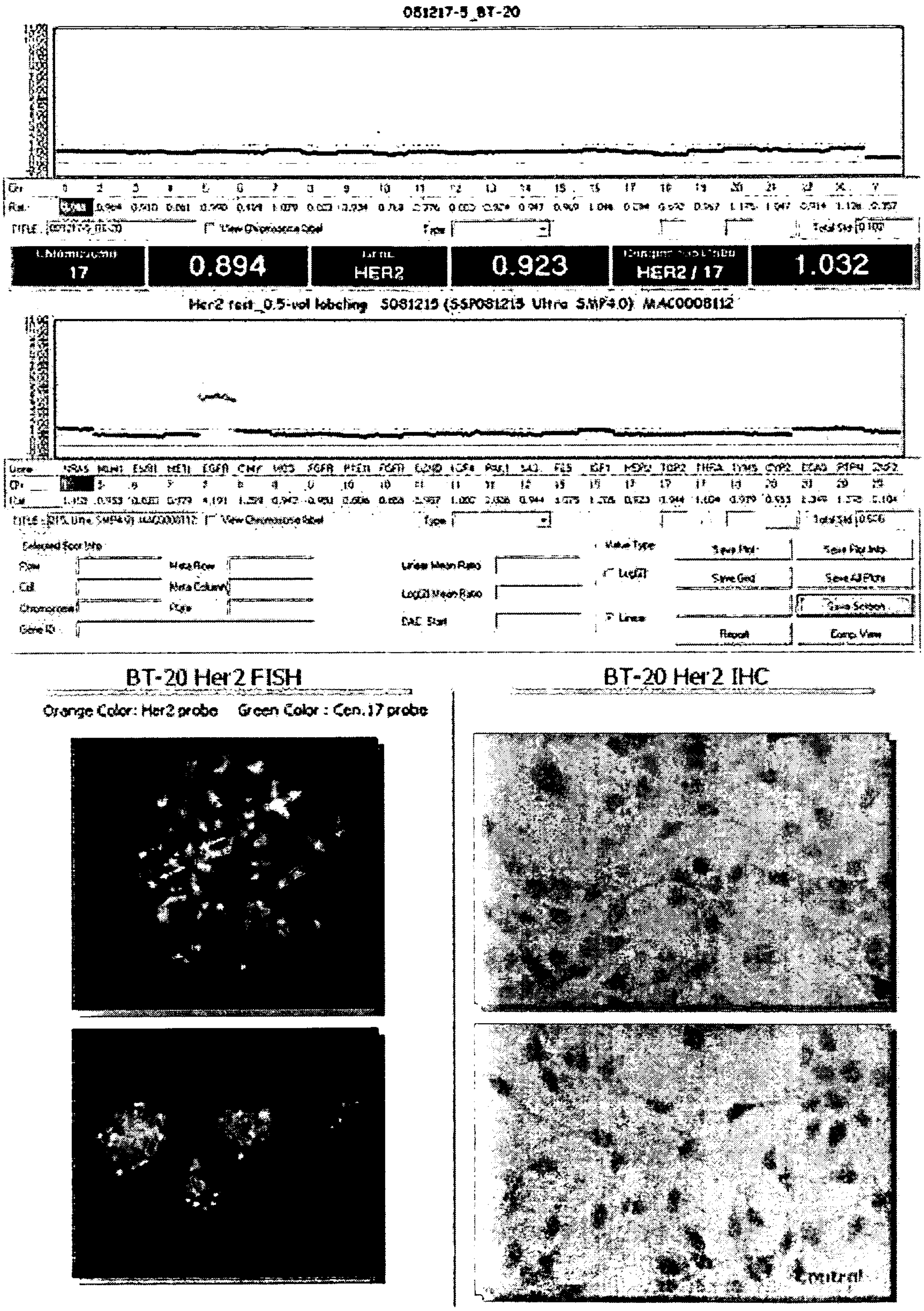
051217-5_BT-20
17
0.894
HER2
0.923
HER2 / 17
1.032
BT-20 Her2 FISH
Orange Color: Her2 probe
Green Color : Cen.17 probe
BT-20 Her2 IHC

**Fig. 11**

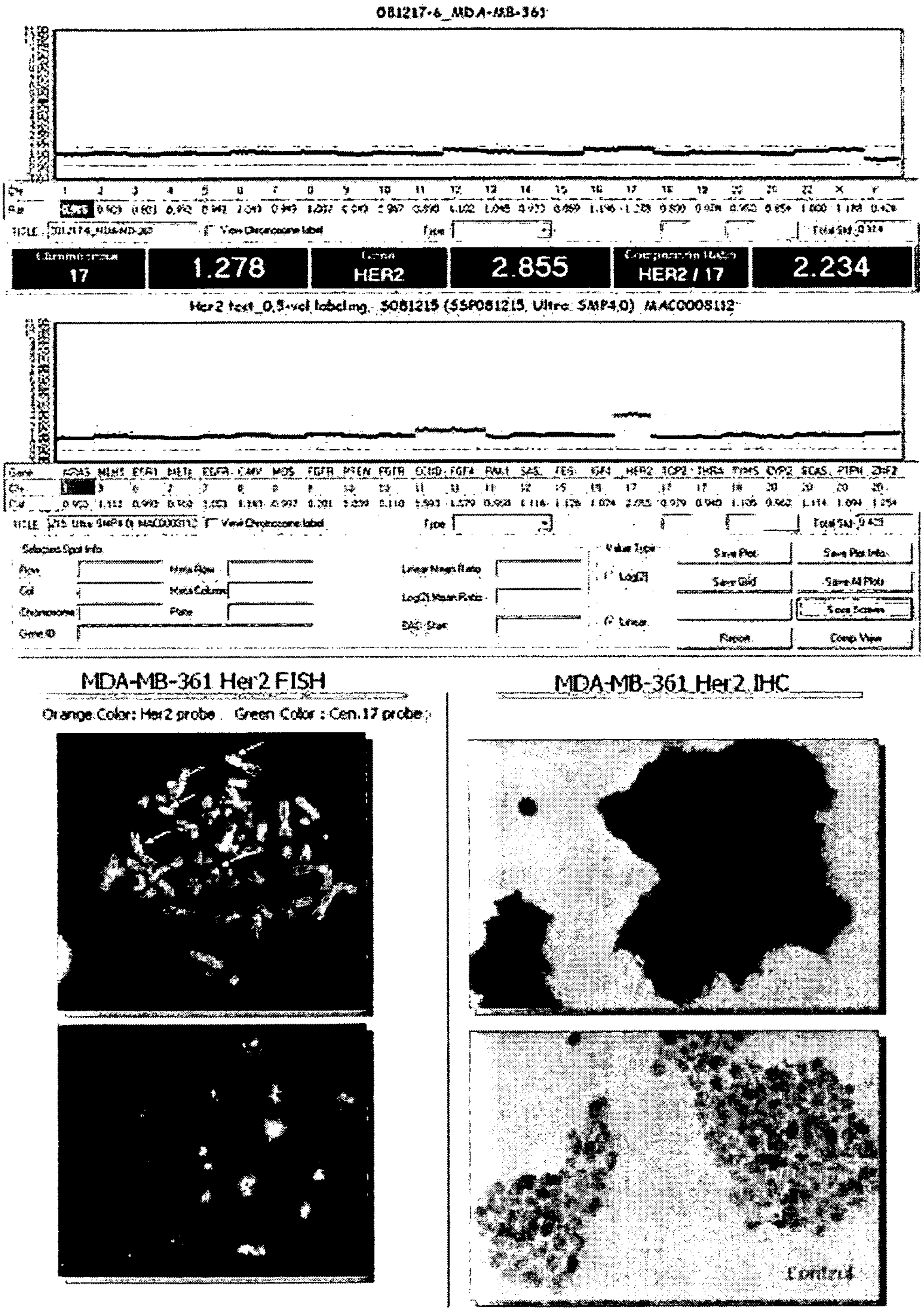
081217-6_MDA-MB-361
Chromosome
17
1.278
Gene
HER2
2.855
Comparison Ratio
HER2 / 17
2.234
MDA-MB-361 Her2 FISH
Orange Color: Her2 probe , Green Color : Cen.17 probe
MDA-MB-361 Her2 IHC
Control

Fig. 12

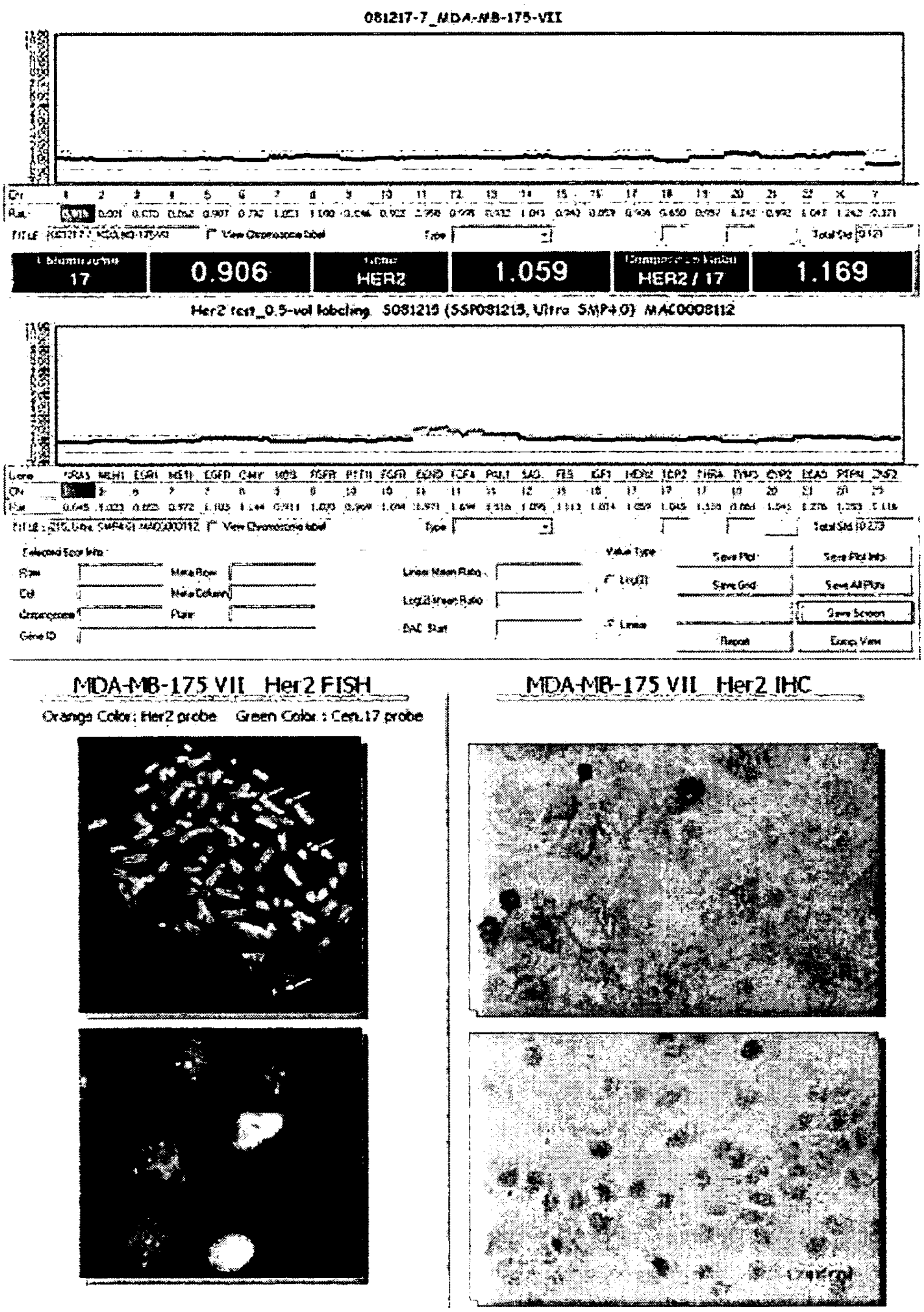
081217-7_MDA-MB-175-VII
17
0.906
HER2
1.059
HER2 / 17
1.169
MDA-MB-175 VII Her2 FISH
Orange Color: Her2 probe Green Color: Cen.17 probe
MDA-MB-175 VII Her2 IHC

# Fig. 13

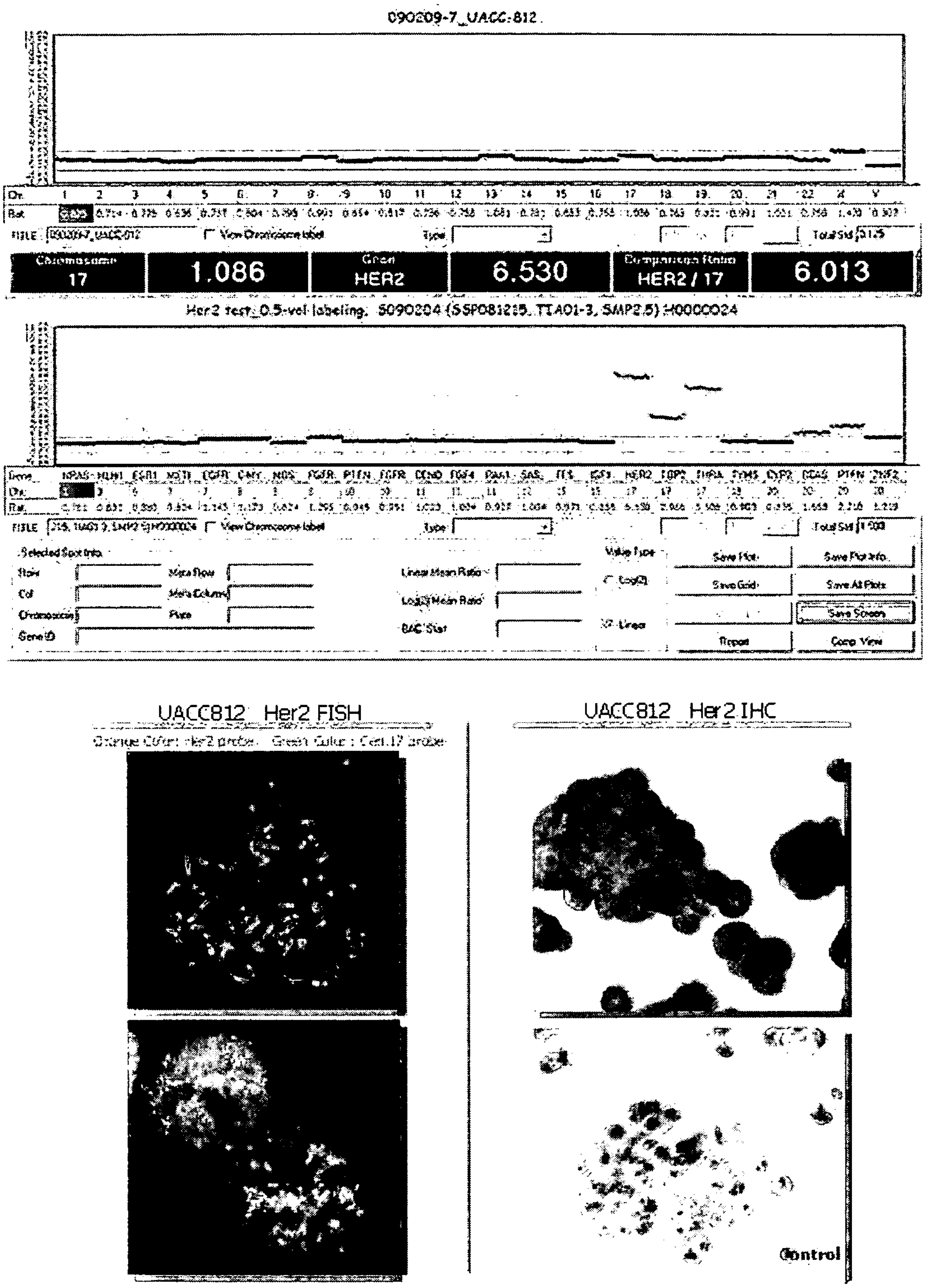
090209-7_UACC-812
Chromosome 17
1.086
Gene HER2
6.530
Comparison Ratio HER2 / 17
6.013
UACC812 Her2 FISH
Orange Color: Her2 probe Green Color: Cen.17 probe
UACC812 Her2 IHC
Control

**Fig. 14**

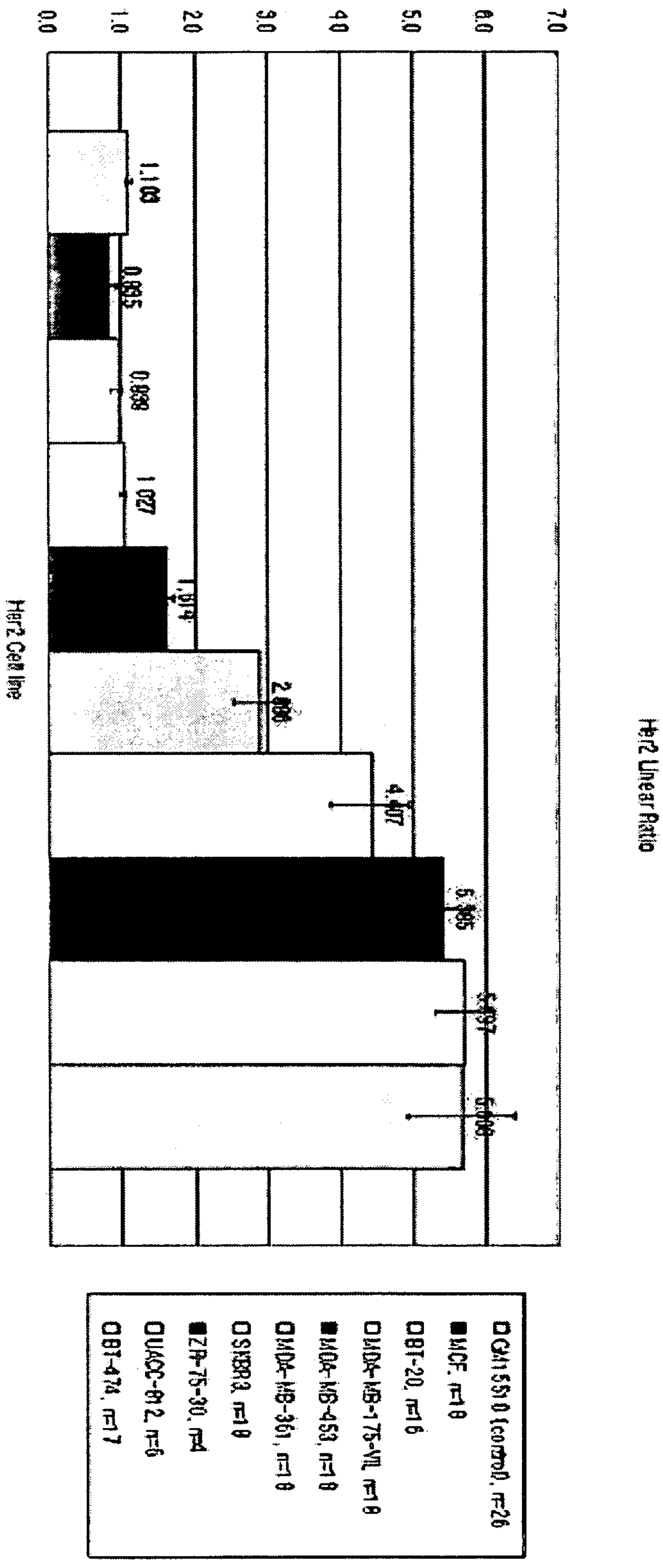
Her2 Linear Ratio
Her2 Cell line
0.0
1.0
2.0
3.0
4.0
5.0
6.0
7.0
MCF, n=18
BT-20, n=16
MDA-MB-175-VII, n=18
MDA-MB-453, n=18
MDA-MB-361, n=18
SKBR3, n=18
ZR-75-30, n=4
UACC-812, n=6
BT-474, n=17

**Fig. 15**

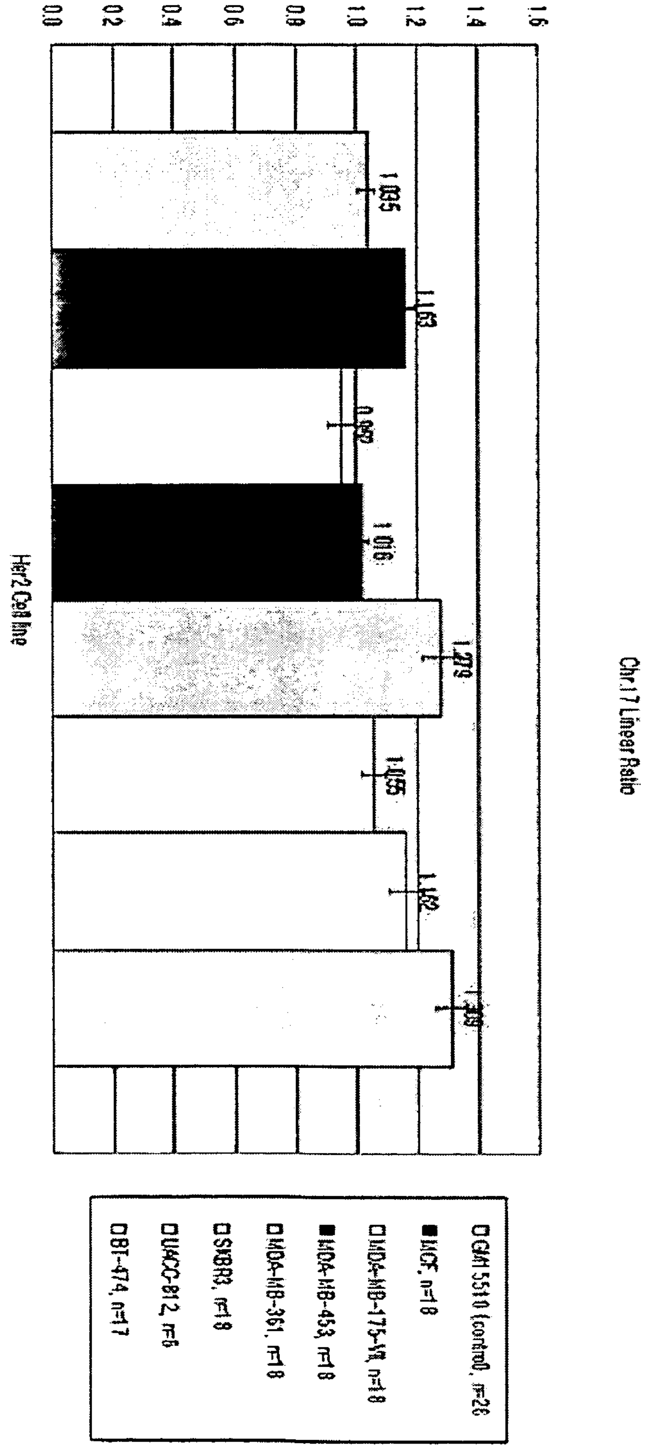
Chr.17 Lineer Ratio
Her2 Cell line
0.0
0.2
0.4
0.6
0.8
1.0
1.2
1.4
1.6
1.035
1.163
0.952
1.016
1.279
1.055
1.162
1.309
GM15510 (control), n=26
MCF, n=18
MDA-MB-175-VII, n=18
MDA-MB-453, n=18
MDA-MB-361, n=18
SKBR3, n=18
UACC-812, n=6
BT-474, n=17

**Fig. 16**

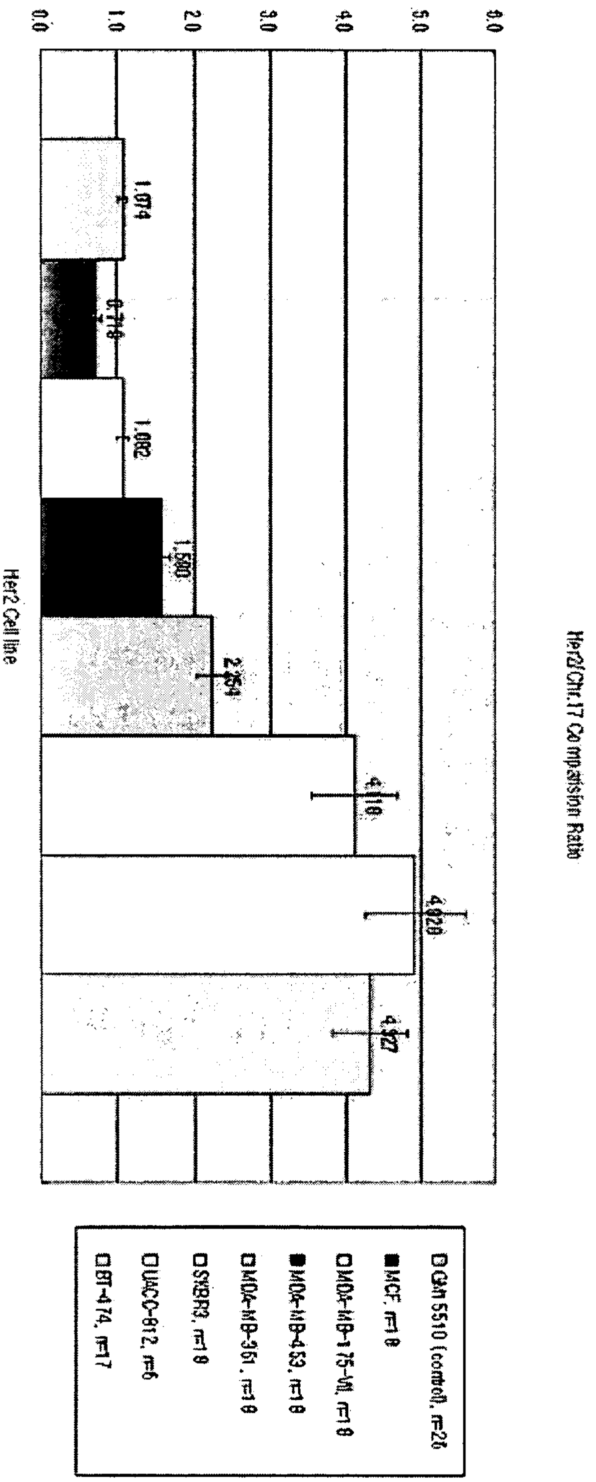
Her2/Chr.17 Comparision Ratio
Her2 Cell line
0.0
1.0
2.0
3.0
4.0
5.0
6.0
1.074
0.718
1.082
1.580
2.254
4.110
GM 5510 (control), n=26
MCF, n=18
MDA-MB-175-VII, n=18
MDA-MB-453, n=18
MDA-MB-361, n=18
SKBR3, n=19
UACC-812, n=6
BT-474, n=17

Fig. 17

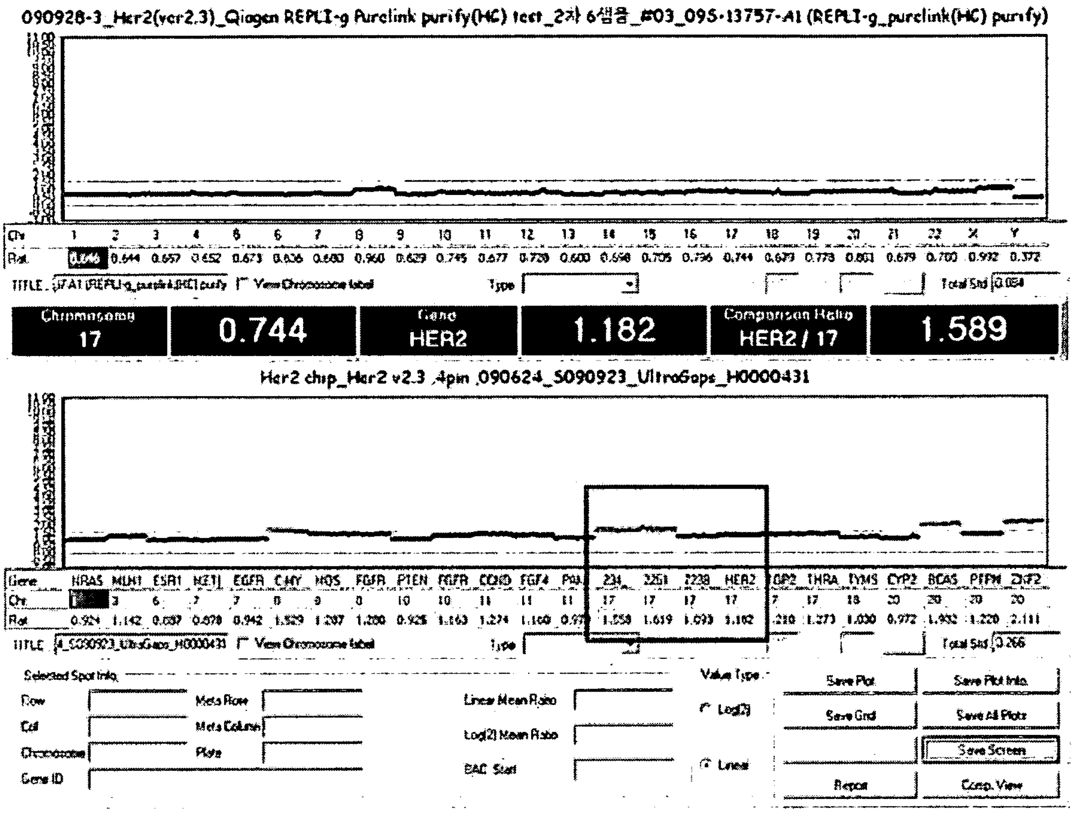
Chromosome 17
0.744
Gene HER2
1.182
Comparison Ratio HER2 / 17
1.589
Her2 chip_Her2 v2.3 .4pin .090624_S090923_UltraGaps_H0000431

Fig. 18

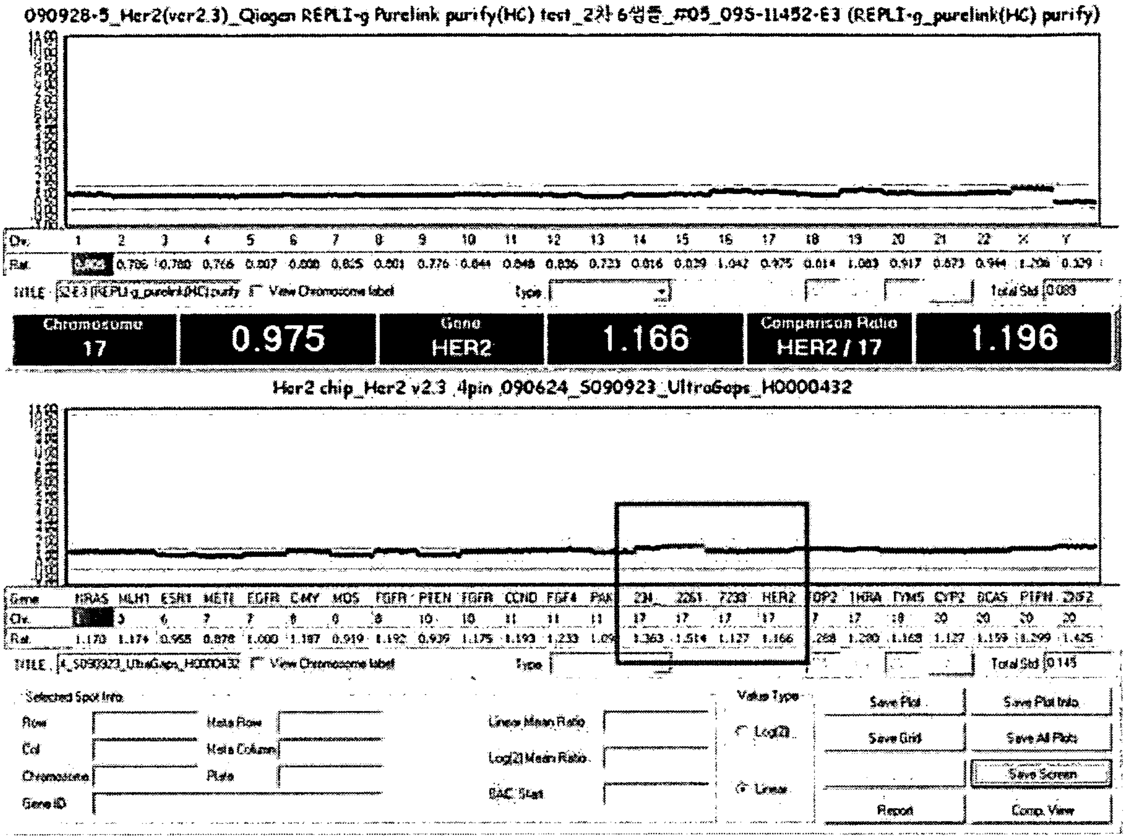
090928-5_Her2(ver2.3)_Qiagen REPLI-g Purelink purify(HC) test_2차 6샘플_#05_095-11452-E3 (REPLI-g_purelink(HC) purify)
Chromosome 17
0.975
Gene HER2
1.166
Comparison Ratio HER2 / 17
1.196
Her2 chip_Her2 v2.3 4pin 090624_S090923_UltraGaps_H0000432

Fig. 19

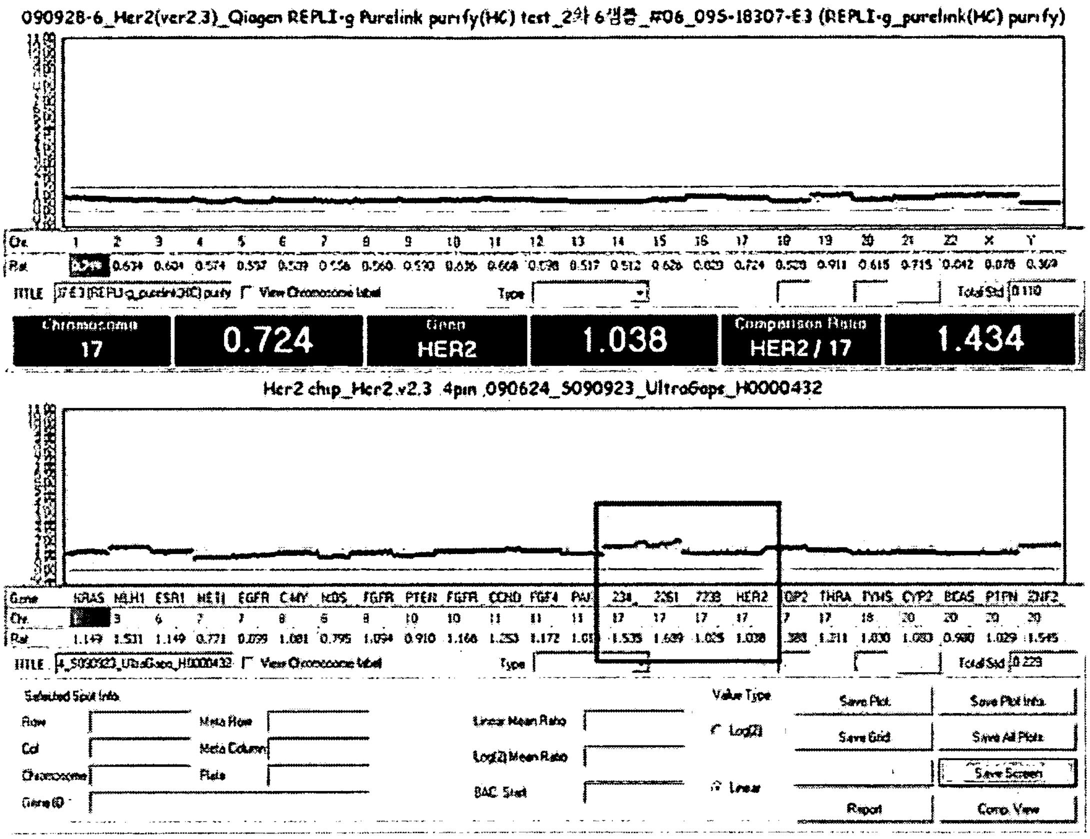

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2007081613 A2
- WO 2008089577 A1
- WO 2006133460 A2
- KR 1020090018575
- KR 100776214


### Non-patent literature cited in the description


- *Journal of Pathology,* vol. 216 (4), 399-407
- *Breast Cancer Research and Treatment,* 24 January 2009, vol. 118 (3), 481-498